# EUROPEAN PATENT APPLICATION

(11) **EP 3 604 281 A1**
(43) Date of publication of application: **05.02.2020**
(21) Application number: 18772161.8
(22) Date of filing: 23.03.2018
(51) Int. Cl.: C07D 215/40, A61K 31/4375, A61K 31/4704, A61K 31/4709, A61K 31/496, A61K 31/498, A61K 31/506, A61K 31/519, A61K 31/5377, A61P 31/04, A61P 43/00, C07D 241/44, C07D 401/12, C07D 401/14, C07D 405/12, C07D 413/12, C07D 417/12, C07D 417/14, C07D 471/04, C07D 487/04

(54) **2(1H)-QUINOLINONE DERIVATIVE**

(30) Priority: 24.03.2017 JP 2017060201
(71) Applicant: Taisho Pharmaceutical Co., Ltd., Tokyo 170-8633 (JP)
(72) Inventor: AMADA, Hideaki, Tokyo 170-8633 (JP); OTAKE, Norikazu, Tokyo 170-8633 (JP); USHIYAMA, Fumihito, Tokyo 170-8633 (JP); KIM, Chunhae, Tokyo 170-8633 (JP); TAKEUCHI, Tomoki, Tokyo 170-8633 (JP); TANAKA, Nozomi, Tokyo 170-8633 (JP)
(74) Representative: Hoffmann Eitle
(86) International application number: PCT/JP2018/011913
(87) International publication number: WO 2018/174288

(57) **Abstract**

Provided are: useful novel compounds that exhibit antibacterial activity based on their actions for inhibiting GyrB of DNA gyrase and ParE of topoisomerase IV; and 2(1H)-quinolinone derivatives represented by formula [1]: 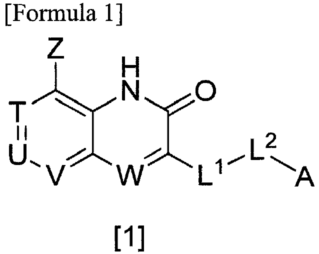 or pharmaceutically acceptable salts thereof.

## Description

### TECHNICAL FIELD

The present invention relates to novel 2(1H)-quinolinone derivatives having inhibitory activity against GyrB subunit of DNA gyrase and ParE subunit of topoisomerase IV or salts thereof, as well as antibacterial agents that comprise such derivatives or salts thereof as an active ingredient.

### BACKGROUND ART

Ever since penicillin was marketed in the nineteen forties, many kinds of antibacterial agents have been developed. While antibacterial agents made great contribution to the treatment of infections, they gave rise to a new problem, emergence of resistant bacteria. In particular, carbapenem-resistant enterobacteriaceae (CRE), multi-drug resistant Pseudomonas aeruginosa (MDRP) and multi-drug resistant Acinetobacter (MDRA), for example, show resistance against β-lactam drugs and many other antibacterial agents including quinolone drugs, which is clinically a big problem. Hence, the development of novel antibacterial agents is strongly desired in clinical settings and among others, the development of antibacterial agents working through novel mechanisms is of significance.

Through simultaneous cleavage and reunion of double-strand DNA, the Type II topoisomerase DNA gyrase changes the conformation (3D structure) of DNA to play an important role as in DNA replication, transcription, recombination, and repair. This enzyme is a holo-enzyme consisting of two molelcules of A subunit (GyrA) which is the gyrA gene product and two molecules of B subunit (GyrB) which is the gyrB gene product, with A subunit being responsible for the cleavage and reunion of DNA strands and B subunit responsible for energy conversion based on ATPase activity.

Bacteria possess topoisomerase IV which is the other subclass of Type II topoisomerase and this is involved in the separation of chromosomes that are formed during DNA replication and which are joined together in a closed annular form. This enzyme consists of two molecules of C subunit (ParC) which is the parC gene product and two molecules of E subunit (ParE) which is the parE gene product, with ParC and ParE having high homology to GyrA and GyrB, respectively.

Since Type II topoisomerase is universally present in bacteria, it is expected that the frequency at which drug resistant bacteria emerge can be suppressed by creating a dual inhibitor that simultaneously inhibits the two subclasses of Type II topoisomerase present in each bacterium.

Quinolone drugs are medications that are widely used in the treatment of bacterial infections and they inhibit DNA gyrase and topoisomerase IV in bacteria. Examples of quinolone drugs include nalidixic and oxolinic acids as early compounds, and more potent fluoroquinolones (e.g., norfloxacin, ciprofloxacin, and levofloxacin, etc.). These compounds bind GyrA to stabilize the cleaved complex, thereby inhibiting the function of DNA gyrase to cause apoptosis or cell death. Problems with quinolone drugs include not only joint disorder in juvenile animals but also photosensitivity which is unique to fluoroquinolones having fluorine (Non-Patent Document 1). What is more, many bacteria have turned out to be resistant to quinolone drugs due, for example, to the occurrence of amino acid variations in the quinolone resistance determining regions (QRDRs) of DNA gyrase and topoisomerase IV (Non-Patent Document 2). Hence, it is desired to develop an antibacterial agent having a different site of action than quinolone drugs.

Examples of inhibitors that bind GyrB include coumarin, novobiocin, and coumermycin A1, etc. These agents inhibit DNA gyrase but find little clinical use for several reasons such as insufficient effectiveness against Gram-negative bacteria and high toxicity against eucaryotic organisms (Non-Patent Document 3).

Other known inhibitors are disclosed in Patent Documents 1-48, etc. and Non-Patent Documents 4-11, etc. but it is not known that the compounds of the present invention have GyrB/ParE inhibiting actions.

Further, 2(1H)-quinolinone derivatives having similar structures to the compounds of the present invention are known by being disclosed in Patnet Documents 49-53, etc. but these compounds are not reported to have GyrB/ParE inhibiting actions.

### CITATION LIST

### PATENT LITERATURE

Patent Document 1: WO01/052845
Patent Document 2: WO01/052846
Patent Document 3: WO02/060879
Patent Document 4: WO03/105846
Patent Document 5: WO2005/012292
Patent Document 6: WO2005/026149
Patent Document 7: WO2005/026162
Patent Document 8: WO2005/089763
Patent Document 9: WO2006/022773
Patent Document 10: WO2006/038116
Patent Document 11: WO2006/092599
Patent Document 12: WO2006/092608
Patent Document 13: WO2006/087543
Patent Document 14: WO2007/056330
Patent Document 15: WO2007/071965
Patent Document 16: WO2007/148093
Patent Document 17: WO2008/020222
Patent Document 18: WO2008/020227
Patent Document 19: WO2008/020229
Patent Document 20: WO2008/068470
Patent Document 21: WO2008/152418
Patent Document 22: WO2009/061875
Patent Document 23: WO2009/027732
Patent Document 24: WO2009/027733
Patent Document 25: WO2009/106885
Patent Document 26: WO2009/147431
Patent Document 27: WO2009/147433
Patent Document 28: WO2009/147440
Patent Document 29: WO2009/074810
Patent Document 30: WO2009/074812
Patent Document 31: WO2009/084614
Patent Document 32: WO2009/156966
Patent Document 33: WO2010/013222
Patent Document 34: WO20l0/038081
Patent Document 35: WO2010/067123
Patent Document 36: WO2010/067125
Patent Document 37: WO2010/136817
Patent Document 38: WO2010/142978
Patent Document 39: WO2011/024004
Patent Document 40: WO2011/032050
Patent Document 41: WO2011/121555
Patent Document 42: WO2012/045124
Patent Document 43: WO2012/097269
Patent Document 44: WO2012/125746
Patent Document 45: WO2012/131588
Patent Document 46: WO2013/091011
Patent Document 47: WO2014/043272
Patent Document 48: WO2015/038661
Patent Document 49: WO2004/103974
Patent Document 50: WO2007/124617
Patent Document 51: WO2008/014307
Patent Document 52: WO2012/098070
Patent Document 53: WO2015/050379

### NON-PATENT LITERATURE

Non-Patent Document 1: Clin. Infect. Dis. (1999), 28, 352-364.
Non-Patent Document 2: J. Infect. Chemother. (2005), 53, 349-356.
Non-Patent Document 3: Trends Microbiol. (1997), 5, 102-109.
Non-Patent Document 4: ACS Infect. Dis. (2015), 1, 4-41.
Non-Patent Document 5: Pharmacol. Ther. (1993), 60, 367-380.
Non-Patent Document 6: J. Med. Chem. (2001), 44, 619-626.
Non-Patent Document 7: Bioorg. Med. Chem. Lett. (2000), 10, 821-826.
Non-Patent Document 8: Bioorg. Med. Chem. Lett. (2007), 17, 4708-4714.
Non-Patent Document 9: Bioorg. Med. Chem. Lett. (2004), 14, 2857-2862.
Non-Patent Document 10: Bioorg. Med. Chem. Lett. (2004), 14, 2863-2866.
Non-Patent Document 11: Bioorg. Med. Chem. Lett. (2009), 19, 5302-5306.

### SUMMARY OF INVENTION

### TECHNICAL PROBLEM

An object of the present invention is to provide novel compounds useful as pharmaceuticals that show strong antibacterial activity against Gram-positive and Gram-negative bacteria as well as their drug resistant variants by inhibiting GyrB of DNA gyrase and ParE of topoisomerase IV.

### SOLUTION TO PROBLEM

As a result of the intensive studies they made with a view to discovering compounds having actions for inhibiting GyrB of DNA gyrase and ParE of topoisomerase IV, the present inventors found that this object could be attained by compounds represented by the following general formula [1] or pharmaceutically acceptable salts thereof. The present invention has been accomplished based on this finding, as will be described below.

The present invention relates to the following.
(1) A compound represented by formula [1]: 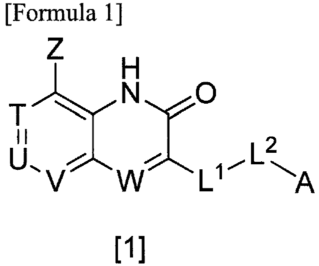 {wherein
   Z represents NH-R¹, a C₁₋₄ alkyl group (said C₁₋₄ alkyl group may be substituted with an amino group or a hydroxy group) or a hydroxy group;
   R¹ represents a hydrogen atom or a C₁₋₄ alkyl group;
   T, U, V and W all represent C-R² or either one of them represents N while the other represent C-R²;
   each R² independently represents a hydrogen atom, a halogen atom, a hydroxy group, a C₁₋₆ alkyl group, a C₁₋₆ alkoxy group (said C₁₋₆ alkyl group and said C₁₋₆ alkoxy group may be substituted with -N(R²¹)(R²²)) or an amino group [said amino group may be substituted with a C₁₋₆ alkyl group (said C₁₋₆ alkyl group may be substituted with a piperidin-4-yl group, a pyrrolidin-3-yl group, an azetidin-3-yl group, a 1-amino-cyclobutan-3-yl group, a morpholinyl group or -N(R²³)(R²⁴)), a 1-amino-cyclobutan-3-yl group or any of the substituents described by formula [2]]: 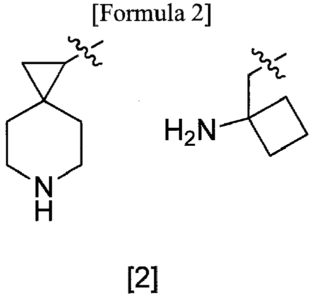
   R²¹, R²², R²³ and R²⁴ are the same or different and each represents a hydrogen atom or a C₁₋₆ alkyl group;
   L¹ represents -CONR³-, -COO-, -(CH₂)ₙNR³- or -NR³CO-;
   R³ represents a hydrogen atom or a C₁₋₆ alkyl group;
   n represents an integer of 1 to 4;
   L² represents a group selected from the group consisting of a bond, a C₁₋₆ alkylene group, a piperidinediyl group, a pyrrolidinediyl group and an azetidinediyl group (said C₁₋₆ alkylene group, said piperidinediyl group, said pyrrolidinediyl group and said azetidinediyl group may be substituted with a carboxy group or an oxo group);
   A represents an aryl group, a heterocyclic group or a C₃₋₈ cycloalkyl group (said aryl group, said heterocyclic group and said C₃₋₈ cycloalkyl group may be substituted with one to four substituents which are the same or different and are each selected from the following substituent group R^{a});
   the substituent group R^{a} represents a C₁₋₆ alkyl group (said C₁₋₆ alkyl group may be substituted with one or two substituents selected from the group consisting of a carboxy group, a hydroxy group, a C3-8 cycloalkyl group, a carbamoyl group and -N(R¹¹)(R¹²)), a carboxy group, a hydroxy group, a heterocyclic group, an amidino
   group, -N(R¹³)(R¹⁴), -CON(R¹⁵)(R¹⁶), a C₁₋₆ alkoxy group (said C₁₋₆ alkoxy group maybe substituted with one or two substituents selected from the group consisting of an amino group, an N-methylpiperazinyl group and a morpholinyl group), a C₂₋₆ alkenyl group (said C₂₋₆ alkenyl group may be substituted with a hydroxy group or -N(R¹⁷)(R¹⁸)), -COOR¹⁹, a 3-aminoazetidinyl group, a piperazinyl group (said piperazinyl group may be substituted with one methyl group), a 4-aminopiperidinyl group or any of the substitutents described by formula [3]: 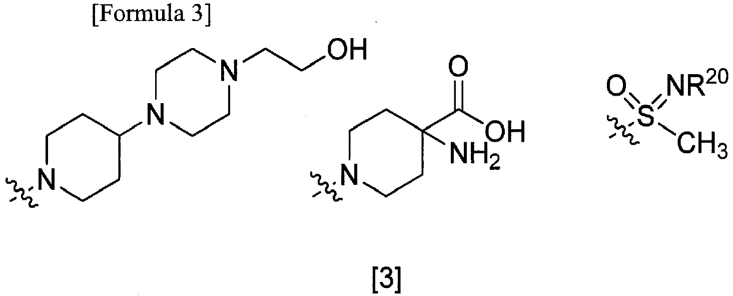
   R¹¹ and R¹² are the same or different and each represents a hydrogen atom, a C₁₋₁₂ alkyl group, a C₃₋₈ cycloalkyl group, a hydroxyethyl group, an N-methylpiperidin-4-yl group, a carboxymethyl group, an N,N-dimethylaminopropyl group or an aminoethyl group; or alternatively
   R¹¹ and R¹², when taken together with the nitrogen atom to which they bind, may form a saturated 3- to 7-membered ring, where said saturated 3- to 7-membered ring may further contain one or more nitrogen atoms, oxygen atoms or sulfur atoms in the ring, and said saturated 3- to 7-membered ring may be substituted with an amino group;
   R¹³ and R¹⁴ are the same or different and each represents a hydrogen atom, a C₁₋₆ alkoxy carbonyl group, a C₃₋₈ cycloalkyl group, -CONHSO₂Me, a C₁₋₆ alkyl group [said C₁₋₆ alkyl group may be substituted with one or two substituents selected from among an amino group, an N-methylpiperazinyl group, a morpholinyl group, -N(CH₂CH₂OH)₂ and a heterocyclic group (said heterocyclic group may be substituted with an amino group)];
   R¹⁵ and R¹⁶ are the same or different and each represents a hydrogen atom, a hydroxy group, a 1,3-dihydroxypropan-2-yl group, a methanesulfonyl group, an N,N-dimethylsulfamoyl group and a C₁₋₆ alkyl group (said C₁₋₆ alkyl group may be substituted with one or two substituents which are the same or different and are each selected from the group consisting of an amino group, a morpholinyl group, a piperidinyl group, a carboxy group, a hydroxy group and the substituent described by formula [4]): 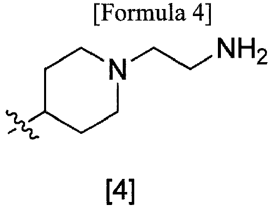
   R¹⁵ and R¹⁶, when taken together with the nitrogen atom to which they bind, may form a saturated 3- to 7-membered ring, where said saturated 3- to 7-membered ring may further contain one or more nitrogen atoms, oxygen atoms or sulfur atoms in the ring;
   R¹⁷ and R¹⁸ are the same or different and each represents a hydrogen atom or a C₁₋₆ alkyl group;
   R¹⁹ represents a C₁₋₆ alkyl group; and
   R²⁰ represents a hydrogen atom or a C₁₋₆ alkyl group}
   or a pharmaceutically acceptable salt thereof.
(2) The compound as recited in (1) wherein Z is NH-R¹, a C₁₋₄ alkyl group or a hydroxy group and R¹ is a C₁₋₄ alkyl group, or a pharmaceutically acceptable salt thereof.
(3) The compound as recited in (2) wherein Z is NH-R¹, an ethyl group or a hydroxy group and R¹ is a methyl group, or a pharmaceutically acceptable salt thereof.
(4) The compound as recited in (3) wherein Z is NH-R¹ and R¹ is a methyl group, or a pharmaceutically acceptable salt thereof.
(5) The compound as recited in (4) wherein T, U, V and W are each C-R², or a pharmaceutically acceptable salt thereof.
(6) The compound as recited in (5) wherein L¹ is -CONR³-, -COO- or -(CH₂)ₙNR³-, or a pharmaceutically acceptable salt thereof.
(7) The compound as recited in (6) wherein L¹ is -CONR³-, or a pharmaceutically acceptable salt thereof.
(8) The compound as recited in (7) wherein R³ is a hydrogen atom, or a pharmaceutically acceptable salt thereof.
(9) The compound as recited in (8) wherein L² is a bond or a group selected from the group consisting of a bond, a methylene group or an ethylene group, a piperidinediyl group, a pyrrolidinediyl group, and an azetidinediyl group, or a pharmaceutically acceptable salt thereof.
(10) The compound as recited in (9) wherein L² is a bond or an ethylene group, or a pharmaceutically acceptable salt thereof.
(11) The compound as recited in (10) wherein L² is a bond, or a pharmaceutically acceptable salt thereof.
(12) The compound as recited in any one of (1) to (11) wherein T, U, V and W are each C-R² and each R² is independently a hydrogen atom, a halogen atom, a hydroxy group, a C₁₋₆ alkyl group (said C₁₋₆ alkyl group may be substituted with -N(R²¹)(R²²)), a C₁₋₆ alkoxy group or an amino group [said amino group may be substituted with a C₁₋₆ alkyl group (said C₁₋₆ alkyl group may be substituted with a piperidin-4-yl group, a pyrrolidin-3-yl group, an azetidin-3-yl group, a 1-amino-cyclobutan-3-yl group, a morpholino group or -N(R²³)(R²⁴)), a 1-amino-cyclobutan-3-yl group or any of the substituents described by formula [2]]: 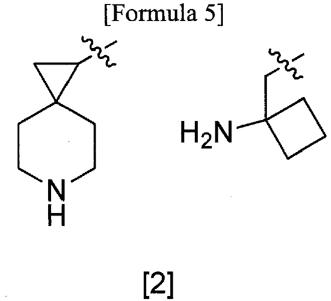 R²¹, R²², R²³ and R²⁴ are the same or different and each is a hydrogen atom or a C₁₋₆ alkyl group, or a pharmaceutically acceptable salt thereof.
(13) The compound as recited in (12) wherein each R² is independently a hydrogen atom, a fluorine atom, a hydroxy group, an ethyl group, a n-propyl group (said ethyl group and said n-propyl group may be substituted with -N(R²¹)(R²²)), a C₁₋₆ alkoxy group, an amino group [said amino group may be substituted with a methyl group, an ethyl group, a n-propyl group (said methyl group, said ethyl group and said n-propyl group may be substituted with one or two substituents selected from the group consisting of a piperidin-4-yl group, a pyrrolidin-3-yl group, an azetidin-3-yl group, a 1-amino-cyclobutan-3-yl group, a morpholino group and -N(R²³)(R²⁴)), a 1-amino-cyclobutan-3-yl group or any of the substituents described by formula [2]]: 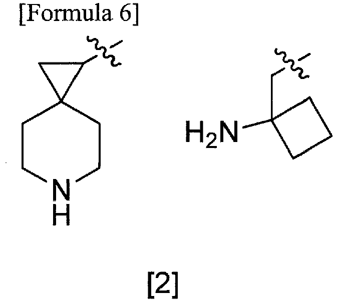 R²¹, R²², R²³ and R²⁴ are the same or different and each is a hydrogen atom or a methyl group, or a pharmaceutically acceptable salt thereof.
(14) The compound as recited in (13) wherein each R² is independentlyl a hydrogen atom, a fluorine atom or a hydroxy group, or a pharmaceutically acceptable salt thereof.
(15) The compound as recited in (13) wherein U is C-F or C-H, T and V are each C-H, and W is C-R², or a pharmaceutically acceptable salt thereof.
(16) The compound as recited in any one of (1) to (15) wherein A is an aryl group or a heterocyclic group (said aryl group and said heterocyclic group may be substituted with one to four substituents which are the same or different and are each selected from the substituent group R^{a}), or a pharmaceutically acceptable salt thereof.
(17) The compound as recited in (16) wherein A is an aryl group or a heterocyclic group (said aryl group and said heterocyclic group may be substituted with one or two substituents which are the same or different and are each selected from the substituent group R^{b});
   R^{b} is a C₁₋₆ alkyl group (said C₁₋₆ alkyl group may be substituted with one or two substituents which are the same or different and are each selected from the group consisting of a carboxy group, a hydroxy group, a carbamoyl group and -N(R¹¹)(R¹²)), a carboxy group, a hydroxy group, a heterocyclic group, an amidino group, -N(R¹³)(R¹⁴), -CON(R¹⁵)(R¹⁶), a C₁₋₆alkoxy group (said C₁₋₆alkoxy group may be substituted with an amino group, an N-methylpiperazinyl group or a morpholino group), a C₂₋₆ alkenyl group (said C₂₋₆ alkenyl group may be substituted with a hydroxy group or -N(R¹⁷)(R¹⁸)), a 3-aminoazetidino group, piperazinyl group (said piperazinyl group may be substituted with one methyl group), a 4-aminopiperidino group or any of the substituents described by formula [3]: 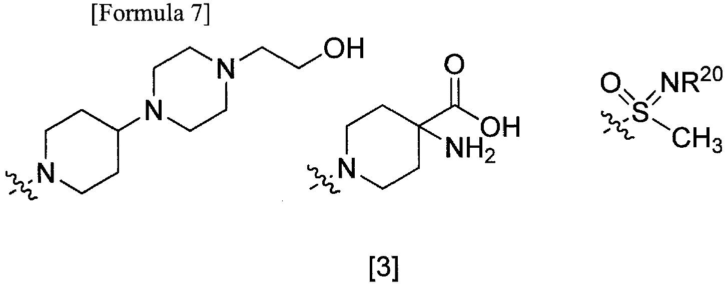
   R¹¹ and R¹² are the same or different and each is an atom or a group selected from the group consisting of a hydrogen atom, a methyl group, a n-pentyl group, a n-octyl group, a cyclopropyl group, a cyclohexyl group, a hydroxyethyl group, an N-methylpiperidin-4-yl group, a carboxymethyl group, an N,N-dimethylaminopropyl group and an aminoethyl group, or alternatively
   R¹¹ and R¹², when taken together with the nitrogen atom to which they bind, form a saturated 3- to 7-membered ring which is an azetidinyl group, a pyrrolidinyl group, a piperidinyl group or a morpholino group;
   R¹³ and R¹⁴ are the same or different and each is a hydrogen atom, a t-butoxycarbonyl group, a cyclohexyl group, -CONHSO₂Me, a methyl group, an ethyl group or a n-propyl group (said methyl group, said ethyl group and said n-propyl group may be substituted with one or two substituents selected from the group consisting of an amino group, an N-methylpiperazino group, a morpholinyl group, -N(CH₂CH₂OH)₂ and a heterocyclic group);
   R¹⁵ and R¹⁶ are the same or different and each is a hydrogen atom, a hydroxy group, a 1,3-dihydroxypropan-2-yl group, a methanesulfonyl group, an N,N-dimethylsulfamoyl group and a C₁₋₆ alkyl group (said C₁₋₆ alkyl group may be substituted with one or two substituents which are the same or different and are each selected from the group consisting of an amino group, a morpholinyl group, a piperidinyl group, a carboxy group, a hydroxy group and the substituent described by formula [4]): 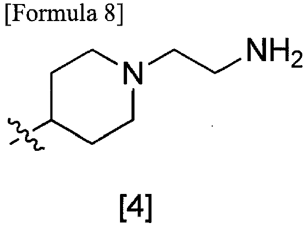
   or alternatively, R¹⁵ and R¹⁶, when taken together with the nitrogen atom to which they bind, form a saturated 3- to 7-membered ring which is a morpholinyl group;
   R¹⁷ and R¹⁸ are the same or different and each is a hydrogen atom or a methyl group;
   R²⁰ is a hydrogen atom or a methyl group, or a pharmaceutically acceptable salt thereof.
(18) The compound as recited in (17) wherein A is a phenyl group or a heterocyclic group (said phenyl group and said heterocyclic group may be substituted with one or two substituents which are the same or different and are each selected from the following substituent group R^{c});
   R^{c} is a methyl group (said methyl group may be substituted with one or two substituents which are the same or different and are each selected from the group consisting of a carboxy group, a hydroxy group, a carbamoyl group and -N(R¹¹)(R¹²)), a carboxy group, a heterocyclic group and -CON(R¹⁵)(R¹⁶);
   R¹¹ and R¹² are the same or different and each is an atom or a group selected from the group consisting of a hydrogen atom, a methyl group, a n-pentyl group, a n-octyl group, a cyclopropyl group, a cyclohexyl group, a hydroxyethyl group, an N-methylpiperidin-4-yl group, a carboxymethyl group, an N,N-dimethylaminopropyl group and an aminoethyl group, or alternatively
   R¹¹ and R¹², when taken together with the nitrogen atom to which bind, form a saturated 3- to 7-membered ring which is an azetidinyl group, a pyrrolidinyl group, a piperidinyl group or a morpholino group;
   R¹⁵ and R¹⁶ are the same or different and each is a hydrogen atom, a methanesulfonyl group, an N,N-dimethylsulfamoyl group, a methyl group, an ethyl group or a n-propyl group (said methyl group, ethyl group or n-propyl group may be substituted with one or two substituents selected from the group consisting of an amino group, a morpholinyl group, a piperidinyl group and a hydroxy group), or a pharmaceutically acceptable salt thereof.
(19) A pharmaceutical composition comprising the compound of (1) to (18) or a pharmaceutically acceptable salt thereof.
(20) A GyrB/ParE inhibitor comprising the compound of (1) to (18) or a pharmaceutically acceptable salt thereof.
(21) An antibacterial agent comprising the compound of (1) to (18) or a pharmaceutically acceptable salt thereof.

### ADVANTAGEOUS EFFECTS OF INVENTION

The compounds of the present invention and pharmaceutically acceptable salts thereof have strong GyrB/ParE inhibiting actions and, hence, are useful as antibacterial agents targeted against Gram-positive and Gram-negative bacteria as causative pathogens.

### DESCRIPTION OF EMBODIMENTS

Hereinafter, the present invention will be described in greater detail.

To begin with, the terminology used herein is explained.

In the present invention, "n-" means normal, "i-" iso, "s-" and "sec-" secondary, "t-" and "tert-" tertiary, "c-" cyclo, "o-" ortho, "m-" meta, and "p-" para.

The term "halogen atom" means a fluorine atom, a chlorine atom, and a bromine atom.

The "C₁₋₄ alkyl group" is a straight- or branched-chain alkyl group with 1 to 4 carbon atoms, and may be exemplified by a methyl group, an ethyl group, a n-propyl group, a n-butyl group, an isopropyl group, an isobutyl group, a t-butyl group, and a s-butyl group.

The "C₁₋₆ alkyl group" is a straight- or branched-chain alkyl group with 1 to 6 carbon atoms and may be exemplified by not only the specific examples of the above-mentioned "C₁₋₄ alkyl group" but also a n-pentyl group, a n-hexyl group, an isopentyl group, a neopentyl group, a t-pentyl group and a 1,2-dimethylpropyl group.

The "C₁₋₁₂ alkyl group" is a straight- or branched-chain alkyl group with 1 to 12 carbon atoms and may be exemplified by not only the specific examples of the above-mentioned "C₁₋₆ alkyl group" but also a 1-ethylpropyl group, a n-heptyl group, a n-octyl group, a n-nonyl group, a n-decyl group and a n-dodecyl group, for example.

The "C₁₋₆ alkylene group" is a straight- or branched-chain alkylene group with 1 to 6 carbon atoms and may be exemplified by -CH₂-, -(CH₂)₂-, -(CH₂)₃-, -CH₂-CH(CH₃)-, -C(CH₃)₂-, -(CH₂)₄-, -(CH₂)₂-CH(CH₃)-, -CH₂-CH(CH₃)-CH₂-, -CH(CH₃)-(CH₂)₂-, -(CH₂)₅-, -(CH₂)₃-CH(CH₃)-, -(CH₂)₂-CH(C₂H₅)-, -(CH₂)₆-, -(CH₂)₃-CH(CH₃)-CH₂- and -CH₂-CH(CH₃)-(CH₂)₃-.
The "C₃₋₈ cycloalkyl group" is a cycloalkyl group with 3 to 8 carbon atoms and may be exemplified by a c-propyl group, a c-butyl group, a c-pentyl group, a c-hexyl group, a c-heptyl group and a c-octyl group.

The "C2-6 alkenyl group" is a straight- or branched-chain alkenyl group with 2 to 6 carbon atoms having one or more double bonds at any position(s) of the aforementioned "C₁₋₆ alkyl group" and may be exemplified by a vinyl group, a 1-propenyl group, a 2-propenyl group, an isopropenyl group, a 2-butenyl group, a 1,3-butadienyl group, a 2-pentenyl group, a 3-pentenyl group and a 2-hexenyl group.

The "C₁₋₆ alkoxy group" is a straight- or branched-chain alkoxy group with 1 to 6 carbon atoms and may be exemplified by a methoxy group, an ethoxy group, a 1-propoxy group, an isopropoxy group, a 1-butoxy group, a 1-methyl-1-propoxy group, a t-butoxy group and a 1-pentyloxy group.

The "C₁₋₆ alkoxycarbonyl group" is a straight- or branched-chain alkoxycarbonyl group with 1 to 6 carbon atoms and may be exemplified by a methoxycarbonyl group, an ethoxycarbonyl group, a n-propoxycarbonyl group, an isopropoxycarbonyl group, a n-butoxycarbonyl group, a t-butoxycarbonyl group, etc.

The "oxo group" refers to a radical composed of a doubly bonded oxygen atom (=O). Hence, if the oxo is bonded to a carbon atom, it is taken together with said carbon atom to form a carbonyl; if one oxo is bonded to one sulfur atom, it is taken together with said sulfur atom to form a sulfinyl; if two oxo groups are bonded to one sulfur atom, they are taken together with said sulfur atom to form a sulfonyl.

The "aryl group" is a monocyclic to a tetracyclic aromatic carbocyclic group composed of 6 to 18 carbon atoms and may be exemplified by a phenyl group, a naphthyl group, an anthryl group, a phenanthrenyl group, a tetracenyl group and a pyrenyl group, for example.

The "heterocyclic group" is "a monocylic heterocyclic group", "a fused cyclic heterocyclic group" or "a spirocyclic heterocyclic group" that contain any one to five ring constituting atoms that are selected from among a nitrogen atom, an oxygen atom and a sulfur atom. If the hetero atom is a sulfur atom, dioxide forms are also embraced by the present invention.

The "monocyclic heterocyclic group" is a type of the above-mentioned "heterocyclic group" that is a monocyclic heterocyclic group composed of 3 to 8 atoms in the ring; it includes "a monocyclic saturated heterocyclic group", "a monocyclic aromatic heterocyclic group" and "a partially saturated monocyclic aromatic heterocyclic group".

The "fused cyclic heterocyclic group" is another type of the above-mentioned "heterocyclic group" that is a fused cyclic heterocyclic group composed of 6 to 14 atoms in the ring; it includes "a fused cyclic saturated heterocyclic group", "a fused cyclic aromatic heterocyclic group" and "a fused cyclic heterocyclic group having a partially saturated monocycle".

The "spirocyclic heterocyclic group" is yet another type of the above-mentioned "heterocyclic group" that is composed of two rings having a total of 6 to 14 atoms and sharing a single spiro carbon atom; the "spirocyclic heterocyclic group" may be substituted with one to three oxo groups. It may be exemplified by a 2-oxa-6-azaspiro[3.3]heptanyl group, a 1-oxa-6-azaspiro[3.3]heptanyl group, a 6-oxa-1-azaspiro[3.3]heptanyl group, a 1-oxo-2,8-diazaspiro[4.5]decanyl group, a 1,4-dioxa-8-azaspiro[4.5]decanyl group, a 2-azaspiro[3.3]heptyl group, a 7-oxa-2-azaspiro[3.5]nonyl group, a 5,8-oxa-2-azaspiro[3.4]octyl group, a 1,4-dioxa-8-azaspiro[4.5]decanyl group and 1-oxaspiro[4.5]decanyl group, for example.

The "monocyclic saturated heterocyclic group" is a monocyclic heterocyclic group whose ring is solely composed of saturated bonds and it may be substituted with one or two oxo groups. It may be exemplified by an aziridinyl group, an azetidinyl group, a pyrrolidinyl group, a 2-oxopyrrolidinyl group, a piperidinyl group, a piperazinyl group, a 3-oxopiperazinyl group, a morpholinyl group, a thiomorpholinyl group (the sulfur atom on the ring may be oxidized), a homopiperazinyl group, a homomorpholinyl group (oxazepanyl group), an imidazolidinyl group, a pyrazolidinyl group, an oxazolidinyl group, a 2-oxo-1,3-oxazolidin-3-yl group, an isoxazolidinyl group, a 2,4-dioxo-1,3-oxazolidin-5-yl group, a 2,3-dioxopiperazinyl group, an oxetan-2-yl group, an oxetan-3-yl group, a 1,3-dioxolanyl group, a tetrahydrofuranyl group, a tetrahydropyranyl group, a tetrahydro-2H-thiopyranyl group, a dithiolanyl group and a thiolanyl group, for example.

The "monocyclic aromatic heterocyclic group" may be exemplified by a pyridyl group, a pyridazinyl group, a pyrimidinyl group, a pyrazinyl group, a thieinyl group, a pyrrolyl group, a thiazolyl group, an isothiazolyl group, a pyrazolyl group, an imidazolyl group, a furyl group, an oxazolyl group, an isoxazolyl group, an oxadiazolyl group, a 1,3,4-thiadiazolyl group, a 1,2,3-triazolyl group, a 1,2,4-triazolyl group and a tetrazolyl group, for example.

The "partially saturated monocyclic aromatic heterocyclic group" is a monocyclic aromatic heterocyclic group in which some of the bonds that constitute the ring are saturated, and those which are substituted with one or two oxo groups are also included. The "partially saturated monocyclic aromatic heterocyclic group" may be exemplified by a 2-pyridonyl group, a 4-pyridonyl group, a 5-oxo-4,5-dihydro-1,2,4-oxadiazol-3-yl group, a 2,4-dioxo-1,3-oxazolidin-5-yl group, a 4,5-dihydro-1H-imidazolyl group, a 1,2,3,6-tetrahydropyridyl group, a 4H-1,3-oxazinyl group and a 5,6-dihydro-4H-1,3-oxazinyl group, for example.

The "fused cyclic saturated heterocyclic group" is a fused cyclic heterocyclic group whose ring is solely composed of saturated bonds and it may be substituted with one to three oxo groups. The "fused cyclic saturated heterocyclic group" may be exemplified by an octahydro-1H-isoindolyl group, a decahydroquinolyl group, a decahydroisoquinolyl group, a hexahydro-2H-[1,4]dioxino[2,3-c]pyrrolyl group and a 3-azabicyclo[3.1.0]hexa-3-yl group, for example.

The "fused cyclic aromatic heterocyclic group" may be exemplified by a quinolyl group, an isoquinolyl group, a naphthyridinyl group (e.g., 1,6-naphthyridinyl group, 1,7-naphthyridinyl group, 1,8-naphthyridinyl group), a quinazolinyl group, a benzofuranyl group, a benzothienyl group, an indolyl group, a benzoxazolyl group, a benzisoxazolyl group (e.g., benzo[c]isoxazolyl group, benzo[d]isoxazolyl group), a 1H-indazolyl group, a 2H-indazolyl group, a benzimidazolyl group, a benzoxadiazolyl group (e.g., benzo[1,2,5]oxadiazolyl group, benzo[1,2,3]oxadiazolyl group, benzo[2,1,3]oxadiazolyl group), a benzothiazolyl group, a benzothiadiazolyl group (e.g., [1,2,5]thiadiazolyl group, benzo[1,2,3]thiadiazolyl group), an indolizinyl group, a benzofurazanyl group, a thienopyridyl group (e.g., thieno[2,3-b]pyridyl group, [3,2-b]pyridyl group), a pyrazolopyridyl group, an imidazopyridyl group (e.g., imidazo[1,5-a]pyridyl group, imidazo[1,2-a]pyridyl group, 3H-imidazo[4,5-b]pyridyl group), an imidazopyrimidinyl group (e.g., imidazo[1,2-a]pyrimidinyl group, imidazo[1,2-c]pyrimidinyl group), an imidazopyrazinyl group (e.g., imidazo[1,5-a]pyrazinyl group, imidazo[1,2-a]pyrazinyl group), a pyrazolopyrimidinyl group (e.g., pyrazolo[1,5-a]pyrimidinyl group, pyrazolo[1,5-c]pyrimidinyl group), a triazolopyrimidinyl group (e.g., [1,2,3]triazolo[1,5-a]pyrimidinyl group, [1,2,3]triazolo[1,5-c]pyrimidinyl group, [1,2,4]triazolo[1,5-a]pyrimidinyl group, [1,2,4]triazolo[1,5-c]pyrimidinyl group), a thienothienyl group (e.g., thieno[2,3-b]thienyl group, thieno[3,2-b]thienyl group) and an imidazothiazolyl group (e.g., imidazo[2,1-b]thiazolyl group, imidazo[5,1-b]thiazolyl group), for example.

The "fused cyclic heterocyclic group having a partially saturated monocycle" is a fused cyclic aromatic heterocyclic group having a monocycle in which some of the ring constituting bonds are saturated and it may be substituted with one to three oxo groups. The "fused cyclic heterocyclic group having a partially saturated monocycle" may be exemplified by a 1,3-dihydrobenzimidazol-2-onyl group, a 2-benzoxazolinonyl group, an octahydroisoindolyl group, a 2H-pyrido[3,2-b]-1,4-oxazin-3(4H)-on-yl group, a 3-oxo-3,4-dihydro-2H-pyrido[3,2-b][1,4]oxazin-6-yl group, a [1,3]dioxolo[4,5-b]pyridyl group, a 2,3-dihydrobenzo[b]thienyl group, a 2,3-dihydro-1-benzofuran-5-yl group, a 2,3-dihydro-1-benzofuran-6-yl group, a 1,3-dihydro-2-benzofuran-5-yl group, a 2,3-dihydro-1H-indol-5-yl group, a 1,3-benzodioxol-5-yl group, a 2,3-dihydro-1,4-benzodioxin-2-yl group, a 2,3-dihydro-1,4-benzodioxin-6-yl group, a 3-oxo-3,4-dihydro-2H-1,4-benzooxazin-6-yl group, a 1,4-benzodioxanyl group, a 2H-benzo[b][1,4]oxazin-3(4H)-on-yl group, a 3,4-dihydro-2H-benzo[b][1,4]dioxepynyl group, an indolinyl group, a 2H-isoindolinyl group, a cromanyl group, a chromonyl group, an isocromanyl group and a 1,2,3,4-tetrahydroisoquinolyl group, for example.

The groups which, "when taken together with the nitrogen atom to which they bind, may form a saturated 3- to 7-membered ring, where said saturated 3- to 7-membered ring may further contain one or more nitrogen atoms, oxygen atoms or sulfur atoms in the ring" may be exemplified by an aziridinyl group, an azetidinyl group, a pyrrolidinyl group, a piperidinyl group, an azepanyl group, a piperazinyl group, a morpholinyl group, a thiomorpholinyl group and a 1,2,3,6-tetrahydropyridyl group, for example.

The groups which, "when taken together with the nitrogen atom to which they bind, may form a saturated 3- to 7-membered ring, where said saturated 3- to 7-membered ring may further contain one or more nitrogen atoms, oxygen atoms or sulfur atoms in the ring, and said saturated 3- to 7-membered ring may be substituted with an amino group" may be exemplified not only by the specific examples of the aforementioned groups which, "when taken together with the nitrogen atom to which they bind, may form a saturated 3- to 7-membered ring, where said saturated 3- to 7-membered ring may further contain one or more nitrogen atoms, oxygen atoms or sulfur atoms in the ring", but also by a 2-aminoaziridinyl group, a 3-aminoazetidinyl group, a 2-aminopyrrolidinyl group, a 3-aminopyrrolidinyl group, a 2-aminopiperidinyl group, a 3-aminopiperidinyl group, a 4-aminopiperidinyl group, a 2-aminoazepanyl group, a 3-aminoazepanyl group and a 4-aminoazepanyl group, for example.

The "leaving group" may be exemplified by a halogen atom, a methylsulfonyloxy group, a trifluoromethylsulfonyloxy group and a p-toluenesulfonyloxy group, for example.

The "antibacterial agents" means substances that act on bacteria such as Gram-positive and Gram-negative bacteria, thereby exhibiting the ability to suppress their growth or kill them. The antibacterial agents may be those which suppress bacterial proliferation or kill some bacterial cells to provide a smaller cell count. Gram-positive bacteria may include, for example, the genus Staphylococcus (e.g., Staphylococcus aureus, Staphylococcus epidermidis), the genus Streptococcus (e.g., Streptococcus pyrogenes, Group B Streptococcus, Streptococcus pneumoniae), and the genus Enterococcus (e.g., Enterococcus faecalis, Enterococcus faecium). Gram-negative bacteria may include, for example, the genus Pseudomonas (e.g., Pseudomonas aeruginosa), the genus Escherichia (e.g., Escherichia coli), the genus Klebsiella (e.g, Krebsiella pneumonia, Krebsiella ocytoca), the genus Haemophilus (e.g., Haemophilus influenzae, Haemophilus parainfluenzae), the genus Bordetella (e.g. Bordetella pertussis, Bordetella bronchiseptica), the genus Serratia (e.g., Serratia marcescens), the genus Proteus (e.g., Proteus mirabilis), the genus Enterobacter (e.g., Enterobacter cloacae), the genus Campylobacter (e.g., Campylobacter jejuni). The genus Citrobacter, the genus Vibrio (e.g., Vibrio parahaemolyticus, Vibrio cholera), the genus Morganella (e.g, Morganella morganii), the genus Salmonella (e.g., Salmonella enterica seovar Typhi, Salmonella enterica seovar Paratyphi), the genus Shigella (e.g., Shigella), the genus Acinetobacter (e.g. Acinetobacter baumannil, Acinetobacter calcoaceticus), the genus Legionella (e.g., Legionella pneumophila), the genus Bacteroides (e.g., Bacteroides fragilis), the genus Neisseria (e.g., Neisseria gonorrhoeae, Neisseria meningitidis), the genus Moraxella (e.g., Moraxella catarrhalis), the genus Chlamydia (e.g., Chlamydia trachomatis, Chlamydia psittaci), the genus Clostridium (e.g, Clostridium dificile, Clostridium tetani) and the genus Helicobacter (e.g., Helicobacter pyroli.)

Preferred embodiments of the compounds of the present invention are as follows.

A preferred example of Z is NH-R¹ or an ethyl group, wherein R¹ is a methyl group. A more preferred example of Z is NH-R¹ wherein R¹ is a methyl group.

A preferred example of T, U, V and W is C-R², wherein R² is a hydrogen atom, a halogen atom, a hydroxy group, a C₁₋₆ alkyl group (said C₁₋₆ alkyl group may be substituted with -N(R²¹)(R²²)), a C₁₋₆alkoxy group, an amino group (said amino group may be substituted with a C₁₋₆ alkyl group [said C₁₋₆ alkyl group may be substituted with a piperidin-4-yl group, a pyrrolidin-3-yl group, an azetidin-3-yl group, a 1-amino-cyclobutan-3-yl group, a morpholino group, -N(R²³)(R²⁴)], a 1-amino-cyclobutan-3-yl group, or any of the substituents described by formula [2]): 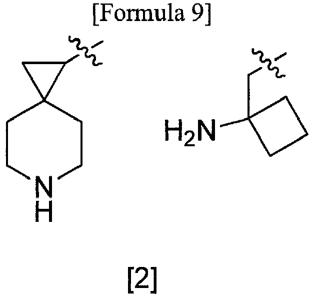 R²¹, R²², R²³ and R²⁴ are the same or different and each is a hydrogen atom or a C₁₋₆ alkyl group.

A more preferred example of T, U, V and W is C-R², wherein R² is a hydrogen atom, a fluorine atom, a hydroxy group, an ethyl group or a n-propyl group (said ethy group or n-propyl group may be substituted with -N(R²¹)(R²²)), a C₁₋₆ alkoxy group, an amino group (said amino group may be substituted with a methyl group, an ethyl group or a n-propyl group [said methyl group, ethyl group or n-propyl group may be substituted with a piperidin-4-yl group, a pyrrolidin-3-yl group, an azetidin-3-yl group, a 1-aminocyclobutan-3-yl group, a morpholino group, or -N(R²³)(R²⁴)], a 1-aminocyclobutan-3-yl group or any of the substituents described by formula [2]): 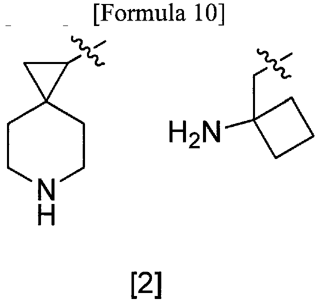 R²¹, R²², R²³ and R²⁴ are the same or different and each is a hydrogen atom or a methyl group.
The most preferred example of T, U, V and W is C-R², wherein R² is a hydrogen atom, a fluorine atom or a hydroxy group. Even more preferably, U is C-F or C-H, T and V are each C-H, and W is C-R² (the preferred examples of R² are as listed above).

A preferred example of L¹ is -CONR³-, -COO- or -(CH₂)ₙNR³-, wherein R³ is a hydrogen atom or a methyl group, and n is 1. A more preferred example of L¹ is -CONR³-wherein R³ is a hydrogen atom.

A preferred example of L² is a bond, a methylene group or an ethylene group, a piperidin-4-yl group, a pyrrolidin-3-yl group or an azetidin-3-yl group. A more preferred example of L² is a bond or an ethylene group. The most preferred example of L² is a bond.

A preferred example of A is an aryl group or a heterocyclic group (said aryl group or heterocyclic group may be substituted with one to four substituens which are the same or different and are each selected from the aforementioned substituent group R^{a}). A more preferred example of A is an aryl group or a heterocyclic group (said aryl group or heterocyclic group may be substituted with one or two substituents which are the same or different and are each selected from the following substituent group R^{b});

R^{b} is a C₁₋₆ alkyl group (said C₁₋₆ alkyl group may be substituted with one or two substituents selected from among a carboxy group, a hydroxy group, a carbamoyl group, and -N(R¹¹)(R¹²), a carboxy group, a hydroxy group, a heterocyclic group, a amidino group, -N(R¹³)(R¹⁴), -CON(R¹⁵)(R¹⁶), a C₁₋₆alkoxy group (said C₁₋₆alkoxy group may be substituted with an amino group, an N-methylpiperazinyl group, or a morpholino group), a C₂₋₆alkenyl group (said C₂₋₆alkenyl group may be substituted with a hydroxy group or -N(R¹⁷)(R¹⁸)), a 3-aminoazetidino group, a piperazinyl group, an N-methylpiperazinyl group, a 4-aminopiperidino group, or any of the substituents described by formula [3]: 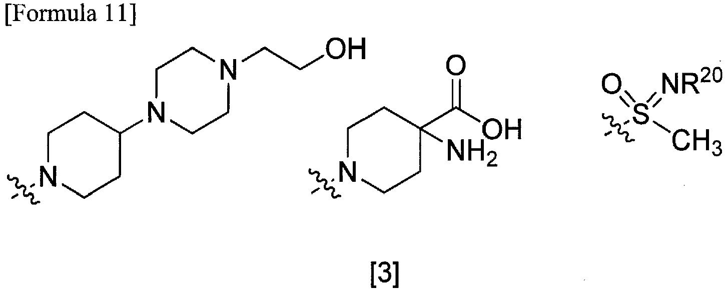
R¹¹ and R¹² are the same or different and each is a hydrogen atom, a methyl group, a n-pentyl group, a n-octyl group, a cyclopropyl group, a cyclohexyl group, a hydroxyethyl group, an N-methylpiperidin-4-yl group, a carboxymethyl group, an N,N-dimethylaminopropyl group, an aminoethyl group, or alternatively,
R¹¹ and R¹², when taken together with the nitrogen atom to which they bind, form an azetidinyl group, a pyrrolidinyl group, a piperidinyl group, or a morpholinyl group;
R¹³ and R¹⁴ are the same or different and each is a hydrogen atom, a t-butoxycarbonyl group, a cyclohexyl group, -CONHSO₂Me, a methyl group, an ethyl group and a n-propyl group (said methyl group, ethyl group and n-propyl group may be substituted with an amino group, an N-methylpiperazinyl group, a morpholino group, -N(CH₂CH₂OH)₂, or a heterocyclic group);
R¹⁵ and R¹⁶ are the same or different and each is a hydrogen atom, a hydroxy group, a 1,3-dihydroxypropan-2-yl group, a methanesulfonyl group, an N,N-dimethylsulfamoyl group, a C₁₋₆ alkyl group (said C₁₋₆ alkyl group may be substituted with one or two substituents selected from among an amino group, a morpholino group, a piperidino group, a piperidin-4-yl group, a carboxy group, a hydroxy group, and the substituent described by formula [4]): 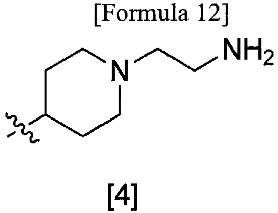
   wherein R¹⁵ and R¹⁶, when taken together with the nitrogen atom to which they bind, form a morpholinyl group;
   R¹⁷ and R¹⁸ are the same or different and each is a hydrogen atom or a methyl group;
   R²⁰ is a hydrogen atom or a methyl group.

The most preferred example of A is a phenyl group or a heterocyclic group (said phenyl group or heterocyclic group may be substituted with one or two substituents which are the same or different and are each selected from the following substituent group R^{c});
R^{c} is a methyl group (said methyl group may be substituted with one or two substituents selected from among a carboxy group, a hydroxy group, a carbamoyl group,
and -N(R¹¹)(R¹²)), a carboxy group, a heterocyclic group, or -CON(R¹⁵)(R¹⁶);
R¹¹ and R¹² are the same or different and each is a hydrogen atom, a methyl group, a n-pentyl group, a n-octyl group, a cyclopropyl group, a cyclohexyl group, a hydroxyethyl group, an N-methylpiperidin-4-yl group, a carboxymethyl group, an N,N-dimethylaminopropyl group or an aminoethyl group, or alternatively,
R¹¹ and R¹², when taken together with the nitrogen atom to which they bind, form an azetidinyl group, a pyrrolidinyl group, a piperidinyl group, or a morpholinyl group;
R¹⁵ and R¹⁶ are the same or different and each is a hydrogen atom, a methanesulfonyl group, a N,N-dimethylsulfamoyl group, a methyl group, an ethyl group or a n-propyl group (said methyl group, ethyl group or n-propyl group may be substituted with an amino group, a morpholino group, a piperidino group, a piperidin-4-yl group, or a hydroxy group).

Preferred examples of the "heterocyclic group" in A are "a monocyclic aromatic heterocyclic group", "a partially saturated monocylic aromatic heterocyclic group", and "a fused cyclic aromatic heterocyclic group" and may be exemplified by a thiazolyl group, a pyrimidinyl group, an imidazolyl group, an oxadiazolonyl group (e.g., 1,2,4-oxadiazol-5(4H)-one), a pyridyl group, an oxodihydropyridyl group (e.g., 1,2-dihydropyridin-2-one), an oxodihydroxadiazolyl group (e.g., 5-oxo-4,5-dihydro-1,2,4-oxadiazol-3-yl), an imidazopyrimidyl group (e.g., imidazo[1,2-a]pyrimidin-2-yl), a pyrazolyl group, and a tetrazolyl group (e.g., 1H-tetrazol-5-yl). Said "heterocyclic group" may be substituted with one to four substituents which are the same or different and are each selected from the above-described substituent groups R^{a} to R^{c}. A preferred example of the "heterocyclic group" in the substituents R^{a} and R^{b} on A is "a monocyclic aromatic heterocyclic group" or "a partially saturated monocyclic aromatic heterocyclic group" and may be exemplified by a thiazolyl group, a pyrimidinyl group, an imidazolyl group, an oxadiazolonyl group (e.g., 1,2,4-oxadiazol-5(4H)-one), a pyridyl group, an oxodihydropyridyl group (e.g., 1,2-dihydropyridin-2-one), an oxodihydroxadiazolyl group (e.g., 5-oxo-4,5-dihydro-1,2,4-oxadiazol-3-yl), a pyrazolyl group, and a tetrazolyl group (e.g., 1H-tetrazol-5-yl).

A preferred example of the "heterocyclic group" in R¹³ and R¹⁴ is "a monocyclic aromatic heterocyclic group" which may be exemplified by a thiazolyl group.

The compounds of the present invention may contain more than one asymmetric center. Hence, those compounds may occur not only as optically active substances but also as racemic modifications thereof; even two or more diastereomers may also occur. All of these forms are included within the scope of the present invention. Individual isomers can be obtained by known methods, such as the use of opticaly active starting materials or intermediates, optically selective reactions or diastereo-selective reactions in the production of intermediates or final products, or chromatography-based separation in the production of intermediates or final products. It should also be noted that in the case where the compunds of the present invention form hydrates or solvates, such hydrates and solvates are also included within the scope of the present invention. Similarly, pharmaceutically acceptable salts of the hydrates or solvates of the compunds of the present invention are also included within the scope of the present invention.

The term "pharmaceutically acceptable salts" as used in the present invention means salts that are used in chemotherapy and prevention of bacterial infections. Examples include: salts with acids such as acetic acid, propionic acid, butyric acid, formic acid, trifluoroacetic acid, maleic acid, tartaric acid, citric acid, stearic acid, succinic acid, ethylsuccinic acid, malonic acid, lactobionic acid, gluconic acid, glucoheptonic acid, benzoic acid, methanesulfonic acid, ethanesulfonic acid, 2-hydroxyethanesulfonic acid, benzenesulfonic acid, paratoluenesulfonic acid (tosic acid), lauryl sulfuric acid, malic acid, aspartic acid, glutamic acid, adipic acid, cysteine, N-acetylcysteine, hydrochloric acid, hydrobromic acid, phosphoric acid, sulfuric acid, hydroiodic acid, nicotinic acid, oxalic acid, picric acid, thiocyanic acid, undecanoic acid, acrylic acid polymers and carboxyvinyl polymers; salts with inorganic bases such as lithium salts, sodium salts, potassium salts, magnesium salts and calcium salts; organic amines such as morpholine and piperidine; as well as salts with amino acids.

The compounds of the present invention can be formulated as pharmaceutical preparations by being combined with one or more pharmaceutically acceptable carriers, excipients or diluents. Examples of such carriers, excipients and diluents include water, lactose, dextrose, fructose, sucrose, sorbitol, mannitol, polyethylene glycol, propylene glycol, starch, gum, gelatin, alginate, calcium silicate, calcium phosphate, cellulose, water syrup, methyl cellulose, polyvinylpyrrolidone, alkyl parahydroxybenzosorbates, talc, magnesium stearate, stearic acid, glycerin, and oils such as sesame oil, olive oil, and soyben oil. Depending on the need, the above-mentioned carriers, excipients or diluents may be mixed with commonly employed additives such as fillers, binders, disintegrants, pH modifiers and solubilizers, so that oral or parenteral medications such as tablets, pills, capsules, granules, powders, liquids, emulsions, suspensions, ointments, injections and cutaneous patches can be prepared by common pharmaceutical formulating procedures.

The compounds of the present invention can be administered to an adult patient either orally or parenterally at a daily dosage of 1-5000 mg that may be given either once a day or in several divided portions per day. It should be noted that the dosage can be adjusted as appropriate for the type of the disease to be treated, the age of the patient, their body weight, the symptom of the disease, and other factors. If desired, the compounds of the present invention can be used in combination with other drugs.

The compounds of the present invention may, for example, be produced by various synthesis methods. The following methods are simply intended as illustrations of the processes for producing the compounds of the present invention and it should be understood that they are by no means limiting. 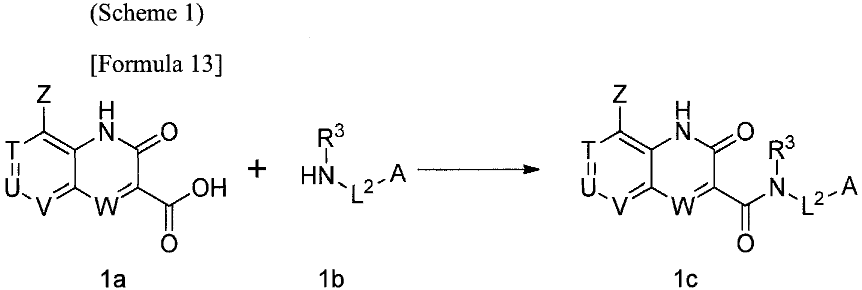 A compound represented by general formula (1a) (wherein Z, T, U, V and W are as defined above) is reacted with a compound represented by general formula (1b) (wherein R³, L² and A are as defined above) in the presence of a condensing agent and in the presence or absence of a base, whereupon a compound represented by general formula (1c) (wherein the respective symbols are as defined above) can be obtained; alternatively, an acid chloride or an acid anhydride of the compound represented by general formula (1a) is reacted with the compound represented by general formula (1b) in the presence or absence of a base, whereupon a compound of the present invention can be obtained that is represented by general formula (1c). 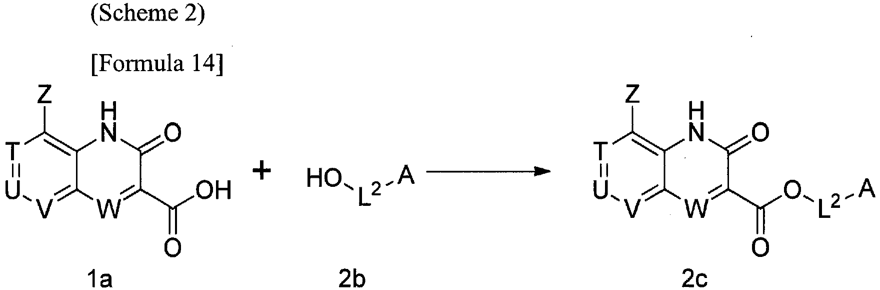 A compound represented by general formula (1a) (wherein Z, T, U, V and W are as defined above) is reacted with a compound represented by general formula (2b) (wherein L² and A are as defined above) in the presence of a condensing agent and in the the presence or absence of a base, whereupon a compound represented by general formula (2c) (wherein the respective symbols are as defined above) can be obtained; alternatively, an acid chloride or an acid anhydride of the compound represented by general formula (1a) is reacted with the compound represented by general formula (2b) in the presence or absence of a base, whereupon a compound of the present invention can be obtained that is represented by general formula (2c). 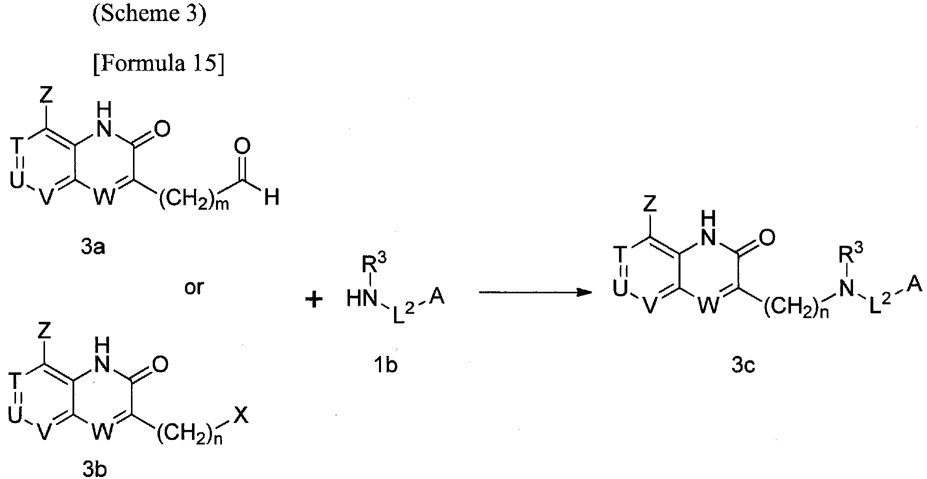 A compound represented by general formula (3a) (wherein Z, T, U, V and W are as defined above, and m is an integer of 0 to 3) amd a compound represented by general formula (1b) (wherein R³, L² and A are as defined above) are reacted in the presence or absence of an acid such as acetic acid and in the presence of a reducing agent such as sodium triacetoxyborohydride, sodium cyanoborohydride, sodium borohydride or 2-picoline borane, whereupon a compound of the present invention can be obtained that is represented by general formula (3c) (wherein the respective symbols are as defined above); alternatively, a compound represented by general formula (3b) (wherein Z, T, U, V and W are as defined above, n is an integer of 1 to 4, and X is a leaving group) and the compound represented by general formula (1b) are reacted in the presence of a base, whereupon a compound of the present invention is obtained that is represented by general formula (3c). 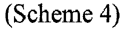 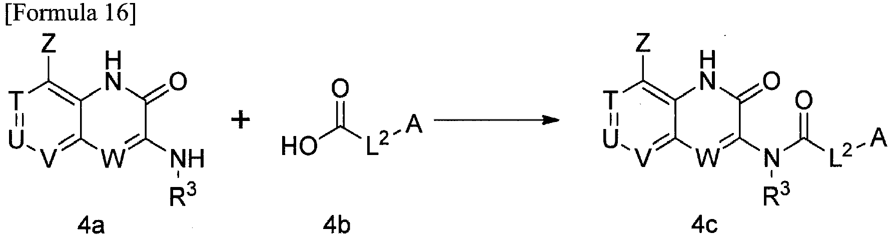 A compound represented by general formula (4a) (wherein Z, T, U, V, W and R³ are as defined above) is reacted with a compound represented by general formula (4b) (wherein L² and A are as defined above) in the presence of a condensing agent and in the presence or absence of a base, whereupon a compound represented by general formula (4c) (wherein the respective symbols are as defined above) can be obtained; alternatively, an acid chloride or an acid anhydride of the compound represented by general formula (4b) is reacted with the compound represented by general formula (4a) in the presence or absence of a base, whereupon a compound of the present invention can be obtained that is represented by general formula (4c). 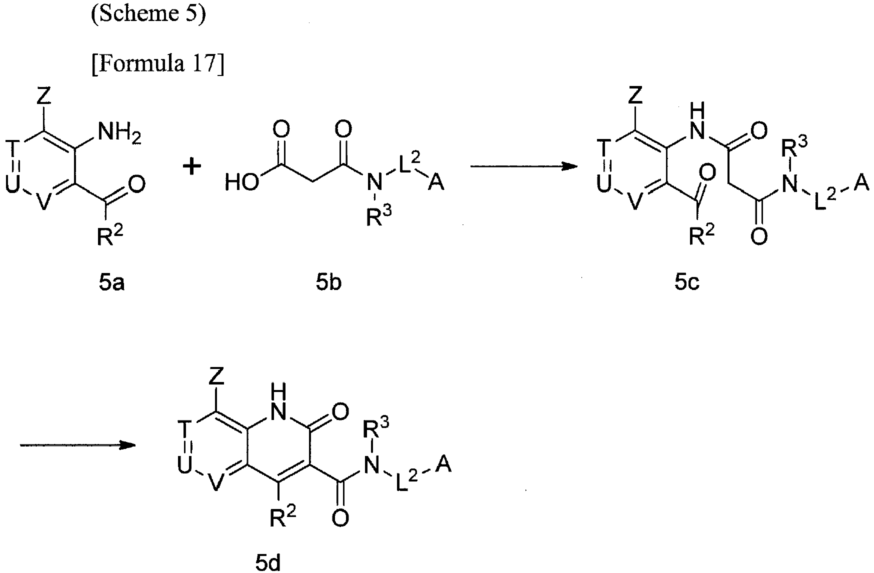 A compound represented by general formula (5a) (wherein Z, T, U, V and R² are as defined above) is reacted with a compound represented by general formula (5b) (wherein R³, L² and A are as defined above) in the presence of a condensing agent and in the presence or absence of a base, whereupon a compound represented by general formula (5c) (wherein the respective symbols are as defined above) cam be obtained; alternatively, an acid chloride or an acid anhydride of the compound represented by general formula (5b) is reacted with the compound represented by general formula (5a) in the presence or absence of a base, whereupon a compound represented by general formula (5c) can be obtained. Subsequently, the compound represented by general formula (5c) is subjected to reaction in the presence of a base such as sodium ethoxide, whereupon a compound of the present invention can be obtained that is represented by general formula (5d) (wherein the respective symbols are as defined above).

In the synthesis methods set forth above, the reaction steps can be changed in order as necessary. What is more, if an amino group, a hydroxy group, a formyl group, a carboxy group and an amide group are present in the compounds or intermediates thereof that are obtained in the respective reaction steps, their protecting groups may be deprotected or suitably recombined to perform the reaction.

In addition, compounds of the present invention can also be synthesized by mutual conversion of Z, T, U, V, W, R², R³, L² and A in those compounds of the present invention which have been obtained by the synthesis methods described above.

Unless otherwise noted, the base to be used in the above-described reactions may be exemplified by the following: hydrides of alkali metals or alkaline earth metals such as lithium hydride, sodium hydride, potassium hydride, and calcium hydride; amides of alkali metals or alkaline earth metals such as lithium amide, sodium amide, lithium diisopropylamide, lithium dicyclohexylamide, lithium hexamethyldisilazide, sodium hexamethyldisilazide, and potassium hexamethyldisilazide; lower alkoxides of alkali metals or alkaline earth metals such as sodium methoxide, sodium ethoxide, and potassium t-butoxide; alkyllithium such as butyllithium, s-butyllithium, t-butyllithium, and methyllithium; hydroxides of alkali metals or alkaline earth metals such as sodium hydroxide, potassium hydroxide, lithium hydroxide, and barium hydroxide; carbonates of alkali metals or alkaline earth metals such as sodium carbonate, potassium carbonate, and cesium carbonate; hydrogencarbonates of alkali metals or alkaline earth metals such as sodium hydrogencarbonate and potassium hydrogencarbonate; amines such as triethylamine, N-methylmorpholine, N,N-diisopropylethylamine, 1,8-diazabicyclo[5.4.0]undec-7-ene (DBU), 1,5-diazabicyclo[4.3.0]non-5-ene (DBN), and N,N-dimethylaniline; and basic heterocyclic compounds such as pyridine, imidazole, and 2,6-lutidine. These bases are chosen as appropriate for various reaction conditions known to persons skilled in the art.

The acid may be exemplified by inorganic acids including hydrochloric acid, hydrobromic acid, sulfuric acid, nitric acid, and phosphoric acid, as well as organic acids including p-toluenesulfonic acid, methanesulfonic acid, trifluoroacetic acid, formic acid, acetic acid, citric acid, and oxalic acid. These acids are chosen as appropriate for various reaction conditions known to persons skilled in the art.

The condensing agent may be exemplified by O-(7-azabenzotriazol-1-yl)-N,N,N',N'-tetramethyluronium hexafluorophosphate, 1-(3-dimethylaminopropyl)-3-ethylcarbodiimide hydrochloride, dicyclohexylcarbodiimide, carbonyldiimidazole, 2-chloro-1-methylpyridinium iodide salt, 4-(4,6-dimethoxy-1,3,5-triazin-2-yl)-4-methylmorpholine chloride salt, O-(7-azabenzotriazol-1-yl)-N,N,N',N'-tetramethyluronium hexafluorophosphate and benzotriazol-1-yl-oxy-tris-pyrrolidino-phosphonium hexafluorophosphate, for example.

The activating agent to be used in the case of employing the method that starts with an acid chloride or an acid anhydride may be exemplified by thionyl chloride, oxalyl chloride, phosphoryl chloride, acetic anhydride and chloroformic acid esters, for example.

The catalyst may be exemplified by palladium acetate, palladium chloride, bis(triphenylphosphine)palladium(II) dichloride, tetrakis(triphenylphosphine)palladium, bis(acetonitrile)dichloropalladium, bis(benzonitrile)dichloropalladium, tris(dibenzylideneacetone)dipalladium, bis(dibenzylideneacetone)palladium, 1,1'-bis(diphenylphosphino)ferrocene dichloropalladium, bis(tricyclohexylphosphine)dichloropalladium, bis(tri-o-tolylphosphine)dichloropalladium, bis(tri-t-butylphosphine)dichloropalladium, (1,3-bis(2,6-diisopropylphenyl)imidazolidene)(3-chloropyridyl)palladium(II) dichloride, palladium on carbon, palladium hydroxide and copper iodide, for example.

The ligand may be exemplified by tri-t-butylphosphine, tricyclohexylphosphine, triphenylphosphine, tritolylphosphine, tributylphosphite, tricyclohexylphosphite, triphenylphosphite, 1,1' -bis(diphenylphosphino)ferrocene, 2,2'-bis(diphenylphosphino)-1,1'-binaphthyl, 2-dicyclohexylphosphino-2',6'-dimethoxybiphenyl, 2-dicyclohexylphosphino-2',4',6'-triisopropylbiphenyl, 2-(di-t-butylphosphino)-2',4',6'-triisopropylbiphenyl and 2-(dit-butylphosphino)biphenyl, for example.

The oxiding agent may be exemplified by inorganic and organic peroxides such as manganese dioxide, Dess-Martin periodinane, 2-iodoxybenzoic acid, potassium permanganate, chromium oxide, potassium dichromate, hydrogen peroxide, m-chloroperbenzoic acid, urea hydrogen peroxide adduct/phthalic anhydride, t-butyl hydroperoxide, cumene hydroperoxide, etc., as well as selenium dioxide, lead (IV) acetate, t-butyl hypochlorite, sodium hypochlorite, and 1, 1,1-triacetoxy-1,1-dihydro-1,2-benziodoxol-3(1H)-one, for example.

The reducing agent may be exemplified by hydrogenated complex compounds such as lithium aluminum hydride, sodium triacetoxyborohydride, sodium cyanoborohydride, sodium borohydride, lithium borohydride, and diisobutylaluminum hydride, as well as boranes, sodium and sodium amalgam, for example.

The metal salt may be exemplified by zinc chloride, zirconium chloride, indium chloride and magnesium chloride, for example.

The solvent is not limited in any particular way as long as it is stable under the reaction conditions of interest and inert (not interfering with the reaction). It may be exemplified by polar solvents (e.g., alcoholic solvents such as water, methanol, ethanol and isopropanol), inert solvents (e.g., halogenated hydrocarbon solvents such as chloroform, methylene chloride, dichloroethane, carbon tetrachloride, etc.; ether solvents such as diethyl ether, diisopropy ether, tetrahydrofuran, 1,4-dioxane, dimethoxyethane, etc.; aprotic solvents such as dimethylformamide, dimethyl sulfoxide, ethyl acetate, t-butyl acetate, acetonitrile, propionitrile, etc.; aromatics such as benzene, toluene, anisole, etc.; or hydrocarbons such as cyclohexane), or mixtures of these solvents.

The reaction may be carried out at a suitable temperature selected within the range from -78°C to the boiling point of the reaction solvent, under atmospheric or superatmospheric pressure, with microwaves being applied.

Hereinbelow, the present invention will be explained in great detail by means of Production Examples, Reference Examples, Working Examples, and Test Examples. However, the present invention is by no means limited to these Production Examples, Reference Examples, Working Examples, and Test Examples, which may be modified without departing from the scope of the present invention.

When purification was performed by column chromatography in the Production Examples, Reference Examples and Working Examples, Biotage® SNAPCartridge ISOLUTE Flash-NH2 (product of Biotage AB) and REVELERIS® Amino 40 µm (product of GRACE) were used as "NH silica gel cartridge" whereas Biotage® SNAP Ultra (product of Biotage AB) and REVELERIS® Silica 40 µm (product of GRACE) were used as "silica gel cartridge".
A commercial product of Silica Gel 60N (product of KANTO KAGAKU) was used as "silica gel 60 N" whereas a commercial product of Chromatorex® NH (product of Fuji Silysia Chemical Ltd.) was used as "chromatorex NH". In the case of purification by TLC, Silica gel 60F254 (Merck) was used as TLC (silica gel plate) whereas TLC plate NH (Fuji Silysia) was used as TLC (NH silica gel plate). As a phase separator, the product of Biotage AB was used.

Purification by reversed phase column chromatography (hereinafter sometimes designated as preparative HPLC or preparative LCMS) was performed under conditions that were suitably selected from the four preparative conditions described below.

As a column, either one of the following models was used:
YMC-Actus Triart C18, 5.0 µm, φ30×50 mm
Xbridge Prep C18, 5.0 µm OBD, φ30×50 mm
Waters XSlect CSH C18, 5.0 µm, φ30×50 mm
Preparative apparatus: Agilent 1260 Infinity or Agilent 6130 (ionization method: Electron Spray Ionization: ESI) (both manufactured by Agilent), with Agilent 385-ELSD being used in combination with an ELSD detector as an attachment.
Solvents: Fluid A (0.1% formic acid containing water); Fluid B (0.1% formic acid containing acetonitrile)

### Preparative Condition 1:

Flow rate: 50 mL/min
0.0-0.5 min (Fluid A/Fluid B = 90/10)
0.5-7.5 min (Fluid A/Fluid B = 90/10 - 20/80)
7.5-7.95 min (Fluid A/Fluid B = 20/80)
7.95-8.0 min (Fluid A/Fluid B = 20/80 - 5/95)
8.0-9.0 min (Fluid A/Fluid B = 5/95)

### Preparative Condition 2:

Flow rate: 50 mL/min
0.0-0.5 min (Fluid A/Fluid B = 95/5)
0.5-7.5 min (Fluid A/Fluid B = 95/5 - 50/50)
7.5-7.95 min (Fluid A/Fluid B = 50/50)
7.95-8.0 min (Fluid A/Fluid B = 50/50 - 5/95)
8.0-9.0 min (Fluid A/Fluid B = 5/95)

### Preparative Condition 3:

Flow rate: 50 mL/min
0.0-0.5 min (Fluid A/Fluid B = 80/20)
0.5-7.0 min (Fluid A/Fluid B = 80/20 - 5/95)
7.0-7.45 min (Fluid A/Fluid B = 5/95)
7.45-7.5 min (Fluid A/Fluid B = 5/95 - 1/99)
7.5-9.0 min (Fluid A/Fluid B = 1/99)

### Preparative Condition 4:

Flow rate: 40 mL/min
0.0-2.0 min (Fluid A/Fluid B = 90/10)
2.0-11.0 min (Fluid A/Fluid B = 90/10 - 20/80)
11.0 -12.0 min (Fluid A/Fluid B = 20/80 - 5/95)
12.0-13.5 min (Fluid A/Fluid B = 5/95)

The instrumental data set out in the Production Examples, Reference Examples and Working Examples were obtained by measurement with the following instruments.
Microwave reactor: Initiator (Biotage AB)
NMR spectra: [1H-NMR] 600 MHz, JNM-ECA600 (JEOL); 500 MHz, JNM-ECA500 (JEOL); 400 MHz, AVANCE III HD 400 (BRUKER)

The high-performance liquid chromatography mass spectra (LCMS) and retention times (RT) set out in the Production Examples, Reference Examples and Working Examples were measured under the following conditions.

### Analytical Condition 1:

Measuring instrument: LCMS-2010EV of Shimadzu Corporation
Column: Shimadzu XR-ODS, 2.2 µm, φ2.0×30 mm
Ionization method: ESI/APCI (atmospheric pressure chemical ionization) dual source
Solvents: Fluid A, 0.1% formic acid containing water; Fluid B, 0.1% formic acid containing acetonitrile
Flow rate: 0.6 mL/min; Detection method: 254 nm
Gradientts:
   0.0-0.5 min (Fluid A/Fluid B = 90/10)
   0.5-1.5 min (Fluid A/Fluid B = 90/10 - 60/40)
   1.5-2.5 min (Fluid A/Fluid B = 60/40 - 1/99)
   2.5-5.0 min (Fluid A/Fluid B = 1/99)

### Analytical Condition 2

Measuring instruments: Agilent 1290 Infinity and Agilent 6130 or 6150 of Agilent, with Agilent 385-ELSD being used in combination with an ELSD detector as an attachment.
Column: Acquity CSH C18, 1.7 µm, φ2.1×50 mm of Waters
Ionization method: ESI
Solvents: Fluid A, 0.1% formic acid containing water; Fluid B, 0.1% formic acid containing acetonitrile
Flow rate: 0.8 mL/min; Detection method: 254 nm
Gradients:
   0.0-0.8 min (Fluid A/Fluid B = 95/5 - 60/40)
   0.8-1.08 min (Fluid A/Fluid B = 60/40 - 1/99)
   1.08-1.38 min (Fluid A/Fluid B = 1/99)

### Analytical Condition 3

Measuring instruments, column, ionization method, solvents, flow rate, and detection method were the same as in Analytical Condition 2.

### Gradients:

0.0-1.2 min (Fluid A/Fluid B = 80/20 - 1/99)
1.2-1.4 min (Fluid A/Fluid B = 1/99)

### Analytical Condition 4

Measuring instruments, column, ionization method, solvents, flow rate, and detection method were the same as in Analytical Condition 2.

### Gradients:

0.0-0.8 min (Fluid A/Fluid B = 70/30 - 1/99)
0.8-1.4 min (Fluid A/Fluid B = 1/99)

The compound names given in the Production Examples, Reference Examples and Workikng Examples were determined with ACD/Name 2015 (ACDLabs 2015 LSM, Advanced Chemistry Development Inc.)

The abbreviations used in the Production Examples, Reference Examples and Workikng Examples are listed below together with their meanings.
AcOEt: Ethyl acetate
APCI: Atmospheric pressure chemical ionization
aq.: Aqueous solution
Boc: t-Butoxycarbonyl
Bn: Benzyl
Bu: Butyl
DEAD: Diethyl azodicarboxylate
DIPEA: N,N-diisopropylethylamine
DMF: N,N-dimethylformamide
DMSO-d6: Dimethyl sulfoxide with 6 hydrogen atoms deuterated
ESI: Electrospray ionization
Et: Ethyl
HATU: O-(7-Azabenzotriazol-1-yl)-N,N,N',N'-tetramethyluronium hexafluorophosphate
HOBt·H₂O: 1-Hydroxybenzotriazole monohydrate
IBX: 2-Iodoxybenzoic acid
IPA: Isopropyl alcohol
IPE: Diisopropyl ether
LC: Liquid chromatography
LDA: Lithium diisopropylamide
Me: Methyl
NMP: 1-Methyl-2-pyrrolidone
PEPPSI: (1,3-Bis(2,6-diisopropylphenyl)imidazolidene)(3-chloropyridyl)palladium(II) dichloride
PdCl₂(dppf)·CH₂Cl₂: 1,1'-Bis(diphenylphosphino)ferrocene palladium(II) chloride dichloromethane complex
PdCl₂(PPh₃)₂: Bis(triphenylphosphine)palladium(II) dichloride
PPTS: Pyridine 4-methylbenzenesulfonate
(p-Tol)₃P: Tri(4-methylphenyl)phosphine
p-TsOH·H₂O: p-Toluenesulfonic acid monohydrate
TBAF: Tetra-n-butylammonium fluoride
TEA: Triethylamine
TFA: Trifluoroacetic acid
THF: Tetrahydrofuran
THP: Tetrahydropyranyl
TMS: Trimethylsilyl
TIPS: Triisopropylsilyl
TsCl: 4-Methylbenzenesulfonyl chloride
WSC·HCl: 1-Ethyl-3-(3-dimethylaminopropyl)carbodiimide hydrochloride
s: Singlet
br s: Broad singlet
d: Doublet
br d: Broad doublet
dd: Double doublet
dt: Double triplet
m: Multiplet
t: Triplet
br t: Broad triplet
td: Triple doublet
tt: Triple triplet
q: Quartet
quin: Quintet
J: Coupling constant
Hz: Hertz

### Production Example 1 8-(Mehylamino)-2-oxo-1,2-dihydroquinoline-3-carboxylic acid

### 1-(1) Ethyl 8-(methylamino)-2-oxo-1,2-dihydroquinoline-3-carboxylate

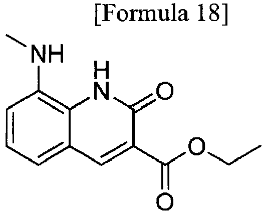

To a solution of 2-amino-3-(methylamino)benzaldehyde (308 mg) in ethanol (4mL), diethyl malonate (434 µL) and piperidine (263 µL) were added and the mixture was stirred under heating for 8 hours at 75°C. After leaving the mixture to cool to room temperature, the precipitating solid was recovered by filtration to obtain the titled compound (291 mg) as a yellow solid.
MS (ESI pos.) m/z : 247 [M+H]+, RT=0.782 min (Analytical Condition 3)

### 1-(2) 8-(Methylamino)-2-oxo-1,2-dihydroquinoline-3-carboxylic acid

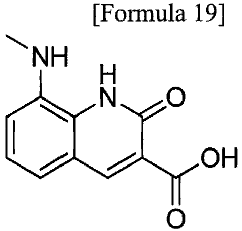

To a solution in tetrahydrofuran (12 mL) of the ethyl 8-(methylamino)-2-oxo-1,2-dihydroquinoline-3-carboxylate (291 mg) obtained in Production Example 1-(1), 1 M sodium hydroxide in aqueous solution (12 mL) was added and the mixture was stirred for 8.5 hours at room temperature. The reaction mixture was neutralized to pH 4 with an aqueous solution of 1 M hydrochloric acid. The precipitating solid was recovered by filtration and then washed with water to obtain the titled compound (281 mg) as a yellow solid.
MS (ESI pos.) m/z : 219 [M+H]+, RT=0.693 min (Analytical Condition 3)
¹H NMR (600 MHz, DMSO-d6) δ ppm 2.86 (d, J=4.5 Hz, 3 H), 6.15 (br d, J=4.5 Hz, 1 H), 6.87 (br d, J=5.8 Hz, 1 H), 7.22 - 7.30 (m, 2 H), 8.87 (s, 1 H), 12.26 (br s, 1 H), 14.83 (br s, 1 H)

### Production Example 2 6-Fluoro-8-(methylamino)-2-oxo-1,2-dihydroquinoline-3-carboxylic acid

### 2-(1) [5-Fluoro-3-(methylamino)-2-nitrophenyl]methanol

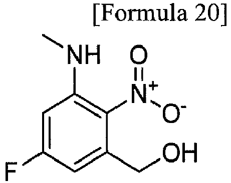

To a solution of (3,5-difluoro-2-nitrophenyl)methanol (20.72 g) in ethanol (50 mL), 33% methylamine in ethanol solution (27.3 mL) was added at room temperature and the mixture was stirred for 2 hours. The resulting insoluble matter was recovered by filtration and washed with ethanol and diethyl ether to obtain the titled compound (12.96 g) as a yellow solid.
MS (ESI pos.) m/z : 201 [M+H]+, RT=0.709 min (Analytical Condition 3)

### 2-(2) 5-Fluoro-3-(methylamino)-2-nitrobenzaldehyde

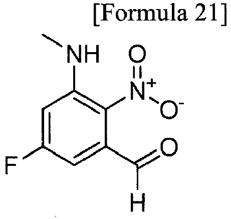

To a solution in chloroform (2840 mL) of the [5-fluoro-3-(methylamino)-2-nitrophenyl]methanol (23.66 g) obtained in Production Example 2-(1), manganese dioxide (51.38 g) was added at 50°C and the mixture was stirred under heating for 7 hours. The insoluble matter was filtered off and the filtrate was concentrated under reduced pressure to obtain the titled compound (20.75 g) as an orange solid.
MS (ESI pos.) m/z : 199 [M+H]+, RT=0.817 min (Analytical Condition 3)

### 2-(3) 2-Amino-5-fluoro-3-(methylamino)benzaldehyde

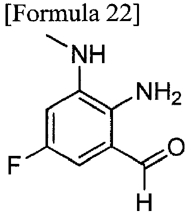

To a solution in ethanol (700 mL) and water (70 mL) of the 5-fluoro-3-(methylamino)-2-nitrobenzaldehyde (10.35 g) obtained in Production Example 2-(2), ammonium chloride (2.79 g) and an iron powder (16.04 g) were added and the mixture was stirred under heating for 1.5 hours at 75°C. The insoluble matter was filtered while hot and the filtrate was concentrated under reduced pressure. To the residue, saturated sodium hydrogencarbonate (300 mL) and ethyl acetate (500 mL) were added and liquid-liquid separation was performed. The aqueous layer was extracted with ethyl aceate (300 mL) and the organic layers were combined, washed with saturated brine, then dried over anhydrous magnesium sulfate, and concentrated under reduced pressure. The thus obtained residue was purified by column chromatography (silica gel cartridge, hexane/ethyl acetate) to obtain the titled compound (6.43 g) as a yellow solid.
MS (ESI pos.) m/z : 169 [M+H]+, RT=0.757 min (Analytical Condition 3)

### 2-(4) Ethyl 6-fluoro-8-(methylamino)-2-oxo-1,2-dihydroquinoline-3-carboxylate

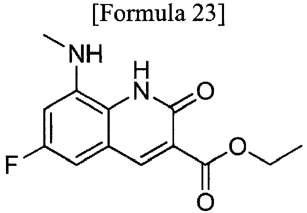

To a solution in ethanol (234 mL) of the 2-amino-5-fluoro-3-(methylamino)benzaldehyde (6.35 g) obtained in Production Example 2-(3), diethyl malonate (7.99 mL) and piperidine (4.84 mL) were added and the mixture was stirred under heating for 17 hours at 75°C. After being left to cool, the reaction mixture was ice cooled and the resulting precipitate was recovered by filtration and washed with cold ethanol to obtain the titled compound (8.60 g) as a yellow solid.
MS (ESI pos.) m/z : 265 [M+H]+, RT=0.864 min (Analytical Condition 3)

### 2-(5) 6-Fluoro-8-(methylamino)-2-oxo-1,2-dihydroquinoline-3-carboxylic acid

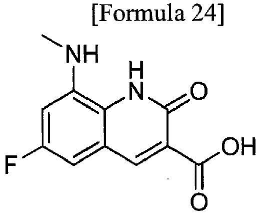

To a solution in tetrahydrofuran (125 mL) of the ethyl 6-fluoro-8-(methylamino)-2-oxo-1,2-dihydroquinoline-3-carboxylate (6.59 g) obtained in Production Example 2-(4), 2 M sodium hydroxide in aqueous solution (125 mL) was added and the mixture was stirred for 16 hours at room temperature. After ice cooling, the reaction mixture was adjusted to pH 4 with an aqueous solution of 1 M hydrochloric acid; the resulting precipitate was recovered by filtration and washed with water to obtain the titled compound (6.75 g) as a yellow solid. MS (ESI pos.) m/z : 237 [M+H]+, RT=0.758 min (Analytical Condition 3)
¹H NMR (600 MHz, DMSO-d6) δ ppm 2.85 (d, J=5.0 Hz, 3 H), 6.28 - 6.43 (m, 2 H), 6.76 (br s, 1 H), 8.47 (br s, 1 H)

### Production Example 3 8-(Methylamino)-2-oxo-1,2-dihydro-1,7-naphthyridine-3-carboxylic acid

### 3-(1) tert-Butyl [2-[benzyl(methyl)amino]pyridin-3-yl]carbamate

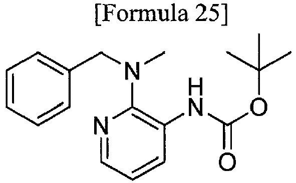

To a solution of N2-benzyl-N2-methylpyridine-2,3-diamine (20.4 g) in tetrahydrofuran (400 mL), 1.9 M sodium (trimethylsilyl)amide in tetrahydrofuran solution (111 mL) and di-tert-butyl dicarbonate (23.0 g) were added under ice cooling and the mixture was stirred for 2 hours at room temperature under a nitrogen gas atmosphere. The reaction mixture was diluted with water and extracted with ethyl acetate. The organic layer was washed with saturated brine, then dried over anhydrous magnesium sulfate, and concentrated under reduced pressure. The residue was purified by column chromatography (NH silica gel cartridge; hexane/ethyl acetate = 88:12) to obtain the titled compound (26.8 g) as a red oil. MS (ESI pos.) m/z : 314 [M+H]+, RT=1.131 min (Analytical Condition 3)

### 3-(2) tert-Butyl [2-[benzyl(methyl)amino]-4-formylpyridin-3-yl]carbamate

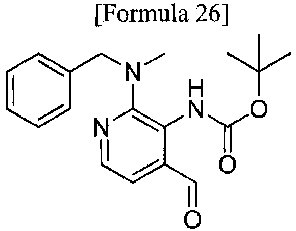

To a solution in tetrahydrofuran (90 mL) of tetramethyl ethylene diamine (8.6 mL) and the tert-butyl [2-[benzyl(methyl)amino]pyridin-3-yl]carbamate (6.0 g) obtained in Production Example 3-(1), 2.6 M n-butyllithium in hexane solution (22 mL) was added dropwise at -78°C under a nitrogen gas atmosphere. After 2-hr stirring at -15°C, the reaction mixture was cooled to -78°C and dimethylformamide (7.5 mL) was added. After 2-hr stirring at -15°C, the reaction mixture was stirred for 4 hours at room temperature. A saturated aqueous solution of ammonium chloride was added to the reaction mixture, which was then extracted with ethyl acetate. The organic layer was washed with saturated brine and thereafter dried over anhydrous magnesium sulfate and concentrated under reduced pressure. The residue was purified by column chromatography (silica gel cartridge, hexane/ethyl acetate = 93:7 - 80:20) to obtain the titled compound (428 mg) as a yellow solid.
MS (ESI pos.) m/z : 342 [M+H]+, RT=1.180 min (Analytical Condition 3)

### 3-(3) 3-Amino-2-[benzyl(methyl)amino]pyridine-4-carbaldehyde

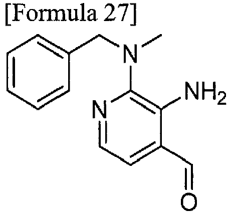

A solution in 1 M hydrochloric acid in aqueous solution (20 mL) of the tert-butyl [2-[benzyl(methyl)amino]-4-formylpyridin-3-yl]carbamate (428 mg) obtained in Production Example 3-(2) was stirred under heating for 0.5 hour at 50°C. To the reaction mixture, an aqueous solution of 2 M sodium hydroxide was added to establish pH 9, followed by extraction with ethyl acetate. The organic layer was washed with saturated brine and thereafter dried over anhydrous magnesium sulfate and concentrated under reduced pressure to obtain the titled compound (308 mg) as a yellow oil.
MS (ESI pos.) m/z : 242 [M+H]+, RT=0.932 min (Analytical Condition 3)

### 3-(4) Ethyl 8-[benzyl(methyl)amino]-2-oxo-1,2-dihydro-1,7-naphthyridine-3-carboxylate

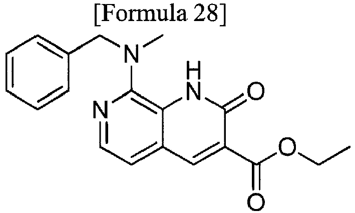

To a solution in ethanol (3 mL) of the 3-amino-2-[benzyl(methyl)amino]pyridine-4-carbaldehyde (308 mg) obtained in Production Example 3-(3), diethyl malonate (270 µL) and piperidine (164 µL) were added and the mixture was stirred under heating for 3 hours at 75°C. The reaction mixture was purified by column chromatography (NH silica gel cartridge, hexane/ethyl acetate = 50:50 to chloroform/methanol = 100:0) to obtain the titled compound (118 mg) as a yellow oil.
MS (ESI pos.) m/z : 338 [M+H]+, RT=0.870 min (Analytical Condition 3)

### 3-(5) Ethyl 8-(methylamino)-2-oxo-1,2-dihydro-1,7-naphthyridine-3-carboxylate

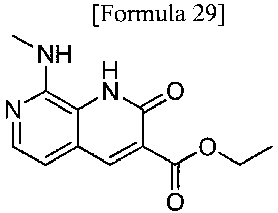

To a solution in ethanol (7 mL) of the ethyl 8-[benzyl(methyl)amino]-2-oxo-1,2-dihydro-1,7-naphthyridine-3-carboxylate (76 mg) obtained in Production Example 3-(4), 5% palladium on carbon (40 mg) was added and the mixture was stirred for 48 hours at room temperature under a hydrogen atmosphere. The insoluble matter was removed by filtration and, thereafter, the filtrate was concentrated under reduced pressure. The residue was purified by column chromatography (NH silica gel cartridge, chloroform/methanol = 100:0 - 91:9) to obtain the titled compound (25 mg) as a yellow solid.
MS (ESI pos.) m/z : 248 [M+H]+, RT=0.639 min (Analytical Condition 2)

### 3-(6) 8-(Methylamino)-2-oxo-1,2-dihydro-1,7-naphthyridine-3-carboxylic acid

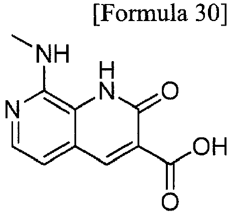

To a solution in tetrahydrofuran (1 mL) of the ethyl 8-(methylamino)-2-oxo-1,2-dihydro-1,7-naphthyridine-3-carboxylate (25 mg) obtained in Production Example 3-(5), 1 M sodium hydroxide in aqueous solution (1 mL) was added and the mixture was stirred for 10 hours at room temperature. The reaction mixture was neutralized to pH 3 with an aqueous solution of 1 M hydrochloric acid. The precipitating solid was recovered by filtration and washed with water, whereafter the titled compound (20 mg) was obtained as a pale yellow solid.
MS (ESI pos.) m/z : 220 [M+H]+, RT=0.600 min (Analytical Condition 3)
¹H NMR (600 MHz, DMSO-d6) δ ppm 3.05 (br s, 3 H), 7.03 - 7.20 (m, 2 H), 7.28 - 7.52 (m, 1 H), 8.54 (s, 1 H), 12.61 (br s, 1 H)

### Production Example 4 8-[(tert-Butoxycarbonyl)(methyl)amino]-2-[(4-methoxyphenyl)methoxy]-1,6-naphthyridine-3-carboxylic acid

### 4-(1) Ethyl 8-iodo-2-oxo-1,2-dihydro-1,6-naphthyridine-3-carboxylate

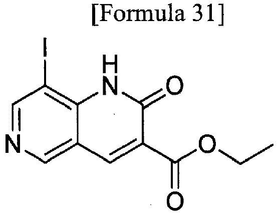

To a solution of ethyl 2-oxo-1,2-dihydro-1,6-naphthyridine-3-carboxylate (5.1 g) in acetic acid (125 mL), N-iodosuccinimide (7.9 g) was added and the mixture was stirred for an hour at 90°C. After further addition of N-iodosuccinimide (5.0 g), the mixture was stirred for 2 hours at 90°C. After adding an aqueous solution of sodium thiosulfate to the reaction mixture, the latter was concentrated under reduced pressure. To the residue, saturated aqueous sodium bicarbonate was added to form a suspension; the suspended matter was recovered by filtration to obtain a 1:1 mixture of the titled compound and the raw material (2.0 g) as a brown solid.
MS (ESI pos.) m/z : 345 [M+H]+, RT=0.607 min (Analytical Condition 3)

### 4-(2) Ethyl 8-iodo-2-[(4-methoxyphenyl)methoxy]-1,6-naphthyridine-3-carboxylate

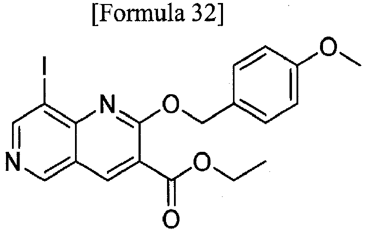

To a solution in tetrahydrofuran (10 mL) of the 1:1 mixture (598 mg) of ethyl 8-iodo-2-oxo-1,2-dihydro-1,6-naphthyridine-3-carboxylate and ethyl 2-oxo-1,2-dihydro-1,6-naphthyridine-3-carboxylate as obtained in Production Example 4-(1), 4-methoxybenzyl alcohol (433 µL) and cyanomethylene tributyl phospholan (1.4 mL) were added and the mixture was stirred for 3 hours at 80°C. After further addition of 4-methoxybenzyl alcohol (433 µL) and cyanomethylene tributyl phospholan (1.4 mL), the mixture was stirred for 2 hours at 100°C. After leaving the reaction mixture to cool to room temperature, water was added to the same. The aqueous layer was extracted with chloroform and separated by means of a phase separator whereas the organic layer was concentrated under reduced pressure. The residue was suspended in hexane and the suspended matter was recovered by filtration to obtain a crude product. The crude product was purified by column chromatography (silica gel cartridge, hexane/ethyl acetate = 97:3 - 60:40) to obtain the titled compound (205 mg) as a white solid.
MS (ESI pos.) m/z : 465 [M+H]+, RT=1.281 min (Analytical Condition 3)

### 4-(3) Ethyl 8-[(tert-butoxycarbonyl)(methyl)amino]-2-[(4-methoxyphenyl)methoxy]-1,6-naphthyridine-3-carboxylate

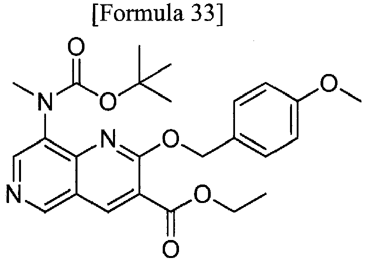

To a solution in 1,4-dioxane (10 mL) of the ethyl 8-iodo-2-[(4-methoxyphenyl)methoxy]-1,6-naphthyridine-3-carboxylate (303 mg) obtained in Production Example 4-(2), tert-butyl methyl carbamate (257 mg), cesium carbonate (362 mg), xantphos (151 mg) and tris(dibenzylideneacetone)palladium(0) (120 mg) were added and the mixture was stirred for 3 hours at 90°C. After being left to cool to room temperature, the reaction mixture was diluted with ethyl acetate and filtered through Celite; the filtrate was concentrated under reduced pressure. The residue was purified by column chromatography (silica gel cartridge, hexane/ethyl acetate = 93:7 - 40:60), whereupon a crude product (169 mg) containing the titled compound was obtained as a brown liquid.
MS (ESI pos.) m/z : 468 [M+H]+, RT=1.167 min (Analytical Condition 3)

### 4-(4) 8-[(tert-Butoxycarbonyl)(methyl)amino]-2-[(4-methoxyphenyl)methoxy]-1,6-naphthyridine-3-carboxylic acid

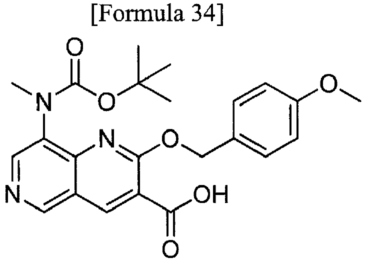

To a solution in tetrahydrofuran (1 mL) of the ethyl 8-[(tert-butoxycarbonyl)(methyl)amino]-2-[(4-methoxyphenyl)methoxy]-1,6-naphthyridine-3-carboxylate obtained as a crude product (169 mg) in Production Example 4-(3), an aqueous solution of 2 M sodium hydroxide was added and the mixture was stirred for 4 hours at room temperature. To the the reaction mixture, an aqueous solution of 1 M hydrochloric acid was added to establish pH 6 and the mixture was allowed to settle for 14 hours undisturbed. The precipitating solid was recovered by filtration to obtain the titled compound (71 mg) as a light brown solid.
MS (ESI pos.) m/z : 440 [M+H]+, RT=0.951 min (Analytical Condition 3)

### Production Example 5 7-Fluoro-5-(methylamino)-3-oxo-3,4-dihydroquinoxaline-2-carboxylic acid

### 5-(1) N1-Benzyl-5-fluoro-N1-methyl-2-nitrobenzene-1,3-diamine

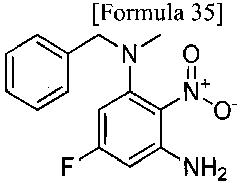

A solution of 3,5-difluoro-2-nitroaniline (1.0 g) and N-methylbenzylamine (810 µL) in ethanol (20 mL) was stirred for 18 hours at room temperature. After further addition of N-methylbenzylamine (220 µL), the mixture was stirred under heating for an additional 5 hours at 50°C. The reaction mixture was concentrated under reduced pressure and thereafter purified by column chromatography (NH silica gel cartridge, hexane/ethyl acetate = 90:10) to obtain the titled compound (880 mg) as a red solid.
MS (ESI pos.) m/z : 276 [M+H]+, RT=1.135 min (Analytical Condition 3)

### 5-(2) N1-Benzyl-5-fluoro-N1-methylbenzene-1,2,3-triamine

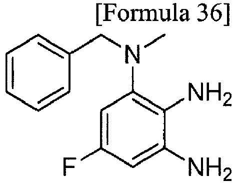

To a solution in ethanol (45 mL) and water (4.5 mL) of ammonium chloride (171 mg) and the N1-benzyl-5-fluoro-N1-methyl-2-nitrobenzene-1,3-diamine (880 mg) obtained in Production Example 5-(1), an iron powder (982 mg) was added and the mixture was stirred under heating for 24 hours at 85°C. The reaction mixture was filtered while hot to thereby remove the insoluble matter, and the filtrate was concentrated under reduced pressure. The residue was purified by column chromatography (NH silica gel cartridge, hexane/ethyl acetate = 90:10 - 75:25) to obtain the titled compound (512 mg) as a red oil. MS (ESI pos.) m/z : 246 [M+H]+, RT=0.693 min (Analytical Condition 3)

### 5-(3) Ethyl 5-[benzyl(methyl)amino]-7-fluoro-3-oxo-3,4-dihydroquinoxaline-2-carboxylate

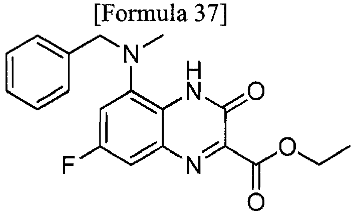

To a solution in ethanol (5 mL) of the N1-benzyl-5-fluoro-N1-methylbenzene-1,2,3-triamine (250 mg) obtained in Production Example 5-(2), diethyl ketomalonate (187 µL) was added and the mixture was stirred under heating for 2 hours at 80°C. The reaction mixture was concentrated under reduced pressure and thereafter purified by column chromatography (NH silica gel cartridge, chloroform/methanol = 100:0 - 95:5) to obtain the titled compound (74 mg) as an orange solid.
MS (ESI pos.) m/z : 356 [M+H]+, RT=1.059 min (Analytical Condition 3)

### 5-(4) Ethyl 7-fluoro-5-(methylamino)-3-oxo-1,2,3,4-tetrahydroquinoxaline-2-carboxylate

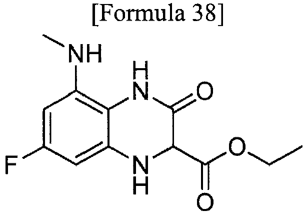

To a solution in ethanol (8 mL) of the ethyl 5-[benzyl(methyl)amino]-7-fluoro-3-oxo-3,4-dihydroquinoxaline-2-carboxylate (74 mg) obtained in Production Example 5-(3), 5% palladium on carbon (50 mg) was added and the mixture was stirred for an hour at room temperature under a hydrogen atmosphere. After removing the insoluble matter by filtration, the filtrate was concentrated under reduced pressure to obtain the titled compound (55 mg) as an orange solid.
MS (ESI pos.) m/z : 268 [M+H]+, RT=0.719 min (Analytical Condition 3)

### 5-(5) Ethyl 7-fluoro-5-(methylamino)-3-oxo-3,4-dihydroquinoxaline-2-carboxylate

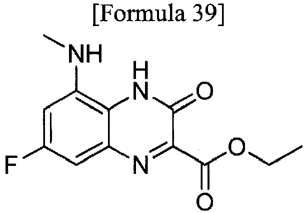

To a solution in chloroform (3 mL) of the ethyl 7-fluoro-5-(methylamino)-3-oxo-1,2,3,4-tetrahydroquinoxaline-2-carboxylate (34 mg) obtained in Production Example 5-(4), 2,3-dichloro-5,6-dicyano-p-benzoquinone (29 mg) was added and the mixture was stirred for an hour at room temperature. The reaction mixture was purified by column chromatography (silica gel cartridge, chloroform/methanol = 98:2) to obtain the titled compound (33 mg) as an orange solid.
MS (ESI pos.) m/z : 266 [M+H]+, RT=0.896 min (Analytical Condition 3)

### 5-(6) 7-Fluoro-5-(methylamino)-3-oxo-3,4-dihydroquinoxaline-2-carboxylic acid

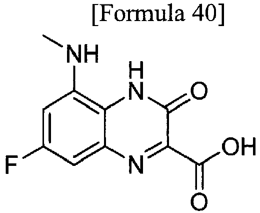

To a solution in tetrahydrofuran (2 mL) of the ethyl 7-fluoro-5-(methylamino)-3-oxo-3,4-dihydroquinoxaline-2-carboxylate (53 mg) obtained in Production Example 5-(5), 1 M sodium hydroxide in aqueous solution (2 mL) was added and the mixture was stirred for 4 hours at room temperature. After the end of the reaction, an aqueous solution of 1 M hydrochloric acid was added to establish pH 1. The precipitating solid was recovered by filtration and washed with water, whereafter the titled compound (47 mg) was obtained as a red solid.
¹H NMR (400 MHz, DMSO-d6) δ ppm 2.86 (s, 3 H), 6.33 (br s, 1 H), 6.58 (dd, J=11.7, 2.3 Hz, 1 H), 6.86 (dd, J=9.2, 2.3 Hz, 1 H)

### Production Example 6 Methyl 2-amino-3-[benzyl(methyl)amino]-5-fluorobenzoate

### 6-(1) Methyl 3-[benzyl(methyl)amino]-5-fluoro-2-nitrobenzoate

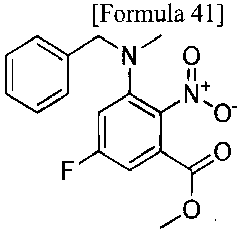

To a solution of methyl 3,5-difluoro-2-nitrobenzoate (21.7 g) in methanol (500 mL), TEA (14 mL) and N-methyl-1-phenyl-methanamine (18.2 mL) were added and the mixture was stirred for 24 hours at room temperature. After the end of the reaction, the solvent was removed by concentrating the reaction mixture under reduced pressure; to the concentrate, chloroform was added and the organic layer was washed with an aqueous solution of ammonium chloride and saturated brine, dried over anhydrous magnesium sulfate, and concentrated under reduced pressure to thereby obtain a crude product. The crude product thus obtained was purified by column chromatography (silica gel cartridge, n-hexane/ethyl acetate = 95:5 - 80:20), whereupon the titled compound (27.4 g) was obtained as a brown oil. MS (ESI pos.) m/z : 319 [M+H]+, RT=0.934 min (Analytical Condition 4)

### 6-(2) Methyl 2-amino-3-[benzyl(methyl)amino]-5-fluorobenzoate

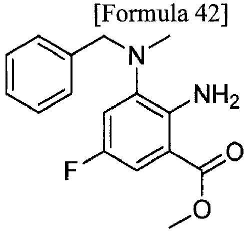

A solution in ethanol (500 mL) and water (50 mL) of an iron powder (164 g), ammonium chloride (15.7 g) and the methyl 3-[benzyl(methyl)amino]-5-fluoro-2-nitrobenzoate (23.3 g) obtained in Production Example 6-(1) was vigorously stirred at 80°C. After the end of the reaction, the reaction mixture was filtered through Celite and the filtrate thus obtained was concentrated under reduced pressure. The residue was dissolved in ethyl acetate and the organic layer was washed with water and saturated brine, dried over anhydrous magnesium sulfate and concentrated under reduced pressure to thereby obtain a crude product. The thus obtained crude product was purified by column chromatography (silica gel cartridge, n-hexane/ethyl acetate = 95:5 - 80:20), whereupon the titled compound (10.64 g) was obtained as a brown oil.
MS (ESI pos.) m/z : 289 [M+H]+, RT=0.997 min (Analytical Condition 4)
¹H NMR (400 MHz, CHLOROFORM-d) δ ppm 2.56 (s, 3 H), 3.87 (s, 3 H), 3.99 (s, 2 H), 6.13 (br s, 2 H), 6.89 (dd, J=9.5, 2.9 Hz, 1 H), 7.22 - 7.39 (m, 6 H)

### Production Example 7 3-[4-(tert-Butoxycarbonyl)anilino]-3-oxopropanoic acid

### 7-(1) tert-Butyl4-(3-ethoxy3-oxopropanamido)benzoate

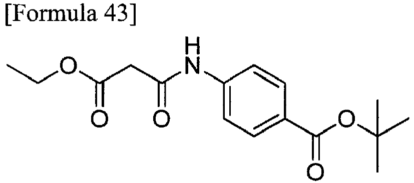

To a solution of tert-butyl 4-aminobenzoate (11.10 g) in chloroform (180 mL), ethyl 3-chloro-3-oxo-propanoate (7.57 mL) and triethylamine (24.0 mL) were added under ice cooling and the mixture was stirred for an hour. After adding chloroform and a saturated aqueous solution of sodium hydrogencarbonate, liquid-liquid separation was performed and the aqueous layer was extracted with chloroform (100 mL). The organic layers were combined, passed through a phase separator and concentrated under reduced pressure. The residue was purified by column chromatography (NH silica gel cartridge, n-hexane/ethyl acetate) to obtain the titled compound (17.33 g) as a colorless oil.
MS (ESI pos.) m/z : 308 [M+H]+, RT=1.043 min (Analytical Condition 3)

### 7-(2) 3-[4-(tert-Butoxycarbonyl)anilino]-3-oxopropanoic acid

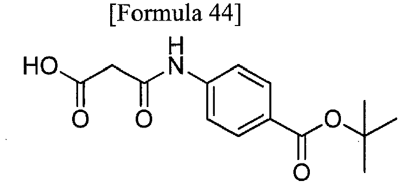

To a solution in tetrahydrofuran (110 mL) of the tert-butyl 4-(3-ethoxy-3-oxopropanamido)benzoate (17.33 g) obtained in Production Example 7-(1), an aqueous solution of 2 M sodium hydroxide (226 mL) was added and the mixture was stirred for 2 hours at room temperature. After ice cooling, an aqueous solution of 1 M hydrochloric acid was added for adjustment to pH 3 and extraction with ethyl acetate was conducted three times. The organic layers were combined, dried over magnesium sulfate and concentrated under reduced pressure to obtain the titled compound (14.21 g) as a colorless solid.
MS (ESI neg.) m/z : 278 [M-H]-, RT=0.882 min (Analytical Condition 3)
¹H NMR (400 MHz, DMSO-d6) δ ppm 1.53 (s, 9 H), 3.35 - 3.39 (m, 2 H), 7.68 (d, J=8.7 Hz, 2 H), 7.86 (d, J=8.7 Hz, 2 H), 10.52 (br s, 1 H), 12.64 (br s, 1 H)

### Production Example 8 3-[[4-(tert-Butoxycarbonyl)phenyl][(2,4-dimethoxyphenyl)methyl]amino]-3-oxopropanoic acid

### 8-(1) tert-Butyl 4-[[(2,4-dimethoxyphenyl)methyl]amino]benzoate

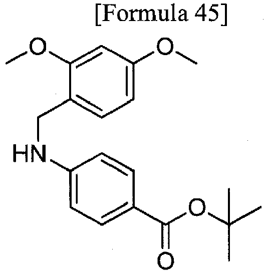

A solution of 2,4-dimethoxybenzaldehyde (5.0 g) and butyl 4-aminobenzoate (5.8 g) in toluene (60 mL) was heated under reflux for 3 hours at 160°C using a Dean-Stark apparatus. After concentrating the reaction mixture under reduced pressure, methanol (60 mL) and sodium borohydride (3.4 g) were added under ice cooling and the mixture was stirred for 19 hours at room temperature. The reaction mixture was diluted with a saturated aqueous solution of sodium hydrogencarbonate and extracted with ethyl acetate. The organic layer was washed with saturated brine, then dried over anhydrous magnesium sulfate and concentrated under reduced pressure. To the residue, ethyl acetate and hexane were added and the precipitating solid was recovered by filtration, whereupon the titled compound (3.43 g) was obtained as a white solid.
¹H NMR (400 MHz, CHLOROFORM-d) δ ppm 1.55 (s, 9 H), 3.79 (s, 3 H), 3.83 (s, 3 H), 4.29 (d, J=5.9 Hz, 2 H), 4.43 (br t, J=5.9 Hz, 1 H), 6.42 (dd, J=8.3, 2.3 Hz, 1 H), 6.47 (d, J=2.3 Hz, 1 H), 6.57 (d, J=8.8 Hz, 2 H), 7.15 (d, J=8.3 Hz, 1 H), 7.79 (d, J=8.8 Hz, 2 H)

### 8-(2) tert-Butyl 4-[[(2,4-dimethoxyphenyl)methyl](3-ethoxy-3-oxopropanoyl)amino]benzoate

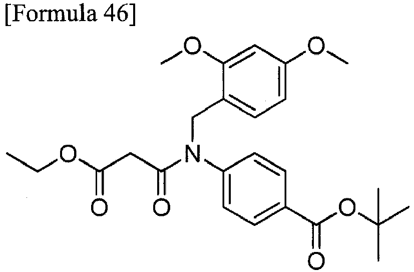

To a solution in chloroform (40 mL) of the tert-butyl 4-[[(2,4-dimethoxyphenyl)methyl]amino]benzoate (3.43 g) obtained in Production Example 8-(1), ethyl malonyl chloride (1.3 mL) and triethylamine (4.17 mL) were added under ice cooling. The mixture was stirred for half an hour under ice cooling; thereafter, a saturated aqueous solution of sodium hydrogencarbonate was added and the mixture was extracted with chloroform. The organic layer was passed through a phase separator and concentrated under reduced pressure. The residue was purified by column chromatography (silica gel cartridge, hexane/ethyl acetate = 80:20 - 70:30) to obtain the titled compound (4.50 g) as a colorless oil. MS (ESI pos.) m/z : 458 [M+H]+, RT=0.920 min (Analytical Condition 4)

### 8-(3) 3-[[4-(tert-Butoxycarbonyl)phenyl] [(2,4-dimethoxyphenyl)methyl]amino]-3-oxopropanoic acid

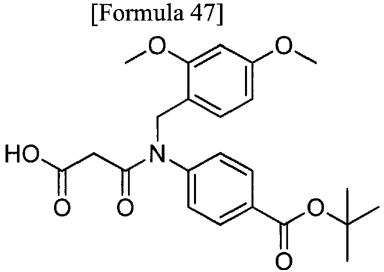

To a solution in tetrahydrofuran (40 mL) of the tert-butyl 4-[[(2,4-dimethoxyphenyl)methyl](3-ethoxy-3-oxopropanoyl)amino]benzoate (4.50 g) obtained in Production Example 8-(2), 2 M sodium hydroxide in aqueous solution (49 mL) was added and the mixture was stirred for 16 hours at room temperature. After the end of the reaction, an aqueous solution of 1 M hydrochloric acid was added to establish pH 1 and extraction was conducted with ethyl acetate. The organic layer was washed with saturated brine, dried over anhydrous magnesium sulfate and concentrated under reduced pressure to obtain the titled compound (4.08 g) as a colorless amorphous.
MS (ESI pos.) m/z : 430 [M+H]+, RT=0.809 min (Analytical Condition 4)
¹H NMR (400 MHz, CHLOROFORM-d) δ ppm 1.59 (s, 9 H), 3.04 (s, 2 H), 3.55 (s, 3 H), 3.78 (s, 3 H), 4.94 (s, 2 H), 6.33 (d, J=2.3 Hz, 1 H), 6.39 (dd, J=8.3, 2.3 Hz, 1 H), 6.99 (d, J=8.4 Hz, 2 H), 7.08 (d, J=8.3 Hz, 1 H), 7.96 (d, J=8.4 Hz, 2 H)

### Production Example 9 6-Fluoro-2-[(4-methoxyphenyl)methoxy]-8-(methylamino)quinoline-3-carbaldehyde

### 9-(1) Ethyl 6-fluoro-2-[(4-methoxyphenyl)methoxy]-8-(methylamino)quinoline-3-carboxylate

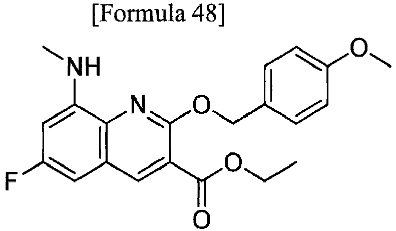

To a solution in tetrahydrofuran (25 mL) of the ethyl 6-fluoro-8-(methylamino)-2-oxo-1,2-dihydroquinoline-3-carboxylate (1.5 g) obtained in Production Example 2-(4), 4-methoxyphenol (0.92 mL) and cyanomethylene tributyl phospholan (4.5 mL) were added amd the mixture was stirred for 2 hours at 80°C. After leaving the reaction mixture to cool to room temperature, water was added to the same. The aqueous layer was extracted with ethyl acetate. The organic layer was dried over anhydrous magnesium sulfate and concentrated under reduced pressure. The residue was purified by column chromatography (silica gel cartridge, hexane/ethyl acetate = 97:3 - 80:20) to obtain the titled compound (1.9 g) as a yellow solid.
MS (ESI pos.) m/z : 385 [M+H]+, RT=1.352 min (Analytical Condition 3)

### 9-(2) [6-Fluoro-2-[(4-methoxyphenyl)methoxy]-8-(methylamino)quinolin-3-yl]methanol

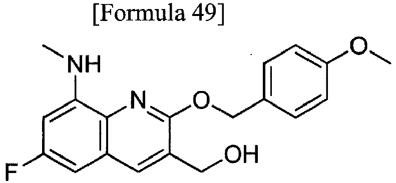

To a solution in tetrahydrofuran (18 mL) of the ethyl 6-fluoro-2-[(4-methoxyphenyl)methoxy]-8-(methylamino)quinoline-3-carboxylate (1.4 g) obtained in Production Example 9-(1), LAH (0.28 g) was added under ice cooling and the mixture was immediately subjected to 2-hr stirring. To the reaction mixture, a Rochelle salt was added, followed by 2.5-day stirring at room temperature. The aqueous layer was extracted with ethyl acetate. The organic layer was dried over anhydrous magnesium sulfate and concentrated under reduced pressure. The residue was purified by column chromatography (silica gel cartridge, hexane/ethyl acetate = 93:7 - 40:60) to obtain the titled compound (0.81 g) as a colorless liquid.
MS (ESI pos.) m/z : 343 [M+H]+, RT=1.161 min (Analytical Condition 3)

### 9-(3) 6-Fluoro-2-[(4-methoxyphenyl)methoxy]-8-(methylamino)quinoline-3-carbaldehyde

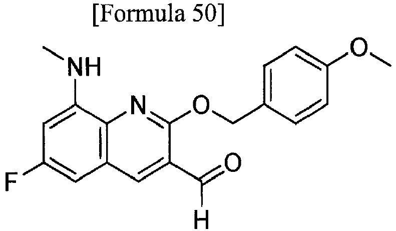

To a solution in chloroform (1.5 mL) of the [6-fluoro-2-[(4-methoxyphenyl)methoxy]-8-(methylamino)quinolin-3-yl]methanol (98 mg) obtained in Production Example 9-(2), a Dess-Martin reagent (134 mg) was added under ice cooling and the mixture was immediately subjected to 10-min stirring. To the reaction mixture, a saturated aqueous solution of ammonium chloride was added under ice cooling. The aqueous layer was extracted with chloroform and the organic layer was passed through a phase separator and concentrated under reduced pressure. The residue was purified by column chromatography (silica gel cartridge, hexane/ethyl acetate = 95:5 - 60:40) to obtain the titled compound (35 mg) as a colorless solid.
MS (ESI pos.) m/z : 341 [M+H]+, RT=1.304 min (Analytical Condition 3)
¹H NMR (400 MHz, CHLOROFORM-d) δ ppm 3.04 (d, J=5.3 Hz, 3 H), 3.83 (s, 3 H), 5.52 (s, 2 H), 5.84 (br s, 1 H), 6.47 (dd, J=11.1, 2.6 Hz, 1 H), 6.72 (dd, J=8.9, 2.6 Hz, 1 H), 6.94 (d, J=8.7 Hz, 2 H), 7.44 (d, J=8.7 Hz, 2 H), 8.47 (s, 1 H), 10.48 (s, 1 H)

### Production Example 10 3-[2-Iodo-4-(methoxycarbonyl)anilino]-3-oxopropanoic acid

### 10-(1) Methyl 4-(3-tert-butoxy-3-oxopropanamido)-3-iodobenzoate

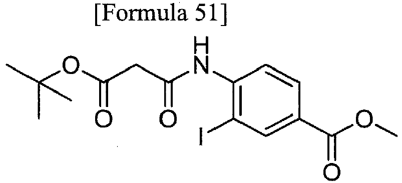

A solution in chloroform (100 mL) of methyl 4-amino-3-iodobenzoate (11.5 g), 3-tert-butoxy-3-oxopropanoic acid (10 g), 4-dimethylaminopyridine (10.1 g) and WSC·HCl (15.9 g) was stirred for 20 hours at room temperature. After the end of the reaction, it was quenched with an aqueous solution of sodium hydrogencarbonate; the resulting organic layer was washed with water and saturated brine, dried over anhydrous magnesium sulfate and concentrated under reduced pressure to obtain a crude product. The crude product thus obtained was used in the next reaction without being purified.
MS (ESI pos.) m/z : 442 [M+Na]+, RT=0.863 min (Analytical Condition 4)

### 10-(2) 3-[2-Iodo-4-(methoxycarbonyl)anilino]-3-oxopropanoic acid

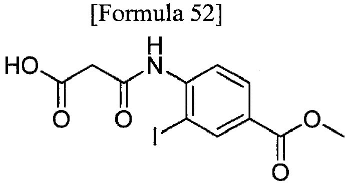

To a solution in chloroform (100 mL) of the methyl 4-(3-tert-butoxy-3-oxopropanamido)-3-iodobenzoate obtained in Production Example 10-(1), TFA (20 mL) was added at room temperature and the mixture was stirred for 3 hours. After the end of the reaction, the reaction mixture was concentrated under reduced pressure and to the residue thus obtained, ethyl acetate was added, followed by extraction with an aqueous solution of sodium hydrogencarbonate. The resulting aqueous layer was washed with ethyl acetate and an aqueous solution of 1 M hydrochloric acid was added until pH 2 or below was established; the precipitating solid was separated by filtration to obtain the titled compound (7.55 g) as a colorless solid.
MS (ESI pos.) m/z : 364 [M+H]+, RT=0.617 min (Analytical Condition 4)
¹H NMR (400 MHz, DMSO-d₆) δ ppm 3.51 (s, 2 H), 3.85 (s, 3 H), 7.86 (d, J=8.5 Hz, 1 H), 7.95 (dd, J=8.5, 2.0 Hz, 1 H), 8.38 (d, J=2.0 Hz, 1 H), 9.79 (s, 1 H), 12.8 (br s, 1 H)

### Production Example 11 3-(Chloromethyl)-6-fluoro-8-(methjylamino)quinolin-2(1H)-one

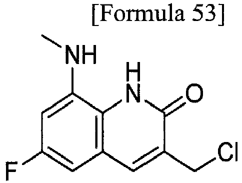

To a solution of 6-fluoro-3-(hydroxymethyl)-8-(methylamino)quinolin-2(1H)-one (49 mg) in chloroform (1 mL), thionyl chloride (24 µL) was added and the mixture was stirred for 2 hours at room temperature. The reaction mixture was concentrated under reduced pressure to obtain the titled compound (50 mg) as a yellow solid.
MS (ESI pos.) m/z : 241 [M+H]+, RT=0.873 min (Analytical Condition 3)

### Production Example 12 tert-Butyl (3-[2-amino-3-[benzyl(methyl)amino]-5-fluorophenyl]-3-[[tert-butyl(dimehyl)silyl]oxy]propyl)methylcarbamate

### 12-(1) 1-[3-[Benzyl(methyl)amino]-5-fluoro-2-nitrophenyl]but-3-en-1-ol

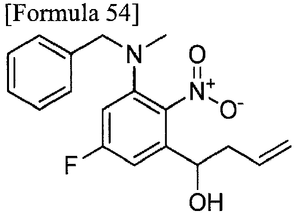

To a solution in THF (100 mL) of the methyl 3-[benzyl(methyl)amino]-5-fluoro-2-nitrobenzoate (5.85 g) obtained in Production Example 6-(1), 1.01 M DIBAL-H in toluene solution (36.8 mL) was added under ice cooling and the mixture was stirred for 5 hours. After the end of the reaction, quenching was done by an aqueous solution of 1 M hydrochloric acid and extraction was conducted with ethyl acetate. The organic layer was washed with saturated brine and thereafter dried over anhydrous magnesium sulfate and concentrated under reduced pressure to thereby obtain a crude product in alcoholic form. The crude product thus obtained was used in the next reaction without being purified. To a solution of the crude product in chloroform (100 mL), manganese dioxide (16 g) was added and the mixture was stirred for 25 hours at 50°C. Afer the end of the reaction, the reaction mixture was filtered through Celite and the filtrate was concentrated under reduced pressure to thereby obtain a crude product in aldehyde form. The crude product thus obtained was used in the next reaction without being purified.

To a solution of the crude product in THF (100 mL), a boron trifluoride diethyl ether complex (2.3 mL) and allylboronic acid pinacol ester (14 mL) were added at -78°C and the mixture was stirred for an hour at that temperature, followed by 17-hr stirring at room temperature. After the end of the reaction, an aqueous solution of sodium hydrogencarbonate was added to the reaction mixture for quenching the reaction. The organic layer was washed with water and saturated brine, dried over anhydrous magnesium sulfate, and concentrated under reduced pressure to thereby obtain a crude product. The crude product thus obtained was purified by column chromatography (silica gel cartridge, n-hexane/ethyl acetate = 90:10 - 80:20) to obtain the titled compound (5.35 g) as a yellow oil.
MS (ESI pos.) m/z : 331 [M+H]+, RT=0.940 min (Analytical Condition 4)

### 12-(2) N-Benzyl-3-(1-[[tert-butyl(dimethyl)silyl]oxy]but-3-en-1-yl)-5-fluoro-N-methyl-2-nitroaniline

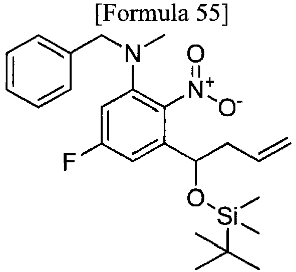

To a solution in chloroform (100 mL) of TEA (7.5 mL), 4-dimethylaminopyridine (217 mg), and the 1-[3-[benzyl(methyl)amino]-5-fluoro-2-nitrophenyl]but-3-en-1-ol (5.88 g) obtained in Production Example 12-(1), tert-butyldimethylsilyl trifluoromethanesulfonate (14.7 mL) was added under ice cooling, and the mixture was stirred for 5 hours at that temperature. After the end of the reaction, an aqueous solution of sodium hydrogencarbonate was added to quench the reaction and thereafter ethyl acetate was added. The organic layer was washed with saturated brine, dried over anhydrous magnesium sulfate and concentrated under reduced pressure to thereby obtain a crude product. The crude product thus obtained was purified by column chromatography (silica gel cartridge, n-hexane/ethyl acetate = 90:10 - 80:20) to obtain the titled compound (7.91 g) as a yellow oil.
¹H NMR (400 MHz, CHLOROFORM-d) δ ppm -0.12 (s, 3 H), 0.03 (s, 3 H), 0.87 (s, 9 H), 2.28 - 2.48 (m, 2 H), 2.66 (s, 3 H), 4.15 (s, 2 H), 4.67 (dd, J=7.3, 3.6 Hz, 1 H), 4.97 - 5.11 (m, 2 H), 5.70 - 5.87 (m, 1 H), 6.72 (dd, J=9.0, 2.6 Hz, 1 H), 6.96 (dd, J=9.0, 2.6 Hz, 1 H), 7.21 - 7.38 (m, 5 H)

### 12-(3) 3-[3-[Benzyl(methyl)amino]-5-fluoro-2-nitrophenyl]-3-[[tert-butyl(dimethyl)silyl]oxy]propanal

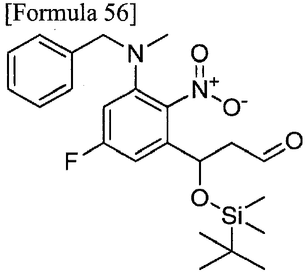

To a solution in 1,4-dioxane (18 mL) and water (4.5 mL) of sodium periodate (1.49 g) and the N-benzyl-3-(1-[[tert-butyl(dimethyl)silyl]oxy]but-3-en-1-yl)-5-fluoro-N-methyl-2-nitroaniline (775 mg) obtained in Production Example 12-(2), 4% osmium oxide in aqueous solution (0.2 mL) was added at room temperature and the mixture was stirred for 3 hours. After the end of the reaction, a saturated aqueous solution of sodium hydrogencarbonate was added to quench the reaction, followed by addition of ethyl acetate. The resulting organic layer was washed with water and saturated brine, dried over anhydrous magnesium sulfate and concentrated under reduced pressure to thereby obtain a crude product. The crude product thus obtained was used in the next reaction without being purified.

### 12-(4) tert-Butyl (3-[3-[benzyl(methyl)amino]-5-fluoro-2-nitrophenyl]-3-[[tert-butyl(dimethyl)silyl]oxy]propyl)methylcarbamate

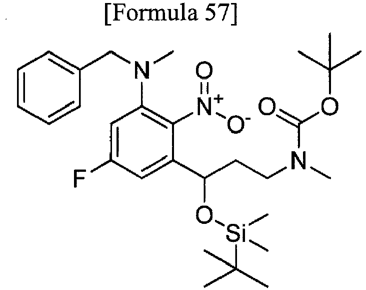

To a solution in chloroform (20 mL) of the 3-[3-[benzyl(methyl)amino]-5-fluoro-2-nitrophenyl]-3-[[tert-butyl(dimethyl)silyl]oxy]propanal obtained in Production Example 12-(3) (as the crude product of Production Example 12-(3)), 40% methylamine in methanol solution (0.54 mL) and acetic acid (0.05 mL) were added and the mixture was stirred for an hour at room temperature. To the reaction mixture, sodium triacetoxyborohydride (1.48 g) was added at room temperature and the mixture was stirred for an additional 3 hours. To the reaction mixture, sodium hydroxide (0.28 g), 1,4-dioxane (10 mL) and water (10 mL) were added, followed by addition of di-tert-butyl dicarbonate (1.89 g) under vigorous stirring, and the mixture was stirred for 2 days at room temperature. After the end of the reaction, saturated sodium hydrogencarbonate was added to the reaction mixture for quenching the reaction, followed by addition of chloroform. The resulting organic layer was washed with saturated brine, dried over anhydrous magnesium sulfate and concentrated under reduced pressure to thereby obtain a crude product. The crude product obtained was purified by column chromatography (silica gel cartridge, n-hexane/ethyl acetate = 80:20 - 60:40) to obtain the titled compound (250 mg) as a yellow oil.
¹H NMR (400 MHz, CHLOROFORM-d) δ ppm -0.15 (s, 3 H), 0.04 (s, 3 H), 0.89 (s, 9 H), 1.44 (s, 9 H), 1.70 - 2.00 (m, 2 H), 2.67 (s, 3 H), 2.82 (s, 3 H), 3.06 - 3.51 (m, 2 H), 4.16 (s, 2 H), 4.61 (br dd, J=8.3, 2.6 Hz, 1 H), 6.65 - 6.79 (m, 1 H), 6.95 (dd, J=8.3, 2.6 Hz, 1 H), 7.18 - 7.38 (m, 5 H)

### 12-(5) tert-Butyl (3-[2-amino-3-[benzyl(methyl)amino]-5-fluorophenyl]-3-[[tert-butyl(dimethyl)silyl]oxy]propyl)methylcarbamate

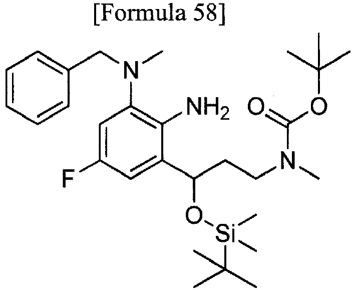

A solution in ethanol (5 mL) of zinc (496 mg), calcium chloride (26 mg), and the tert-butyl (3-[3-[benzyl(methyl)amino]-5-fluoro-2-nitrophenyl]-3-[[tert-butyl(dimethyl)silyl]oxy]propyl)methylcarbamate (133 mg) obtained in Production Example 12-(4) was stirred for an hour at 80°C. After the end of the reaction, the reaction mixture was filtered through Celite and the filtrate was concentrated under reduced pressure to thereby obtain a crude product. The crude product thus obtained was purified by column chromatography (silica gel cartridge, n-hexane/ethyl acetate = 80:20 - 50:50) to obtain the titled compound (80 mg) as a pale yellow oil.
¹H NMR (400 MHz, CHLOROFORM-d) δ ppm -0.15 (s, 3 H), 0.05 (s, 3 H), 0.88 (s, 9 H), 1.43 (s, 9 H), 1.92 - 2.06 (m, 1 H), 2.13 - 2.27 (m, 1 H), 2.55 (s, 3 H), 2.81 (s, 3 H), 2.97 - 3.43 (m, 2 H), 3.87 - 4.05 (m, 2 H), 4.51 - 4.64 (m, 3 H), 6.52 (br d, J=7.9 Hz, 1 H), 6.68 (dd, J=10.1, 2.3 Hz, 1 H), 7.20 - 7.35 (m, 5 H)

### Production Example 13 tert-Butyl (4-[2-amino-3-[benzyl(methyl)amino]-5-fluorophenyl]-4-[[tert-butyl(dimethyl)silyl]oxy]butyl)methylcarbamate

### 13-(1) 4-[3-[Benzyl(methyl)amino]-5-fluoro-2-nitrophenyl]-4-[[tert-butyl(dimethyl)silyl]oxy]butan-1-ol

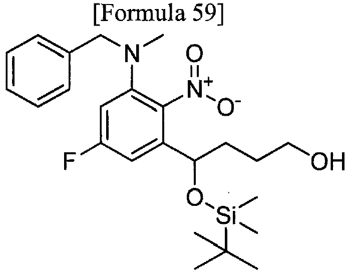

To a solution in THF (300 mL) of the N-benzyl-3-(1-[[tert-butyl(dimethyl)silyl]oxy]but-3-en-1-yl)-5-fluoro-N-methyl-2-nitroaniline (14.1 g) obtained in Production Example 12-(2), a 1.0 M borane-THF complex in THF solution (38 mL) was added at -78°C and the mixture was stirred for 6 hours as it was warmed to the temperature of ice cooling. To the reaction mixture, 3.0 M sodium hydroxide in aqueous solution (16 mL) and 30% hydrogen peroxide in aqueous solution (5.4 mL) were added and the mixture was stirred for 3 hours under ice cooling. After the end of the reaction, diethyl ether and an aqueous solution of ammonium chloride were added to the reaction mixture for quenching the reaction and, thereafter, diethyl ether was further added. The resulting organic layer was washed with water and saturated brine, dried over anhydrous magnesium sulfate and concentrated under reduced pressure to thereby obtain a crude product. The crude product thus obtained was purified by column chromatography (silica gel cartridge, n-hexane/ethyl acetate = 80:20 - 50:50) to obtain the titled compound (10.1 g) as a pale yellow oil.
¹H NMR (400 MHz, CHLOROFORM-d) δ ppm -0.13 (s, 3 H), 0.04 (s, 3 H), 0.88 (s, 9 H), 1.50 - 1.84 (m, 4 H), 2.66 (s, 3 H), 3.58 - 3.68 (m, 2 H), 4.15 (s, 2 H), 4.61 - 4.70 (m, 1 H), 6.72 (dd, J=9.0, 2.7 Hz, 1 H), 6.96 (dd, J=9.0, 2.7 Hz, 1 H), 7.17 - 7.37 (m, 5 H)

### 13-(2) 4-[3-[Benzyl(methyl)amino]-5-fluoro-2-nitrophenyl]-4-[[tert-butyl(dimethyl)silyl]oxy]butanal

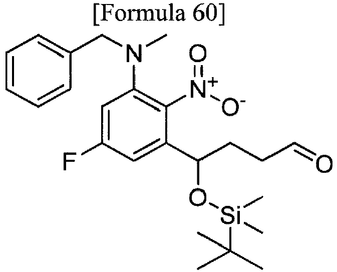

To a solution in DMSO (5 mL) of the 4-[3-[benzyl(methyl)amino]-5-fluoro-2-nitrophenyl]-4-[[tert-butyl(dimethyl)silyl]oxy]butan-1-ol (461 mg) obtained in Production Example 13-(1), 40% IBX (837 mg) was added and the mixture was stirred for one day and night at room temperature. After the end of the reaction, an aqueous solution of sodium hydrogencarbonate was added to the reaction mixture for quenching the reaction, followed by addition of ethyl acetate. The resulting organic layer was washed with a saturated solution of sodium hydrogencarbonate, water and saturated brine, dried over anhydrous magnesium sulfate and concentrated under reduced pressure to thereby obtain a crude product. The crude product thus obtained was used in the next reaction without being purified.
MS (ESI pos.) m/z : 461 [M+H]+, RT=1.154 min (Analytical Condition 4)

### 13-(3) tert-Butyl (4-[3-[benzyl(methyl)amino]-5-fluoro-2-nitrophenyl]-4-[[tert-butyl(dimethyl)silyl]oxy]butyl)methylcarbamate

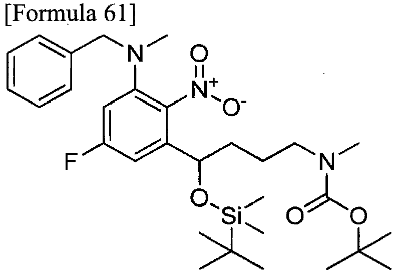

To a solution in chloroform (10 mL) of the 4-[3-[benzyl(methyl)amino]-5-fluoro-2-nitrophenyl]-4-[[tert-butyl(dimethyl)silyl]oxy]butanal obtained in Production Example 13-(2) (as the crude product of Production Example 13-(2)), 40% methylamine in methanol solution (0.31 mL) and acetic acid (0.03 mL) were added and the mixture was stirred for an hour at room temperature. To the reaction mixture, sodium triacetoxyborohydride (848 mg) was added at room temperature and the mixture was stirred for 3 hours. To the reaction mixture, sodium hydroxide (160 mg), 1,4-dioxane (5 mL) and water (5 mL) were added and vigorous stirring followed; di-tert-butyl bicarbonate (1.09 g) was then added and the mixture was stirred for 3 hours at room temperature. After the end of the reaction, saturated sodium hydrogencarbonate was added to the reaction mixture for quenching the reaction and, thereafter, chloroform was added. The resulting organic layer was washed with saturated brine, dried over anhydrous magnesium sulfate and concentrated under reduced pressure to thereby obtain a crude product. The crude product thus obtained was purified by column chromatography (silica gel cartridge, n-hexane/ethyl acetate = 80:20 - 50:50) to obtain the titled compound (160 mg) as a yellow oil.
¹H NMR (400 MHz, CHLOROFORM-d) δ ppm -0.14 (s, 3 H), 0.03 (s, 3 H), 0.88 (s, 9 H), 1.43 (s, 9 H), 1.56 - 1.76 (m, 4 H), 2.66 (s, 3 H), 2.82 (br s, 3 H), 3.18 (br s, 2 H), 4.15 (s, 2 H), 4.55 - 4.65 (m, 1 H), 6.72 (dd, J=9.3, 2.6 Hz, 1 H), 6.94 (dd, J=9.3, 2.6 Hz, 1 H), 7.18 - 7.40 (m, 5 H)

### 13-(4) tert-Butyl (4-[2-amino-3-[benzyl(methyl)amino]-5-fluorophenyl]-4-[[tert-butyl(dimethyl)silyl]oxy]butyl)methylcarbamate

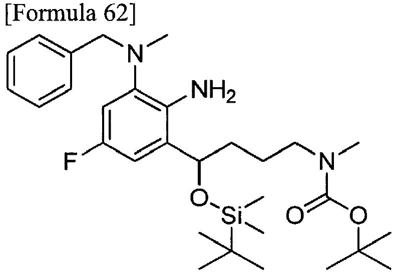

A solution in ethanol (3 mL) of zinc (580 mg), calcium chloride (31 mg), and the tert-butyl (4-[3-[benzyl(methyl)amino]-5-fluoro-2-nitrophenyl]-4-[[tert-butyl(dimethyl)silyl]oxy]butyl)methylcarbamate (160 mg) obtained in Production Example 13-(3) was stirred for an hour at 80°C. After the end of the reaction, the reaction mixture was filtered through Celite and the filtrate was concentrated under reduced pressure to thereby obtain a crude product. The crude product thus obtained was purified by column chromatography (silica gel cartridge, n-hexane/ethyl acetate = 60:40 - 40:60) to obtain the titled compound (87 mg) as a yellow oil.
¹H NMR (400 MHz, CHLOROFORM-d) δ ppm -0.13 (s, 3 H), 0.06 (s, 3 H), 0.88 (s, 9 H), 1.43 (s, 9 H), 1.59 - 1.79 (m, 2 H), 1.90 (br s, 2 H), 2.55 (s, 3 H), 2.80 (br s, 3 H), 3.03 - 3.26 (m, 1 H), 3.39 (br s, 1 H), 3.87 - 4.07 (m, 2 H), 4.56 (s, 2 H), 4.63 (br s, 1 H), 6.53 (br s, 1 H), 6.67 (dd, J=10.0, 2.5 Hz, 1 H), 7.19 - 7.39 (m, 5 H)

### Production Example 14 3-[4-(Methoxycarbonyl)-2-[(morpholin-4-yl)methyl]anilino]-3-oxopropanoic acid

### 14-(1) Methyl 4-[(tert-butoxycarbonyl)amino]-3-ethenylbenzoate

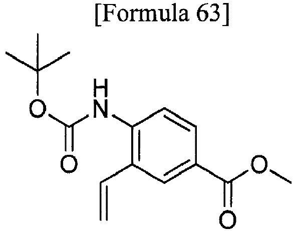

A solution in THF (20 mL) of methyl 4-amino-3-ethenylbenzoate (3.66 g) and di-tert-butyl bicarbonate (22.5 g) was stirred for 28 hours at 90°C. After the end of the reaction, the reaction mixture was concentrated under reduced pressure to thereby obtain a crude product. The crude product thus obtained was purified by column chromatography (silica gel cartridge, n-hexane/ethyl acetate = 95:5 - 80:20) to obtain the titled compound (5.02 g) as a brown oil.
MS (ESI pos.) m/z : 300 [M+Na]+, RT=0.900 min (Analytical Condition 4)

### 14-(2) Methyl 4-[(tert-butoxycarbonyl)amino]-3-formylbenzoate

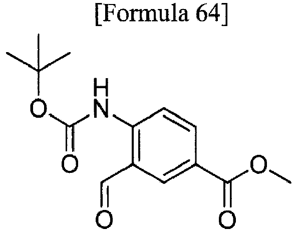

To a solution in 1.4-dioxane (80 mL) and water (20 mL) of sodium periodate (7.74 g), 2,6-dimethylpyridine (4.2 mL), and the methyl 4-[(tert-butoxycarbonyl)amino]-3-ethenylbenzoate (5.02 g) obtained in Production Example 14-(1), 4% osmium oxide in aqueous solution (1.17 mL) was added at room temperature and the mixture was stirred for 2 days. After the end of the reaction, chloroform and water were added to the reaction mixture and the resulting organic layer was washed with saturated brine, dried over anhydrous magnesium sulfate and concentrated under reduced pressure to thereby obtain a crude product. The crude product thus obtained was purified by column chromatography (silica gel cartridge, n-hexane/ethyl acetate = 95:5 - 80:20), whereby the titled compound (3.68 g) was obtained as a pale yellow solid.
¹H NMR (400 MHz, CHLOROFORM-d) δ ppm 1.55 (s, 9 H), 3.94 (s, 3 H), 8.20 (dd, J=8.9, 2.1 Hz, 1 H), 8.35 (d, J=2.1 Hz, 1 H), 8.54 (d, J=8.9 Hz, 1 H), 9.89 - 10.01 (m, 1 H), 10.59 (br s, 1 H)

### 14-(3) Methyl 4-[(tert-butoxycarbonyl)amino]-3-[(morpholin-4-yl)methyl]benzoate

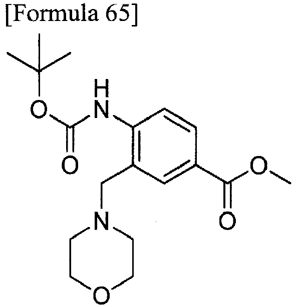

To a solution in chloroform (100 mL) of acetic acid (0.13 mL), morpholine (1.16 mL), and the methyl 4-[(tert-butoxycarbonyl)amino]-3-formylbenzoate (1.25 g) obtained in Production Example 14-(2), sodium triacetoxyborohydride (4.74 g) was added and the mixture was stirred for 17 hours at room temperature. After the end of the reaction, an aqueous solution of sodium hydrogencarbonate was added to quench the reaction and extraction was conducted with chloroform. The resulting organic layer was washed with water and saturated brine, dried over anhydrous magnesium sulfate and concentrated under reduced pressure to thereby obtain a crude product. The crude product thus obtained was purified by column chromatography (silica gel cartridge, n-hexane/ethyl acetate = 95:5 - 60:40) to obtain the titled compound (1.29 g) as a colorless solid.
MS (ESI pos.) m/z : 351 [M+H]+, RT=0.622 min (Analytical Condition 4)

### 14-(4) Methyl 4-amino-3-[(morpholin-4-yl)methyl]benzoate

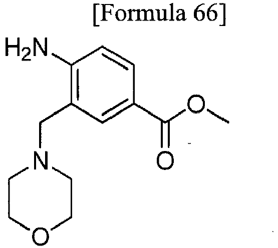

To a solution in chloroform (10 mL) of the methyl 4-[(tert-butoxycarbonyl)amino]-3-[(morpholin-4-yl)methyl]benzoate (1.29 g) obtained in Production Example 14-(3), TFA (5 mL) was added and the mixture was stirred for 7 hours at room temperature. After the end of the reaction, the solvent was removed by concentrating the reaction mixture under reduced pressure and the concentrate was dissolved in chloroform. The resulting organic layer was washed with an aqueous solution of sodium hydrogencarbonate, water and saturated brine, dried over anhydrous magnesium sulfate and concentrated under reduced pressure to thereby obtain a crude product. The crude product thus obtained was used in the next reaction without being purified.
MS (ESI pos.) m/z : 251 [M+H]+, RT=0.210 min (Analytical Condition 4)

### 14-(5) Methyl 4-(3-tert-butoxy-3-oxopropanamido)-3-[(morpholin-4-yl)methyl]benzoate

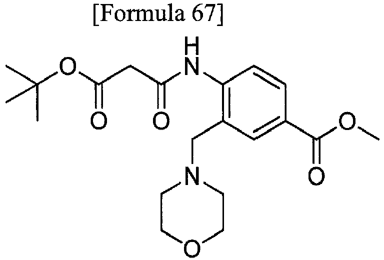

To a solution of 3-tert-butoxy-3-oxopropanoic acid (1.77 g) in chloroform (55 mL), DMF (0.03 mL) and oxalyl chloride (1.1 mL) were added and the mixture was stirred for 3 hours at room temperature. After the end of the reaction, the solvent was removed by concentrating the reaction mixture under reduced pressure, whereupon acid chloride was obtained. The acid chloride thus obtained was used in the next reaction without being further purified.
To a solution in chloroform (50 mL) of the methyl 4-amino-3-[(morpholin-4-yl)methyl]benzoate obtained in Production Example 14-(4) (as crude product), TEA (2.05 mL) was added; a chloroform solution of the separately prepared acid chloride was added under ice cooling and the mixture was stirred for 17 hours at room temperature. After the end of the reaction, an aqueous solution of sodium hydrogencarbonate was added to quench the solution; thereafter, the resulting organic layer was washed with saturated brine, dried over anhydrous magnesium sulfate and concentrated under reduced pressure to thereby obtain a crude product. The crude product thus obtained was purified by column chromatography (silica gel cartridge, n-hexane/ethyl acetate = 80:20 - 50:50) to obtain the titled compound (1.4 g) as a colorless solid.
MS (ESI pos.) m/z : 393 [M+H]+, RT=0.374 min (Analytical Condition 4)

### 14-(6) 3-[4-(Methoxycarbonyl)-2-[(morpholin-4-yl)methyl]anilino]-3-oxopropanoic acid

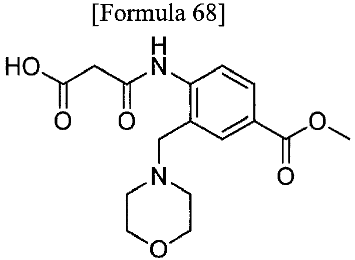

To a solution in 1,4-dioxane (10 mL) of the methyl 4-(3-tert-butoxy-3-oxopropanamido)-3-[(morpholin-4-yl)methyl]benzoate (1.4 g) obtained in Production Example 14-(5), 4 M hydrochloric acid in dioxane solution (20 mL) was added and the mixture was stirred for 3 days at room temperature. After the end of the reaction, the solvent was removed by concentrating the reaction mixture under reduced pressure, whereupon a crude product was obtained. The crude product thus obtained was used in the next reaction without being purified.
MS (ESI pos.) m/z : 337 [M+H]+, RT=0.575 min (Analytical Condition 2)

### Production Example 15 8-(Hydroxymethyl)quinolin-2(1H)-one

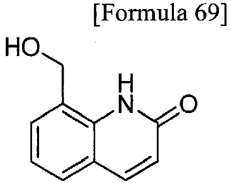

To a solution of 2-oxo-1,2-dihydroquinoline-8-carbaldehyde (35 mg) in methanol (3.5 mL), sodium borohydride (30 mg) was added and the mixture was stirred for 2 hours under ice cooling. After the end of the reaction, an aqueous solution of sodium hydrogencarbonate was added to quench the reaction and extraction was conducted with chloroform. The organic layer was passed through a phase separator and thereafter concentrated under reduced pressure to obtain the titled compound (31 mg) as a colorless solid.
MS (ESI pos.) m/z : 176 [M+H]+, RT=0.416 min (Analytical Condition 3)
¹H NMR (600 MHz, CHLOROFORM-d) δ ppm 4.21 (br t, J=5.4 Hz, 1 H), 5.01 (d, J=5.4 Hz, 2 H), 6.70 (d, J=9.5 Hz, 1 H), 7.19 (t, J=7.6 Hz, 1 H), 7.43 (d, J=7.6 Hz, 1 H), 7.54 (d, J=7.6 Hz, 1 H), 7.83 (d, J=9.5 Hz, 1 H), 11.33 (br s, 1 H)

### Production Example 16 8-(1-Hydroxyethyl)quinolin-2(1H)-one

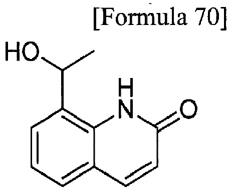

To a solution of 8-ethenylquinolin-2(1H)-one (10 mg) in tetrahydrofuran (1.5 mL), a borane-dimethyl sulfide complex (10 µL) was added and the mixtsure was stirred for 1.5 hours at room temperature. Under ice cooling, 2 M sodium hydroxide in aqueous solution (0.7 mL) and 30% hydrogen peroxide in aqueous solution (0.7 mL) were slowly added and the mixture was stirred for 2.5 hours at room temperature. After quenching the reaction by a saturated aqueous solution of ammonium chloride, the reaction mixture was extracted with ethyl acetate. The organic layer was washed with saturated brine and thereafter dried over anhydrous magnesium sulfate and concentrated under reduced pressure. The residue was purified by column chromatography (silica gel cartridge, chloroform/methanol = 100:0 - 92:8) to obtain the titled compound (6.4 mg) as a colorless oil.
MS (ESI pos.) m/z : 190 [M+H]+, RT=0.528 min (Analytical Condition 3)
¹H NMR (600 MHz, CHLOROFORM-d) δ ppm 1.67 (d, J=6.6 Hz, 3 H), 4.80 - 4.90 (m, 1 H), 5.28 - 5.35 (m, 1 H), 6.63 (d, J=9.5 Hz, 1 H), 7.16 (t, J=7.6 Hz, 1 H), 7.32 (d, J=7.6 Hz, 1 H), 7.46 (d, J=7.6 Hz, 1 H), 7.77 (d, J=9.5 Hz, 1 H), 11.17 (br s, 1 H)

### Production Example 17 8-(Ethylamino)quinolin-2(1H)-one

### Production Example 17-(1) Ethyl (2E)-3-[3-(ethylamino)-2-nitrophenyl]prop-2-enoate

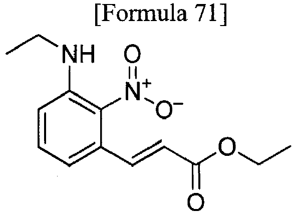

To 3-chloro-N-ethyl-2-nitroaniline (426 mg), ethyl acrylate (2.3 mL), bis(tri-tert-butylphosphine)palladium(0) (216 mg) and DIPEA (12 mL) were added and the mixture was stirred for 15 minutes at 160°C under microwave irradiation. After being left to cool, the reaction mixture was concentrated under reduced pressure. The residue was purified by column chromatography (NH silica gel cartridge, hexane/ethyl acetate = 98:2) to obtain the titled compound (341 mg) as an orange oil.
MS (ESI pos.) m/z : 265 [M+H]+, RT=1.139 min (Analytical Condition 3)

### Production Example 17-(2) Ethyl (2E)-3-[2-amino-3-(ethylamino)phenyl]prop-2-enoate

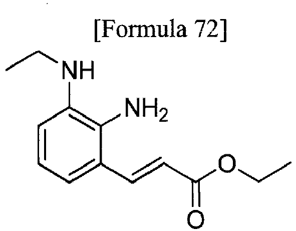

To a solution in ethanol (8.5 mL) and water (1.0 mL) of the ethyl (2E)-3-[3-(ethylamino)-2-nitrophenyl]prop-2-enoate (341 mg) obtained in Production Example 17-(1), ammonium chloride (69 mg) and an iron powder (398 mg) were added and the mixture was stirred for 5 hours at 75°C. After being left to cool, the reaction mixture was filtered through Celite and a saturated aqueous solution of sodium hydrogencarbonate was added to the filtrate. The organic layer was washed with saturated brine and thereafter dried over anhydrous magnesium sulfate and concentrated under reduced pressure. The residue was purified by column chromatograph (NH silica gel cartridge, hexane/ethyl acetate = 94:6 - 85:15) to obtain the titled compound (214 mg) as a yellow solid.
MS (ESI pos.) m/z : 235 [M+H]+, RT=0.712 min (Analytical Condition 3)

### Production Example 17-(3) 8-(Ethylamino)quinolin-2(1H)-one

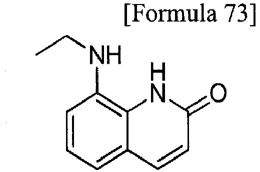

To a solution in methanol (11 mL) of the ethyl (2E)-3-[2-amino-3-(ethylamino)phenyl]prop-2-enoate (214 mg) obtained in Production Example 17-(2), 28% sodium methoxide in methanol solution (529 mg) was added and the mixture was stirred for 7.5 hours at 85°C. After being left to cool, the reaction mixture was concentrated under reduced pressure, and chloroform and water were added to the residue. The organic layer was passed through a phase separator and thereafter concentrated under reduced pressure. The residue was purified by column chromatography (NH silica gel cartridge, hexane/ethyl acetate = 80:20 - 60:40) to obtain the titled compound (17 mg) as a colorless solid.
MS (ESI pos.) m/z : 189 [M+H]+, RT=0.716 min (Analytical Condition 3)
¹H NMR (600 MHz, DMSO-d6) δ ppm 1.27 (t, J=7.2 Hz, 3 H), 3.14 (qd, J=7.2, 4.5 Hz, 2 H), 5.72 (t, J=4.5 Hz, 1 H), 6.46 (d, J=9.5 Hz, 1 H), 6.67 (d, J=7.8 Hz, 1 H), 6.91 (dd, J=7.8, 0.8 Hz, 1 H), 7.02 (t, J=7.8 Hz, 1 H), 7.82 (d, J=9.5 Hz, 1 H), 10.98 (br s, 1 H)

In the next place, the methods of producing compounds similar to those of the present invention are described in detail as Reference Examples.

### Reference Example 1 4-[(2-Oxo-1,2-dihydroquinoline-3-carbonyl)amino]benzoic acid

### 1-(1) Methyl 4-[(2-oxo-1,2-dihydroquinoline-3-carbonyl)amino]benzoate

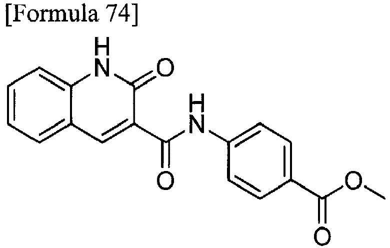

A suspension of 2-oxo-1,2-dihydroquinoline-3-carboxylic acid (111 mg) in thionyl chloride (8 mL) was stirred under reflux for 2 hours. After being left to cool to room temperature, the reaction mixture was concentrated under reduced pressure. To a solution of the residue in THF (4 mL), methyl 4-aminobenzoate (106 mg) was added under ice cooling and immediately subjected to 1-hr stirring. The reaction mixture was filtered and the solid obtained was washed with methanol to obtain the titled compound (101 mg) as a yellow solid.
MS (ESI pos.) m/z : 323[M+H]+, RT=0.969 min (Analytical Condition 3)

### 1-(2) 4-[(2-Oxo-1,2-dihydroquinoline-3-carbonyl)amino]benzoic acid

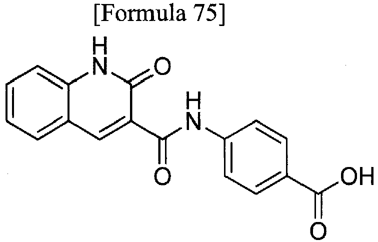

To a solution in tetrahydrofuran (3 mL) of the methyl 4-[(2-oxo-1,2-dihydroquinoline-3-carbonyl)amino]benzoate (101 mg) obtained in Reference Example 1-(1), 1 M sodium hydroxide in aqueous solution (3 mL) was added and the mixture was stirred for 5 hours at room temperature. To the reaction mixture, an aqueous solution of 1 M hydrochloric acid was added and the precipitating solid was recovered by filtration and thereafter washed with water to obtain the titled compound (36 mg) as a colorless solid.
MS (ESI pos.) m/z : 309[M+H]+, RT=0.802 min (Analytical Condition 3)
¹H NMR (600 MHz, DMSO-d6) δ ppm 7.32 - 7.42 (m, 1 H), 7.49 (d, J=8.3 Hz, 1 H), 7.68 - 7.78 (m, 1 H), 7.85 (d, J=8.7 Hz, 2 H), 7.97 (d, J=8.7 Hz, 2 H), 8.03 (d, J=7.4 Hz, 1 H), 9.00 (s, 1 H), 12.43 (s, 1 H), 12.71 (s, 1 H), 12.78 (br s, 1 H)

Now, the methods of producing compounds of the present invention are described in detail by means of Working Examples.

### Example 1 3-[[8-(Methylamino)-2-oxo-1,2-dihydroquinoline-3-carbonyl]amino]benzoic acid

### 1-(1) Methyl 3-[[8-(methylamino)-2-oxo-1,2-dihydroquinoline-3-carbonyl]amino]benzoate

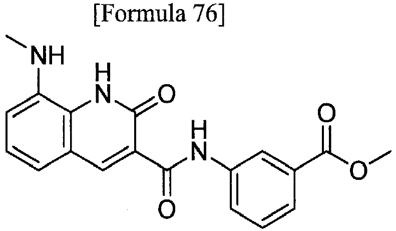

To a solution in DMF (3 mL) of the 8-(methylamino)-2-oxo-1,2-dihydroquinoline-3-carboxylic acid (40 mg) obtained in Production Example 1-(2), methyl 3-aminobenzoate (30 mg), HATU (76 mg) and DIPEA (96 µL) were added and the mixture was stirred for 21 hours at room temperature. The reaction mixture was concentrated under reduced pressure and water was added to the residue; the mixture was then stirred for 5 minutes. The precipitate was recovered by filtration to obtain the titled compound as a crude product. The crude product thus obtained was used in the next reaction without being purified.
MS (ESI neg.) m/z : 350 [M-H]-, RT=1.035 min (Analytical Condition 3)

### 1-(2) 3-[[8-(Methylamino)-2-oxo-1,2-dihydroquinoline-3-carbonyl]amino]benzoic acid

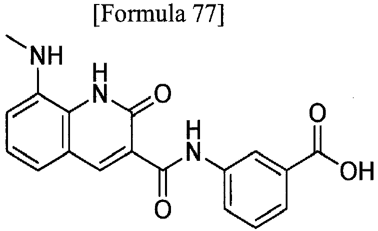

To a solution in tetrahydrofuran (1.8 mL) of the methyl 3-[[8-(methylamino)-2-oxo-1,2-dihydroquinoline-3-carbonyl]amino]benzoate obtained in Example 1-(1), 1 M sodium hydroxide in aqueous solution (1.8 mL) was added and the mixture was stirred for 4 hours at room temperature. The reaction mixture was neutralized to pH 4 with an aqueous solution of 1 M hydrochloric acid. The precipitating solid was recovered by filtration and thereafter washed with water to obtain the titled compound (36 mg) as a yellow solid.
MS (ESI pos.) m/z : 338 [M+H]+, RT=0.885 min (Analytical Condition 3)
¹H NMR (600 MHz, DMSO-d6) δ ppm 2.87 (d, J=4.5 Hz, 3 H), 6.18 (d, J=4.5 Hz, 1 H), 6.83 (d, J=7.8 Hz, 1 H), 7.19 - 7.24 (m, 1 H), 7.24 - 7.29 (m, 1 H), 7.52 (t, J=7.8 Hz, 1 H), 7.71 (d, J=7.8 Hz, 1 H), 7.95 (dd, J=7.8, 1.9 Hz, 1 H), 8.33 (t, J=1.9 Hz, 1 H), 8.90 (s, 1 H), 11.77 (s, 1 H), 12.30 (s, 1 H), 13.06 (br s, 1 H)

The same procedures as in Example 1 were employed to obtain the Example compounds identified below from the corresponding raw materials.

**[Table 1-1]**

| Example | Structural Formula | Compound Name | MS | NMR |
|---|---|---|---|---|
| 1 | 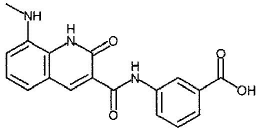 | 3-{[8-(methylamino)-2-oxo-1,2-dihydroquinoline-3-carbonyl]amino}benzoic acid | MS (ESI pos.) m/z: 338 [M+H]+ RT=0.885 min (Analytical Condition 3) | 1H NMR (600 MHz, DMSO-d6) δ ppm 2.87 (d, J=4.5 Hz, 3 H), 6.18 (d, J=4.5 Hz, 1 H), 6.83 (d, J=7.8 Hz, 1 H), 7.19 - 7.24 (m, 1 H), 7.24 - 7.29 (m, 1 H), 7.52 (t, J=7.8 Hz, 1 H), 7.71 (d, J=7.8 Hz, 1 H), 7.95 (dd, J=7.8, 1.9 Hz, 1 H), 8.33 (t, J=1.9 Hz, 1 H), 8.90 (s, 1 H), 11.77 (s, 1 H), 12.30 (s, 1 H), 13.06 (br s, 1 H) |
| 2 | 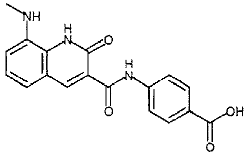 | 4-{[8-(methylamino)-2-oxo-1,2-dihydroquinoline-3-carbonyl]amino}benzoic acid | MS (ESI pos.) m/z : 338 [M+H]+ RT=0.890 min (Analytical Condition 3) | 1H NMR (600 MHz, DMSO-d6) δ ppm 2.82 (d, J=4.5 Hz, 3 H), 6.13 (br d, J=4.5 Hz, 1 H), 6.76 (br d, J=7.0 Hz, 1 H), 7.12 - 7.23 (m, 2 H), 7.77 (br d, J=8.3 Hz, 2 H), 7.91 (d, J=8.3 Hz, 2 H), 8.84 (s, 1 H) |
| 3 | 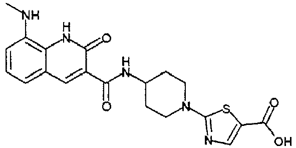 | 2-(4-{[8-(methylamino)-2-oxo-1,2-dihydroquinoline-3-carbonyl]amino}piperidine-1-yl)-1,3-thiazole-5-carboxylic acid | MS (ESI pos.) m/z : 428 [M+H]+ RT=0.766 min (Analytical Condition 3) | 1H NMR (600 MHz, DMSO-d6) δ ppm 1.54 - 1.66 (m, 2 H), 1.99 - 2.07 (m, 2 H), 2.83 (d, J=4.1 Hz, 3 H), 3.35 - 3.41 (m, 2 H), 3.86 - 3.96 (m, 2 H), 4.07 - 4.17 (m, 1 H), 6.13 (br d, J=4.1 Hz, 1 H), 6.78 (d, J=6.6 Hz, 1 H), 7.14 - 7.20 (m, 2 H), 7.76 (s, 1 H), 8.76 (s, 1 H), 9.88 (d, J=7.4 Hz, 1 H), 11.52 (s, 1 H), 12.64 (br s, 1 H) |
| 4 | 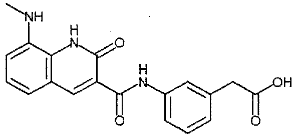 | (3-{[8-(methylamino)-2-oxo-1,2-dihydroquinoline-3-carboyl]amino}phenyl)acetic acid | MS (ESI pos.) m/z : 352 [M+H]+ RT=0.856 min (Analytical Condition 3) | 1H NMR (600 MHz, DMSO-d6) δ ppm 2.86 (d, J=4.5 Hz, 3 H), 3.60 (s, 2 H), 6.18 (br d, J=4.5 Hz, 1 H), 6.82 (dd, J=7.4, 1.2 Hz, 1 H), 7.03 (d, J=7.4 Hz, 1 H), 7.18 - 7.28 (m, 2 H), 7.33 (t, J=7.8 Hz, 1 H), 7.58 (s, 1 H), 7.70 (br dd, J=7.4, 1.2 Hz, 1 H), 8.88 (s, 1 H), 11.73 (s, 1 H), 12.13 (s, 1 H), 12.35 (brs, 1 H) |
| 5 | 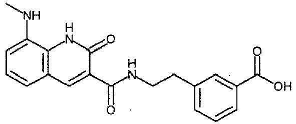 | 3-(2-{[8-(methylamino)-2-oxo-1,2-dihydroquinoline-3-carbonyl]amino}ethyl)benzoic acid | MS (ESI pos.) m/z: 366 [M+H]+ RT=0.814 min (Analytical Condition 3) | 1H NMR (600 MHz, DMSO-d6) δ ppm 2.83 (d, J=4.5 Hz, 3 H), 2.92 (t, J=7.2 Hz, 2 H), 3.44 - 3.64 (m, 2 H), 6.07 (br d, J=4.5 Hz, 1 H), 6.77 (dd, J=6.4, 2.7 Hz, 1 H), 7.13 - 7.20 (m, 2 H), 7.41 (t, J=7.8 Hz, 1 H), 7.51 (br d, J=7.8 Hz, 1 H), 7.79 (d, J=7.8 Hz, 1 H), 7.85 (s, 1 H), 8.73 (s, 1 H), 9.83 (t, J=5.6 Hz, 1 H) |
| 6 | 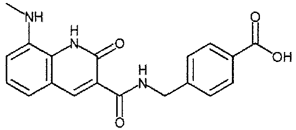 | 4-({[8-(methylamino)-2-oxo-1,2-dihydroquinoline-3-carbonyl]amino}methyl)benzoic acid | MS (ESI pos.) m/z: 351 [M+H]+ RT=0.784 min (Analytical Condition 3) | 1H NMR (600 MHz, DMSO-d6) δ ppm 2.84 (d, J=4.5 Hz, 3 H), 4.65 (d, J=6.0 Hz, 2 H), 6.08 - 6.16 (m, 1 H), 6.79 (dd, J=7.0, 2.1 Hz, 1 H), 7.15 - 7.21 (m, 2 H), 7.45 (m, J=8.3 Hz, 2 H), 7.92 (m, J=8.3 Hz, 2 H), 8.78 (s, 1 H), 10.22 (t, J=6.0 Hz, 1 H), 11.56 (br s, 1 H) |
| 8 | 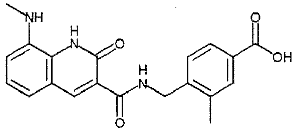 | 3-methyl-4-({[8-(methylamino)-2-oxo-1,2-dihydroquinoline-3-carbonyl]amino}methyl)benzoic acid | MS (ESI pos.) m/z : 366 [M+H]+ RT=0.834 min (Analytical Condition 3) | 1H NMR (600 MHz, DMSO-d6) δ ppm 2.39 (s, 3 H), 2.84 (d, J=4.5 Hz, 3 H), 4.62 (d, J=5.8 Hz, 2 H), 6.13 (brd, J=4.5 Hz, 1 H), 6.78 (dd, J=7.4, 1.6 Hz, 1 H), 7.14 - 7.26 (m, 2 H), 7.36 (d, J=7.4 Hz, 1 H), 7.74 (d, J=7.8 Hz, 1 H), 7.78 (s, 1 H), 8.79 (s, 1 H), 10.16 (brs, 1 H), 11.57 (br s, 1 H), 12.63 -13.09 (m, 1 H) |
| 9 | 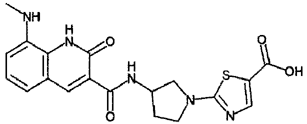 | 2-(3-{[8-(methylamino)-2-oxo-1,2-dihydroquinoline-3-carbonyl]amino)pyrrolidine-1-yl)-1,3-thiazole-5-carboxylic acid | MS (ESI pos.) m/z : 414 [M+H]+ RT=0.697 min (Analytical Condition 3) | 1H NMR (600 MHz, DMSO-d6) δ ppm 2.03 - 2.17 (m, 1 H), 2.28 - 2.44 (m, 1 H), 2.83 (d, J=4.5 Hz, 3 H), 3.43 (br d, J=5.8 Hz, 1 H), 3.58 (br s, 2 H), 3.81 (br dd, J=10.5, 6.4 Hz, 1 H), 4.60 - 4.72 (m, 1 H), 6.12 (br d, J=4.5 Hz, 1 H), 6.78 (dd, J=7.2, 1.4 Hz, 1 H), 7.11 - 7.24 (m, 2 H), 7.80 (s, 1 H), 8.76 (s, 1 H), 10.06 (d, J=7.0 Hz, 1 H), 11.52 (s, 1 H), 12.58 (brs, 1 H) |

**[Table 1-2]**

| Example | Structural Formula | Compound Name | MS | NMR |
|---|---|---|---|---|
| 10 | 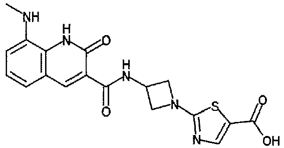 | 2-(3-{[8-(methylamino)-2-oxo-1,2-dihydroquinoline-3-carbonyl]amino}azetidine-1-yl)-1,3-thiazole-5-carboxylic acid | MS (ESI pos.) m/z : 400 [M+H]+ RT=0.705 min (Analytical Condition 3) | 1H NMR (600 MHz, DMSO-d6) δ ppm 2.84 (d, J=4.5 Hz, 3 H), 4.12 (dd, J=8.6, 5.4 Hz, 2 H), 4.42 (t, J=8.6 Hz, 2 H), 4.93 -4.99 (m, 1 H), 6.13 (brd, J=4.5 Hz, 1 H), 6.79 (dd, J=7.4, 1.7 Hz, 1 H), 7.15 - 7.21 (m, 2 H), 7.77 (s, 1 H), 8.75 (s, 1 H), 10.29 (d, J=7.0 Hz, 1 H), 11.56 (s, 1 H, 12.71 (br s, 1 H) |
| 13 | 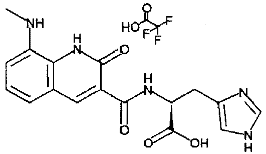 | N-[8-(methylamino)-2-oxo-1,2-dihydroquinoline-3-carbonyl]-L-histidine-trifluoroacetate | MS (ESI pos.) m/z : 356 [M+H]+ RT=0.747 min (Analytical Condition 2) | 1H NMR (600 MHz, DMSO-d6) δ ppm 2.84 (s, 3 H), ,3.21- 3.30 (m, 2 H), 4.89 (td, J=7.8, 5.2 Hz, 1 H), 6.13 (br s, 1 H), 6.77 - 6.81 (m, 1 H), 7.15 - 7.19 (m, 2 H), 7.44 (s, 1 H), 8.71 (s, 1 H), 8.99 (s, 1 H), 10.22 (d, J=7.8 Hz, 1 H), 11.52 (s, 1 H), 14.07 (br s, 1 H) |
| 20 | 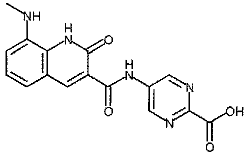 | 5-{[8-(methylamino)-2-oxo-1,2-dihydroquinoline-3-carbonyl]amino}pyrimidine-2-carboxylic acid | | 1H NMR (600 MHz, DMSO-d6) δ ppm 2.86 (d, J=4.5 Hz, 3 H), 6.21 (br d, J=4.5 Hz, 1 H), 6.85 (d, J=7.4 Hz, 1 H), 7.23 (t, J=7.8 Hz, 1 H), 7.26 (d, J=7.8 Hz, 1 H), 8.95 (s, 1 H), 9.28 (s, 2 H), 11.82 (br s, 1 H), 12.44 (brs, 1 H) |
| 44 | 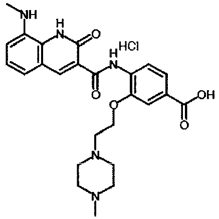 | 4-{[8-(methylamino)-2-oxo-1,2-dihydroquinoline-3-carbonyl]amino}-3-[2-(4-methylpiperazine-1-yl)ethoxy]benzoic acid-hydrochloride | MS (ESI pos.) m/z : 480 [M+H]+ RT=0.525 min (Analytical Condition 3) | 1H NMR (400 MHz, DMSO-d6) δ ppm 2.60 - 4.70 (m, 18 H), 6.81 - 6.87 (m, 1 H), 7.15 - 7.28 (m, 2 H), 7.61 (d, J=1.7 Hz, 1 H), 7.66 (br d, J=8.3 Hz, 1 H), 8.63 (d, J=8.3 Hz, 1 H), 8.93 (s, 1 H), 11.82 (br s, 1 H), 12.68 (s, 1 H) |
| 166 | 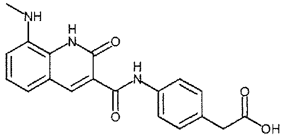 | (4-{[8-(methylamino)-2-oxo-1,2-dihydroquinoline-3-carbonyl]amino}phenyl)acetic acid | MS (ESI pos.) m/z : 352 [M+H]+ RT=0.853 min (Analytical Condition 3) | 1H NMR (600 MHz, DMSO-d6) δ ppm 2.86 (d, J=4.5 Hz, 3 H), 3.55 (s, 2 H), 6.19 (br d, J=4.5 Hz, 1 H), 6.82 (d, J=7.8 Hz, 1 H), 7.18 - 7.25 (m, 2 H), 7.27 (d, J=8.7 Hz, 2 H), 7.67 (d, J=8.7 Hz, 2 H), 8.88 (s, 1 H), 11.72 (brs, 1 H), 12.12 (s, 1 H) |
| 167 | 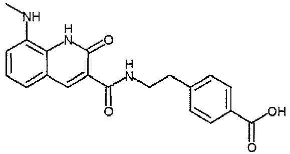 | 4-(2-{[8-(methylamino)-2-oxo-1,2-dihydroquinoline-3-carbonyl]amino}ethyl)benzoic acid | MS (ESI pos.) m/z : 366 [M+H]+ RT=0.814 min (Analytical Condition 3) | 1H NMR (600 MHz, DMSO-d6) δ ppm 2.83 (d, J=4.5 Hz, 3 H), 2.94 (t, J=7.0 Hz, 2 H), 3.57 - 3.67 (m, 2 H), 6.06 (br d, J=4.5 Hz, 1 H), 6.77 (dd, J=6.8, 1.9 Hz, 1 H), 7.12 - 7.20 (m, 2 H), 7.41 (d, J=8.3 Hz, 2 H), 7.88 (d, J=8.3 Hz, 2 H), 8.74 (s, 1 H), 9.83 (brt, J=5.8 Hz, 1 H), 11.53 (s, 1 H), 12.83 (brs, 1 H) |
| 168 | 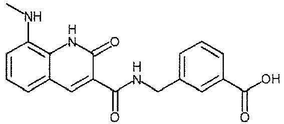 | 3-({[8-(methylamino)-2-oxo-1,2-dihydroquinoline-3-carbonyl]amino}methyl)benzoic acid | MS (ESI pos.) m/z : 352 [M+H]+ RT=0.806 min (Analytical Condition 3) | 1H NMR (600 MHz, DMSO-d6) δ ppm 2.84 (d, J=4.5 Hz, 3 H), 4.64 (d, J=6.1 Hz, 2 H), 6.12 (br d, J=4.5 Hz, 1 H), 6.79 (dd, J=7.2, 1.9 Hz, 1 H), 7.14 - 7.21 (m, 2 H), 7.47 (t, J=7.8 Hz, 1 H), 7.60 (d, J=7.8 Hz, 1 H), 7.84 (d, J=7.8 Hz, 1 H), 7.93 (s, 1 H), 8.79 (s, 1 H), 10.22 (t, J=6.1 Hz, 1 H), 11.56 (br s, 1 H), 12.98 (br s, 1 H) |

### Example 7 N-(2-[[Cyclohexyl(methyl)amino]methyl]phenyl)-8-(methylamino)-2-oxo-1,2-dihydroquinoline-3-carboxamide

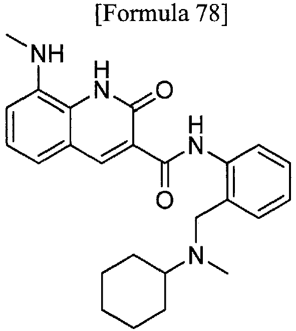

To a solution in DMF (3 mL) of the 8-(methylamino)-2-oxo-1,2-dihydroquinoline-3-carboxylic acid (39 mg) obtained in Production Example 1-(2), HATU (75 mg), 2-[[cyclohexyl(methyl)amino]methyl]aniline (294 mg) and DIPEA (140 µL) were added and the mixture was stirred for 7 hours at room temperature. The reaction mixture was diluted with ethyl acetate and washed with saturated aqueous sodium bicarbonate and water. The organic layer was dried over anhydrous magnesium sulfate and concentrated under reduced pressure. The resulting residue was purified by column chromatography (silica gel cartridge, chloroform/methanol = 100:0 - 90:10) to obtain the titled compound (15 mg) as a yellow solid.
MS (ESI pos.) m/z : 419 [M+H]+, RT=0.641 min (Analytical Condition 3)
¹H NMR (600 MHz, CHLOROFORM-d) δ ppm 0.77 - 1.32 (m, 10 H), 2.20 (s, 3 H), 2.80 (s, 3 H), 3.00 - 3.11 (m, 1 H), 3.77 (s, 2 H), 6.76 - 6.92 (m, 1 H), 7.11 - 7.16 (m, 1 H), 7.16 - 7.21 (m, 1 H), 7.17 - 7.20 (m, 1 H), 7.22 - 7.39 (m, 4 H), 8.15 - 8.33 (m, 1 H), 8.96 (br s, 1 H), 11.71 (br s, 1 H)

The same procedures as in Example 7 were employed to obtain the Example compounds identified below from the corresponding raw materials.

**[Table 2-1]**

| Example | Structural Formula | Compound Name | MS | NMR |
|---|---|---|---|---|
| 7 | 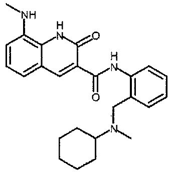 | N-(2-{[cyclohexyl(methyl) amino]methyl}phenyl)-8-(methylamino)-2-oxo-1,2-dihydroquinoline-3-carboxamide | MS (ESI pos.) m/z : 419 [M+H]+ RT=0.641 min (Analytical Condition 3) | 1H NMR (600 MHz, CHLOROFORM-d) δ ppm 0.77 - 1.32 (m, 10 H), 2.20 (s, 3 H), 2.80 (s, 3 H), 3.00 - 3.11 (m, 1 H), 3.77 (s, 2 H), 6.76 - 6.92 (m, 1 H), 7.11 - 7.16 (m, 1 H), 7.16 - 7.21 (m, 1 H), 7.17 - 7.20 (m, 1 H), 7.22 - 7.39 (m, 4 H), 8.15 - 8.33 (m, 1 H), 8.96 (br s, 1 H), 11.71 (br s, 1 H) |
| 11 | 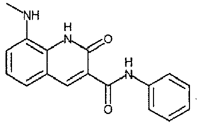 | 8-(methylamino)-2-oxo-N-dihydroquinoline-3-carboxamide | MS (ESI pos.) m/z : 294 [M+H]+ RT=1.014 min (Analytical Condition 3) | 1H NMR (600 MHz, DMSO-d6) δ ppm 2.86 (d, J=4.5 Hz, 3 H), 6.17 (br d, J=4.5 Hz, 1 H), 6.81 (d, J=7.4 Hz, 1 H), 7.11 - 7.15 (m, 1 H), 7.18 - 7.21 (m, 1 H), 7.22 - 7.25 (m, 1 H), 7.39 (t, J=7.8 Hz, 2 H), 7.73 (d, J=7.8 Hz, 2 H), 8.88 (s, 1 H), 11.74 (brs, 1 H), 12.23 (br s, 1 H) |
| 12 | 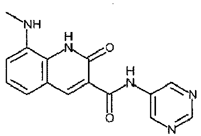 | 8-(methylamino)-2-oxo-N-(pyrimidine-5-yl)-1,2-dihydroquinoline-3-carboxamide | MS (ESI pos.) m/z : 296 [M+H]+ RT=0.740 min (Analytical Condition 3) | 1H NMR (600 MHz, DMSO-d6) δ ppm 2.86 (d, J=4.5 Hz, 3 H), 6.14 - 6.24 (m, 1 H), 6.84 (dd, J=7.8, 0.8 Hz, 1 H), 7.22 (t, J=7.8 Hz, 1 H), 7.26 (dd, J=7.8, 0.8 Hz, 1 H), 8.93 (s, 1 H), 8.96 (s, 1 H), 9.19 (s, 2 H), 11.79 (s, 1 H), 12.24 (s, 1 H) |
| 14 | 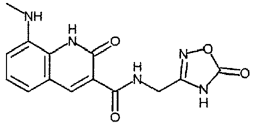 | 8-(methylamino)-2-oxo-N-[(5-oxo-4,5-dihydro-1,2,4-oxadiazole-3-yl)methyl]-1,2-dihydroquinoline-3-carboxamide | MS (ESI pos.) m/z : 316 [M+H]+ RT=0.655 min (Analytical Condition 3) | 1H NMR (600 MHz, DMSO-d6) δ ppm 2.84 (d, J=4.5 Hz, 3 H), 4.50 (d, J=5.8 Hz, 2 H), 6.12 (q, J=4.5 Hz, 1 H), 6.80 (dd, J=7.6, 1.4 Hz, 1 H), 7.15 - 7.23 (m, 2 H), 8.78 (s, 1 H), 10.14 (t, J=5.8 Hz, 1 H), 11.60 (s, 1 H), 12.39 (br s, 1 H) |
| 15 | 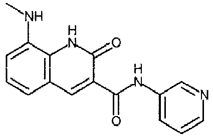 | 8-(methylamino)-2-oxo-N-(pyridine-3-yl)-1,2-dihydroquinoline-3-carboxamide | MS (ESI pos.) m/z : 295 [M+H]+ RT=0.545 min (Analytical Condition 3) | 1H NMR (600 MHz, DMSO-d6) δ ppm 2.86 (d, J=4.5 Hz, 3 H), 6.18 (br q, J=4.5 Hz, 1 H), 6.83 (d, J=7.8 Hz, 1 H), 7.21 (t, J=7.8 Hz, 1 H), 7.25 (dd, J=7.8, 0.8 Hz, 1 H), 7.43 (dd, J=8.3, 4.5 Hz, 1 H), 8.19 - 8.25 (m, 1 H), 8.34 (dd, J=4.5, 1.4 Hz, 1 H), 8.88 - 8.92 (m, 2 H), 11.77 (br s, 1 H), 12.23 (s, 1 H) |
| 16 | 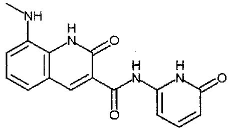 | 8-(methylamino)-2-oxo-N-(6-oxo-1,6-dihydropyridine-2-yl)-1,2-dihydroquinoline-3-carboxamide | MS (ESI pos.) m/z : 311 [M+H]+ RT=0.730 min (Analytical Condition 3) | 1H NMR (600 MHz, DMSO-d6) δ ppm 2.85 (d, J=4.5 Hz, 3 H), 6.17 (q, J=4.5 Hz, 1 H), 6.34 - 6.46 (m, 1 H), 6.79 - 6.86 (m, 1 H), 7.21 (t, J=7.8 Hz, 1 H), 7.25 (d, J=7.8 Hz, 1 H), 7.67 (br t, J=7.8 Hz, 1 H), 7.70 - 7.85 (m, 1 H), 8.92 (s, 1 H), 10.82 (br s, 1 H), 11.74 (s, 1 H), 12.24 (s, 1 H) |
| 18 | 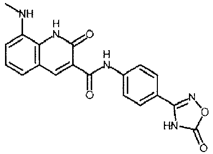 | 8-(methylamino)-2-oxo-N-[4-(5-oxo-4,5-dihydro-1,2,4-oxadiazole-3-yl)phenyl]-1,2-dihydroquinoline-3-carboxamide | MS (ESI pos.) m/z : 378 [M+H]+ RT=0.913 min (Analytical Condition 3) | 1H NMR (600 MHz, DMSO-d6) δ ppm 2.86 (d, J=4.5 Hz, 3 H), 6.18 (br q, J=4.5 Hz, 1 H), 6.84 (d, J=7.8 Hz, 1 H), 7.22 (t, J=7.8 Hz, 1 H), 7.26 (d, J=7.8 Hz, 1 H), 7.84 (d, J=8.7 Hz, 2 H), 7.89 - 7.98 (m, 3 H), 8.91 (s, 1 H), 11.79 (s, 1 H), 12.43 (s, 1 H) |
| 19 | 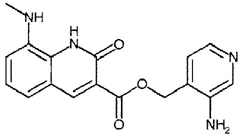 | (3-aminopyridine-4-yl)methyl 8-(methylamino)-2-oxo-1,2-dihydroquinoline-3-carboxylate | MS (ESI pos.) m/z : 325 [M+H]+ RT=0.333 min (Analytical Condition 3) | 1H NMR (600 MHz, DMSO-d6) δ ppm 2.82 (d, J=5.0 Hz, 3 H), 5.19 (s, 2 H), 5.48 (s, 2 H), 6.03 (br d, J=5.0 Hz, 1 H), 6.76 (d, J=6.6 Hz, 1 H), 7.09 - 7.14 (m, 2 H), 7.21 (d, J=5.0 Hz, 1 H), 7.79 (d, J=5.0 Hz, 1 H), 8.04 (s, 1 H), 8.54 (s, 1 H), 11.22 (br s, 1 H) |

**[Table 2-2]**

| Example | Structural Formula | Compound Name | MS | NMR |
|---|---|---|---|---|
| 21 | 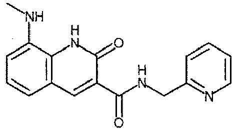 | 8-(methylamino)-2-oxo-N-[(pyridine-2-yl)methyl]-1,2-dihydroquinoline-3-carboxamide | MS (ESI pos.) m/z : 309 [M+H]+ RT=0.464 min (Analytical Condition 3) | 1H NMR (600 MHz, CHLOROFORM-d) δ ppm 2.78 (br d, J=3.3 Hz, 3 H), 4.75 (br s, 1 H), 4.89 (d, J=5.4 Hz, 2 H), 6.87 (d, J=7.4 Hz, 1 H), 7.16 - 7.29 (m, 3 H), 7.39 (d, J=7.6 Hz, 1 H), 7.67 (td, J=7.6, 1.7 Hz, 1 H), 8.54 (br d, J=4.1 Hz, 1 H), 9.03 (s, 1 H), 10.32 (br s, 1 H), 11.66 (brs, 1 H) |
| 22 | 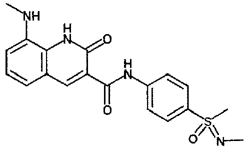 | N-[4-(N,S-dimethanesulfonimidoyl)phenyl]-8-(methylamino)-2-oxo-1,2-dihydroquinoline-3-carboxamide | MS (ESI pos.) m/z : 385 [M+H]+ RT=0.657 min (Analytical Condition 3) | 1H NMR (600 MHz, DMSO-d6) δ ppm 2.48 (s, 3 H), 2.86 (d, J=4.5 Hz, 3 H), 3.11 (s, 3 H), 6.19 (q, J=4.5 Hz, 1 H), 6.84 (d, J=7.0 Hz, 1 H), 7.22 (t, J=7.8 Hz, 1 H), 7.26 (d, J=7.8 Hz, 1 H), 7.84 (d, J=8.7 Hz, 2 H), 7.97 (d, J=8.7 Hz, 2 H), 8.92 (s, 1 H), 11.79 (s, 1 H), 12.47 (s, 1 H) |
| 23 | 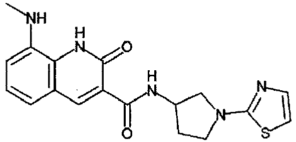 | 8-(methylamino)-2-oxo-N-[1-(1,3-thiazole-2-yl)pyrrolidine-3-yl]-1,2-dihydroquinoline-3-carboxamide | MS (ESI pos.) m/z : 370 [M+H]+ RT=0.463 min (Analytical Condition 3) | 1H NMR (600 MHz, DMSO-d6) δ ppm 2.03 - 2.14 (m, 1 H), 2.31 - 2.41 (m, 1 H), 2.83 (d, J=5.0 Hz, 3 H), 3.36 (dd, J=10.3, 4.1 Hz, 1 H), 3.46 - 3.58 (m, 2 H), 3.75 (dd, J=10.3, 6.2 Hz, 1 H), 4.63 (dq, J=11.3, 5.9 Hz, 1 H), 6.12 (br d, J=5.0 Hz, 1 H), 6.75 (d, J=3.7 Hz, 1 H), 6.78 (d, J=7.4 Hz, 1 H), 7.15 (d, J=3.7 Hz, 1 H), 7.16 - 7.22 (m, 2 H), 8.76 (s, 1 H), 10.06 (br d, J=6.2 Hz, 1 H), 11.52 (brs, 1 H) |
| 24 | 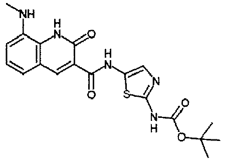 | tert-butyl(5-{[8-(methylamino)-2-oxo-1,2-dihydroquinoline-3-carbonyl]amino}-1,3-thiazole-2-yl)carbamate | MS (ESI pos.) m/z : 416 [M+H]+ RT=1.021 min (Analytical Condition 3) | 1H NMR (600 MHz, DMSO-d6) δ ppm 1.50 (s, 9 H), 2.86 (d, J=4.5 Hz, 3 H), 6.18 (brd, J=4.5 Hz, 1 H), 6.83 (d, J=7.8 Hz, 1 H), 7.21 (t, J=7.8 Hz, 1 H), 7.26 (d, J=7.8 Hz, 1 H), 7.36 (s, 1 H), 8.85 (s, 1 H), 11.16 (brs, 1 H), 11.77 (s, 1 H), 12.59 (br s, 1 H) |
| 26 | 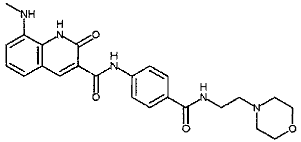 | 8-(methylamino)-N-(4-{[2-(morpholine-4-yl)ethyl]carbamoyl}phenyl)-2-oxo-1,2-dihydroquinoline-3-carboxamide | MS (ESI pos.) m/z : 450 [M+H]+ RT=0.498 min (Analytical Condition 3) | 1H NMR (600 MHz, DMSO-d6) δ ppm 2.87 (d, J=4.5 Hz, 3 H), 3.34 - 3.46 (m, 4 H), 3.58 (br t, J=4.5 Hz, 4 H), 6.18 (br d, J=4.5 Hz, 1 H), 6.82 (br d, J=7.0 Hz, 1 H), 7.21 (t, J=7.8 Hz, 1 H), 7.24 (d, J=7.8 Hz, 1 H), 7.82 (d, J=8.7 Hz, 2 H), 7.88 (d, J=8.7 Hz, 2 H), 8.36 (brt, J=5.6 Hz, 1 H), 8.89 (s, 1 H) |
| 27 | 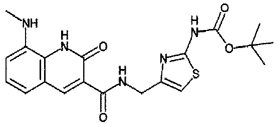 | tert-butyl[4-({[8-(methylamino)-2-oxo-1,2-dihydroquinoline-3-carbonyl]amino}methyl)-1,3-thiazole-2-yl]carbamate | MS (ESI pos.) m/z: 430 [M+H]+ RT=0.960 min (Analytical Condition 3) | 1H NMR (600 MHz, DMSO-d6) δ ppm 1.47 (s, 9 H), 2.84 (d, J=4.5 Hz, 3 H), 4.50 (d, J=5.8 Hz, 2 H), 6.11 (br d, J=4.5 Hz, 1 H), 6.76 (br d, J=7.0 Hz, 1 H), 6.92 (s, 1 H), 7.11 - 7.22 (m, 2 H), 8.75 (s, 1 H), 10.14 (br s, 1 H), 11.47 (br s, 2 H) |
| 28 | 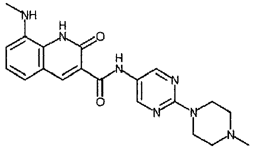 | 8-(methylamino)-N-[2-(4-methylpiperazine-1-yl)pyrimidine-5-yl]-2-oxo-1,2-dihydroquinoline-3-carboxamide | MS (ESI pos.) m/z : 394 [M+H]+ RT=0.427 min (Analitical Condition 3) | 1H NMR (600 MHz, DMSO-d6) δ ppm 2.22 (s, 3 H), 2.36 (t, J=5.0 Hz, 4 H), 2.85 (d, J=4.5 Hz, 3 H), 3.71 (t, J=5.0 Hz, 4 H), 6.17 (br d, J=4.5 Hz, 1 H), 6.82 (d, J=7.4 Hz, 1 H), 7.20 (t, J=7.4 Hz, 1 H), 7.22 (d, J=7.4 Hz, 1 H), 8.71 (s, 2 H), 8.86 (s, 1 H), 11.69 (br s, 1 H), 11.76 (s, 1 H) |
| 29 | 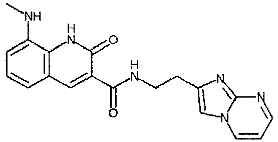 | N-[2-(imidazo[1,2-a]pyrimidine-2-yl)ethyl]-8-(methylamino)-2-oxo-1,2-dihydroquinoline-3-carboxamide | MS (ESI pos.) m/z : 363 [M+H]+ RT=0.292 min (Analytical Condition 3) | 1H NMR (600 MHz, DMSO-d6) δ ppm 2.82 (d, J=4.5 Hz, 3 H), 3.00 (t, J=7.0 Hz, 2 H), 3.72 - 3.77 (m, 2 H), 6.06 (br d, J=4.5 Hz, 1 H), 6.77 (dd, J=7.4, 1.7 Hz, 1 H), 7.01 (dd, J=6.6, 4.1 Hz, 1 H), 7.13 - 7.20 (m, 2 H), 7.77 (s, 1 H), 8.45 - 8.50 (m, 1 H), 8.75 (s, 1 H), 8.91 (dd, J=6.6, 2.1 Hz, 1 H), 9.86 (t, J=5.6 Hz, 1 H), 11.48 (s, 1 H) |

**[Table 2-3]**

| Example | Structural Formula | Compound Name | MS | NMR |
|---|---|---|---|---|
| 30 | 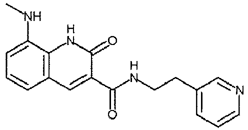 | 8-(methylamino)-2-oxo-N-[2-(pyridine-3-yl)ethyl]-1,2-dihydroquinoline-3-carboxamide | MS (ESI pos.) m/z: 323 [M+H]+ RT=0.278 min (Analytical Condition 3) | 1H NMR (600 MHz, DMSO-d6) δ ppm 2.84 (d, J=4.5 Hz, 3 H), 2.87 - 2.92 (m, 2 H), 3.57 - 3.65 (m, 2 H), 6.07 (br d, J=4.5 Hz, 1 H), 6.78 (dd, J=6.6, 2.5 Hz, 1 H), 7.14 - 7.19 (m, 2 H), 7.33 (dd, J=7.6, 4.7 Hz, 1 H), 7.71 (br d, J=7.6 Hz, 1 H), 8.43 (dd, J=4.7, 1.4 Hz, 1 H), 8.49 (d, J=2.1 Hz, 1 H), 8.73 (s, 1 H), 9.84 (br t, J=5.6 Hz, 1 H), 11.54 (s, 1 H) |
| 32 | 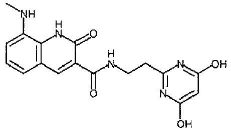 | N-[2-(4,6-dihydroxypyrimidine-2-yl)ethyl]-8-(methylamino)-2-oxo-1,2-dihydroquinoline-3-carboxamide | MS (ESI pos.) m/z : 356 [M+H]+ RT=0.525 min (Analytical Condition 3) | 1H NMR (600 MHz, DMSO-d6) δ ppm 2.78 (t, J=6.6 Hz, 2 H), 2.83 (d, J=4.5 Hz, 3 H), 3.71 (dd, J=12.8, 6.6 Hz, 2 H), 5.11 (br s, 1 H), 6.07 (br d, J=4.5 Hz, 1 H), 6.77 (dd, J=7.0, 1.7 Hz, 1 H), 7.09 - 7.25 (m, 2 H), 8.73 (s, 1 H), 9.88 - 9.93 (m, 1 H), 11.54 (s, 2 H) |
| 34 | 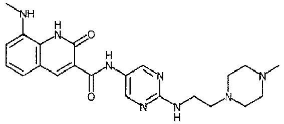 | 8-(methylamino)-N-(2-{[2-(4-methylpiperazine-1-yl)ethyl]amino}pyrimidine-5-yl)-2-oxo-1,2-dihydroquinoline-3-carboxamide | MS (ESI pos.) m/z : 437 [M+H]+ RT=0.290 min (Analytical Condition 3) | 1H NMR (600 MHz, DMSO-d6) δ ppm 2.87 (br s, 6 H), 3.00-3.85 (m, 12 H), 6.18 (s, 1 H), 6.83 (dd, J=7.4, 1.7 Hz, 1 H), 7.17 - 7.27 2 H), 8.69 (s, 2 H), 8.86 s 1 H), 11.71 (br s, 1 H), 11.74 (s, 1 H) |
| 36 | 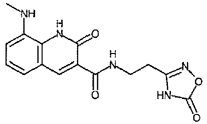 | 8-(methylamino)-2-oxo-N-[2-(5-oxo-4,5-dihydro-1,2,4-oxadiazole-3-yl)ethyl]-1,2-dihydroquinoline-3-carboxamide | MS (ESI pos.) m/z : 330 [M+H]+ RT=0.646 min (Analytical Condition 3) | 1H NMR (600 MHz, DMSO-d6) δ ppm 2.64 - 2.70 (m, 2 H), 2.84 (d, J=5.0 Hz, 3 H), 3.63 (q, J=6.6 Hz, 2 H), 6.10 (brd, J=5.0 Hz, 1 H), 6.78 (d, J=7.0 Hz, 1 H), 7.13 - 7.20 (m, 2 H), 8.74 (s, 1 H), 9.87 (t, J=6.6 Hz, 1 H), 11.51 (br s, 1 H) |
| 37 | 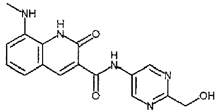 | N-[2-(hydroxymethyl)pyrimidine-5-yl]-8-(methylamino)-2-oxo-1,2-dihydroquinoline-3-carboxamide | MS (ESI pos.) m/z : 326 [M+H]+ RT=0.651 min (Analytical Condition 3) | 1H NMR (600 MHz, DMSO-d6) δ ppm 2.86 (d, J=4.5 Hz, 3 H), 4.59 (d, J=6.2 Hz, 2 H), 5.29 (t, J=6.2 Hz, 1 H), 6.15 (d, J=4.5 Hz, 1 H), 6.78 (br d, J=5.4 Hz, 1 H), 7.08 - 7.26 (m, 2 H), 8.87 (br s, 1 H), 9.15 (s, 2 H) |
| 38 | 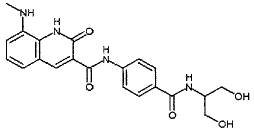 | N-{4-[(1,3-dihydroxypropane-2-yl)carbamoyl]phenyl}-8-(methylamino)-2-oxo-1,2-dihydroquinoline-3-carboxamide | MS (ESI pos.) m/z : 411 [M+H]+ RT=0.714 min (Analytical Condition 3) | 1H NMR (600 MHz, DMSO-d6) δ ppm 2.83 (s, 3 H), 3.55 (m, 1 H), 3.64 (m, 1 H), 4.25 (m, 1 H), 4.30 - 4.39 (m, 2 H), 5.86 - 6.18 (m, 3 H), 6.55 (d, J=8.7 Hz, 2 H), 6.78 (d, J=7.4 Hz, 1 H), 7.12 - 7.21 (m, 2 H), 7.68 (d, J=8.7 Hz, 2 H), 8.77 (s, 1 H), 10.11 (d, J=8.3 Hz, 1 H), 11.52 (s, 1 H) |
| 41 | 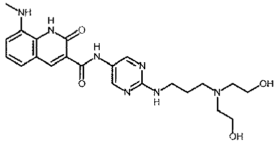 | N-[2-({3-[bis(2-hydroxyethyl)amino]propyl} amino)pyrimidine-5-yl]-8-(methylamino)-2-oxo-1,2-dihydroquinoline-3-carboxamide | MS (ESI pos.) m/z : 456 [M+H]+ RT=0.344 min (Analytical Condition 3) | 1H NMR (600 MHz, DMSO-d6) δ ppm 1.89 - 1.98 (m, 2 H), 2.86 (d, J=4.5 Hz, 3 H), 3.13 - 3.28 (m, 6 H), 3.33 - 3.42 (m, 2 H), 3.74 (br s, 4 H), 5.27 (br s, 2 H), 6.17 (br d, J=4.5 Hz, 1 H), 6.82 (dd, J=7.4, 1.7 Hz, 1 H), 7.17 - 7.26 (m, 2 H), 7.28 (br s, 1 H), 8.64 (s, 2 H), 8.85 (s, 1 H), 11.70 (s, 2 H) |
| 42 | 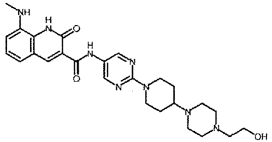 | N-(2-{4-[4-(2-hydroxyethyl)piperazine-1-yl]piperidine-1-yl)pyrimidine-5-yl)-8-(methylamino)-2-oxo-1,2-dihydroquinoline-3-carboxamide | MS (ESI pos.) m/z : 507 [M+H]+ RT=0.338 min (Analytical Condition 3) | 1H NMR (400 MHz, DMSO-d6) δ ppm 1.28 - 1.41 (m, 2 H), 1.84 (br d, J=11.5 Hz, 2 H), 2.80 - 2.96 (m, 5 H), 3.46 - 3.57 (m, 2 H), 4.45 (br s, 1 H), 4.58 - 4.70 (m, 2 H), 6.17 (br d, J=4.5 Hz, 1 H), 6.82 (dd, J=7.3, 1.9 Hz, 1 H), 7.17 - 7.25 (m, 2 H), 8.69 (s, 2 H), 8.86 (s, 1 H), 11.72 (s, 2 H) |
| 43 | 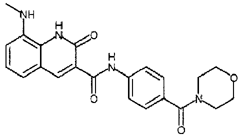 | 8-(methylamino)-N-[4-(morpholine-4-carbonyl)phenyl]-2-oxo-1,2-dihydroquinoline-3-carboxamide | MS (ESI pos.) m/z : 407 [M+H]+ RT=0.807 min (Analytical Condition 3) | 1H NMR (400 MHz, CHLOROFORM-d) δ ppm 2.93 (d, J=3.9 Hz, 3 H), 3.65 - 3.73 (m, 8 H), 6.63 - 6.70 (m, 1 H), 6.91 - 6.98 (m, 1 H), 7.28 - 7.34 (m, 1 H), 7.47 (d, J=8.5 Hz, 2 H), 7.85 (d, J=8.5 Hz, 2 H), 9.09 (s, 1 H), 11.71 (br s, 1 H), 11.88 (s, 1 H) |

### Example 17 4-[[8-(Methylamino)-2-oxo-1,2-dihydro-1,7-naphthyridine-3-carbonyl]amino]benzoic acid

### 17-(1) Methyl 4-[[8-(methylamino)-2-oxo-1,2-dihydro-1,7-naphthyridine-3-carbonyl]amino]benzoate

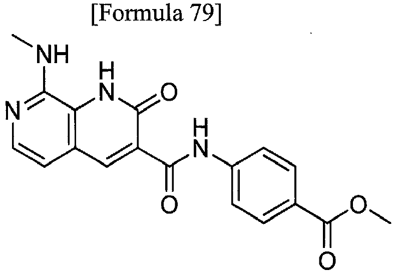

To a solution in DMF (4 mL) of methyl 4-aminobenzoate (14 mg), HATU (36 mg), and the 8-(methylamino)-2-oxo-1,2-dihydro-1,7-naphthyridine-3-carboxylic acid (17 mg) obtained in Production Example 3-(6), DIPEA (41 µL) was added and the mixture was stirred for 41 hours at room temperature. Following further addition of methyl 4-aminobenzoate (7 mg), HATU (15 mg) and DIPEA (25 µL), the mixture was stirred for 8 hours at room temperature. After concentrating the reaction mixture under reduced pressure, a saturated aqueous solution of sodium hydrogencarbonate was added to the residue; the organic layer was washed with saturated brine and thereafter dried over anhydrous magnesium sulfate. The dried organic layer was concentrated under reduced pressure to obtain the titled compound (27 mg) as a yellow solid.
MS (ESI pos.) m/z : 353 [M+H]+, RT=1.008 min (Analytical Condition 2)

### 17-(2) 4-[[8-(Methylamino)-2-oxo-1,2-dihydro-1,7-naphthyridine-3-carbonyl]amino]benzoic acid

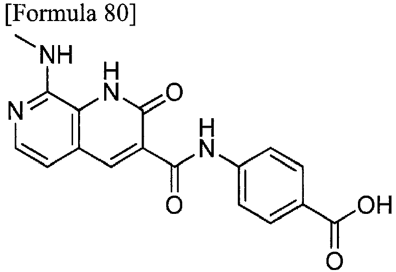

To a solution in tetrahydrofuran (1 mL) of the methyl 4-[[8-(methylamino)-2-oxo-1,2-dihydro-1,7-naphthyridine-3-carbonyl]amino]benzoate (27 mg) obtained in Example 17-(1), 1 M sodium hydroxide in aqueous solution (1 mL) was added and the mixture was stirred for 14 hours at room temperature. After the end of the reaction, an aqueous solution of 1 M hydrochloric acid was added to establish pH 3. The precipitating solid was recovered by filtration and washed with water, whereupon the titled compound (4 mg) was obtained as a yellow solid.
MS (ESI pos.) m/z : 339 [M+H]+, RT=0.827 min (Analytical Condition 3)
¹H NMR (600 MHz, DMSO-d6) δ ppm 2.97 (d, J=4.5 Hz, 3 H), 7.08 (d, J=5.4 Hz, 1 H), 7.18 (br d, J=4.5 Hz, 1 H), 7.85 (d, J=8.7 Hz, 2 H), 7.92 (d, J=5.4 Hz, 1 H), 7.97 (d, J=8.7 Hz, 2 H), 8.85 (s, 1 H), 12.14 (s, 1 H), 12.30 (br s, 1 H), 12.82 (br s, 1 H)

### Example 25 N-(2-Amino-1,3-thiazol-5-yl)-8-(methylamino)-2-oxo-1,2-dihydroquinoline-3-carboxamide-hydrochloride

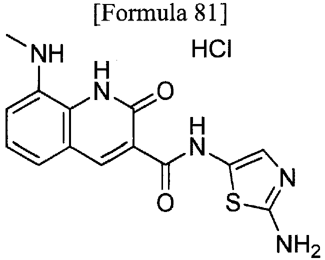

To the tert-butyl (5-[[8-(methylamino)-2-oxo-1,2-dihydroquinoline-3-carbonyl]amino]-1,3-thiazol-2-yl)carbamate (34 mg) obtained in Example 24, 4 M hydrochloric acid in ethyl acetate solution (6 mL) and 2 M hydrochloric acid in methanol solution (6 mL) were added and the mixture was stirred under heating for 6 hours at 50°C. The reaction mixture was concentrated under reduced pressure to obtain the titled compound (29 mg) as a brown solid.
MS (ESI pos.) m/z : 316 [M+H]+, RT=0.568 min (Analytical Condition 3)
¹H NMR (600 MHz, DMSO-d6) δ ppm 2.85 (s, 3 H), 6.85 (dd, J=7.6, 1.4 Hz, 1 H), 7.08 (s, 1 H), 7.16 (s, 1 H), 7.19 - 7.27 (m, 2 H), 7.53 (s, 1 H), 8.87 (s, 1 H), 9.00 (br s, 2 H), 11.83 (s, 1 H), 12.67 (s, 1 H)

The same procedures as in Example 25 were employed to obtain the compound of Example 31 from the corresponding raw material.

**[Table 3]**

| Example | Structural Formula | Compound Name | MS | NMR |
|---|---|---|---|---|
| 25 | 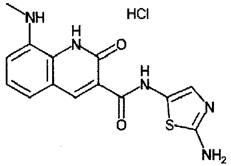 | N-(2-amino-1,3-thiazole-5-yl)-8-(methylamino)-2-oxo-1,2-dihydroquinoline-3-carboxamide-hydrochloride | MS (ESI pos.) m/z : 316 [M+H]+ RT=0.568 min (Analytical Condition 3) | 1H NMR (600 MHz, DMSO-d6) δ ppm 2.85 (s, 3 H), 6.85 (dd, J=7.6, 1.4 Hz, 1 H), 7.08 (s, 1 H), 7.16 (s, 1 H), 7.19 - 7.27 (m, 2 H), 7.53 (s, 1 H), 8.87 (s, 1 H), 9.00 (br s, 2 H), 11.83 (s, 1 H), 12.67 (s, 1 H) |
| 31 | 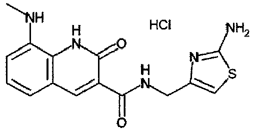 | N-[(2-amino-1,3-thiazole-4-yl)methyl]-8-(methylamino)-2-oxo-1,2-dihydroquinoline-3-carboxamide-hydrochloride | MS (ESI pos.) m/z : 330 [M+H]+ RT=0.322 min (Analytical Condition 3) | 1H NMR (600 MHz, DMSO-d6) δ ppm 2.84 (s, 3 H), 4.45 (d, J=5.8 Hz, 2 H), 6.69 (s, 1 H), 6.80 (dd, J=6.8, 2.3 Hz, 1 H), 7.11 - 7.26 (m, 2 H), 8.77 (s, 1 H), 9.17 (br s, 2 H), 10.15 (t, J=5.8 Hz, 1 H), 11.61 (s, 1 H) |

### Example 33 4-[[6-Fluoro-8-(methylamino)-2-oxo-1,2-dihydroquinoline-3-carbonyl]amino]benzoic acid

### 33-(1) Methyl 4-[[6-fluoro-8-(methylamino)-2-oxo-1,2-dihydroquinoline-3-carbonyl] amino]benzoate

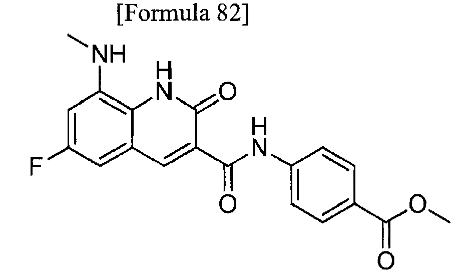

To a solution in DMF (5 mL) of methyl 4-aminobenzoate (39 mg), HATU (101 mg), and the 6-fluoro-8-(methylamino)-2-oxo-1,2-dihydroquinoline-3-carboxylic acid (50 mg) obtained in Production Example 2-(5), DIPEA (45 µL) was added and the mixture was stirred for 19.5 hours at room temperature. To the reaction mixture, ethyl acetate and saturated aqueous sodium bicarbonate were added and a liquid-liquid separating operation was performed; the precipitating solid was recovered by filtration to obtain the titled compound (20 mg) as a yellow solid.
MS (ESI pos.) m/z : 370 [M+H]+, RT=1.084 min (Analytical Condition 3)

### 33-(2) 4-[[6-Fluoro-8-(methylamino)-2-oxo-1,2-dihydroquinoline-3-carbonyl]amino]benzoic acid

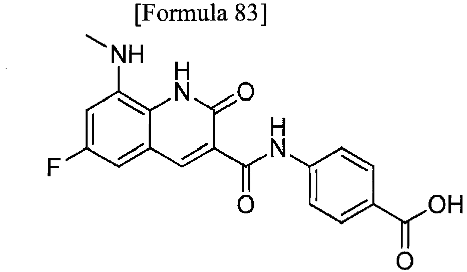

To a solution in THF (2 mL) of the methyl 4-[[6-fluoro-8-(methylamino)-2-oxo-1,2-dihydroquinoline-3-carbonyl]amino]benzoate (20 mg) obtained in Example 33-(1), 1 M sodium hydroxide in aqueous solution (2 mL) and ethanol (200 µL) were added and the mixture was stirred for 4.5 hours at room temperature. Following further addition of 1 M sodium hydroxide in aqueous solution (1 mL), the mixture was stirred for 18 hours at room temperature. The reaction mixture was neutralized (pH 6-7) with 1 M hydrochloric acid and saturated aqueous sodium bicarbonate; the precipitate was recovered by filtration to obtain the titled compound (13 mg) as a brown solid.
MS (ESI neg.) m/z : 354 [M-H]-, RT=0.939 min (Analytical Condition 3)
¹H NMR (600 MHz, DMSO-d6) δ ppm 2.86 (d, J=4.5 Hz, 3 H), 6.54 (br d, J=4.5 Hz, 1 H), 6.63 (dd, J=11.6, 2.5 Hz, 1 H), 7.06 (dd, J=8.7, 2.5 Hz, 1 H), 7.83 (d, J=8.7 Hz, 2 H), 7.97 (d, J=8.7 Hz, 2 H), 8.87 (s, 1 H), 12.53 (br s, 1 H)

The same procedures as in Example 33 were employed to obtain the Example compounds identified below from the corresponding raw materials.

**[Table 4-1]**

| Example | Structural Formula | Compound Name | MS | NMR |
|---|---|---|---|---|
| 33 | 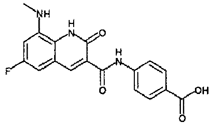 | 4-{[6-fluoro-8-(methylamino)-2-oxo-1,2-dihydroquinoline-3-carbonyl]amino} benzoic acid | MS (ESI neg.) m/z : 354 [M- H]-RT=0.939 min (Analytical Condition 3) | 1H NMR (600 MHz, DMSO-d6) δ ppm 2.86 (d, J=4.5 Hz, 3 H), 6.54 (br d, J=4.5 Hz, 1 H), 6.63 (dd, J=11.6, 2.5 Hz, 1 H), 7.06 (dd, J=8.7, 2.5 Hz, 1 H), 7.83 (d, J=8.7 Hz, 2 H), 7.97 (d, J=8.7 Hz, 2 H), 8.87 (s, 1 H), 12.53 (br s, 1 H) |
| 54 | 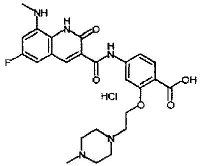 | 4-{[6-fluoro-8-(methylamino)-2-oxo-1,2-dihydroquinoline-3-carbonyl]amino}-2-[2-(4-methylpiperazine-1-yl)ethoxy]benzoic acid-hydrochloride | MS (ESI pos.) m/z : 498 [M+H]+ RT=0.546 min (Analytical Condition 3) | 1H NMR (400 MHz, DMSO-d6) δ ppm 2.73 - 2.92 (m, 12 H), 2.95 - 3.19 (m, 4 H), 4.29 (br s, 2 H), 6.47 - 6.73 (m, 1 H), 7.03 - 7.11 (m, 1 H), 7.31 (dd, J=8.4, 1.7 Hz, 1 H), 7.69 (d, J=1.7 Hz, 1 H), 7.78 (d, J=8.4 Hz, 1 H), 8.86 (s, 1 H), 11.87 (br s, 1 H), 12.33 (br s, 2 H) |
| 56 | 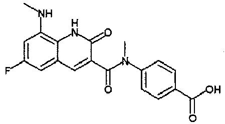 | 4-{[6-fluoro-8-(methylamino)-2-oxo-1,2-dihydroquinoline-3-carbonyl](methyl) amino}benzoic acid | MS (ESI pos.) m/z : 370 [M+H]+ RT=0.712 min (Analytical Condition 3) | 1H NMR (600 MHz, DMSO-d6) δ ppm 2.76 (br s, 3 H), 3.37 (s, 3 H), 6.18 (br s, 1 H), 6.46 (br d, J=11.1 Hz, 1 H), 6.58 - 6.79 (m, 1 H), 7.39 (br s, 2 H), 7.68 - 8.03 (m, 3 H), 11.01 (br s, 1 H), 12.94 (br s, 1 H) |
| 58 | 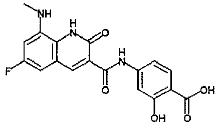 | 4-{[6-fluoro-8-(methylamino)-2-oxo-1,2-dihydroquinoline-3-carbonyl]amino}-2-hydroxybenzoic acid | NT | 1H NMR (400 MHz, DMSO-d6) δ ppm 2.86 (d, J=4.5 Hz, 3 H), 6.53 (brs, 1 H), 6.58 - 6.71 (m, 1 H), 7.01 - 7.12 (m, 2 H), 7.54 (s, 1 H), 7.78 (d, J=8.4 Hz, 1 H), 8.88 (s, 1 H), 11.88 (s, 1 H), 12.34 (br s, 1 H) |
| 66 | 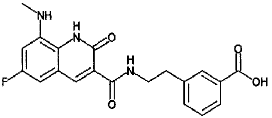 | 3-(2-{[6-fluoro-8-(methylamino)-2-oxo-1,2-dihydroquinoline-3-carbonyl]amino)ethyl) benzoic acid | MS (ESI pos.) m/z: 384 [M+H]+ RT=0.840 min (Analytical Condition 3) | 1H NMR (600 MHz, DMSO-d6) δ ppm 2.83 (d, J=4.5 Hz, 3 H), 2.92 (t, J=7.2 Hz, 2 H), 3.61 (q, J=7.2 Hz, 2 H), 6.44 (br d, J=4.5 Hz, 1 H), 6.59 (dd, J=12.0, 2.5 Hz, 1 H), 6.99 (dd, J=8.9, 2.5 Hz, 1 H), 7.42 (t, J=7.6 Hz, 1 H), 7.52 (br d, J=7.6 Hz, 1 H), 7.79 (d, J=7.6 Hz, 1 H), 7.85 (s, 1 H), 8.72 (s, 1 H), 9.83 (brs, 1 H), 11.63 (br s, 1 H), 12.91 (br s, 1 H) |
| 70 | 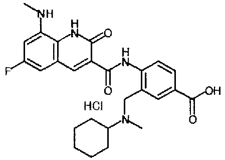 | 3-{[cyclohexyl(methyl) fluoro-8-(methylamino)-2-oxo-1,2-dihydroquinoline-3-carbonyl]amino}bezoic acid-hydrochloride | amino]methyl}-4-{[6-MS (ESI pos.) m/z:481 [M+H]+ RT=0.666 min (Analytical Condition 3) | 1H NMR (400 MHz, DMSO-d6) δ ppm 1.07 -1.42 (m, 4 H), 1.46 -1.71 (m, 2 H), 1.76 -1.94 (m, 2 H), 2.05 (br s, 1 H), 2.15 (br s, 1 H), 2.62 (br s, 3 H), 2.87 (d, J=4.4 Hz, 3 H), 3.48 (br s, 1 H), 4.41 (br s, 1 H), 4.54 - 4.67 (m, 1 H), 6.62 - 6.75 (m, 2 H), 7.11 (br d, J=7.6 Hz, 1 H), 8.08 (br d, J=7.6 Hz, 1 H), 8.27 (br s, 1 H), 8.43 (d, J=8.6 Hz, 1 H), 8.95 (s, 1 H), 9.37 (br s, 1 H), 11.99 (br s, 1 H), 12.49 (brs, 1 H), 13.08 (brs, 1 H) |
| 74 | 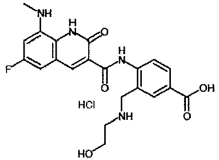 | 4-{[6-fluoro-8-(methylamino)-2-oxo-1,2-dihydroquinoline-3-carbonyl]amino}-3-{[(2-hydroxyethyl)amino] methyl}benzoic acid-hydrochloride | MS (ESI pos.) m/z : 429 [M+H]+ RT=0.638 min (Analytical Condition 3) | 1H NMR (400 MHz, DMSO-d6) δ ppm 2.78 - 2.86 (m, 3 H), 3.11 - 3.21 (m, 2 H), 3.43 (q, J=6.0 Hz, 2 H), 4.55 - 4.77 (m, 3 H), 6.01 (s, 2 H), 6.33 (br d, J=4.0 Hz, 1 H), 6.52 (dd, J=11.7, 2.6 Hz, 1 H), 6.69 (d, J=8.4 Hz, 1 H), 6.79 (dd, J=8.4, 2.6 Hz, 1 H), 7.63 (dd, J=8.4, 2.6 Hz, 1 H), 7.78 (s, 1 H), 7.88 (s, 1 H), 11.30 (br s, 1 H), 12.02 (br s, 1 H) |
| 83 | 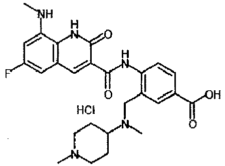 | 4-{[6-fluoro-8-(methylamino)-2-oxo-1,2-dihydroquinoline-3-carbonyl]amino}-3-{[methyl(1-methylpiperidine-4-yl)amino]methyl} benzoic acid-hydrochloride | MS (ESI pos.) m/z : 496 [M+H]+ RT=0.345 min (Analytical Condition 3) | 1H NMR (400 MHz, DMSO-d6) δ ppm 1.78 - 2.02 (m, 4 H), 2.15 (s, 3 H), 2.69 (s, 3 H), 2.72 - 2.79 (m, 2 H), 2.81 - 2.94 (m, 6 H), 3.75 (s, 2 H), 6.63 (dd, J=11.9, 2.6 Hz, 1 H), 6.68 (br d, J=4.5 Hz, 1 H), 7.05 (dd, J=8.8, 2.6 Hz, 1 H), 7.90 (d, J=8.7 Hz, 1 H), 7.93 (s, 1 H), 8.43 (d, J=8.7 Hz, 1 H), 8.86 (s, 1 H), 11.85 (br s, 1 H), 12.23 (brs, 1 H), 12.73 (brs, 1 H) |

**[Table 4-2]**

| Example | Structural Formula | Compound Name | MS | NMR |
|---|---|---|---|---|
| 85 | 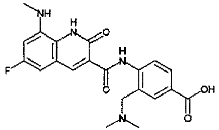 | 3-[(dimethylamino) methyl]-4-{[6-fluoro-8-(methylamino)-2-oxo-1,2-dihydroquinoline-3-carbonyl]amino} benzoic acid | MS (ESI pos.) m/z: 413 [M+H]+ RT=0.532 min (Analytical Condition 3) | 1H NMR (400 MHz, DMSO-d6) δ ppm 2.21 (s, 6 H), 2.85 (d, J=4.4 Hz, 3 H), 3.53 (s, 2 H), 6.53 (br d, J=4.4 Hz, 1 H), 6.61 (dd, J=11.8, 2.6 Hz, 1 H), 7.03 (dd, J=8.8, 2.6 Hz, 1 H), 7.83 (s, 1 H), 7.89 (d, J=8.6 Hz, 1 H), 8.52 (d, J=8.6 Hz, 1 H), 8.86 (s, 1 H), 12.41 (brs, 1 H) |
| 93 | 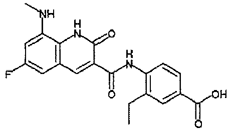 | 3-ethyl-4-{[6-fluoro-8-(methylamino)-2-oxo-1,2-dihydroquinoline-3-carbonyl]amino} benzoic acid | MS (ESI pos.) m/z : 384 [M+H]+ RT=1.011 min (Analytical Condition 3) | 1H NMR (400 MHz, DMSO-d6) δ ppm 1.26 (t, J=7.5 Hz, 3 H), 2.82 (q, J=7.5 Hz, 2 H), 2.86 (d, J=4.5 Hz, 3 H), 6.43 (br d, J=4.5 Hz, 1 H), 6.65 (dd, J=11.8, 2.5 Hz, 1 H), 7.09 (dd, J=8.7, 2.5 Hz, 1 H), 7.81 - 7.88 (m, 2 H), 8.54 (d, J=8.3 Hz, 1 H), 8.92 (s, 1 H), 12.03 (br s, 1 H), 12.52 (s, 1 H), 12.73 (br s, 1 H) |
| 96 | 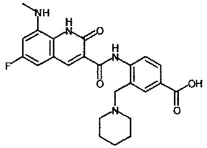 | 4-{[6-fluoro-8-(methylamino)-2-oxo-1,2-dihydroquinoline-3-carbonyl]amino}-3-[(piperidine-1-yl)methyl]benzoic acid | MS (ESI pos.) m/z : 453 [M+H]+ RT=0.609 min (Analytical Condition 3) | 1H NMR (400 MHz, DMSO-d6) δ ppm 1.38 (brs, 2 H), 1.46 - 1.55 (m, 4 H), 2.30 - 2.45 (m, 4 H), 2.85 (d, J=4.5 Hz, 3 H), 3.56 (s, 2 H), 6.55 (br d, J=4.5 Hz, 1 H), 6.63 (dd, J=11.7, 2.6 Hz, 1 H), 7.04 (dd, J=8.8, 2.6 Hz, 1 H), 7.83 (s, 1 H), 7.90 (d, J=8.6 Hz, 1 H), 8.50 (d, J=8.6 Hz, 1 H), 8.85 (s, 1 H), 11.69 (brs, 1 H), 12.22 (br s, 1 H), 12.76 (brs, 1 H) |
| 97 | 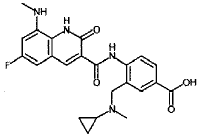 | 3-{[cyclopropyl(methyl) amino]methyl}-4-{[6-fluoro-8-(methylamino)-2-oxo-1,2-dihydroquinoline-3-carbonyl]amino} benzoic acid | MS (ESI pos.) m/z : 439 [M+H]+ RT=0.595 min (Analytical Condition 3) | 1H NMR (400 MHz, DMSO-d6) δ ppm 0.22 - 0.33 (m, 4 H), 1.69 - 1.81 (m, 1 H), 2.27 - 2.36 (m, 3 H), 2.85 (d, J=4.5 Hz, 3 H), 3.77 (s, 2 H), 6.51 (d, J=4.5 Hz, 1 H), 6.64 (dd, J=11.8, 2.3 Hz, 1 H), 7.05 (dd, J=8.7, 2.3 Hz, 1 H), 7.86 - 7.93 (m, 2 H), 8.36 (d, J=9.0 Hz, 1 H), 8.85 (s, 1 H), 11.79 (s, 1 H), 12.09 (s, 1 H), 12.67 (s, 1 H) |
| 98 | 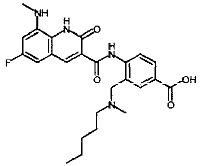 | 4-{[6-fluoro-8-(methylamino)-2-oxo-1,2-dihydroquinoine-3-carbonyl]amino}-3-{[methyl(pentyl)amino]methyl}benzoic acid | MS (ESI pos.) m/z : 469 [M+H]+ RT=0.681 min (Analytical Condition 3) | 1H NMR (400 MHz, DMSO-d6) δ ppm 0.70 (t, J=7.1 Hz, 3 H), 1.06 - 1.20 (m, 4 H), 1.34 - 1.45 (m, 2 H), 2.22 (s, 3 H), 2.31 (t, J=7.1 Hz, 2 H), 2.85 (d, J=4.5 Hz, 3 H), 3.61 (s, 2 H), 6.50 (br d, J=4.5 Hz, 1 H), 6.62 (dd, J=11.8, 2.5 Hz, 1 H), 7.04 (dd, J=8.7, 2.5 Hz, 1 H), 7.87 (s, 1 H), 7.89 (d, J=8.6 Hz, 1 H), 8.48 (d, J=8.6 Hz, 1 H), 8.85 (s, 1 H), 11.79 (br s, 1 H), 12.27 (brs, 1 H), 12.74 (s, 1 H) |
| 99 | 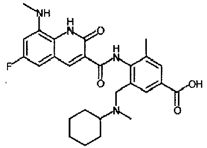 | 3-{[cyclohexyl(methyl)amino]methyl}-{[6-fluoro-8-(methylamino)-2-oxo-1,2-dihydroquinoline-3-carbonyl]amino}-5-methylbenzoic acid | MS (ESI pos.) m/z : 495 [M+H]+ RT=0.680 min (Analytical Condition 3) | 1.13-1.24 (m, 1 H), 1.30 -1.40 (m, 2 H), 1.41 - 1.49 (m, 1 H), 1.51 -1.60 (m, 2 H), 1.63 -1.71 (m, 1 H), 1.83 - 1.91 (m, 2 H), 2.04 - 2.12 (m, 2 H), 2.39 (s, 3 H), 2.77 (br s, 3 H), 2.96 (s, 3 H), 4.31 (br s, 2 H), 6.70 (br d, J=11.6 Hz, 1 H), 6.83 - 6.95 (m, 1H), 7.94 (s, 1 H), 8.03 (s, Hz, 1 H), 8.50 (s, 1 H), 8.90 (s, 1 H), |
| 101 | 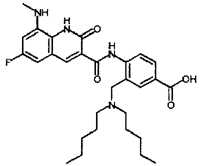 | 3-[(dipentylamino)methyl] -4-{[6-fluoro-8-(methylamino)-2-oxo-1,2-dihydroquinoline-3-carbonyl]amino}benzoic acid | MS (ESI pos) m/z : 525 [M+H]+ RT=0.816 min (Analycal Condition 3) | 1H NMR (400 MHz, DMSO-d6) δ ppm 0.69 - 0.80 (m, 6 H), 1.11 - 1.20 (m, 8 H), 1.40 (br s, 4 H), 2.40 - 2.47 (m, 4 H), 2.85 (d, J=4.4 Hz, 3 H), 3.69 (s, 2 H), 6.49 (br d, J=4.4 Hz, 1 H), 6.61 (dd, J=11.7,2.6 Hz, 1 H), 7.03 (dd, J=8.8, 2.6 Hz, 1 H), 7.87 (dd, J=8.6, 2.0 Hz, 1 H), 7.97 (d, J=2.0 Hz, 1 H), 8.37 (d, J=8.6 Hz, 1 H), 8.82 (s, 1 H), 12.26 (br s, 2 H) |
| 102 | 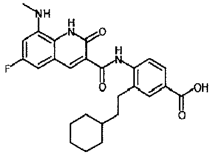 | 3-(2-cyclohexylethyl)-4-{[6-fluoro-8-(methylamino)-2-oxo-1,2-dihydroquinoline-3-carbonyl]amino}benzoic acid | MS (ESI pos.) m/z : 466 [M+H]+ RT=1.318 min (Analytical Condition 3) | 1H NMR (600 MHz, DMSO-d6) δ ppm 1.11 - 1.29 (m, 4 H), 1.30 -1.43 (m, 2 H), 1.47 -1.57 (m, 2 H), 1.60 - 1.65 (m, 1 H), 1.65 -1.71 (m, 2 H), 1.75 -1.84 (m, 2 H), 2.73 - 2.83 (m, 2 H), 2.86 (d, J=4.1 Hz, 3 H), 6.54 (br d, J=4.1 Hz, 1 H), 6.65 (dd, J=11.8, 2.7 Hz, 1 H), 7.08 (dd, J=8.7, 2.7 Hz, 1 H), 7.81 - 7.84 (m, 2 H), 8.51 (d, J=9.1 Hz, 1 H), 8.93 (s, 1 H), 11.94 (s, 1 H), 12.40 (s, 1 H) |

**[Table 4-3]**

| Example | Structural Formula | Compound Name | MS | NMR |
|---|---|---|---|---|
| 103 | 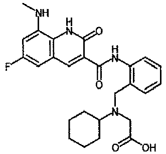 | N-cyclohexyl-N-[(2-{[6-fluoro-8-(methylamino)-2-oxo-1,2-dihydroquinoline-3-carbonyl]amino} phenyl)methyl]glycine | MS (ESI pos.) m/z: 481 [M+H]+ RT=0.761 min (Analytical Condition 3) | 1H NMR (400 MHz, DMSO-d6) δ ppm 0.95 -1.93 (m, 10 H), 2.52 - 2.59 (m, 1 H), 2.85 (d, J=4.4 Hz, 3 H), 3.22 - 3.28 (m, 2 H), 3.90 (s, 2 H), 6.49 (br d, J=4.4 Hz, 1 H), 6.61 (dd, J=11.7, 2.6 Hz, 1 H), 7.02 (dd, J=8.8, 2.6 Hz, 1 H), 7.12 (td, J=7.5, 1.1 Hz, 1 H), 7.29 (t, J=7.5 Hz, 1 H), 7.48 (d, J=7.5 Hz, 1 H), 8.11 (d, J=7.5 Hz, 1 H), 8.78 (s, 1 H), 11.70 (br s, 1 H), 11.82 (brs, 1 H) |
| 104 | 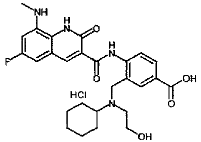 | 3-{[cydohexyl(2-hydroxyethyl)amino] methyl}-4-{[6-fluoro-8-(methylamino)-2-oxo-1,2-dihydroquinoline-3-carbonyl]amino} benzoic acid-hydrochloride | MS (ESI pos.) m/z: 511 [M+H]+ RT=0.645min (Analytical Condition 3) | 1H NMR (400 MHz, DMSO-d6) δ ppm 1.18 -2.34 (m, 10 H), 2.86 (br d, J=3.3 Hz, 3 H), 3.02 (br s, 1 H), 3.38 - 3.77 (m, 4 H), 4.38 - 4.64 (m, 2 H), 6.62 - 6.71 (m, 2 H), 7.10 (br d, J=8.1 Hz, 1 H), 8.04 - 8.11 (m, 1 H), 8.25 - 8.37 (m, 2 H), 8.93 (s, 1 H), 9.28 (br s, 1 H), 11.99 (br s, 1 H), 12.42 (br s, 1 H), 13.13 (brs, 1 H) |
| 105 | 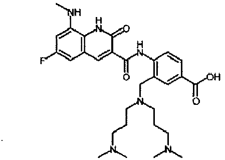 | 3-({bis[3-(dimethylamino)propyl] amino}methyl)-4-{[6-fluoro-8-(methylamino)-2-oxo-1,2-dihydroquinoline-3-carbonyl]amino} benzoic acid | MS (ESI pos.) m/z : 555 [M+H]+ RT=0.223 min (Analytical Condition 3) | NT |
| 106 | 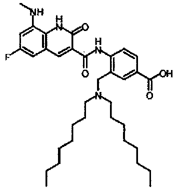 | 3-[(dioctylamino)methyl]-4-{[6-fluoro-8-(methylamino)-2-oxo-1,2-dihydroquinoline-3-carbonyl]amino} benzoic acid | MS (ESI pos.) m/z : 609 [M+H]+ RT=1.036 min (Analytical Condition 3) | 1H NMR (400 MHz, DMSO-d6) δ ppm 0.77 (t, J=6.8 Hz, 6 H), 1.04 - 1.43 (m, 24 H), 2.35 - 2.45 (m, 4 H), 2.85 (d, J=4.5 Hz, 3 H), 3.67 (s, 2 H), 6.45 (br s, 1 H), 6.53 (br d, J=12.7 Hz, 1 H), 6.94 (br d, J=10.0 Hz, 1 H), 7.83 (br d, J=8.6 Hz, 1 H), 7.94 (s, 1 H), 8.33 (br d, J=8.6 Hz, 1 H), 8.74 (s, 1 H) |
| 107 | 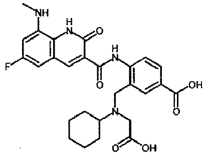 | 3-{[(carboxymethyl) methyl}-4-{[6-fluoro-8-(methylamino)-2-oxo-1,2-dihydroquinoline-3-carbonyl]amino} | MS (ESI pos.) m/z : 525 [M+H]+ RT=0.566 min (Analytical Condition 3) | 1H NMR (400 MHz, DMSO-d6) δ ppm 0.95 - 1.95 (m, 10 H), 2.54 - 2.61 (m, 1 H), 2.85 (d, J=4.5 Hz, 3 H), 3.29 (s, 2 H), 3.96 (s, 2 H), 6.50 (d, J=4.5 Hz, 1 H), 6.63 (dd, J=11.8, 2.5 Hz, 1 H), 7.04 (dd, J=8.8, 2.5 Hz, 1 H), 7.87 (dd, J=8.5, 1.9 Hz, 1 H), 8.07 (d, J=1.9 Hz, 1 H), 8.37 (d, J=8.5 Hz, 1 H), 8.80 (s, 1 H), 11.76 (br s, 1 H), 12.13 (br s, 1 H) |
| 111 | 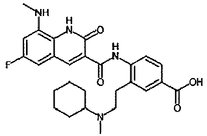 | 3-{2-[cyclohexyl(methyl) amino]ethyl}-4-{[6-fluoro-8-(methylamino)-2-oxo-1,2-dihydroquinoline-3-carbonyl]amino} benzoic acid | MS (ESI pos.) m/z : 495 [M+H]+ RT=0.694 min (Analytical Condition 3) | 1H NMR (600 MHz, PYRIDINE-d5) δ ppm 1.05 -1.20 (m, 5 H), 1.42 - 1.48 (m, 1 H), 1.59 - 1.64 (m, 3 H), 1.74 - 1.79 (m, 2 H), 2.42 (s, 3 H), 2.88 (s, 3 H), 2.89 - 2.93 (m, 2 H), 3.17-3.22 (m, 2 H), 6.47 (brs, 1 H), 6.74 (dd, J=11.4, 2.7 Hz, 1 H), 6.99 (dd, J=8.5, 2.7 Hz, 1 H), 8.50 (dd, J=8.3, 1.9 Hz, 1 H), 8.53 (d, J=1.9 Hz, 1 H), 9.18 (d, J=8.3 Hz, 1 H), 9.25 (s, 1 H) |
| 115 | 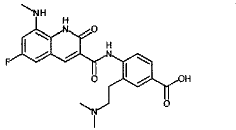 | 3-[2-(dimethylamino)ethyl]-4-{[6-fluoro-8-(methylamino)-2-oxo-1,2-dihydroquinoline-3-carbonyl]amino} benzoic acid | MS (ESI pos.) m/z : 427 [M+H]+ RT=0.586 min (Analytical Condition 3) | 1H NMR (400 MHz, DMSO-d6) δ ppm 2.62 (br s, 6 H), 2.86 (d, J=4.4 Hz, 3 H), 2.96 - 3.13 (m, 4 H), 6.61 - 6.65 (m, 1 H), 6.65 - 6.69 (m, 1 H), 7.08 (dd, J=8.7, 2.6 Hz, 1 H), 7.85 - 7.93 (m, 2 H), 8.48 (d, J=8.6 Hz, 1 H), 8.92 (s, 1 H), 12.47 (brs, 1 H) |
| 116 | 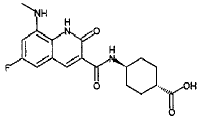 | trans-4-{[6-fluoro-8-(methylamino)-2-oxo-1,2-dihydroquinoline-3-carbonyl]amino} cyclohexane-1-carboxylic acid | MS (ESI pos.) m/z : 362 [M+H]+ RT=0.787 min (Analytical Condition 3) | 1H NMR (600 MHz, DMSO-d6) δ ppm 1.26 -1.36 (m, 2 H), 1.38 - 1.50 (m, 2 H), 1.89 - 2.02 (m, 4 H), 2.19 - 2.28 (m, 1 H), 2.83 (d, J=4.5 Hz, 3 H), 3.69 - 3.80 (m, 1 H), 6.49 (br d, J=4.5 Hz, 1 H), 6.59 (dd, J=11.6, 2.5 Hz, 1 H), 7.00 (dd, J=8.7, 2.5 Hz, 1 H), 8.72 (s, 1 H), 9.73 (br d, J=7.8 Hz,1 H), 11.59 (br s, 1 H), 12.04 (br |

### Example 35 N-(2-[[(2-Amino-1,3-thiazol-4-yl)methyl]amino]pyrimidin-5-yl)-8-(methylamino)-2-oxo-1,2-dihydroquinoline-3-carboxamide

### 35-(1) tert-Butyl (4-[[(5-[[8-(methylamino)-2-oxo-1,2-dihydroquinoline-3-carbonyl]amino]pyrimidin-2-yl)amino]methyl]-1,3-thiazol-2-yl)carbamate

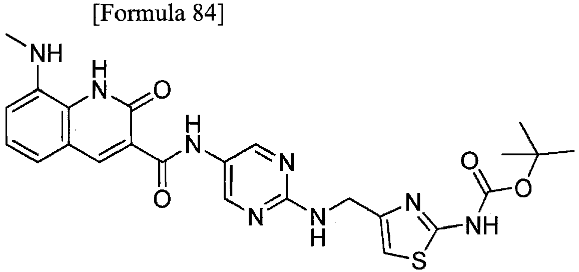

To a solution in DMF (2.5 mL) of tert-butyl (4-[[(5-aminopyrimidin-2-yl)amino]methyl]-1,3-thiazol-2-yl)carbamate (78 mg), HATU (105 mg), and the 8-(methylamino)-2-oxo-1,2-dihydroquinoline-3-carboxylic acid (50 mg) obtained in Production Example 1-(2), DIPEA (140 µL) was added and the mixture was stirred for 17 hours at room temperature. The precipitating solid was recovered by filtration and thereafter washed with water to obtain the titled compound (26 mg) as a yellow solid.
MS (ESI pos.) m/z : 523 [M+H]+, RT=0.989 min (Analytical Condition 3)

### 35-(2) N-(2-[[(2-Amino-1,3-thiazol-4-yl)methyl]amino]pyrimidin-5-yl)-8-(methylamino)-2-oxo-1,2-dihydroquinoline-3-carboxamide

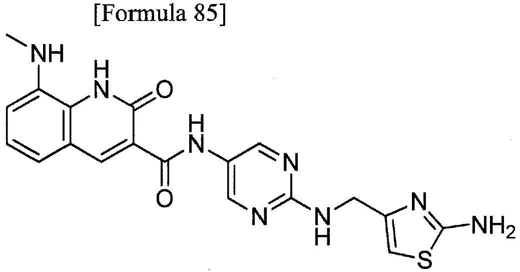

A solution in TFA (5 mL) of the tert-butyl (4-[[(5-[[8-(methylamino)-2-oxo-1,2-dihydroquinoline-3-carbonyl]amino]pyrimidin-2-yl)amino]methyl]-1,3-thiazol-2-yl)carbamate (26 mg) obtained in Example 35-(1) was stirred for half an hour at room temperature. To the reaction mixture, ethyl acetate and a saturated aqueous solution of sodium hydrogencarbonate were added; the precipitating solid was recovered by filtration and thereafter washed with ethyl acetate, dimethylformamide, methanol and ethanol, whereupon the titled compound (4 mg) was obtained as a yellow solid.
MS (ESI pos.) m/z : 423 [M+H]+, RT=0.497 min (Analytical Condition 3)
¹H NMR (600 MHz, DMSO-d6) δ ppm 2.86 (d, J=4.5 Hz, 3 H), 4.31 (d, J=5.4 Hz, 2 H), 6.16 (br d, J=4.5 Hz, 1 H), 6.18 (s, 1 H), 6.82 (d, J=6.6 Hz, 1 H), 6.85 (s, 2 H), 7.16 - 7.27 (m, 2 H), 7.37 (t, J=6.6 Hz, 1 H), 8.61 (s, 2 H), 8.86 (s, 1 H), 11.69 (br s, 2 H)

The same procedures as in Example 35 were employed to obtain the Example compounds identified below from the corresponding raw materials.

**[Table 5]**

| Example' | Structural Formula | Compound Name | MS | NMR |
|---|---|---|---|---|
| 35 | 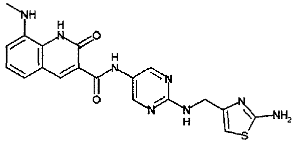 | N-(2-{[(2-amino-1,3-thiazole-4-yl)methyl] amino}pyrimidine-5-yl)-8-(methylamino)-2-oxo-1,2-dihydroquinoline-3-carboxamide | MS (ESI pos.) m/z: 423 [M+H]+ RT=0.497 min (Analytical Condition 3) | 1H NMR (600 MHz, DMSO-d6) δ ppm 2.86 (d, J=4.5 Hz, 3 H), 4.31 (d, J=5.4 Hz, 2 H), 6.16 (br d, J=4.5 Hz, 1 H), 6.18 (s, 1 H), 6.82 (d, J=6.6 Hz, 1 H), 6.85 (s, 2 H), 7.16 - 7.27 (m, 2 H), 7.37 (t, J=6.6 Hz, 1 H), 8.61 (s, 2 H), 8.86 (s, 1 H), 11.69 (br s, 2 H) |
| 45 | 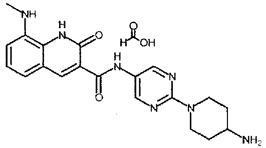 | N-[2-(4-aminopiperidine-1-yl)pydmidine-5-yl]-8-(methylamino)-2-oxo-1,2-dihydroquinoline-3-carboxamide-formate | MS (ESI pos.) m/z: 394 [M+H]+ RT=0.458 min (Analytical Condition 3) | 1H NMR (600 MHz, DMSO-d6) δ ppm 1.25 - 1.35 (m, 2 H), 1.79 - 1.92 (m, 2 H), 2.86 (d, J=4.5 Hz, 3 H), 2.95 - 3.05 (m, 3 H), 4.52 - 4.57 (m, 2 H), 6.17 (br d, J=4.5 Hz, 1 H), 6.78 (d, J=7.4 Hz, 1 H), 7.11 - 7.21 (m, 2 H), 8.38 (s, 1 H), 8.70 (s, 2 H), 8.82 (s, 1 H) |
| 46 | 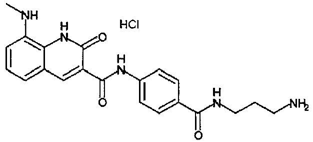 | N-{4-[(3-aminopropyl) carbamoyl]phenyl}-8-(methylamino )-2-oxo-1,2-dihydroquinoline-3-carboxamide-hydrochloride | MS (ESI pos.) m/z : 394 [M+H]+ RT=0.480 min (Analytical Condition 3) | 1H NMR (600 MHz, DMSO-d6) δ ppm 1.77 - 1.85 (m, 2 H), 2.76 - 2.97 (m, 7 H), 6.21 (br d, J=4.5 Hz, 1 H), 6.84 (d, J=7.6 Hz, 1 H), 7.18 - 7.28 (m, 2 H), 7.83 (d, J=8.7 Hz, 2 H), 7.90 (d, J=8.7 Hz, 2 H), 8.60 (t, J=6.0 Hz, 1 H), 8.90 (s, 1 H), 12.36 (s, 1 H) |
| 47 | 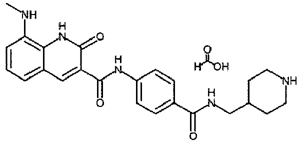 | 8-(methylamino)-2-oxo-N-(4-{[(piperidine-4-yl)methyl]carbamoyl} phenyl)-1,2-dihydroquinoline-3-carboxamide-formate | MS (ESI pos.) m/z: 434 [M+H]+ RT=0.503 min (Analytical Condition 3) | 1H NMR (600 MHz, DMSO-d6) δ ppm 1.21 - 1.31 (m, 2 H), 1.71 - 1.80 (m, 3 H), 2.64 - 2.76 (m, 2 H), 2.86 (br s, 3 H), 3.08 - 3.25 (m, 4 H), 6.21 (br s, 1 H), 6.79 (d, J=7.4 Hz, 1 H), 7.14 - 7.22 (m, 2 H), 7.81 (d, J=8.7 Hz, 2 H), 7.88 (d, J=8.7 Hz, 2 H), 8.38 (s, 1 H), 8.47 (br t, J=5.8 Hz, 1 H), 8.86 (s, 1 H), 12.58 (br s, 1 H) |
| 49 | 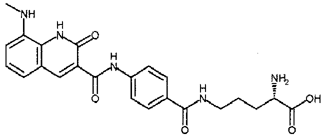 | N5-(4-{[8-(methylamino)-2-oxo-1,2-dihydroquinoline-3-carbonyl]amino} benzoyl)-L-omithine | MS (ESI pos.) m/z: 452 [M+H]+ RT=0.597 min (Analytical Condition 3) | 1H NMR (600 MHz, DMSO-d6) δ ppm 1.40 - 3.19 (m, 10 H), 6.18 (d, J=4.5 Hz, 1 H), 6.82 (br d, J=7.4 Hz, 1 H), 7.24 (s, 2 H), 7.81 (d, J=8.7 Hz, 2 H), 7.91 (d, J=8.7 Hz, 2 H), 8.68 - 8.76 (m, 1 H), 8.90 (s, 1 H) |

### Example 39 4-Amino-1-(5-[[8-(methylamino)-2-oxo-1,2-dihydroquinoline-3-carbonyl]amino]pyrimidin-2-yl)piperidine-4-carboxylic acid-hydrochloride

### 39-(1) Methyl 4-[(tert-butoxycarbonyl)amino]-1-(5-[[8-(methylamino)-2-oxo-1,2-dihydroquinoline-3-carbonyl]amino]pyrimidin-2-yl)piperidine-4-carboxylate

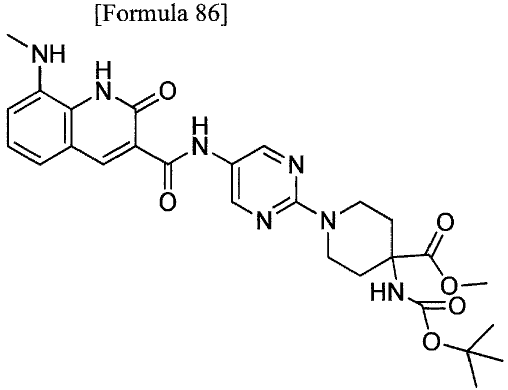

To a solution in DMF (2.5 mL) of methyl 1-(5-aminopyrimidin-2-yl)-4-[(tert-butoxycarbonyl)amino]piperidine-4-carboxylate (45 mg) and the 8-(methylamino)-2-oxo-1,2-dihydroquinoline-3-carboxylic acid (23 mg) obtained in Production Example 1-(2), HATU (48 mg) and DIPEA (64 µL) were added and the mixture was stirred for 17 hours at room temperature. The reaction mixture was concentrated under reduced pressure to obtain the titled compound as a crude product.
MS (ESI pos.) m/z : 552 [M+H]+, RT=1.060 min (Analytical Condition 3)

### 39-(2) 4-[(tert-Butoxycarbonyl)amino]-1-(5-[[8-(methylamino)-2-oxo-1,2-dihydroquinoline-3 -carbonyl] amino]pyrimidin-2-yl)piperidine-4-carboxylic acid

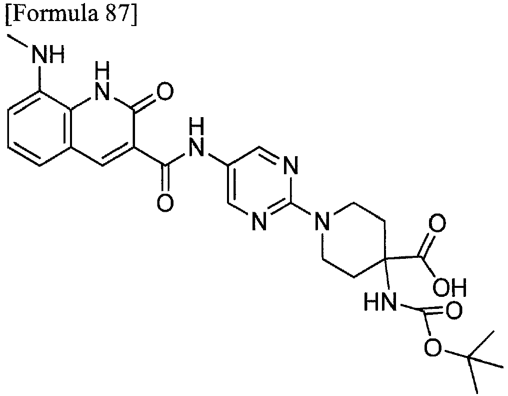

To a solution in tetrahydrofuran (1 mL) of the methyl 4-[(tert-butoxycarbonyl)amino]-1-(5-[[8-(methylamino)-2-oxo-1,2-dihydroquinoline-3-carbonyl]amino]pyrimidin-2-yl)piperidine-4-carboxylate obtained in Example 39-(1), 1 M sodium hydroxide in aqueous solution (1 mL) was added and the mixture was stirred for 52 hours at room temperature. To the reaction mixture, an aqueous solution of 1 M hydrochloric acid was added to establish pH 6 and the insoluble matter was removed by filtration. The filtrate was concentrated under reduced pressure and the residue was purified by reversed phase column chromatography to obtain the titled compound as a yellow solid.
MS (ESI pos.) m/z : 538 [M+H]+, RT=0.944 min (Analytical Condition 3)

### 39-(3) 4-Amino-1-(5-[[8-(methylamino)-2-oxo-1,2-dihydroquinoline-3-carbonyl]amino]pyrimidin-2-yl)piperidine-4-carboxylic acid-hydrochloride

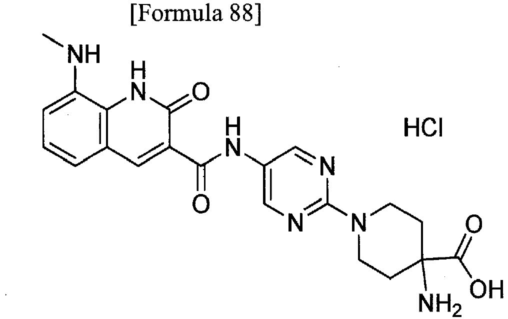

A solution in 4 M hydrochloric acid in ethyl acetate solution (8 mL) of the 4-[(tert-butoxycarbonyl)amino]-1-(5-[[8-(methylamino)-2-oxo-1,2-dihydroquinoline-3-carbonyl]amino]pyrimidin-2-yl)piperidine-4-carboxylic acid obtained in Example 39-(2) was stirred for 2 hours at room temperature. The reaction mixture was concentrated under reduced pressure to obtain the titled compound (0.4 mg) as a yellow solid.
MS (ESI pos.) m/z : 438 [M+H]+, RT=0.368 min (Analytical Condition 3)
¹H NMR (600 MHz, DMSO-d6) δ ppm 1.76 - 1.86 (m, 2 H), 2.06 - 2.14 (m, 2 H), 2.86 (s, 3 H), 3.79 - 3.85 (m, 2 H), 4.00 - 4.08 (m, 2 H), 6.21 (br s, 1 H), 6.83 (d, J=7.4 Hz, 1 H), 7.18 - 7.24 (m, 2 H), 8.76 (s, 2 H), 8.87 (s, 1 H), 11.71 (s, 1 H), 11.77 (s, 1 H)

The same procedures as in Example 39 were employed to obtain the compound of Example 48 from the corresponding raw material.

**[Table 6]**

| Example | Structural Formula | Compound Name | MS | NMR |
|---|---|---|---|---|
| 39 | 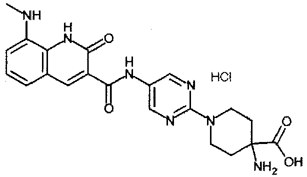 | 4-amino-1-(5-{[8-(methylamino)-2-oxo-1,2-dihydroquinoline-3-carbonyl]amino}pyrimidine-2-yl)pipeddine-4-carboxylyc acid-hydrochloride | MS (ESI pos.) m/z : 438 [M+H]+ RT=0.368 min (Analytical Condition 3) | 1H NMR (600 MHz, DMSO-d6) δ ppm 1.76 -1.86 (m, 2 H), 2.06 - 2.14 (m, 2 H), 2.86 (s, 3 H), 3.79 - 3.85 (m, 2 H), 4.00 - 4.08 (m, 2 H), 6.21 (br s, 1 H), 6.83 (d, J=7.4 Hz, 1 H), 7.18 - 7.24 (m, 2 H), 8.76 (s, 2 H), 8.87 (s, 1 H), 11.71 (s, 1 H), 11.77 (s, 1 H) |
| 48 | 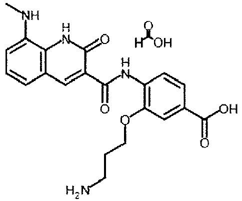 | 3-(3-aminopropoxy)-4-{[8-(methylamino)-2-oxo-1,2-dihydroquinoline-3-carbonyl]amino}benzoicacid-formate | MS : (ESI 411 pos.) [M+H]+ RT=0.553 min (Analytical Condition 3) | 1H NMR (600 MHz, DMSO-d6) δ ppm 2.16 (dt, J=12.5, 6.3 Hz, 2 H), 2.86 (brs, 3 H), 3.18 (br t, J=6.3 Hz, 2 H), 4.24 (t, J=6.3 Hz, 2 H), 6.39 (br s, 1 H), 6.76 (d, J=8.0 Hz, 1 H), 7.16 (t, J=8.0 Hz, 1 H), 7.19 (d, J=8.0 Hz, 1 H), 7.56 - 7.59 (m, 2 H), 7.57 (s, 1 H), 8.38 (s, 1 H), 8.57 (d, J=8.0 Hz, 1 H), 8.86 (s, 1 H), 12.94 (brs, 1 H) |

### Example 40 6-Fluoro-8-(methylamino)-N-(4-[[2-(morpholin-4-yl)ethyl]carbamoyl]phenyl)-2-oxo-1,2-dihydroquinoline-3-carboxamide

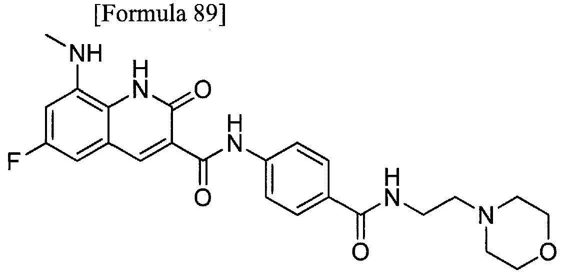

To a solution in DMF (1 mL) of the 6-fluoro-8-(methylamino)-2-oxo-1,2-dihydroquinoline-3-carboxylic acid (39 mg) obtained in Production Example 2-(5), HATU (68 mg), 4-amino-N-[2-(morpholin-4-yl)ethyl]benzamide (49 mg) and DIPEA (285 µL) were added and the mixture was stirred for 18 hours at room temperature. The reaction mixture was purified by preparative LCMS. Upon further purification by preparative TLC, the titled compound (2.4 mg) was obtained as a colorless solid.
MS (ESI pos.) m/z : 468 [M+H]+, RT=0.543 min (Analytical Condition 3)
¹H NMR (600 MHz, DMSO-d6) δ ppm 2.42 - 2.49 (m, 4 H), 2.86 (d, J=4.5 Hz, 3 H), 3.09 - 3.22 (m, 2 H), 3.36 - 3.41 (m, 2 H), 3.56 - 3.60 (m, 4 H), 6.55 (br d, J=4.5 Hz, 1 H), 6.65 (dd, J=11.6, 2.5 Hz, 1 H), 7.07 (dd, J=8.3, 2.5 Hz, 1 H), 7.81 (d, J=8.7 Hz, 2 H), 7.87 (d, J=8.7 Hz, 2 H), 8.35 (t, J=5.6 Hz, 1 H), 8.88 (s, 1 H), 11.86 (br s, 1 H), 12.32 (s, 1 H)

The same procedures as in Example 40 were employed to obtain the Example compounds identified below from the corresponding raw materials.

**[Table 7-1]**

| Example | Structural Formula | Compound Name | MS | NMR |
|---|---|---|---|---|
| 40 | 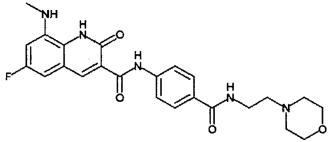 | 6-fluoro-8-(methylamino)-N-(4-{[2-(morpholine-4-yl)ethyl]carbamoyl}phenyl)-2-oxo-1,2-dihydroquinoline-3-carboxamide | MS (ESI pos.) m/z : 468 [M+H]+ RT=0.543 min (Analytical Condition 3) | 1H NMR (600 MHz, DMSO-d6) δ ppm 2.42 - 2.49 (m, 4 H), 2.86 (d, J=4.5 Hz, 3 H), 3.09 - 3.22 (m, 2 H), 3.36 - 3.41 (m, 2 H), 3.56 - 3.60 (m, 4 H), 6.55 (br d, J=4.5 Hz, 1 H), 6.65 (dd, J=11.6, 2.5 Hz, 1 H), 7.07 (dd, J=8.3, 2.5 Hz, 1 H), 7.81 (d, J=8.7 Hz, 2 H), 7.87 (d, J=8.7 Hz, 2 H), 8.35 (t, J=5.6 Hz, 1 H), 8.88 (s, 1 H), 11.86 (br s, 1 H), 12.32 (s, 1 H) |
| 50 | 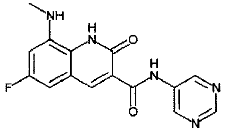 | 6-fluoro-8-(methylamino)-2-oxo-N-(pyrimidine-5-yl)-1,2-dihydroquinoline-3-carboxamide | MS (ESI pos.) m/z: 314 [M+H]+ RT=0.764 min (Analytical Condition 3) | 1H NMR (600 MHz, DMSO-d6) δ ppm 2.86 (d, J=4.5 Hz, 3 H), 6.55 (br d, J=4.5 Hz, 1 H), 6.66 (dd, J=11.8, 2.5 Hz, 1 H), 7.08 (dd, J=8.7, 2.5 Hz, 1 H), 8.91 (s, 1 H), 8.96 (s, 1 H), 9.19 (s, 2 H), 11.88 (brs, 1 H), 12.23 (brs, 1 H) |
| 51 | 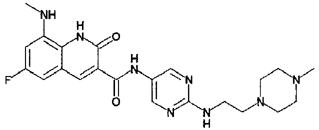 | 6-fluoro-8-(methylamino)-N-(2-{[2-(4-methylpiperazine-1-yl)ethyl]amino} pyrimidine-5-yl)-2-oxo-1,2-dihydroquinoline-3-carboxamide | MS (ESI pos.) m/z. 455 [M+H]+ RT=0.337 min (Analytical Condition 3) | 1H NMR (600 MHz, DMSO-d6) δ ppm 2.26 (s, 3 H), 2.34 - 2.58 (m, 6 H), 2.85 (d, J=4.5 Hz, 3 H), 3.06 - 3.67 (m, 6 H), 6.53 (br d, J=4.5 Hz, 1 H), 6.63 (dd, J=11.6, 2.5 Hz, 1 H), 6.94 (t, J=5.8 Hz, 1 H), 7.04 (dd, J=8.7, 2.5 Hz, 1 H), 8.15 (s, 1 H), 8.59 (s, 2 H), 8.83 (s, 1 H), 11.67 (s, 1 H) |
| 52 | 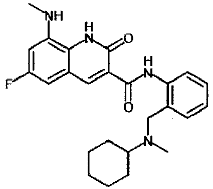 | N-(2-{[cyclohexyl (methyl)amino]methyl} phenyl)-6-fluoro-8-(methylamino)-2-oxo-1,2-dihydroquinoline-3-carboxamide | MS (ESI pos.) m/z: 437 [M+H]+ RT=0.661 min (Analytical Condition 3) | 1H NMR (600 MHz, DMSO-d6) δ ppm 1.11 - 1.18 (m, 1 H), 1.24 - 1.33 (m, 2 H), 1.45 -1.58 (m, 2 H), 1.58 -1.67 (m, 1 H), 1.79 -1.91 (m, 2 H), 2.00 - 2.12 (m, 2 H), 2.64 (s, 3 H), 2.87 (s, 3 H), 3.29 - 3.44 (m, 1 H), 4.26 (dd, J=13.6, 8.7 Hz, 1 H), 4.52 (dd, J=13.6,2.1 Hz, 1 H), 6.67 (dd, J=11.8, 2.7 Hz, 1 H), 7.09 (dd, J=8.5, 2.7 Hz, 1 H), 7.34 (td, J=7.6, 1.2 Hz, 1 H), 7.55 (td, J=7.6, 1.4 Hz, 1 H), 7.62 (dd, J=7.6, 1.4 Hz, 1 H), 8.08 (dd, J=7.6, 1.2 Hz, 1 H), 8.91 (s, 1 H), 9.09 (br s, 1 H), 12.08 (s, 1 H) |
| 53 | 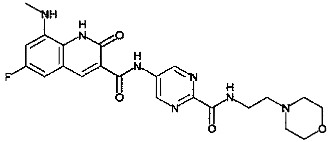 | 6-fluoro-8-(methylamino)-N-(2-{[2-(morpholine-4-yl)ethyl]carbamoyl} pyrimidine-5-yl)-2-oxo-1,2-dihydroquinoline-3-carboxamide | MS (ESI pos.) m/z: 470 [M+H]+ RT=0.486 min (Analytical Condition 3) | 1H NMR (400 MHz, DMSO-d6) δ ppm 2.42 (br s, 4 H), 2.45 - 2.55 (m, 2 H), 2.86 (d, J=4.5 Hz, 3 H), 3.39 - 3.48 (m, 2 H), 3.54 - 3.62 (m, 4 H), 6.49 - 6.58 (m, 1 H), 6.63 (dd, J=8.8, 2.6 Hz, 1 H), 7.04 (dd, J=8.8, 2.6 Hz, 1 H), 8.75 (t, J=5.9 Hz, 1 H), 8.90 (s, 1 H), 9.31 (s, 2 H), 12.17 (br s, 1 H), 12.73 (br s, 1 H) |
| 61 | 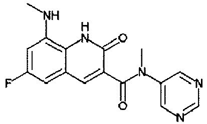 | 6-fluoro-N-methyl-8-(methylamino)-2-oxo-N-(pyrimidine-5-yl)-1,2-dihydroquinoline-3-carboxamide | MS (ESI pos.) m/z: 328 [M+H]+ RT=0.599 min (Analytical Condition 3) | 1H NMR (600 MHz, CHLOROFORM-d) δ ppm 2.93 (br s, 3 H), 3.55 (br s, 3 H), 5.69 (br s, 1 H), 6.50 (brd, J=10.7 Hz, 1 H), 6.59 (brs, 1 H), 7.96 (br s, 1 H), 8.70 (br s, 2 H), 9.02 (br s, 1 H) |
| 63 | 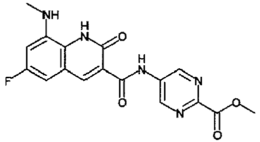 | methyl 5-{[6-fluoro-8-(methylamino)-2-oxo-1,2-dihydroquinoline-3-carbonyl]amino} pyrimidine-2-carboxylate | MS (ESI pos.) m/z: 372 [M+H]+ RT=0.816 min (Analytical Condition 3) | 2.97 (m, (400 MHz, DMSO-d6) δ - ppm 2.77 - 2 H), 7.04 - 7.23 (m, 1 H), 8.92 (s, 1 H), 9.34 (s, 2 H), 11.90 (br s, 1 H), 12.56 (br s, 1 H) |
| 64 | 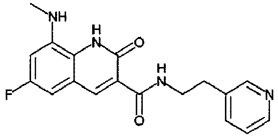 | 6-fluoro-8-(methylamino)-2-oxoN-[2-(pyridine-3-yl)ethyl]-1,2-dihydroquinoline-3-carboxamide | MS (ESI pos.) m/z: 341 [M+H]+ RT=0.373 min (Analytical Condition 3) | 1H NMR (600 MHz, DMSO-d6) δ ppm 2.83 (d, J=4.5 Hz, 3 H), 2.88 (t, J=7.2 Hz, 2 H), 3.57 - 3.65 (m, 2 H), 6.40 - 6.45 (m, 1 H), 6.58 (dd, J=11.6, 2.5 Hz, 1 H), 6.98 (dd, J=8.7, 2.5 Hz, 1 H), 7.32 (dd, J=7.8, 5.0 Hz, 1 H), 7.70 (brd, J=7.8 Hz, 1 H), 8.42 (dd, J=5.0, 1.4 Hz, 1 H), 8.48 (d, J=2.1 Hz, 1 H), 8.70 (s, 1 H), 9.90 (br s, 1 H), 11.63 (br s, 1 H) |

**[Table 7-2]**

| Example | Structural Formula | Compound Name | MS | NMR |
|---|---|---|---|---|
| 67 | 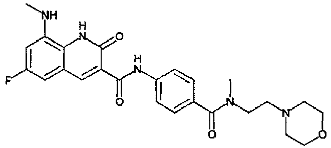 | 6-fluoro-8-(methylamino)-N-(4-{methyl[2-(morpholine-4-yl)ethyl]carbamoyl} phenyl)-2-oxo-1,2-dihydroquinoline-3-carboxamide | MS (ESI pos.) m/z : 482 [M+H]+ RT=0.545 min (Analytical Condition 3) | 1H NMR (600 MHz, DMSO-d6) δ ppm 2.20 (br s, 2 H), 2.45 (br s, 2 H), 2.86 (d, J=4.5 Hz, 3 H), 2.96 (s, 3 H), 3.34 - 3.65 (m, 7 H), 3.35 - 3.47 (m, 1 H), 6.50 - 6.54 (m, 1 H), 6.62 (dd, J=11.6, 2.5 Hz, 1 H), 7.05 (dd, J=8.7, 2.5 Hz, 1 H), 7.43 (d, J=8.7 Hz, 2 H), 7.78 (d, J=8.7 Hz, 2 H), 8.86 (s, 1 H), 11.84 (brs, 1 H), 12.41 (br s, 1 H) |
| 69 | 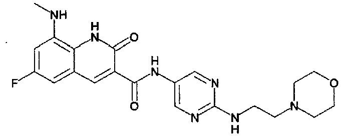 | 6-fluoro-8-(methylamino)-N-(2-{[2-(morpholine-4-yl)ethyl]amino} pydmidine-5-yl)-2-oxo-1,2-dihydroquinoline-3-carboxamide | MS (ESI pos.) m/z: 442 [M+H]+ RT=0.495 min (Analytical Condition 3) | 1H NMR (600 MHz, DMSO-d6) δ ppm 2.85 (s, 3 H), 3.32 - 3.36 (m, 2 H), 3.65 - 3.68 (m, 2 H), 3.94 - 4.04 (m, 8 H), 6.65 (dd, J=11.8, 2.5 Hz, 1 H), 7.05 (dd, J=8.7, 2.5 Hz, 1 H), 7.36 (br s, 1 H), 8.70 (s, 2 H), 8.84 (s,1 H), 11.74 (s, 1 H), 11.81 (br s, 1 H) |
| 72 | 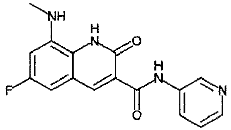 | 6-fluoro-8-(methylamino)-2-oxo-N-(pyridine-3-yl)-1,2-dihydroquinoline-3-carboxamide | MS (ESI neg.) m/z: 313[M+H]+ RT=0.607 min (Analytical Condition 3) | 1H NMR (600 MHz, DMSO-d6) δ ppm 2.86 (d, J=4.5 Hz, 3 H), 6.54 (br d, J=4.5 Hz, 1 H), 6.65 (dd, J=11.8, 2.7 Hz, 1 H), 7.07 (dd, J=8.9, 2.7 Hz, 1 H), 7.43 (dd, J=8.4,4.5 Hz, 1 H), 8.21 (ddd, J=8.4, 2.5, 1.2 Hz, 1 H), 8.35 (dd, J=4.5, 1.2 Hz, 1 H), 8.88 (d, J=2.5 Hz, 1 H), 8.89 (s, 1 H), 11.86 (br s, 1 H), 12.20 (s, 1 H) |
| 73 | 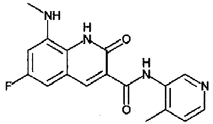 | 6-fluoro-8-(methylamino)-N-(4- methylpyridine-3-yl)-2-oxo-1,2-dihydroquinoline-3-carboxamide | MS (ESI neg.) m/z: 327 [M+H]+ RT=0.515 min (Analytical Condition 3) | 1H NMR (600 MHz, DMSO-d6) δ ppm 2.38 (s, 3 H), 2.86 (d, J=4.5 Hz, 3 H), 6.50 (br d, J=4.5 Hz, 1 H), 6.65 (dd, J=11.8, 2.7 Hz, 1 H), 7.08 (dd, J=8.9, 2.7 Hz, 1 H), 7.33 (d, J=5.0 Hz, 1 H), 8.24 (d, J=5.0 Hz, 1 H), 8.93 (s, 1 H), 9.37 (s,1 H), 11.93 (br s, 1 H), 12.21 (brs, 1 H) |
| 76 | 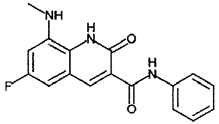 | 6-fluoro-8-(methylamino)-2-oxoN-phenyl-1,2-dihydroquinoline-3-carboxamide | MS (ESI pos.) m/z: 312 [M+H]+ RT=1.045 min (Analytical Condition 3) | 1H NMR (600 MHz, DMSO-d6) δ ppm 2.86 (d, J=4.5 Hz, 3 H), 6.47 - 6.55 (m, 1 H), 6.64 (dd, J=11.6, 2.5 Hz, 1 H), 7.06 (dd, J=8.7, 2.5 Hz, 1 H), 7.10 - 7.17 (m, 1 H), 7.36 - 7.43 (m, 2 H), 7.73 (d, J=7.7 Hz, 2 H), 8.87 (s, 1 H), 11.82 (br s, 1 H), 12.12 (s, 1 H) |
| 77 | 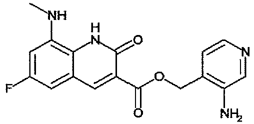 | (3-aminopyridine-4-yl)methyl 6-fluoro-8-(methylamino)-2-oxo-1,2-dihydroquinoline-3-carboxylate | MS (ESI pos.) m/z: 343 [M+H]+ RT=0.420 min (Analytical Condition 3) | 1H NMR (600 MHz, DMSO-d6) δ ppm 2.82 (d, J=4.5 Hz, 3 H), 5.19 (s, 2 H), 5.45 (brs, 2 H), 6.39 (br d, J=4.5 Hz, 1 H), 6.58 (dd, J=11.6, 2.5 Hz, 1 H), 6.91 (dd, J=8.7, 2.5 Hz, 1 H), 7.21 (d, J=4.7 Hz, 1 H), 7.79 (d, J=4.7 Hz, 1 H), 8.04 (s, 1 H), 8.51 (s, 1 H), 11.32 (brs, 1 H) |
| 78 | 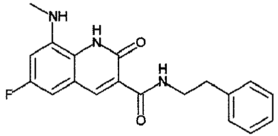 | 6-fluoro-8-(methylamino)-2-oxo-N-(2-phenylethyl)-1,2-dihydroquinoline-3-carboxamide | MS (ESI pos.) m/z: 340 [M+H]+ RT=0.973 min (Analytical Condition 3) | 1H NMR (600 MHz, DMSO-d6) δ ppm 2.83 (d, J=4.5 Hz, 3 H), 2.85 (t, J=7.2 Hz, 2 H), 3.55 - 3.61 (m, 2 H), 6.42 (br d, J=4.5 Hz, 1 H), 6.58 (dd, J=11.8, 2.3 Hz, 1 H), 6.98 (dd, J=8.5, 2.3 Hz, 1 H), 7.19 - 7.23 (m, 1 H), 7.26 - 7.32 (m, 4 H), 8.71 (s, 1 H), 9.89 (br s, 1 H), 11.63 (br s, 1 H) |
| 79 | 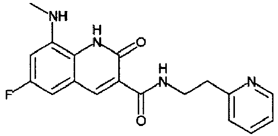 | 6-fluoro-8-(methylamino)-2-oxoN-[2-(pyridine-2-yl)ethyl]-1,2-dihydroquinoline-3-carboxamide | MS (ESI pos.) m/z: 341 [M+H]+ RT=0.390 min (Analytical Condition 3) | 1H NMR (600 MHz, DMSO-d6) δ ppm 2.82 (br d, J=4.5 Hz, 3 H), 3.01 (brt, J=7.0 Hz, 2 H), 3.69 - 3.76 (m, 2 H), 6.40 (br s, 1 H), 6.57 (br d, J=11.6 Hz, 1 H), 6.97 (br d, J=9.1 Hz, 1 H), 7.21 - 7.25 (m, 1 H), 7.31 (d, J=7.5 Hz, 1 H), 7.71 (td, J=7.5, 1.9 Hz, 1 H), 8.50 - 8.53 (m, 1 H), 8.70 (s, 1 H), 9.97 (br s, 1 H), 11.60 (br s, 1 H) |

**[Table 7-3]**

| Example | Structural Formula | Compound Name | MS | NMR |
|---|---|---|---|---|
| 80 | 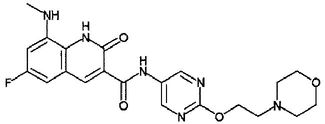 | 6-fluoro-8-(methylamino)-N-{2-[2-(morpholine-4-yl)ethoxy]pyrimidine-5-yl)-2-oxo-1,2-dihydroquinoline-3-carboxamide | MS (ESI pos.) m/z 443 [M+H]+ RT=0.513 min (Analytical Condition 3) | 1H NMR (400 MHz, DMSO-d6) δ ppm 2.40-2.55 (m, 4 H), 2.70 (t, J=5.8 Hz, 2 H), 2.85 (d, J=4.2 Hz, 3 H), 3.53 - 3.59 (m, 4 H), 4.40 (t, J=5.8 Hz, 2 H), 6.11 (dd, J=11.6, 2.6 Hz, 1 H), 6.18 (br d, J=4.2 Hz, 1 H), 6.51 (dd, J=10.0, 2.6 Hz, 1 H), 8.39 (s, 1 H), 8.90 (s, 2 H) |
| 81 | 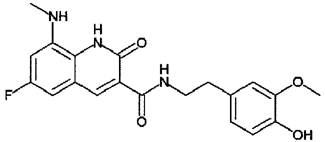 | 6-fluoro-N-[2-(4-hydroxy-3-methoxyphenyl)ethyl]-8-(methylamino)-2-oxo-1,2-dihydroquinoline-3-carboxamide | MS (ESI pos.) m/z : 386 [M+H]+ RT=0.833 min (Analytical Condition 3) | 1H NMR (600 MHz, DMSO-d6) δ ppm 2.73 (t, J=7.0 Hz, 2 H), 2.83 (d, J=4.5 Hz, 3 H), 3.52 - 3.56 (m, 2 H), 3.75 (s, 3 H), 6.43 (br d, J=4.5 Hz, 1 H), 6.58 (dd, J=12.0, 2.5 Hz, 1 H), 6.62 - 6.66 (m, 1 H), 6.66 - 6.70 (m, 1 H), 6.83 (d, J=2.5 Hz, 1 H), 6.98 (dd, J=8.7,2.5 Hz, 1 H), 8.70 (s, 1 H), 9.85 (br s, 1 H), 11.59 (br s, 1 H) |
| 82 | 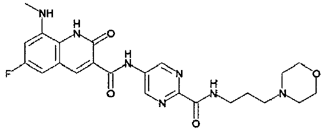 | 6-fluoro-8-(methylamino)-N-(2-{[3-(morpholine-4-yl)propyl]carbamoyl} pydmidine-5-yl)-2-oxo-1,2-dihydroquinoline-3-carboxamide | MS (ESI pos.) m/z: 484 [M+H]+ RT=0.510 min (Analytical Condition 3) | 1H NMR (400 MHz, DMSO-d6) δ ppm 1.71 (quin, J=6.7 Hz, 2 H), 2.31 - 2.47 (m, 6 H), 2.86 (d, J=4.5 Hz, 3 H), 3.33 - 3.40 (m, 2 H), 3.62 (t, J=4.6 Hz, 4 H), 6.56 (brd, J=4.5 Hz, 1 H), 6.66 (dd, J=11.7, 2.6 Hz, 1 H), 7.06 (dd, J=8.7, 2.6 Hz, 1 H), 8.92 (s, 1 H), 9.10 (t, J=5.7 Hz, 1 H), 9.31 (s, 2 H), 11.85 (br s, 1 H), 12.43 (s, 1 H) |
| 86 | 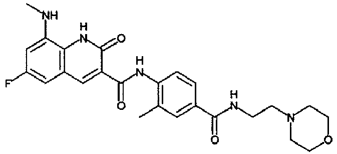 | 6-fluoro-8-(methylamino)-N-(2-methyl-4-{[2-(morpholine-4-yl)ethyl]carbamoyl} phenyl)-2-oxo-1,2-dihydroquinoline-3-carboxamide | MS (ESI ( pos.) m/z : 482 [M+H]+ RT=0.561 (Analytical Condition 3) | 1H NMR (400 MHz, DMSO-d6) δ ppm 2.38 - 2.44 (m, 1 H), 2.43 (s, 6 H), 2.83 - 2.89 (m, 3 H), 3.33 - 3.44 (m, 4 H), 3.54 - 3.62 (m, 4 H), 6.47 (br s, 1 H), 6.62 (br d, J=11.5 Hz, 1 H), 7.05 (br d, J=7.6 Hz, 1 H), 7.72 (d, J=8.7 Hz, 1 J=8.7 Hz, 7.76 (s, 1 H), 8.88 (s, 1 H), (11.94 (br s, 1 H), 12.53 (br s, 1 H) |
| 87 | 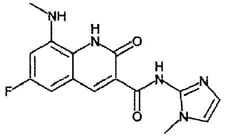 | 6-fluoro-8-(methylamino)-N-(1- methyl-1 H-imidazole-2-yl)-2-oxo-1,2-dihydroquinoline-3-carboxamide | MS (ESI pos.) m/z: 316 [M+H]+ RT=0.353 min (Analytical Condition 3) | 1H NMR (600 MHz, DMSO-d6) δ ppm 2.87 (d, J=4.5 Hz, 3 H), 3.56 (br s, 3H), 6.40 - 6.59 (m, 2 H), 6.84 - 7.02 (m, 2 H), 7.16 (s, 1 H), 8.83 (br s, 1 H) |
| 88 | 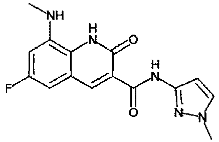 | 6-fluoro-8-(methylamino)-N-(1-methyl-1H-pyrazole-3-yl)-2-oxo-1,2-dihydroquinoline-3-carboxamide | MS (ESI pos.) m/z: 316 [M+H]+ RT=0.802 min (Analytical Condition 3) | 1H NMR (600 MHz, DMSO-d6) δ ppm 2.85 (d, J=4.5 Hz, 3 H), 3.77 (s, 3 H), 6.49 (br d, J=4.5 Hz, 1 H), 6.61 - 6.63 (m, 2 H), 7.03 (dd, J=8.7, 2.5 Hz, 1 H), 7.62 (d, J=2.5 Hz, 1 H), 8.84 (s, 1 H), 11.87 (br s, 1 H), 12.32 (br s, 1 H) |
| 89 | 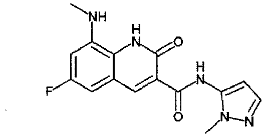 | 6-fluoro-8-(methylamino)-N-( 1-methyl-1H-pyrazole-5-yl)-2-oxo-1,2-dihydroquinoline-3-carboxamide | MS (ESI pos.) m/z: 316 [M+H]+ RT=0.794 min (Analytical Condition 3) | 1H NMR (600 MHz, DMSO-d6) δ ppm 2.86 (br d, J=4.5 Hz, 3 H), 3.78 (s, 3 H), 6.46 - 6.48 (m, 1 H), 6.50 - 6.55 (m, 1 H), 6.63 - 6.67 (m, 1 H), 7.06 (br d, J=8.7 Hz, 1 H), 7.36 - 7.38 (m, 1 H), 8.88 (s, 1 H), 11.97 (br s, 1 H), 12.63 (br s, 1 H) |
| 90 | 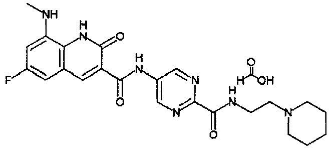 | 6-fluoro-8-(methylamino)-2-oxoN-(2-{[2-(piperidine-1-yl)ethyl]carbamoyl} pydmidine-5-yl)-1,2-dihydroquinoline-3-carboxamide-formate | MS (ESI pos.) m/z : 468 [M+H]+ RT=0.521 min (Analytical Condition 3) | 1H NMR (400 MHz, DMSO-d6) δ ppm 1.38 (s, 2 H), 1.45 - 1.54 (m, 4 H), 2.39 (br s, 4 H), 2.42 - 2.48 (m, 2 H), 2.86 (d, J=4.8 Hz, 3 H), 3.79 - 4.00 (m, 2 H), 6.41 - 6.49 (m, 2 H), 6.86 (dd, J=9.1, 2.4 Hz, 1 H), 8.69 (t, J=5.8 Hz, 1 H), 8.73 (s, 1 H), 9.28 (s, 2 H) |

**[Table 7-4]**

| Example | Structural Formula | Compound Name | MS | NMR |
|---|---|---|---|---|
| 95 | 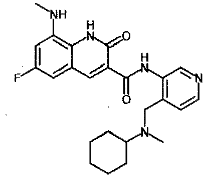 | N-(4-{[cyclohexyl (methyl)amino]methyl} pyridine-3-yl)-6-fluoro-8-(methylamino)-2-oxo-1,2-dihydroquinoline-3-carboxamide | MS (ESI pos.) m/z : 438 [M+H]+ RT=0.581 min (Analytical Condition 3) | 1H NMR (600 MHz, CHLOROFORMS) δ ppm 0.89 - 0.98 (m, 1 H), 1.02 - 1.12 (m, 2H), 1.13 -1.22 (m, 2 H), 1.47 -1.54 (m, 1 H), 1.59-1.65 (m, 2 H), 1.70 - 1.84 (m, 2 H), 2.15 (s, 3 H), 2.37 - 2.46 (m, 1 H), 2.75 (br d, J=4.5 Hz, 3 H), 3.70 (s, 2 H), 5.49 (br d, J=4.5 Hz, 1 H), 6.55 (d, J=10.9 Hz, 1 H), 6.81 (d, J=5.8 Hz, 1 H), 7.27 (d, J=5.0 Hz, 1 H), 8.41 (d, J=5.0 Hz, 1 H), 8.90 (br s, 1 H), 9.46 (s, 1 H), 11.61 (br s, 1H), 12.46 (br s, 1 H) |
| 121 | 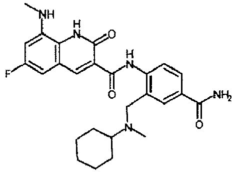 | N-(4-carbamoyl-2-{[cyclohexyl(methyl) amino]methyl)phenyl)-6-fluoro-8-(methylamino)-2-oxo-1,2-dihydroquinoline-3-carboxamide | MS (ESI pos.) m/z: 480 [M+H]+ RT=0.598 min (Analytical Condition 3) | 1H NMR (400 MHz, DMSO-d6) δ ppm 1.04 - 2.21 (m, 10 H), 2.54 (br s, 3 H), 2.86 (br d, J=4.2 Hz, 3 H), 3.01 - 3.26 (m, 1 H), 4.31 (br s, 2H), 6.65 (br dd, J=11.7, 2.4 Hz, 1 H), 6.83 (br s, 1 H), 7.08 (dd, J=8.7, 2.4 Hz, 1 H), 7.92 - 8.05 (m, 2 H), 8.30 (d, J=8.4 Hz, 1H), 8.91 (s, 1H), 12.04 (br s, 1 H), 12.37 (br s, 1 H) |
| 144 | 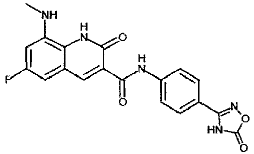 | 6-fluoro-8-(methylamino)-2-oxoN-[4-(5-oxo-4,5-dihydro-1,2,4-oxadiazole-3-yl)phenyl]-1,2-dihydroquinoline-3-carboxamide | MS (ESI pos.) m/z: 396 [M+H]+ RT=0.938 min (Analytical Condition 3) | 1H NMR (600 MHz, DMSO-d6) δ ppm 2.86 (d, J=4.5 Hz, 3 H), 6.52 (br d, J=4.5 Hz, 1 H), 6.61 (dd, J=11.6, 2.5 Hz, 1 H), 7.04 (dd, J=8.7, 2.5 Hz, 1 H), 7.72 - 7.80 (m, 4 H), 8.85 (br s, 1 H), 11.88 (br s, 1H), 12.41 (br s, 1 H) |
| 149 | 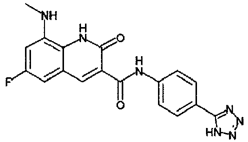 | 6-fluoro-8-(methylamino)-2-oxoN-[4-(1H-tetrazole-5-yl)phenyl]-1,2-dihydroquinoline-3-carboxamide | MS (ESI pos.) m/z: 380 [M+H]+ RT=0.883 min (Analytical Condition 3) | 1H NMR (600 MHz, DMSO-d6) δ ppm 2.86 (d, J=4.5 Hz, 3 H), 6.55 (br d, J=4.5 Hz, 1 H), 6.65 (dd, J=11.6, 2.5 Hz, 1 H), 7.08 (dd, J=8.5, 2.5 Hz, 1H), 7.91 - 7.98 (m, 2 H), 8.03 - 8.10 (m, 2 H), 8.89 (s, 1 H), 11.88 (br s, 1 H), 12.38 (s, 1 H) |
| 154 | 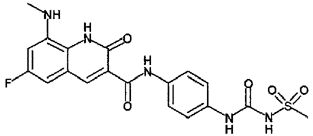 | 6-fluoro-N-(4-{[(methanesulfonyl) carbamoyl]amino }phenyl)-8-(methylamino)-2-oxo-1,2-dihydroquinoline-3-carboxamide | MS (ESI neg.) m/z: 446 [M-H]-RT=0.836 min (Analytical Condition 3) | 1H NMR (600 MHz, DMSO-d6) δ ppm 2.85 (d, J=4.5 Hz, 3 H), 3.26 (s, 3 H), 6.54 (br d, J=4.5 Hz, 1 H), 6.64 (dd, J=11.6, 2.5 Hz, 1H), 7.05 (dd, J=8.7, 2.5 Hz, 1H), 7.42 -7.47 (m, 2 H), 7.64 - 7.69 (m, 2 H), 8.85 (s, 1 H), 8.88 (br s, 1 H), 11.76 (s, 1H), 11.82 (brs, 1 H), 12.05 (s, 1 H) |

### Example 55 6-Fluoro-8-(methylamino)-2-oxo-N-[2-(piperazin-1-yl)pyrimidin-5-yl]-1,2-dihydroquinoline-3-carboxamide-hydrochloride

### 55-(1) tert-Butyl 4-(5-[[6-fluoro-8-(methylamino)-2-oxo-1,2-dihydroquinoline-3-carbonyl]amino]pyrimidin-2-yl)piperazine-1-carboxylate

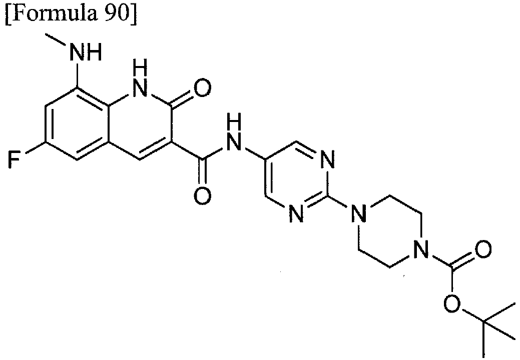

To a solution in DMF (1 mL) of the 6-fluoro-8-(methylamino)-2-oxo-1,2-dihydroquinoline-3-carboxylic acid (42 mg) obtained in Production Example 2-(5), HATU (74 mg), tert-butyl 4-(5-aminopyrimidin-2-yl)piperazine-1-carboxylate (49 mg) and DIPEA (92 µL) were added and the mixture was stirred for 16 hours at room temperature.
The reaction mixture was purified by preparative LCMS to obtain the titled compound (9 mg) as an orange solid.
MS (ESI pos.) m/z : 498 [M+H]+, RT=1.147 min (Analytical Condition 3)

### 55-(2) 6-Fluoro-8-(methylamino)-2-oxo-N-[2-(piperazin-1-yl)pyrimidin-5-yl]-1,2-dihydroquinoline-3-carboxamide-hydrochloride

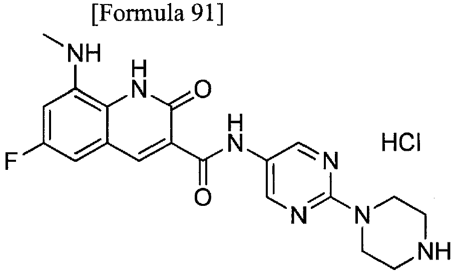

To the tert-butyl 4-(5-[[6-fluoro-8-(methylamino)-2-oxo-1,2-dihydroquinoline-3-carbonyl]amino]pyrimidin-2-yl)piperazine-1-carboxylate (9 mg) obtained in Example 55-(1), 4 M hydrochloric acid in dioxane (1 mL) was added and the mixture was stirred for an hour at room temperature. The reaction mixture was concentrated to obtain the titled compound (8 mg) as an orange solid.
MS (ESI pos.) m/z : 398 [M+H]+, RT=0.517 min (Analytical Condition 3)
¹H NMR (600 MHz, DMSO-d6) δ ppm 2.85 (s, 3 H), 3.15 - 3.19 (m, 4 H), 3.92 - 3.99 (m, 4 H), 6.63 (dd, J=11.6, 2.5 Hz, 1 H), 7.04 (dd, J=8.7, 2.5 Hz, 1 H), 8.79 (s, 2 H), 8.85 (s, 1 H), 9.26 (br s, 1 H), 11.82 (s, 1 H), 11.88 (br s, 1 H)

The same procedures as in Example 55 were employed to obtain the Example compounds identified below from the corresponding raw materials.

**[Table 8]**

| Example | Structural Formula | Compound Name | MS | NMR |
|---|---|---|---|---|
| 55 | 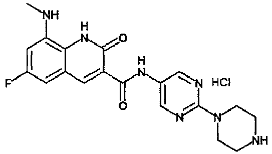 | 6-fluoro-8-(methylamino)-2-oxo-N-[2-(piperazine-1-yl)pyrimidine-5-yl]-1,2-dihydroquinoline-3-carboxamide-hydrochrolide | MS (ESI pos.) m/z: 398 [M+H]+ RT=0.517 min (Analytical Condition 3) | 1H NMR (600 MHz, DMSO-d6) δ ppm 2.85 (s, 3 H), 3.15 - 3.19 (m, 4 H), 3.92 - 3.99 (m, 4 H), 6.63 (dd, J=11.6, 2.5 Hz, 1 H), 7.04 (dd, J=8.7, 2.5 Hz, 1 H), 8.79 (s, 2 H), 8.85 (s, 1 H), 9.26 (br s, 1 H), 11.82 (s, 1 H), 11.88 (br s, 1H) |
| 57 | 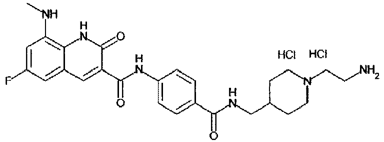 | N-[4-({[1-(2-aminoethyl)piperidine-4-yl]methyl}carbamoyl) phenyl]-6-fluoro-8-(methylamino)-2-oxo-1,2-dihydroquinoline-3-carboxamide-dihydrochloride | MS (ESI pos.) m/z: 495 [M+H]+ RT=0.363 min (Analytical Condition 3) | 1H NMR (600 MHz, DMSO-d6) δ ppm 1.58 -1.63 (m, 1 H), 1.77 -1.92 (m, 4 H), 2.85 (s, 3 H), 2.91-3.73 (m, 10 H), 6.64 (d, J=12.0 Hz, 1 H), 7.06 (d, J=8.3 Hz, 1 H), 7.81 (d, J=8.3 Hz, 2 H), 7.91 (d, J=8.3 Hz, 2 H), 8.34 (br s, 3 H), 8.58 (br s, 1 H), 8.87 (s, 1 H), 10.64 (br s, 1 H), 11.91 (br s, 1 H), 12.33 (s, 1 H) |
| 59 | 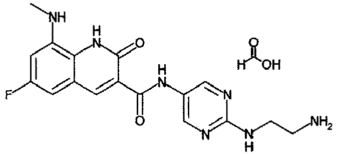 | N-{2-[(2-aminoethyl)amino] pyrimidine-5-yl}-6-fluoro-8-(methylamino)-2-oxo-1,2-dihydroquinoline-3-carboxamide-formate | MS (ESI pos.) m/z: 372 [M+H]+ RT=0.436 min (Analytical Condition 3) | 1H NMR (600 MHz, DMSO-d6) δ ppm 2.85 (br s, 3 H), 2.89 - 2.94 (m, 2 H), 3.43 - 3.49 (m, 2 H), 6.51 (d, J=9.1 Hz, 1H), 6.91 (d, J=9.1 Hz, 1 H), 7.20 (br s, 1 H), 8.34 (s, 1 H), 8.65 (s, 2 H), 8.73 (s, 1 H), 12.55 (br s, 1H) |
| 60 | 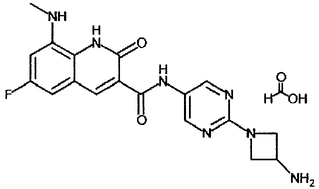 | N-[2-(3-aminoazetidine-1-yl)pyrimidine-5-yl]-6-fluoro-8-(methylamino)-2-oxo-1,2-dihydroquinoline-3-carboxamide-formate | MS (ESI pos.) m/z: 384 [M+H]+ RT=0.435 min (Analytical Condition 3) | 1H NMR (600 MHz, DMSO-d6) δ ppm 2.85 (d, J=4.5 Hz, 3 H), 3.67 - 3.73 (m, 2 H), 3.77 - 3.85 (m, 1 H), 4.17 -4.21 (m, 2 H), 6.55 (br d, J=4.5 Hz, 1 H), 6.61 (dd, J=11.6, 2.5 Hz, 1 H), 7.01 (dd, J=8.7, 2.5 Hz, 1 H), 8.23 (s, 1 H), 8.67 (s, 2 H), 8.82 (s, 1 H), 11.90 (brs, 1 H) |
| 68 | 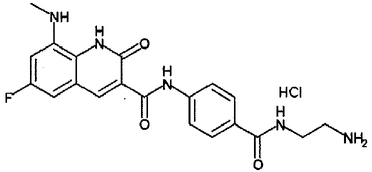 | N-{4-[(2-aminoethyl)carbamoyl] phenyl}-6-fluoro-8-(methylamino)-2-oxo-1,2-dihydroquinoline-3-carboxamide-hydrochloride | MS (ESI pos.) m/z: 398 [M+H]+ RT=0.541 min (Analytical Condition 3) | 1H NMR (600 MHz, DMSO-d6) δ ppm 2.85 (s, 3 H), 2.95 - 3.01 (m, 2 H), 3.46 - 3.57 (m, 2 H), 6.64 (dd, J=11.6, 2.5 Hz, 1 H), 7.06 (dd, J=8.9, 2.5 Hz, 1H), 7.83 (d, J=8.8 Hz, 2 H), 7.97 (d, J=8.8 Hz, 2 H), 8.04 (br s, 3 H), 8.71 (t, J=5.6 Hz, 1 H), 8.88 (s, 1 H), 11.94 (brs, 1 H), 12.36 (s, 1 H) |

### Example 62 2-(3-Aminopropoxy)-4-[[6-fluoro-8-(methylamino)-2-oxo-1,2-dihydroquinoline-3 -carbonyl] amino]benzoic acid-formate

### 62-(1) Methyl 2-[3-[(tert-butoxycarbonyl)amino]propoxy]-4-[[6-fluoro-8-(methylamino)-2-oxo-1,2-dihydroquinoline-3-carbonyl]amino]benzoate

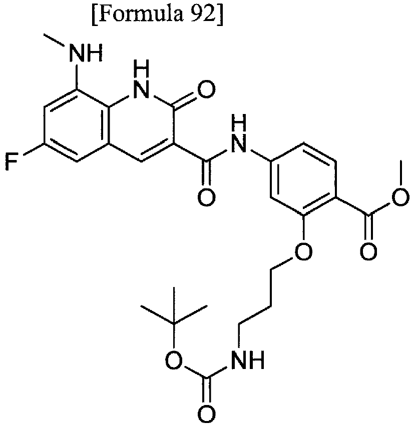

To a solution in DMF (7.5 mL) of methyl 4-amino-2-[3-[(tert-butoxycarbonyl)amino]propoxy]benzoate (297 mg) and the 6-fluoro-8-(methylamino)-2-oxo-1,2-dihydroquinoline-3-carboxylic acid (180 mg) obtained in Production Example 2-(5), HATU (348 mg) and DIPEA (465 µL) were added and the mixture was stirred for 3.5 hours at room temperature. The reaction mixture was concentrated under reduced pressure and, thereafter, a saturated aqueous solutioln of sodium hydrogencarbonate was added and the mixture was extracted with ethyl acetate. The organic layer was washed with saturated brine, dried over anhydrous magnesium sulfate, and concentrated under reduced pressure. The resulting residue was purified by column chromatography (silica gel cartridge, chloroform/methanol = 97:3) to obtain the titled compound (197 mg) as a yellow solid.
MS (ESI pos.) m/z : 543 [M+H]+, RT=1.194 min (Analytical Condition 3)

### 62-(2) 2-[3-[(tert-Butoxycarbonyl)amino]propoxy]-4-[[6-fluoro-8-(methylamino)-2-oxo-1,2-dihydroquinoline-3-carbonyl]amino]benzoic acid

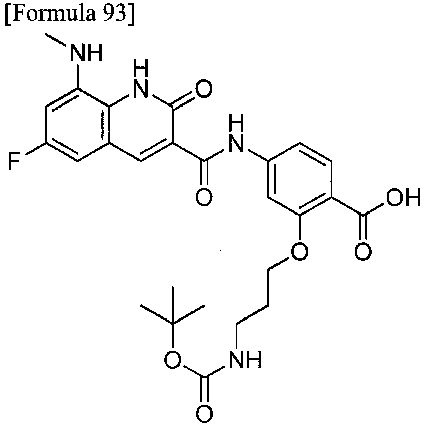

To a solution in tetrahydrofuran (4 mL) of the methyl 2-[3-[(tert-butoxycarbonyl)amino]propoxy]-4-[[6-fluoro-8-(methylamino)-2-oxo-1,2-dihydroquinoline-3-carbonyl]amino]benzoate (197 mg) obtained in Example 62-(1), 1 M sodium hydroxide in aqueous solultion (4 mL) was added and the mixture was stirred under heating for 7.5 hours at 65°C. After leaving the reaction mixture to cool, an aqueous solution of 1 M hydrochloric acid was added to establish pH 3 and the mixture was concentrated under reduced pressure to distill off tetrahydrofuran. The residue was ice cooled and the precipitating solid was recovered by filtration and thereafter washed with water to obtain the titled compound (120 mg) as a brown solid.
MS (ESI pos.) m/z : 529 [M+H]+, RT=1.078 min (Analytical Condition 3)

### 62-(3) 2-(3-Aminopropoxy)-4-[[6-fluoro-8-(methylamino)-2-oxo-1,2-dihydroquinoline-3-carbonyl]amino]benzoic acid-formate

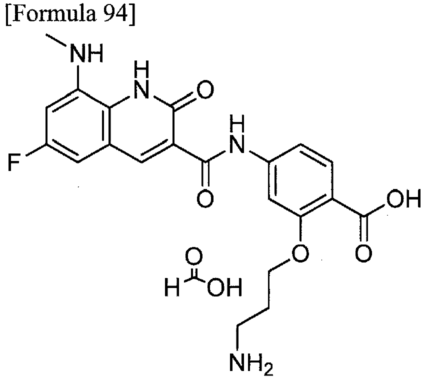

To the 2-[3-[(tert-butoxycarbonyl)amino]propoxy]-4-[[6-fluoro-8-(methylamino)-2-oxo-1,2-dihydroquinoline-3-carbonyl]amino]benzoic acid (120mg) obtained in Example 62-(2), 4 M hydrochloric acid in ethyl acetate solution (30 mL) was added and the mixture was stirred for 19 hours at room temperature. The reaction mixture was concentrated under reduced pressure and thereafter purified by reversed phase column chromatography to obtain the titled compound (6 mg) as a yellow solid.
MS (ESI pos.) m/z : 429 [M+H]+, RT=0.601 min (Analytical Condition 3)
¹H NMR (400 MHz, DMSO-d6) δ ppm 2.07 (br s, 2 H), 2.86 (d, J=4.4 Hz, 3 H), 3.00 - 3.07 (m, 2 H), 4.15 (br t, J=5.4 Hz, 2 H), 6.55 (d, J=4.4 Hz, 1 H), 6.61 (dd, J=8.8, 2.6 Hz, 1 H), 7.01 (dd, J=8.8, 2.6 Hz, 1 H), 7.14 (dd, J=8.4, 1.7 Hz, 1 H), 7.51 (d, J=1.7 Hz, 1 H), 7.71 (d, J=8.4 Hz, 1 H), 8.82 (s, 1 H), 12.38 (br s, 1 H)

### Example 65 5-[[6-Fluoro-8-(methylamino)-2-oxo-1,2-dihydroquinoline-3-carbonyl] amino]pyrimidine-2-carboxylic acid

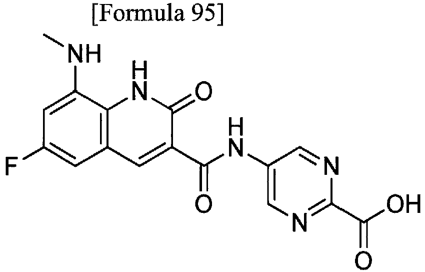

To a solution in tetrahydrofuran (2 mL) of the ethyl 5-[[6-fluoro-8-(methylamino)-2-oxo-1,2-dihydroquinoline-3-carbonyl]amino]pyrimidine-2-carboxylate (40 mg) obtained in Example 63, 1 M sodium hydroxide in aqueous solution (2 mL) was added and the mixture was stirred for 4 hours at room temperature. To the reaction mixture, an aqueous solution of 1 M hydrochloric acid was added to establish pH 1; the precipitating solid was recovered by filtration and washed with water; thereafter, the titled compound (37 mg) was obtained as a yellow solid.
¹H NMR (400 MHz, DMSO-d6) δ ppm 2.86 (d, J=4.4 Hz, 3 H), 6.56 (br d, J=4.4 Hz, 1 H), 6.66 (dd, J=11.8, 2.6 Hz, 1 H), 7.07 (dd, J=8.7, 2.6 Hz, 1 H), 8.92 (s, 1 H), 9.32 (s, 2 H), 11.89 (br s, 1 H), 12.44 (s, 1 H), 13.39 (br s, 1 H)

### Example 71 6-Fluoro-N-[2-[(2-hydroxyethyl)carbamoyl]pyrimidin-5-yl]-8-(methylamino)-2-oxo-1,2-dihydroquinoline-3-carboxamide

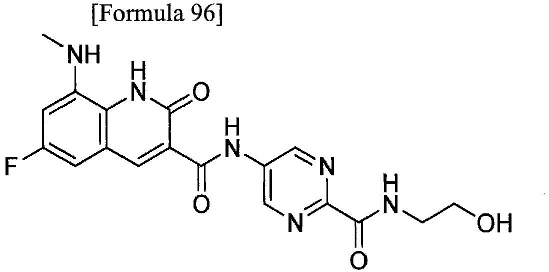

To a solution in DMF (2.5 mL) of 2-aminoethan-1-ol (39 µL) and the 5-[[6-fluoro-8-(methylamino)-2-oxo-1,2-dihydroquinoline-3-carbonyl]amino]pyrimidine-2-carboxylic acid (15 mg) obtained in Example 65, HATU (39 mg) and DIPEA (26 µL) were added and the mixture was stirred for 23 hours at room temperature. The insoluble matter was removed by filtration and the filtrate was concentrated under reduced pressure. The residue was purified by reversed phase column chromatography to obtain the titled compound (0.6 mg) as a yellow solid.
MS (ESI neg.) m/z : 399 [M-H]-, RT=0.678 min (Analytical Condition 3)

The same procedures as in Example 71 were employed to obtain the Example compounds identified below from the corresponding raw materials.

**[Table 9]**

| Example | Structural Formula | Compound Name | MS | NMR |
|---|---|---|---|---|
| 71 | 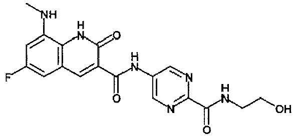 | 6-fluoro-N-{2-[(2-hydroxyethyl) carbamoyl] pyrimidine-5-yl}-8-(methylamino)-2-oxo-1,2-dihydroquinoline-3-carboxamide | MS (ESI neg.) m/z: 399 [M-H]-RT=0.678 min (Analytical Condition 3) | NT |
| 75 | 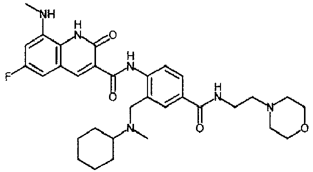 | N-(2-{[cyclohexyl(methyl) amino]methyl}-4-{[2-yl)ethyl]carbamoyl} phenyl)-6-fluoro-8-(methylamino)-2-oxo-1,2-dihydroquinoline-3-carboxamide | MS (ESI pos.) m/z: 593 [M+H]+ RT=0.464 min (Analytical Condition 3) | 1H NMR (400 MHz, CHLOROFORM-d) δ ppm 0.78 -1.44 (m, 8 H), 1.79 (brd, J=11.5 Hz, 2 H), 2.20 (s, 3 H), 2.50 - 2.58 (m, 5 H), 2.63 (t, J=6.0 Hz, 2 H), 2.77 (br s, 3 H), 3.58 (q, J=6.0 Hz, 2 H), 3.71 - 3.78 (m, 4 H), 3.80 (br s, 2 H), 5.62 (br s, 1H), 6.51 (br d, J=10.8 Hz, 1 H), 6.71 - 6.83 (m, 2 H), 7.70 (dd, J=8.4, 1.8 Hz, 1 H), 7.78 (s, 1 H), 8.43 (d, J=8.4 Hz, 1 H), 8.74 (br s, 1 H), 11.44 -13.06 (m, 2 H) |
| 84 | 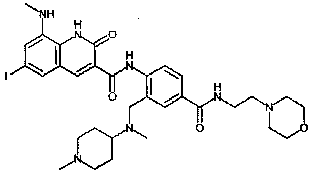 | 6-fluoro-8-(methylamino)-N-(2-{[methyl(1-methylpiperidine-4-yl)amino]methyl}-4-{[2-(morpholine-4-yl)ethyl]carbamoyl} phenyl)-2-oxo-1,2-dihydroquinoline-3-carboxamide | MS (ESI pos.) m/z: 608 [M+H]+ RT=0.179 min (Analytical Condition 3) | 1H NMR (400 MHz, CHLOROFORM-d) δ ppm 0.78 - 2.93 (m, 24 H), 3.57 (q, J=5.6 Hz, 2H), 3.72 - 3.78 (m, 6 H), 5.36 (br s, 1 H), 6.55 (dd, J=11.0, 2.2 Hz, 1 H), 6.74-6.80 (m, 2 H), 7.66 (dd, J=8.4, 2.2 Hz, 1 H), 7.77 (s, 1H), 8.46 (d, J=8.4 Hz, 1 H), 8.75 (s, 1 H) |
| 94 | 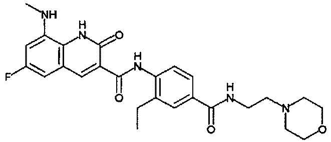 | N-(2-ethyl-4-{[2-(morpholine-4-yl)ethyl]carbamoyl) phenyl)-6-fluoro-8-(methylamino)-2-oxo-1,2-dihydroquinoline-3-carboxamide | MS (ESI pos.) m/z: 496 [M+H]+ RT=0.609 min (Analytical Condition 3) | 1H NMR (400 MHz, CHLOROFORM-d) δ ppm 1.18 -1.30 (m, 3 H), 2.48 - 2.58 (m, 4 H), 2.63 (t, J=6.0 Hz, 2 H), 2.70 - 2.80 (m, 5 H), 3.59 (q, J=5.6 Hz, 2 H), 3.70 - 3.79 (m, 4 H), 5.29 (br s, 1 H), 6.57 (brd, J=10.3 Hz, 1H), 6.80 - 6.88 (m, 2 H), 7.61 (dd, J=8.4, 1.9 Hz, 1 H), 7.75 (s, 1 H), 8.44 (d, J=8.4 Hz, 1 H), 8.98 (br s, 1 H), 11.72 (br s, 1H) |
| 145 | 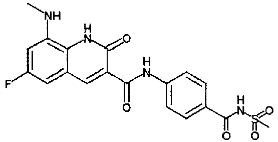 | 6-fluoro-N-{4-[(methanesulfonyl) carbamoyl]phenyl}-8-(methylamino)-2-oxo-1,2-dihydroquinoline-3-carboxamide | MS (ESI pos.) m/z : 433 [M+H]+ RT=0.889 min (Analytical Condition 3) | 1H NMR (600 MHz, DMSO-d6) δ ppm 2.86 (d, J=4.5 Hz, 3 H), 6.55 (br d, J=4.5 Hz, 1 H), 6.65 (dd, J=12.0, 2.5 Hz, 1H), 7.08 (dd, J=8.7, 2.5 Hz, 1H), 7.79 - 7.90 (m, 2 H), 7.96 - 8.04 (m, 2 H), 8.89 (s, 1 H), 11.89 (s, 1 H), 12.05 (brs, 1 H), 12.42 (s,1 H) |
| 146 | 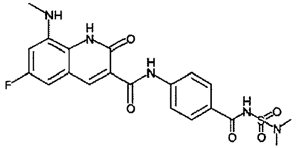 | N-{4-[(dimethylsulfamoyl) carbamoyl]phenyl}-6-fluoro-8-(methylamino)-2-oxo-1,2-dihydroquinoline-3-carboxamide | MS (ESI pos.) m/z : 462 [M+H]+ RT=0.950 min (Analytical Condition 3) | 1H NMR (600 MHz, DMSO-d6) δ ppm 2.86 (d, J=4.5 Hz, 3 H), 2.88 (s, 6 H), 6.55 (br d, J=4.5 Hz, 1 H), 6.65 (dd, J=11.6, 2.5 Hz, 1 H), 7.08 (dd, J=8.7, 2.5 Hz, 1 H), 7.79 - 7.91 (m, 2 H), 7.92 - 8.04 (m, 2 H), 8.89 (s, 1 H), 11.73 (br s, 1 H), 11.88 (br s, 1 H), 12.41 (s, 1 H) |

### 91 6-Fluoro-N-[4-(hydroxymethyl)pyridin-3-yl]-8-(methylamino)-2-oxo-1,2-dihydroquinoline-3 -carboxamide

### Example 91 6-Fluoro-N-[4-(hydroxymethyl)pyridin-3-yl]-8-(methylamino)-2-oxo-1,2-dihydroquinoline-3-carboxamide

### 91-(1) N-[4-([[tert-Butyl(diphenyl)silyl]oxy]methyl)pyridin-3-yl]-6-fluoro-8-(methylamino)-2-oxo-1,2-dihydroquinoline-3-carboxamide

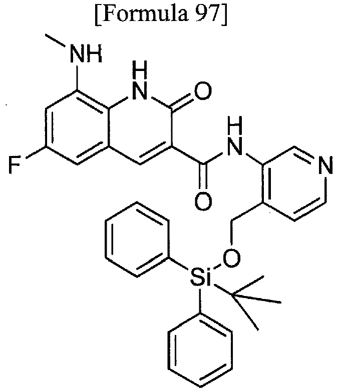

To a solution in DMF (1 mL) of 4-([[tert-butyl(diphenyl)silyl]oxy]methyl)pyridin-3-amine (69 mg) and the 6-fluoro-8-(methylamino)-2-oxo-1,2-dihydroquinoline-3-carboxylic acid (41 mg) obtained in Production Example 2-(5), HATU (73 mg) and DIPEA (91 µL) were added and the mixture was stirred for 15 hours at room temperature. The precipitating solid was recovered by filtration to obtain the titled compound (30 mg) as a yellow solid. MS (ESI pos.) m/z : 581 [M+H]+, RT=1.283 min (Analytical Condition 3)

### 91-(2) 6-Fluoro-N-[4-(hydroxymethyl)pyridin-3-yl]-8-(methylamino)-2-oxo-1,2-dihydroquinoline-3 -carboxamide

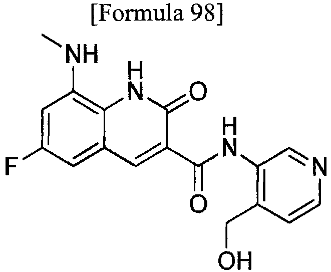

To the N-[4-([[tert-butyl(diphenyl)silyl]oxy]methyl)pyridin-3-yl]-6-fluoro-8-(methylamino)-2-oxo-1,2-dihydroquinoline-3-carboxamide (30 mg) obtained in Example 91-(1), 1 M TBAF in THF solution (258 µL) was added and the mixture was stirred for 15 hours at room temperature. To the reaction mixture, water and ethyl acetate were added. The precipitating solid was recovered by filtration to obtain the titled compound (7 mg) as a yellow solid.
MS (ESI pos.) m/z : 343 [M+H]+, RT=0.474 min (Analytical Condition 3)
¹H NMR (600 MHz, DMSO-d6) δ ppm 2.86 (s, 3 H), 4.75 (s, 2 H), 6.60 (br s, 1 H), 6.64 - 6.68 (m, 1 H), 7.05 - 7.10 (m, 1H), 7.82 (d, J=5.4 Hz, 1 H), 8.54 (d, J=5.4 Hz, 1 H), 8.93 (s, 1 H), 9.47 (s, 1 H), 12.01 (br s, 1 H), 12.35 (s, 1 H)

The same procedures as in Example 91 were employed to obtain the Example compounds identified below from the corresponding raw materials.

**[Table 10]**

| Example | Structural Formula | Compound Name | MS | NMR |
|---|---|---|---|---|
| 91 | 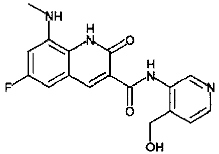 | 6-fluoro-N-[4-(hydroxymethyl)pyridine-3-yl]-8-(methylamino)-2-oxo-1,2-dihydroquinoline-3-carboxamide | MS (ESI pos.) m/z: 343 [M+H]+ RT=0.474 min (Analytical Condition 3) | 1H NMR (600 MHz, DMSO-d6) δ ppm 2.86 (s, 3 H), 4.75 (s, 2 H), 6.60 (br s, 1 H), 6.64 - 6.68 (m, 1 H), 7.05 - 7.10 (m, 1 H), 7.82 (d, J=5.4 Hz, 1H), 8.54 (d, J=5.4 Hz, 1H), 8.93 (s, 1 H), 9.47 (s, 1 H), 12.01 (br s, 1 H), 12.35 (s, 1 H) |
| 122 | 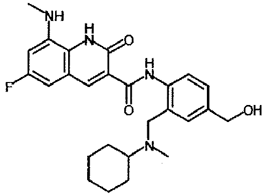 | N-[24[cyclohexyl(methyl) amino]methyl}-4-(hydroxymethyl)phenyl]-6-fluoro-8-(methylamino)-2-oxo-1,2-dihydroquinoline-3-carboxamide | MS (ESI pos.) m/z: 467 [M+H]+ RT=0.609 min (Analytical Condition 3) | 1H NMR (400 MHz, DMSO-d6) δ ppm 0.85 - 1.90 (m, 10 H), 2.16 (s, 3 H), 2.85 (d, J=4.5 Hz, 3 H), 3.11 - 3.19 (m, 1 H), 3.70 (s, 2 H), 4.45 (d, J=5.7 Hz, 2 H), 5.04 (t, J=5.7 Hz, 1 H), 6.12 (br d, J=11.5 Hz, 1 H), 6.19 (br d, J=4.5 Hz, 1H), 6.53 (br d, J=10.4 Hz, 1 H), 7.12 (br d, J=8.2 Hz, 1 H), 7.40 (s, 1 H), 8.29 (d, J=8.2 Hz, 1 H), 8.40 (s, 1 H) |
| 123 | 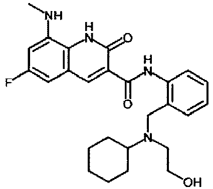 | N-(2-{[cyclohexyl(2-hydroxyethyl)amino] methyl)phenyl)-6-fluoro-8-(methylamino)-2-oxo-1,2-dihydroquinoline-3-carboxamide | MS (ESI pos.) m/z: 467 [M+H]+ RT=0.661 min (Analytical Condition 3) | 1H NMR (400 MHz, CHLOROFORM-d) δ ppm 0.87 -1.92 (m, 10 H), 2.39 - 2.60 (m, 1 H), 2.61 - 2.73 (m, 5 H), 3.60 (t, J=4.6 Hz, 2 H), 3.77 (s, 2 H), 5.39 (br d, J=4.5 Hz, 1 H), 6.43 (dd, J=11.1, 2.7 Hz, 1 H), 6.72 (dd, J=8.1, 2.7 Hz, 1 H), 7.10 - 7.16 (m, 1 H), 7.21 - 7.26 (m, 1 H), 7.32 - 7.39 (m, 1 H), 8.10 (d, J=8.1 Hz, 1 H), 8.85 (s, 1 H), 11.59 (brs, 1 H) |
| 124 | 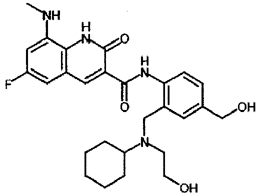 | N-[2-{[cyclohexyl(2-hydroxyethyl)amino] methyl}-4-(hydroxymethyl)phenyl]-6-fluoro-8-(methylamino)-2-oxo-1,2-dihydroquinoline-3-carboxamide | MS (ESI pos.) m/z: 497 [M+H]+ RT=0.603 min (Analytical Condition 3) | 1H NMR (400 MHz, CHLOROFORM-d) δ ppm 0.77 -1.92 (m, 10 H), 2.83 (brd, J=3.8 Hz, 3 H), 3.29 - 3.44 (m, 5 H), 3.76 (s, 2 H), 4.68 (s, 2 H), 5.97 (brd, J=3.8 Hz, 1H), 6.44 - 6.52 (m, 1 H), 6.72 (dt, J=5.5, 2.5 Hz, 1 H), 7.27 - 7.35 (m, 2 H), 8.04 (dd, J=5.3, 2.6 Hz, 1 H), 8.83 (s, 1 H), 11.76 (brs, 1 H) |
| 158 | 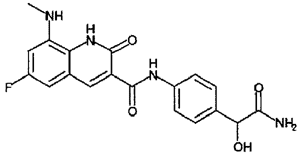 | N-[4-(2-amino-1-hydroxy-2-oxoethyl)phenyl]-6-fluoro-8-(methylamino)-2-oxo-1,2-dihydroquinoline-3-carboxamide | MS (ESI pos.) m/z: 385 [M+H]+ RT=0.750 min (Analytical Condition 3) | 1H NMR (600 MHz, DMSO-d6) δ ppm 2.85 (d, J=4.5 Hz, 3 H), 4.83 (br d, J=4.0 Hz, 1 H), 5.99 (br d, J=4.0 Hz, 1 H), 6.53 (br d, J=4.5 Hz, 1 H), 6.62 (br d, J=11.6 Hz, 1H), 7.04 (br d, J=8.7 Hz, 1 H), 7.18 (br s, 1 H), 7.38 (br s, 1 H), 7.42 (d, J=8.7 Hz, 2 H), 7.68 (d, J=8.7 Hz, 2 H), 8.85 (s, 1 H), 11.83 (brs, 1H), 12.22 (br s, 1 H) |

### Example 92 6-Fluoro-N-[4-(hydroxycarbamoyl)phenyl]-8-(methylamino)-2-oxo-1 ,2-dihydroquinoline-3 -carboxamide

### 92-(1) 6-Fluoro-8-(methylamino)-N-(4-[[(oxan-2-yl)oxy]carbamoyl]phenyl)-2-oxo-1 ,2-dihydroquinoline-3 -carboxamide

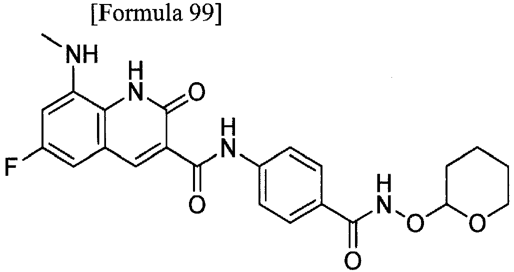

To a solution in DMF (4.5 mL) of 4-amino-N-[(oxan-2-yl)oxy]benzamide (160 mg) and the 6-fluoro-8-(methylamino)-2-oxo-1,2-dihydroquinoline-3-carboxylic acid (133 mg) obtained in Production Example 2-(5), HATU (257 mg) and DIPEA (343 µL) were added and the mixture was stirred for 15 hours at room temperature. To the reaction mixture, water was added and extraction was conducted with ethyl acetate. The organic layer was washed with a saturated aqueous solution of sodium hydrogencarbonate and saturated brine, dried over anhydrous magnesium sulfate, and concentrated under reduced pressure, whereupon the titled compound (192 mg) was obtained as a yellow solid.
MS (ESI neg.) m/z : 453 [M-H]-, RT=0.926 min (Analytical Condition 3)

### 92-(2) 6-Fluoro-N-[4-(hydroxycarbamoyl)phenyl]-8-(methylamino)-2-oxo-1,2-dihydroquinoline-3 -carboxamide

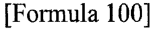

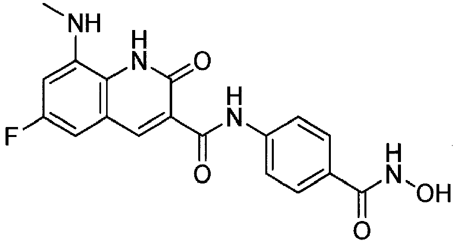

To a solution in methanol (8 mL) of the 6-fluoro-8-(methylamino)-N-(4-[[(oxan-2-yl)oxy]carbamoyl]phenyl)-2-oxo-1,2-dihydroquinoline-3-carboxamide (192 mg) obtained in Example 92-(1), tosic acid monohydrate (40 mg) was added and the mixture was stirred for 65 hours at room temperature. The precipitating solid was recovered by filtration and dissolved in ethyl acetate, followed by a liquid-liquid seprating operation with an aqueous solution of 2 M sodium hydroxide. The solid precipitating from the aqueous layer was recovered by filtration and thereafter washed with water to obtain the titled compound (8 mg) as a yellow solid.
MS (ESI pos.) m/z : 371 [M+H]+, RT=0.738 min (Analytical Condition 3)
¹H NMR (400 MHz, DMSO-d6) δ ppm 2.85 (d, J=4.6 Hz, 3 H), 6.38 - 6.53 (m, 2 H), 6.89 (br d, J=8.6 Hz, 1 H), 7.78 (s, 4 H), 8.72 (s, 1H), 8.94 (br s, 1H), 11.14 (br s, 1 H), 11.86 (br s, 1 H), 13.43 (s, 1 H)

### Example 100 3-[[Cyclohexyl(methyl)amino]methyl]-4-[[7-fluoro-5-(methylamino)-3 -oxo-3,4-dihydroquinoxaline-2-carbonyl] amino]benzoic acid

### 100-(1) Methyl 3-[[cyclohexyl(methyl)amino]methyl]-4-[[7-fluoro-5-(methylamino)-3-oxo-3,4-dihydroquinoxaline-2-carbonyl]amino]benzoate

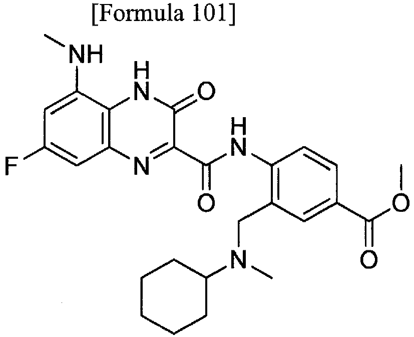

To a solution in DMF (3.5mL) of methyl 4-amino-3-[[cyclohexyl(methyl)amino]methyl]benzoate-hydrochloride (47 mg) and the 7-fluoro-5-(methylamino)-3-oxo-3,4-dihydroquinoxaline-2-carboxylic acid (30 mg) obtained in Production Example 5-(6), HATU (58 mg) and DIPEA (110 µL) were added and the mixture was stirred for 16 hours at room temperature. The reaction mixture was concentrated under reduced pressure; thereafter, a saturated aqueous solution of sodium hydrogencarbonate was added and extraction was conducted with ethyl acetate. The organic layer was washed with saturated brine, dried over anhydrous magnesium sulfate, and concentrated under reduced pressure. The residue was purified by column chromatography (silica gel cartridge, hexane/ethyl acetate = 80:20) to obtain the titled compound (15 mg) as a red solid.
MS (ESI pos.) m/z : 496 [M+H]+, RT=0.824 min (Analytical Condition 3)

### 100-(2) 3-[[Cyclohexyl(methyl)amino]methyl]-4-[[7-fluoro-5-(methylamino)-3-oxo-3,4-dihydroquinoxaline-2-carbonyl] amino]benzoic acid

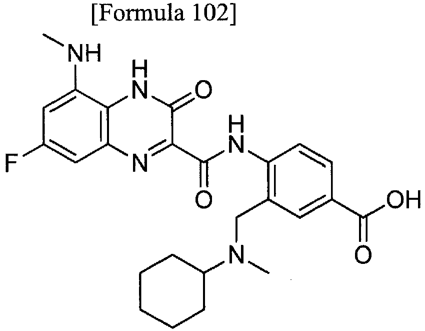

To a solution in tetrahydrofuran (1 mL) of the methyl 3-[[cyclohexyl(methyl)amino]methyl]-4-[[7-fluoro-5-(methylamino)-3-oxo-3,4-dihydroquinoxaline-2-carbonyl]amino]benzoate (15 mg) obtained in Example 100-(1), 1 M sodium hydroxide in aqueous solution (1 mL) was added and the mixture was stirred for 19 hours at room temperature. To the reaction mixture, an aqueous solution of 1 M hydrochloric acid was added to establish pH 5; the precipitating solid was recovered by filtration and washed with water, whereafter the titled compound (7 mg) was obtained as a red solid.
MS (ESI pos.) m/z : 482 [M+H]+, RT=0.680 min (Analytical Condition 3)
¹H NMR (400 MHz, Pyridine-d5) δ ppm 0.96 - 1.76 (m, 10 H), 2.41 (s, 3 H), 2.68 (br t, J=11.7 Hz, 1 H), 2.95 (s, 3 H), 3.86 - 3.97 (m, 2 H), 6.61 (dd, J=11.6, 2.5 Hz, 1 H), 7.13 (dd, J=9.5, 2.5 Hz, 1 H), 8.42 (d, J=1.7 Hz, 1 H), 8.49 - 8.57 (m, 1H), 8.87 (d, J=8.6 Hz, 1 H), 13.53 (br s, 1 H)

### Example 108 3-[[Cyclohexyl(methyl)amino]methyl]-4-[[8-(methylamino)-2-oxo-1,2-dihydro-1,6-naphthyridine-3-carbonyl]amino]benzoic acid

### 108-(1) Methyl 4-([8-[(tert-butoxycarbonyl)(methyl)amino]-2-[(4-methoxyphenyl)methoxy]-1,6-naphthyridine-3-carbonyl]amino)-3-[[cyclohexyl(methyl)amino]methyl]benzoate

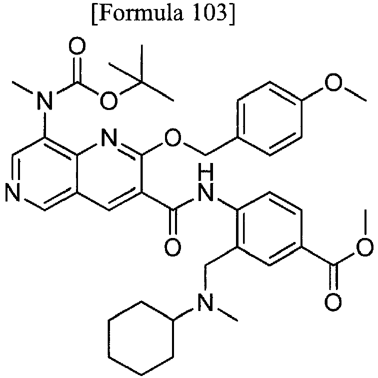

To a solution in DMF (1 mL) of the 8-[(tert-butoxycarbonyl)(methyl)amino]-2-[(4-methoxyphenyl)methoxy]-1,6-naphthyridine-3-carboxylic acid (71 mg) obtained in Production Example 4-(4), HATU (68 mg), methyl 4-amino-3-[[cyclohexyl(methyl)amino]methyl]benzoate-hydrochloride (56 mg) and DIPEA (170 µL) were added and the mixture was stirred for 2.5 days at room temperature. The reaction mixture was diluted with ethyl acetate and washed with water. The organic layer was dried over anhydrous magnesium sulfate and concentrated under reduced pressure. The residue was purified by column chromatography (silica gel cartridge, hexane/ethyl acetate = 93:7 - 0:100) to obtain the titled compound (23 mg) as a yellow solid.
MS (ESI pos.) m/z : 698[M+H]+, RT=0.820 min (Analytical Condition 3)

### 108-(2) Methyl 3-[[cyclohexyl(methyl)amino]methyl]-4-[[8-(methylamino)-2-oxo-1,2-dihydro-1,6-naphthyridine-3-carbonyl] amino]benzoate

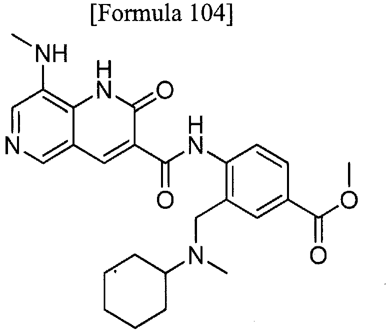

To a solution in chloroform (200 µL) of the methyl 4-([8-[(tert-butoxycarbonyl)(methyl)amino]-2-[(4-methoxyphenyl)methoxy]-1,6-naphthyridine-3-carbonyl]amino)-3-[[cyclohexyl(methyl)amino]methyl]benzoate (23 mg) obtained in Example 108-(1), TFA (20 µL) was added and the mixture was stirred for 6 hours at room temperature. The reaction mixture was concentrated. The residue was purified by column chromatography (NH silica gel cartridge, chloroform/methanol = 100:0 - 70:30) to obtain the titled compound (8 mg) as a yellow solid.
MS (ESI pos.) m/z : 478[M+H]+, RT=0.206 min (Analytical Condition 3)

### 108-(3) 3-[[Cyclohexyl(methyl)amino]methyl]-4-[[8-(methylamino)-2-oxo-1,2-dihydro-1,6-naphthyridine-3-carbonyl]amino]benzoic acid

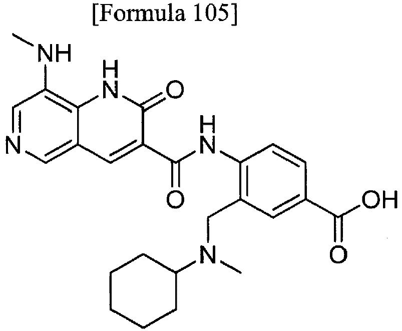

To a solution in THF (0.5 mL) of the methyl 3-[[cyclohexyl(methyl)amino]methyl]-4-[[8-(methylamino)-2-oxo-1,2-dihydro-1,6-naphthyridine-3-carbonyl]amino]benzoate (8 mg) obtained in Example 108-(2), 2 M sodium hydroxide in aqueous solution (0.5 mL) was added and the mixture was stirred for 2 hours at room temperature, followed by warming to 50°C and stirring for 4 hours. The THF in the reaction mixture was concentrated. To the aqueous solution obtained, 1 M hydrochloric acid was added to establish pH 5 and the precipitating solid was recovered by filtration, whereupon the titled compound (7 mg) was obtained as a yellow solid.
MS (ESI pos.) m/z : 464 [M+H]+, RT=0.618 min (Analytical Condition 3)
¹H NMR (400 MHz, DMSO-d6) δ ppm 0.98 - 2.24 (m, 10 H), 2.64 - 2.73 (m, 1 H), 2.91 (d, J=4.8 Hz, 3 H), 3.78 (br s, 3 H), 4.40 (s, 2 H), 6.19 (br s, 1 H), 7.84 - 8.11 (m, 3 H), 8.42 (d, J=8.1 Hz, 1 H), 8.53 (br s, 1 H), 8.99 (s, 1 H), 12.10 (br s, 2 H), 12.87 (br s, 1 H)

The same procedures as in Example 108 were employed to obtain the compound of Example 112 from the corresponding raw material.

**[Table 11]**

| Example | Structural Formula | Compound Name | MS | NMR |
|---|---|---|---|---|
| 108 | 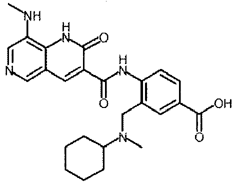 | 3-{[cyclohexyl(methyl) amino]methyl}-4-{[8-(methylamino)-2-oxo-1,2-dihydro-1,6-naphthyridine-3-carbonyl]amino}benzoic acid | MS (ESI pos.) m/z: 464 [M+H]+ RT=0.618 min (Analytical Condition 3) | 1H NMR (400 MHz, DMSO-d6) δ ppm 0.98 - 2.24 (m, 10 H), 2.64 - 2.73 (m, 1 H), 2.91 (d, J=4.8 Hz, 3 H), 3.78 (br s, 3 H), 4.40 (s, 2 H), 6.19 (br s, 1 H), 7.84 - 8.11 (m, 3 H), 8.42 (d, J=8.1 Hz, 1 H), 8.53 (brs, 1 H), 8.99 (s, 1 H), 12.10 (brs, 2 H), 12.87 (br s, 1 H) |
| 112 | 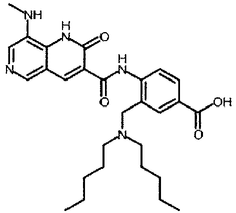 | 3-[(dipentylamino) methyl]4-{[8-(methylamino)-2-oxo-1,,2-dihydro-1,6-naphthyridine-3-carbonyl]amino}benzoic acid | MS (ESI pos.) m/z: 508 [M+H]+ RT=0.475 min (Analytical Condition 3) | 1H NMR (400 MHz, Solvent) δ ppm 0.74 - 0.83 (m, 6 H), 1.14 -1.23 (m, 8 H), 1.48 -1.59 (m, 4 H), 2.60 - 2.69 (m, 4 H), 2.93 (s, 3 H), 3.93 (s, 2 H), 8.26 (s, 1 H), 8.55 (dd, J=8.6, 1.7 Hz, 1 H), 8.64 (d, J=1.7 Hz, 1 H), 8.69 (s, 1 H), 9.12 (d, J=8.6 Hz, 1 H), 9.31 (s, 1 H), 12.60 (br s, 1 H) |

### Example 109 3-[(3-Aminopiperidin-1-yl)methyl]-4-[[6-fluoro-8-(methylamino)-2-oxo-1,2-dihydroquinoline-3-carbonyl]amino]benzoic acid-hydrochloride

### 109-(1) tert-Butyl 3-([3-[(tert-butoxycarbonyl)amino]piperidin-1-yl]methyl)-4-[[6-fluoro-8-(methylamino)-2-oxo-1,2-dihydroquinoline-3-carbonyl]amino]benzoate

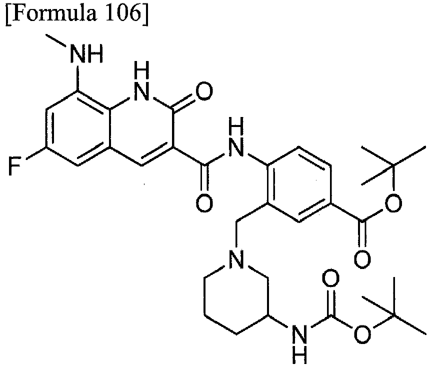

To a solution in DMF (1 mL) of tert-butyl 4-amino-3-([3-[(tert-butoxycarbonyl)amino]piperidin-1-yl]methyl)benzoate (68 mg) and the 6-fluoro-8-(methylamino)-2-oxo-1,2-dihydroquinoline-3-carboxylic acid (36 mg) obtained in Production Example 2-(5), HATU (64 mg) and DIPEA (80 µL) were added and the mixture was stirred for 18 hours at room temperature. The reaction mixture was diluted with ethyl acetate and washed with water and saturated aqueous sodium bicarbonate. The organic layer was dried over anhydrous magnesium sulfate and concentrated under reduced pressure. The residue was purified by column chromatography (silica gel cartridge, hexane/ethyl acetate = 95:5 - 60:40) to obtain the titled compound (32 mg) as a colorless solid.
MS (ESI pos.) m/z : 624[M+H]+, RT=0.985 min (Analytical Condition 3)

### 109-(2) 3-[(3-Aminopiperidin-1-yl)methyl]-4-[[6-fluoro-8-(methylamino)-2-oxo-1,2-dihydroquinoline-3-carbonyl]amino]benzoic acid-hydrochloride

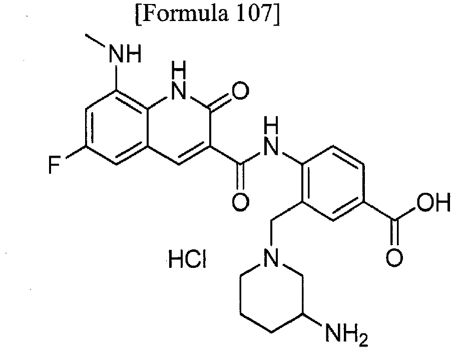

To the tert-butyl 3-([3-[(tert-butoxycarbonyl)amino]piperidin-1-yl]methyl)-4-[[6-fluoro-8-(methylamino)-2-oxo-1,2-dihydroquinoline-3-carbonyl]amino]benzoate (32 mg) obtained in Example 109-(1), 4 M hydrochloric acid in dioxane solution (1 mL) was added and the mixture was stirred for 5 hours at room temperature. The reaction mixture was concentrated to obtain the titled compound (15mg) as a yellow solid.
MS (ESI pos.) m/z : 468 [M+H]+, RT=0.573 min (Analytical Condition 3)
¹H NMR (400 MHz, DMSO-d6) δ ppm 1.31 - 2.17 (m, 9 H), 2.86 (s, 3 H), 3.57 (s, 2 H), 6.60 - 6.76 (m, 2 H), 7.10 (br d, J=7.9 Hz, 1 H), 7.61 - 7.81 (m, 1 H), 8.37 - 8.43 (m, 1 H), 8.53 (br d, J=8.6 Hz, 1 H), 8.92 (br s, 1 H), 12.21 (br s, 1 H), 12.69 (br s, 1 H)

The same procedures as in Example 109 were employed to obtain the Example compounds identified below from the corresponding raw materials.

**[Table 12]**

| Example | Structural Formula | Compound Name | MS | NMR |
|---|---|---|---|---|
| 109 | 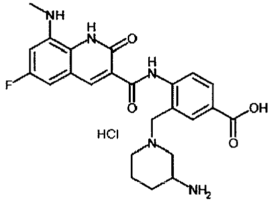 | 3-[(3-aminopiperidine-1-yl)methyl]-4-{[6-fluoro-8-(methylamino)-2-oxo-1,2-dihydroquinoline-3-carbonyl]amino }benzoic acid-hydrochloride | MS (ESI pos.) m/z: 468 [M+H]+ RT=0.573 min (Analytical Condition 3) | 1H NMR (400 MHz, DMSO-d6) δ ppm 1.31 - 2.17 (m 9 H), 2.86 (s, 3 H), 3.57 (s, 2 H), 6.60 - 6.76 (m, 2 H), 7.10 (br d, J=7.9 Hz, 1 H), 7.61 - 7.81 (m, 1H), 8.37 - 8.43 (m, 1H), 8.53 (br d, J=8.6 Hz, 1H), 8.92 (br s, 1H), 12.21 (br s, 1H), 12.69 (br s, 1H) |
| 110 | 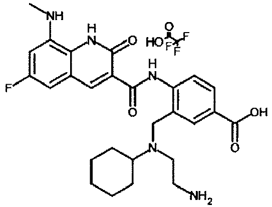 | 3-{[(2-aminoethyl) (cyclohexyl)amino] methyl}-4-{[6-fluoro-8-(methylamino)-2-oxo-1,2-dihydroquinoline-3-carbonyl]amino}benzoic acid-trifluroacetate | MS (ESI pos.) m/z: 510 [M+H]+ RT=0.655 min (Analytical Condition 3) | 1H NMR (600 MHz, PYRIDINE-d5) δ ppm 1.02 - 1.82 (m, 13 H), 2.88 (s, 3 H), 2.88 -2.93 (m, 2 H), 3.16 - 3.22 (m, 2 H), 6.47 (br s, 1 H), 6.74 (dd, J=11.4, 2.7 Hz, 1 H), 6.99 (dd, J=8.5, 2.7 Hz, 1 H), 8.48 - 8.60 (m, 3 H), 9.25 (s, 1 H) |
| 113 | 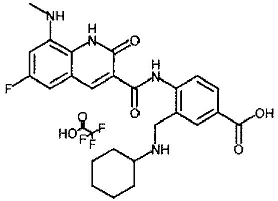 | 3-[(cyclohexylamino) methyl]-4-{[6-fluoro-8-(methylamino)-2-oxo-1,2-dihydroquinoline-3-carbonyl]amino)benzoic acid-trifluoroacetate | MS (ESI pos.) m/z: 467 [M+H]+ RT=0.660 min (Analytical Condition 3) | 1H NMR (400 MHz, METHANOL-d4) δ ppm 1.36 -1.53 (m, 5 H), 1.72 -1.80 (m, 1 H), 1.88 -1.96 (m, 2 H), 2.23 - 2.32 (m, 2 H), 2.96 (s, 3 H), 3.10 - 3.16 (m, 1 H), 4.41 (s, 2 H), 6.70 (dd, J=11.4, 2.3 Hz, 1 H), 6.92 (dd, J=8.4, 2.3 Hz, 1 H), 8.15 (dd, J=8.4, 1.5 Hz, 1 H), 8.26 (d, J=1.5 Hz, 1 H), 8.31 (d, J=8.4 Hz, 1H), 8.95 (s, 1 H) |

### Example 114 3-[Cyclohexyl(methyl)amino]-4-[[6-fluoro-8-(methylamino)-2-oxo-1,2-dihydroquinoline-3-carbonyl]amino]benzoic acid

### 114-(1) tert-Butyl 3-[cyclohexyl(methyl)amino]-4-[[6-fluoro-8-(methylamino)-2-oxo-1,2-dihydroquinoline-3-carbonyl] amino]benzoate

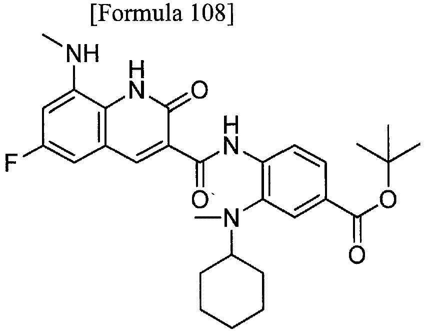

To a solution in DMF (1 mL) of the 6-fluoro-8-(methylamino)-2-oxo-1,2-dihydroquinoline-3-carboxylic acid (60 mg) obtained in Production Example 2-(5), tert-butyl 4-amino-3-[cyclohexyl(methyl)amino]benzoate (85 mg), HATU (106 mg) and DIPEA (133 µL) were added and the mixture was stirred for 17 hours at room temperature. The suspended matter in the reaction mixture was recovered by filtration. The solid obtained was washed with saturated aqueous sodium bicarbonate and water. Upon drying, the titled compound (70 mg) was obtained as a yellow solid.
MS (ESI pos.) m/z : 523[M+H]+, RT=1.177 min (Analytical Condition 4)

### 114-(2) 3-[Cyclohexyl(methyl)amino]-4-[[6-fluoro-8-(methylamino)-2-oxo-1,2-dihydroquinoline-3 -carbonyl] amino]benzoic acid

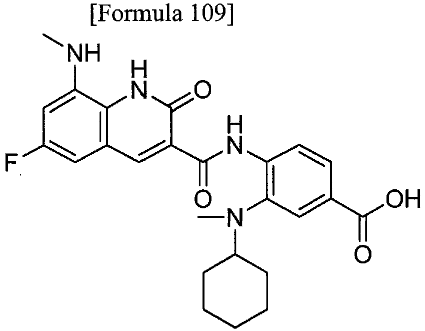

To a solution in chloroform (1 mL) of the tert-butyl 3-[cyclohexyl(methyl)amino]-4-[[6-fluoro-8-(methylamino)-2-oxo-1,2-dihydroquinoline-3-carbonyl]amino]benzoate (61 mg) obtained in Example 114-(1), TFA (1 mL) was added and the mixture was stirred for 20 hours at room temperature. The reaction mixture was concentrated to obtain the titled compound (51 mg) as a yellow solid.
MS (ESI pos.) m/z : 467 [M+H]+, RT=1.217 min (Analytical Condition 3)
¹H NMR (400 MHz, DMSO-d6) δ ppm 1.02 - 1.22 (m, 3 H), 1.29 - 1.41 (m, 2 H), 1.48 - 1.56 (m, 1 H), 1.66 - 1.75 (m, 2 H), 1.84 - 1.92 (m, 2 H), 2.64 (s, 3 H), 2.76 - 2.83 (m, 1H), 2.85 (s, 3 H), 6.63 (dd, J=11.7, 2.6 Hz, 1 H), 7.06 (dd, J=8.8, 2.6 Hz, 1 H), 7.75 (dd, J=8.6, 2.0 Hz, 1 H), 7.81 (d, J=2.0 Hz, 1 H), 8.66 (d, J=8.6 Hz, 1 H), 8.89 (s, 1 H), 11.82 (s, 1 H), 12.70 (s, 1H)

The same procedures as in Example 114 were employed to obtain the Example compounds identified below from the corresponding raw materials.

**[Table 13]**

| Example | Structural Formula | Compound Name | MS | NMR |
|---|---|---|---|---|
| 114 | 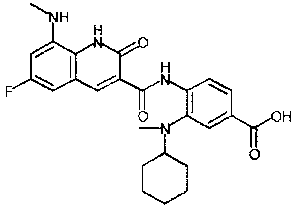 | -[cyclohexyl(methyl) amino]-4-{[6-fluoro-8-(methylamino)-2-oxo-1,2-dihydroquinoline-3-carbonyl]amino}benzoic acid | MS (ESI pos.) m/z: 467 [M+H]+ RT=1.217 min (Analytical Condition 3) | 1H NMR (400 MHz, DMSO-d6) δ ppm 1.02 - 1.22 (m, 3 H), 1.29 -1.41 (m, 2 H), 1.48 - 1.56 (m, 1 H), 1.66 -1.75 (m, 2 H), 1.84 - 1.92 (m, 2 H), 2.64 (s, 3 H), 2.76 - 2.83 (m, 1 H), 2.85 (s, 3 H), 6.63 (dd, J=11.7, 2.6 Hz, 1 H), 7.06 (dd, J=8.8, 2.6 Hz, 1 H), 7.75 (dd, J=8.6, 2.0 Hz, 1 H), 7.81 (d, J=2.0 Hz, 1 H), 8.66 (d, J=8.6 Hz, 1 H), 8.89 (s, 1 H), 11.82 (s, 1 H), 12.70 (s, 1 H) |
| 125 | 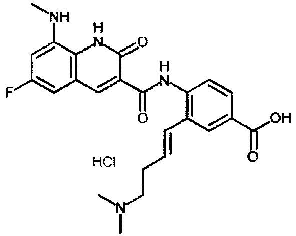 | 3-[(1E)-4-(dimethylamino)but-1-en-1-yl]-4-{[6-fluoro-8-(methylamino)-2-oxo-1,2-dihydroquinoline-3-carbonyl]amino}benzoic acid-hydrochloride | MS (ESI pos.) m/z: 453 [M+H]+ RT=0.625 min (Analytical Condition 3) | 1H NMR (400 MHz, DMSO-d6) δ ppm 2.64 - 2.74 (m, 2 H), 2.77 - 2.88 (m, 9 H), 3.57 - 3.62 (m, 1 H), 3.66 - 3.72 (m, 1 H), 6.21 - 6.33 (m, 1 H), 6.66 (br dd, J=11.6, 2.7 Hz, 1 H), 6.82 - 6.94 (m, 1 H), 7.09 (dd, J=8.7, 2.7 Hz, 1 H), 7.90 (br dd, J=8.5, 1.5 Hz, 1 H), 8.08 (td, J=8.5, 1.2 Hz, 1 H), 8.43 - 8.51 (m, 1 H), 8.93 (s, 1 H), 9.75 (br s, 1 H), 12.08 (s, 1 H) |
| 162 | 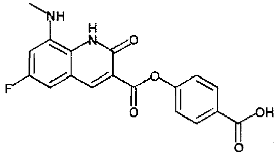 | 4-{[6-fluoro-8-(methylamino)-2-oxo-1,2-dihydroquinoline-3-carbonyl]oxy}benzoic acid | MS (ESI pos.) m/z: 357 [M+H]+ RT=0.859 min (Analytical Condition 3) | 1H NMR (600 MHz, DMSO-d6) δ ppm 2.84 (d, J=4.5 Hz, 3 H), 6.44 (brd, J=4.5 Hz, 1 H), 6.61 (dd, J=11.8, 2.7 Hz, 1 H), 6.95 (dd, J=8.7, 2.7 Hz, 1 H), 7.39 (d, J=8.7 Hz, 2 H), 8.02 - 8.07 (m, 2 H), 8.77 (s, 1 H), 11.39 (br s, 1 H), 13.07 (br s, 1 H) |

### Example 117 4-[[6-Fluoro-4-hydroxy-8-(methylamino)-2-oxo-1,2-dihydroquinoline-3 -carbonyl] amino]benzoic acid

### 117-(1) Methyl 3-[benzyl(methyl)amino]-2-[3-[4-(tert-butoxycarbonyl)anilino]-3-oxopropanamido]-5-fluorobenzoate

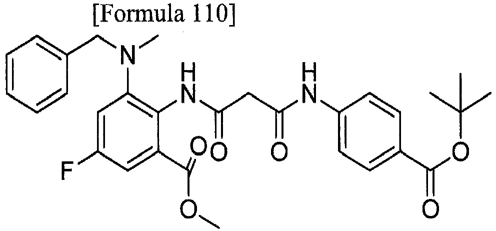

To a solution in chloroform (60 mL) of the 3-[4-(tert-butoxycarbonyl)anilino]-3-oxopropanoic acid (5.00 g) obtained in Production Example 7-(2), dimethylformamide (140 µL) and oxalyl chloride (3.03 mL) were added and the mixture was stirred for an hour at room temperature. The reaction mixture was concentrated under reduced pressure and, thereafter, the residue was dissolved in chloroform (60 mL); triethylamine (4.98 mL) and a solution in chloroform (100 mL) of the methyl 2-amino-3-[benzyl(methyl)amino]-5-fluorobenzoate (4.90 g) obtained in Production Example 6-(2) were added and the resulting mixture was stirred for half an hour at room temperature. To the reaction mixture, a saturated aqueous solution of sodium hydrogencarbonate was added and extraction was conducted with chloroform. The organic layer was passed through a phase separator and concentrated under reduced pressure. The residue was purified by column chromatography (silica gel cartridge, hexane/ethyl acetate = 75:25 - 45:55) to obtain the titled compound (4.02 g) as a pale yellow amorphous.
MS (ESI pos.) m/z : 550 [M+H]+, RT=0.980 min (Analytical Condition 4)

### 117-(2) tert-Butyl 4-([8-[benzyl(methyl)amino]-6-fluoro-4-hydroxy-2-oxo-1,2-dihydroquinoline-3 -carbonyl] amino)benzoate

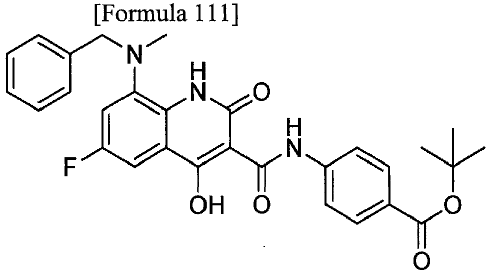

To a solution in ethanol (150 mL) of the methyl 3-[benzyl(methyl)amino]-2-[3-[4-(tert-butoxycarbonyl)anilino]-3-oxopropanamido]-5-fluorobenzoate (4.02 g) obtained in Example 117-(1), 20% sodium ethoxide in ethanol solution (4.8 mL) was added and the mixture was stirred for 2 hours at room temperature. After being diluted with water, the reaction mixture was brought to pH 1 with an aqueous solution of 1 M hydrochloric acid. The precipitating solid was recovered by filtration and therafter washed with water to obtain the titled compound (3.77 g) as a yellow solid.
MS (ESI pos.) m/z : 518 [M+H]+, RT=1.233 min (Analytical Condition 4)

### 117-(3) tert-Butyl 4-[[6-fluoro-4-hydroxy-8-(methylamino)-2-oxo-1,2-dihydroquinoline-3-carbonyl]amino]benzoate

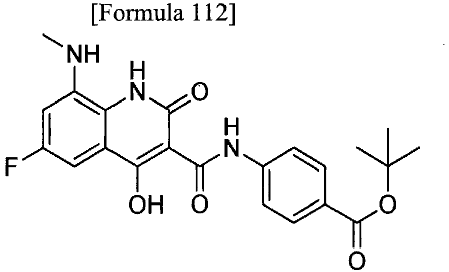

To a solution in methanol (30 mL) of the tert-butyl 4-([8-[benzyl(methyl)amino]-6-fluoro-4-hydroxy-2-oxo-1,2-dihydroquinoline-3-carbonyl]amino)benzoate (150 mg) obtained in Example 117-(2), 5% palladium on carbon (100 mg) was added and the mixture was stirred for an hour at room temperature under a hydrogen atmosphere. The insoluble matter was removed by filtration and, thereafter, the filtrate was concentrated under reduced pressure to obtain the titled compound (99 mg) as a pale yellow solid.
MS (ESI neg.) m/z : 426 [M-H]-, RT=1.456 min (Analytical Condition 3)

### 117-(4) 4-[[6-Fluoro-4-hydroxy-8-(methylamino)-2-oxo-1,2-dihydroquinoline-3-carbonyl]amino]benzoic acid

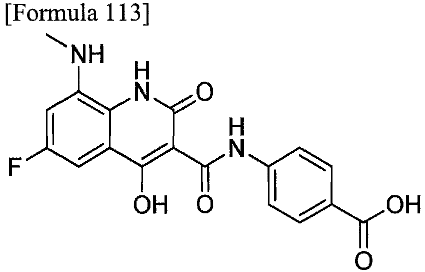

To a solution in chloroform (5 mL) of the tert-butyl 4-[[6-fluoro-4-hydroxy-8-(methylamino)-2-oxo-1,2-dihydroquinoline-3-carbonyl]amino]benzoate (99 mg) obtained in Example 117-(3), TFA (8 mL) was added and the mixture was stirred for 2 hours at room temperature. After concentrating the mixture under reduced pressure, chloroform was added to the residue. The precipitating solid was recovered by filtration and washed with chloroform to obtain the titled compound (85 mg) as a pale yellow solid.
MS (ESI pos.) m/z : 372 [M+H]+, RT=1.133 min (Analytical Condition 3)
¹H NMR (400 MHz, DMSO-d6) δ ppm 2.84 (d, J=4.2 Hz, 3 H), 6.54 (br d, J=4.2 Hz, 1 H), 6.66 (dd, J=11.5, 1.8 Hz, 1 H), 6.92 (dd, J=8.4, 1.8 Hz, 1 H), 7.78 (br d, J=8.6 Hz, 2 H), 7.99 (br d, J=8.6 Hz, 2 H), 11.32 (s, 1 H), 12.86 (br s, 1 H), 12.93 (s, 1 H), 16.06 (s, 1 H)

The same procedures as in Example 117 were employed to obtain the Example compounds identified below from the corresponding raw materials.

**[Table 14]**

| Example | Structural Formula | Compound Name | MS | NMR |
|---|---|---|---|---|
| 117 | 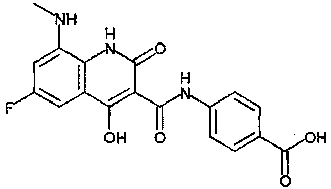 | 4-{[6-fluoro-4-hydroxy-8-(methylamino)-2-oxo-1,2-dihydroquinoline-3-carbonyl]amino}benzoic acid | MS (ESI pos.) m/z: 372 [M+H]+ RT=1.133 min (Analytical Condition 3) | 1H NMR (400 MHz, DMSO-d6) δ ppm 2.84 (d, J=4.2 Hz, 3 H), 6.54 (br d, J=4.2 Hz, 1 H), 6.66 (dd, J=11.5, 1.8 Hz, 1 H), 6.92 (dd, J=8.4, 1.8 Hz, 1H), 7.78 (br d, J=8.6 Hz, 2 H), 7.99 (br d, J=8.6 Hz, 2 H), 11.32 (s, 1 H), 12.86 (brs, 1 H), 12.93 (s, 1 H), 16.06 (s, 1 H) |
| 129 | 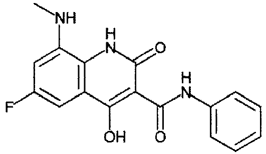 | 6-fluoro-4-hydroxy-8-(methylamino)-2-oxo-N-dihydroquinoline-3-carboxamide | MS (ESI pos.) m/z: 326 [M-H]-RT=0.990 min (Analytical Condition 4) | 1H NMR (400 MHz, DMSO-d6) δ ppm 2.84 (d, J=4.5 Hz, 3 H), 6.53 (d, J=4.5 Hz, 1H), 6.66 (dd, J=11.6, 2.5 Hz, 1 H), 6.92 (dd, J=9.0, 2.5 Hz, 1 H), 7.21 (t, J=7.7 Hz, 1 H), 7.43 (t, J=7.7 Hz, 2 H), 7.66 (br d, J=7.7 Hz, 2 H), 11.26 (s, 1 H), 12.64 (s, 1 H) |
| 138 | 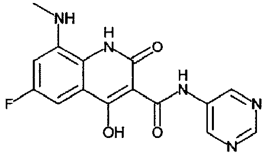 | 6-fluoro-4-hydroxy-8-(methylamino)-2-oxo-N-(pyrimidine-5-yl)-1,2-dihydroquinoline-3-carboxamide | MS (ESI pos.) m/z: 330 [M+H]+ RT=0.994 min (Analytical Condition 3) | NT |
| 153 | 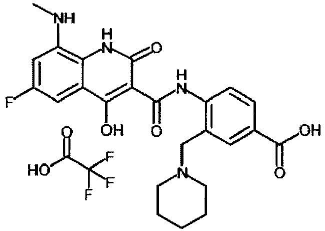 | 4-{[6-fluoro-4-hydroxy-8-(methylamino)-2-oxo-1,2-dihydroquinoline-3-carbonyl]amino}-3-[(piperidine-1-yl)methyl]benzoic acid-trifluoroacetate | MS (ESI pos.) m/z : 469 [M+H]+ RT=0.662 min (Analytical Condition 4) | 1H NMR (400 MHz, DMSO-d6) δ ppm 1.24 -1.46 (m, 1 H), 1.53 -1.77 (m, 3 H), 1.78 - 1.90 (m, 2 H), 2.86 (s, 3 H), 2.95 - 3.13 (m, 2 H), 3.49 (br d, J=11.2 Hz, 2 H), 4.47 (br s, 2 H), 6.57 (brs, 1 H), 6.71 (dd, J=11.7, 2.6 Hz, 1 H), 6.95 (dd, J=8.7, 2.6 Hz, 1 H), 8.10 (br d, J=7.3 Hz, 1 H), 8.23 (s, 1 H), 8.28 (d, J=8.6 Hz, 1 H), 9.36 (br s, 1 H), 11.48 (s, 1 H), 12.96 (s, 1 H), 13.19 (br s, 1 H) |

### Example 118 4-[[6-Fluoro-4-methoxy-8-(methylamino)-2-oxo-1,2-dihydroquinoline-3-carbonyl]amino]benzoic acid

### 118-(1) Methyl 3-[benzyl(methyl)amino]-2-(3-[[4-(tert-butoxycarbonyl)phenyl][(2,4-dimethoxyphenyl)methyl]amino]-3-oxopropanamido)-5-fluorobenzoate

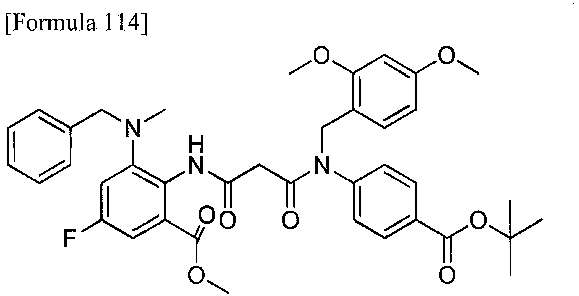

To a solution in chloroform (48 mL) of the 3-[[4-(tert-butoxycarbonyl)phenyl][(2,4-dimethoxyphenyl)methyl]amino]-3-oxopropanoic acid (4.08 g) obtained in Production Example 8-(3), dimethylformamide (73 µL) and oxalyl chloride (1.61 mL) were added and the mixture was stirred for an hour at room temperature. After concentrating the reaction mkxture under reduced pressure, the residue was dissolved in chloroform (48 mL); triethylamine (2.65 mL) and a solution in chloroform (48 mL) of the methyl 2-amino-3-[benzyl(methyl)amino]-5-fluorobenzoate (2.60 g) obtained in Production Example 6-(2) were added and the mixture was stirred for an hour at room temperature. To the reaction mixture, a saturated aqueous solution of sodium hydrogencarbonate was added and extraction was conducted with chloroform. The organic layer was passed through a phase separator and concentrated under reduced pressure. The residue was purified by column chromatography (silica gel cartridge, hexane/ethyl acetate = 80:20 - 70:30) to obtain the titled compound (4.35 g) as a pale yellow amorphous.
MS (ESI pos.) m/z : 700 [M+H]+, RT=1.048 min (Analytical Condition 4)

### 118-(2) tert-Butyl 4-([8-[benzyl(methyl)amino]-6-fluoro-4-hydroxy-2-oxo-1,2-dihydroquinoline-3-carbonyl][(2,4-dimethoxyphenyl)methyl]amino)benzoate

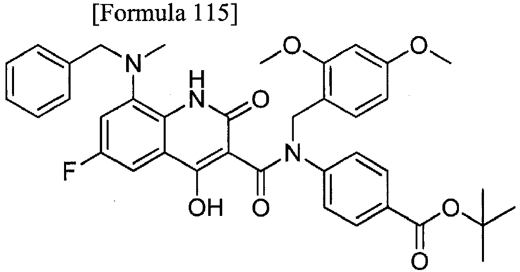

To a solution in ethanol (150 mL) of the methyl 3-[benzyl(methyl)amino]-2-(3-[[4-(tert-butoxycarbonyl)phenyl][(2,4-dimethoxyphenyl)methyl]amino]-3-oxopropanamido)-5-fluorobenzoate (4.35 g) obtained in Example 118-(1), 20% sodium ethoxide in ethanol solution (8.1 mL) was added and the mixture was stirred under heating for an hour at 75°C. After being left to cool, the reaction mixture was diluted with water and brought to pH 1 with an aqueous solution of 1 M hydrochloric acid. The precipitating solid was recovered by filtration and thereafter washed with water to obtain the titled compound (3.35 g) as a colorless solid.
MS (ESI neg.) m/z : 666 [M-H]-, RT=0.997 min (Analytical Condition 4)

### 118-(3) tert-Butyl 4-([8-[benzyl(methyl)amino]-6-fluoro-4-methoxy-2-oxo-1,2-dihydroquinoline-3-carbonyl][(2,4-dimethoxyphenyl)methyl]amino)benzoate

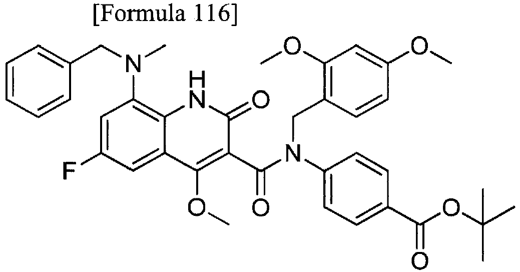

To a solution in acetonitrile (4.5 mL) and methanol (1.5 mL) of DIPEA (78 µL) and the tert-butyl 4-([8-[benzyl(methyl)amino]-6-fluoro-4-hydroxy-2-oxo-1,2-dihydroquinoline-3-carbonyl][(2,4-dimethoxyphenyl)methyl]amino)benzoate (150 mg) obtained in Example 118-(2), 2 M trimethylsilyldiazomethane in diethyl ether solution (169 µL) was added and the mixture was stirred for half an hour at room temperature. The reaction mixture was concentrated under reduced pressure and thereafter purified by column chromatography (silica gel cartridge, hexane/ethyl acetate = 60:40 - 25:75) to obtain the titled compound (117 mg) as a yellow oil.
MS (ESI pos.) m/z : 704 [M+Na]+, RT=1.057 min (Analytical Condition 4)

### 118-(4) tert-Butyl 4-[[(2,4-dimethoxyphenyl)methyl][6-fluoro-4-methoxy-8-(methylamino)-2-oxo-1,2-dihydroquinoline-3-carbonyl]amino]benzoate

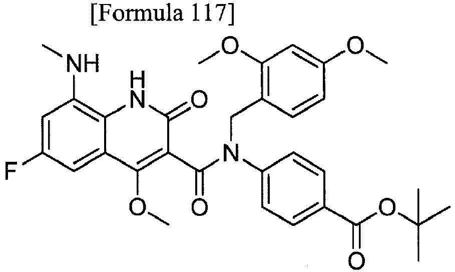

To a solution in methanol (20 mL) of the tert-butyl 4-([8-[benzyl(methyl)amino]-6-fluoro-4-methoxy-2-oxo-1 ,2-dihydroquinoline-3-carbonyl] [(2,4-dimethoxyphenyl)methyl]amino)benzoate (103 mg) obtained in Example 118-(3), 5% palladium on carbon (80 mg) was added and the mixture was stirred for an hour at room temperature under a hydrogen atmosphere. After removing the insoluble matter by filtration, the filtrate was concentrated under reduced pressure to obtain the titled compound (15 mg) as a colorless solid.
MS (ESI neg.) m/z : 590 [M-H]-, RT=0.951 min (Analytical Condition 4)

### 118-(5) 4-[[6-Fluoro-4-methoxy-8-(methylamino)-2-oxo-1,2-dihydroquinoline-3-carbonyl](trifluoroacetyl)amino]benzoic acid

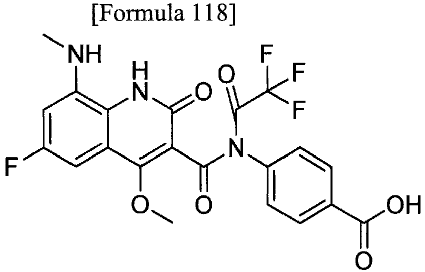

To a solution in chloroform (5 mL) of the tert-butyl 4-[[(2,4-dimethoxyphenyl)methyl] [6-fluoro-4-methoxy-8-(methylamino)-2-oxo-1 ,2-dihydroquinoline-3-carbonyl]amino]benzoate (15 mg) obtained in Example 118-(4), TFA (8 mL) was added and the mixture was stirred for 1.5 hours at room temperature. The reaction mixtsure was concentrated under reduced pressure to obtain the titled compound (12 mg) as a violet oil.
MS (ESI pos.) m/z : 482 [M+H]+, RT=0.872 min (Analytical Condition 3)

### 118-(6) 4-[[6-Fluoro-4-methoxy-8-(methylamino)-2-oxo-1,2-dihydroquinoline-3-carbonyl]amino]benzoic acid

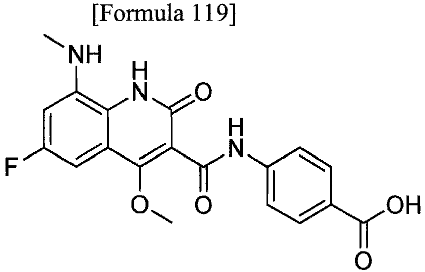

A solution in methanol (6 mL) and water (1 mL) of potassium carbonate (34 mg) and the 4-[[6-fluoro-4-methoxy-8-(methylamino)-2-oxo-1,2-dihydroquinoline-3-carbonyl](trifluoroacetyl)amino]benzoic acid (12 mg) obtained in Example 118-(5) was stirred for 2 hours at room temperature. After concentrating the reaction mixture under reduced pressure, an aqueouos solution of 1 M hydrochloric acid was added and the precipitating solid was recovered by filtration. The solid thus obtained was purified by reversed phase column chromatography, whereupon the titled compound (0.3 mg) was obtained as a pale yellow solid.
MS (ESI pos.) m/z : 386 [M+H]+, RT=0.811 min (Analytical Condition 3)
¹H NMR (400 MHz, DMSO-d6) δ ppm 2.82 (d, J=4.5 Hz, 3 H), 4.04 (s, 3 H), 6.32 (d, J=4.5 Hz, 1 H), 6.54 (dd, J=11.5, 2.6 Hz, 1 H), 6.82 (dd, J=9.5, 2.6 Hz, 1 H), 7.77 (d, J=8.6 Hz, 2 H), 7.92 (d, J=8.6 Hz, 2 H), 10.83 (br s, 1 H), 11.04 (s, 1 H)

### Example 119 4-[[6-Fluoro-8-(methylamino)-2-oxo-1,2-dihydroquinoline-3-carbonyl]amino]-3-[(1E)-4-hydroxybut-1-en-1-yl]benzoic acid

### 119-(1) tert-Butyl 3-[(1E)-4-[[tert-butyl(dimethyl)silyl]oxy]but-1-en-1-yl]-4-[[6-fluoro-8-(methylamino)-2-oxo-1,2-dihydroquinoline-3-carbonyl]amino]benzoate

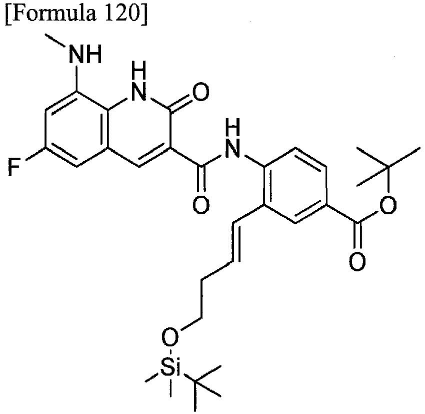

To a solution in DMF (18 mL) of tert-butyl 4-amino-3-[(1E)-4-[[tert-butyl(dimethyl)silyl]oxy]but-1-en-1-yl]benzoate (0.23 g), HATU (0.69 g), and the 6-fluoro-8-(methylamino)-2-oxo-1,2-dihydroquinoline-3-carboxylic acid (0.22 g) obtained in Production Example 2-(5), DIPEA (0.42 mL) was added; the mixture was stirred for 3 days at room temperature and thereafter concentrated under reduced pressure. The residue was purified by column chromatography (NH silica gel cartridge, chloroform/methanol) to obtain the titled compound (0.16 g) as a brown oil.
MS (ESI pos.) m/z : 596 [M+H]+, RT=1.295 min (Analytical Condition 4)

### 119-(2) tert-Butyl 4-[[6-fluoro-8-(methylamino)-2-oxo-1,2-dihydroquinoline-3-carbonyl] amino]-3-[(1E)-4-hydroxybut-1-en-1-yl]benzoate

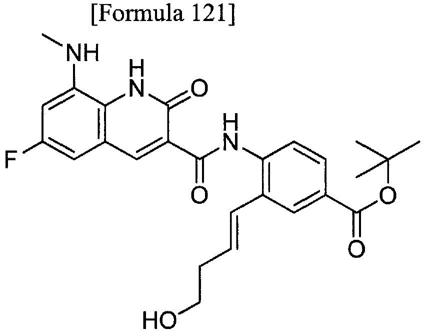

To a solution in THF (6 mL) of the tert-butyl 3-[(lE)-4-[[tert-butyl(dimethyl)silyl]oxy]but-1-en-1-yl]-4-[[6-fluoro-8-(methylamino)-2-oxo-1,2-dihydroquinoline-3-carbonyl]amino]benzoate (0.15 g) obtained in Production Example 119-(1), 1 M TBAF in THF solution (0.63 mL) was added; the mixture was stirred for 15 hours at room temperature and thereafter concentrated under reduced pressure. The residue was purified by column chromatography (silica gel cartridge, chloroform/methanol) to obtain the titled compound (0.05 g) as a yellow solid.
MS (ESI pos.) m/z : 482 [M+H]+, RT=1.256 min (Analytical Condition 3)

### 119-(3) 4-[[6-Fluoro-8-(methylamino)-2-oxo-1,2-dihydroquinoline-3-carbonyl]amino]-3-[(1E)-4-hydroxybut-1-en-1-yl]benzoic acid

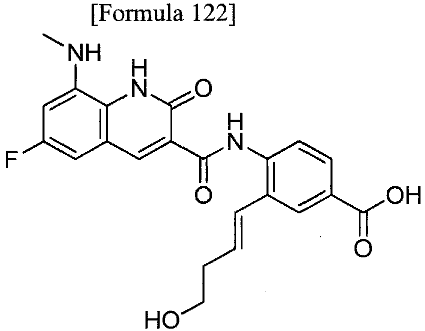

To a solution in chloroform (0.5 mL) of the tert-butyl 4-[[6-fluoro-8-(methylamino)-2-oxo-1,2-dihydroquinoline-3-carbonyl]amino]-3-[(1E)-4-hydroxybut-1-en-1-yl]benzoate (9 mg) obtained in Production Example 119-(2), trifluoroacetic acid (0.5 mL) was added at room temperature; the mixture was stirred for 1.5 hours and thereafter concentrated under reduced pressure. The residue was dissdolved in methanol (1 mL) and an aqueous solution of 2 M sodium hydroxide was added (in three drops); the mixture was stirred for 50 minutes and thereafter concentrated under reduced pressure. To the residue, an aqueous solution of 1 M hydrochloric acid was added and, thereafter, extraction was conducted with a 10% methanol/chloroform solution; by concentrating the organic layer under reduced pressure, the titled compound (3 mg) was obtained as a brown solid.
MS (ESI pos.) m/z : 426 [M+H]+, RT=0.926 min (Analytical Condition 3)
¹H NMR (400 MHz, DMSO-d6) δ ppm 0.76 - 0.91 (m, 2 H), 2.86 (br d, J=3.7 Hz, 3 H), 3.29 - 3.36 (m, 2 H), 3.56 - 3.66 (m, 1 H), 6.46 - 6.54 (m, 1 H), 6.66 (br d, J=11.4 Hz, 1 H), 6.75 - 6.87 (m, 2 H), 7.10 (dd, J=8.4, 2.7 Hz, 1 H), 7.87 (br d, J=8.4 Hz, 1 H), 8.04 (s, 1 H), 8.54 (d, J=8.4 Hz, 1 H), 8.93 (s, 1 H), 12.05 (s, 1 H), 12.55 (s, 1 H)

### Example 120 4-[[6-Fluoro-8-(methylamino)-2-oxo-1,2-dihydroquinoline-3-carbonyl]amino]-3-(4-hydroxybutyl)benzoic acid

### 120-(1) tert-Butyl 4-[[6-fluoro-8-(methylamino)-2-oxo-1,2-dihydroquinoline-3-carbonyl]amino]-3-(4-hydroxybutyl)benzoate

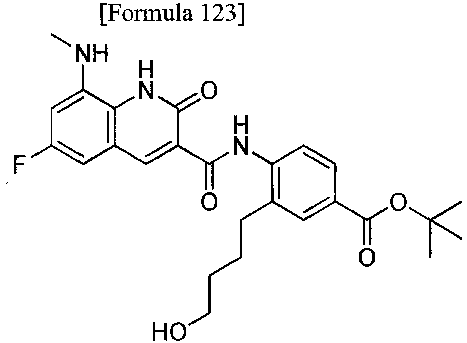

To a solution in THF (5 mL) and methanol (3 mL) of the tert-butyl 4-[[6-fluoro-8-(methylamino)-2-oxo-1,2-dihydroquinoline-3-carbonyl]amino]-3-[(1E)-4-hydroxybut-1-en-1-yl]benzoate (0.04 g) obtained in Production Example 119-(2), 10% palladium on carbon (17 mg) was added and the mixture was stirred for 22 hours at room temperature under a hydrogen atmosphere. Afte removing the insoluble matter by filration, the the filtrate was concentrated under reduced pressure. The residue was suspended in chloroform and thereafter recovered by filtration to obtain the titled compound (20 mg) as a pale green solid. MS (ESI pos.) m/z : 484 [M+H]+, RT=1.249 min (Analytical Condition 3)

### 120-(2) 4-[[6-Fluoro-8-(methylamino)-2-oxo-1,2-dihydroquinoline-3-carbonyl]amino]-3-(4-hydroxybutyl)benzoic acid

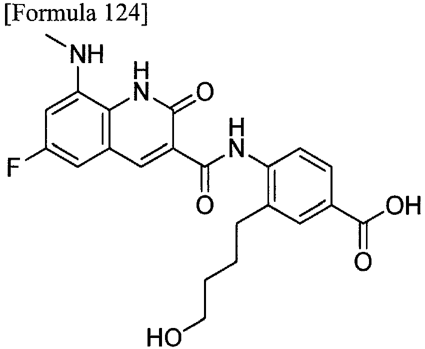

To a solution in 1,4-dioxane (2 mL) of the tert-butyl 4-[[6-fluoro-8-(methylamino)-2-oxo-1,2-dihydroquinoline-3 -carbonyl]amino]-3-(4-hydroxybutyl)benzoate (15 mg) obtained in Example 120-(1), 4 M hydrochloric acid in 1,4-dioxane solution (2 mL) was added at room temperature; the mixture was stirred for 20 hours and thereafter concentrated under reduced pressure to obtain the titled compound (15 mg) as a pale green solid.
MS (ESI pos.) m/z : 428 [M+H]+, RT=0.913 min (Analytical Condition 3)
1H NMR (400 MHz, DMSO-d6) δ ppm 1.51 - 1.71 (m, 4 H), 2.80 (br t, J=7.4 Hz, 2 H), 2.86 (s, 3 H), 3.44 (t, J=6.4 Hz, 2 H), 6.65 (dd, J=11.7, 2.6 Hz, 1 H), 7.09 (dd, J=8.7, 2.6 Hz, 1 H), 7.80 - 7.88 (m, 2 H), 8.55 (d, J=9.2 Hz, 1 H), 8.93 (s, 1 H), 12.02 (s, 1 H), 12.48 (s, 1 H)

The same procedures as in Example 120 were employed to obtain the compound of Example 126 from the corresponding raw material.

**[Table 15]**

| Example | Structural Formula | Compound Name | MS | NMR |
|---|---|---|---|---|
| 120 | 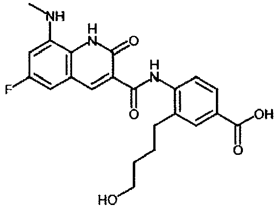 | 4-{[6-fluoro-8-(methylamino)-2-oxo-1,2-dihydroquinoline-3-carbonyl]aminoy3-(4-hydroxybutyl)benzoic acid | MS (ESI pos.) m/z: 428 [M+H]+ RT=0.913 min (Analytical Condition 3) | 1H NMR (400 MHz, DMSO-d6) δ ppm 1.51 -1.71 (m, 4 H), 2.80 (br t, J=7.4 Hz, 2 H), 2.86 (s, 3 H), 3.44 (t, J=6.4 Hz, 2 H), 6.65 (dd, J=11.7, 2.6 Hz, 1 H), 7.09 (dd, J=8.7, 2.6 Hz, 1 H), 7.80 - 7.88 (m, 2 H), 8.55 (d, J=9.2 Hz, 1 H), 8.93 (s, 1 H), 12.02 (s, 1 H), 12.48 (s, 1 H) |
| 126 | 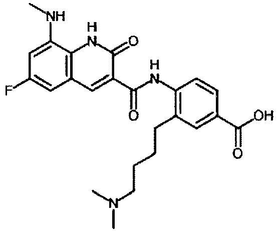 | 3-[4-(dimethylamino)butyl]-4-{[6-fluoro-8-(methylamino)-2-oxo-1,2-dihydroquinoline-3-carbonyl]amino}benzoic acid | MS (ESI pos.) m/z: 455 [M+H]+ RT=0.614 min (Analytical Condition 3) | 1H NMR (400 MHz, DMSO-d6) δ ppm 1.60 -1.71 (m, 4 H), 2.54 (br s, 6 H), 2.78 - 2.86 (m, 4 H), 2.87 (d, J=4.5 Hz, 3 H), 6.40 - 6.67 (m, 2 H), 7.03 (br d, J=10.4 Hz, 1 H), 7.77 - 7.91 (m, 2 H), 8.56 (d, J=8.3 Hz, 1 H), 8.88 (s, 1 H) |

### Example 127 Ethyl 4-[[6-fluoro-4-hydroxy-8-(methylamino)-2-oxo-1,2-dihydroquinoline-3 -carbonyl] amino]benzoate

### 127-(1) Methyl 3-[benzyl(methyl)amino]-2-[3-[4-(ethoxycarbonyl)anilino]-3-oxopropanamido]-5-fluorobenzoate

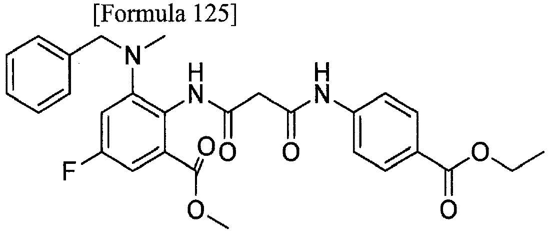

To a solution in dimethylformamide (8 mL) of the methyl 2-amino-3-[benzyl(methyl)amino]-5-fluorobenzoate (250 mg) obtained in Production Example 6-(2), 3-[4-(ethoxycarbonyl)anilino]-3-oxopropanoic acid (218 mg), HATU (396 mg) and DIPEA (529 µL) were added and the mixture was stirred for 64 hours at room temperature. To the reaction mixture, a saturated aqueous solution of sodium hydrogencarbonate was added and extraction was conducted with ethyl acetate. The organic layer was washed with saturated brine and thereafter dried over anhydrous magnesium sulfate and concentrated under reduced pressure. The residue was purified by column chromatography (silica gel cartridge, hexane/ethyl acetate = 80:20 to chloroform/methanol = 90:10) to obtain the titled compound (67 mg) as a yellow oil.
MS (ESI pos.) m/z : 522 [M+H]+, RT=1.210 min (Analytical Condition 3)

### 127-(2) Ethyl 4-([8-[benzyl(methyl)amino]-6-fluoro-4-hydroxy-2-oxo-1,2-dihydroquinoline-3-carbonyl]amino)benzoate

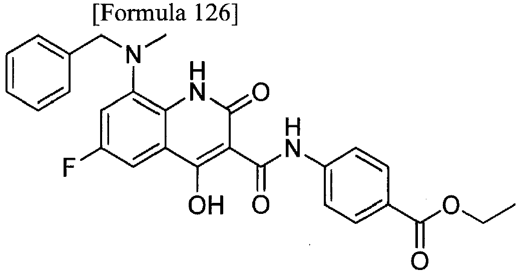

To a solution in ethanol (4 mL) of the methyl 3-[benzyl(methyl)amino]-2-[3-[4-(ethoxycarbonyl)anilino]-3-oxopropanamido]-5-fluorobenzoate (67 mg) obtained in Example 127-(1), 20% sodium ethoxide in ethanol solution (84 µL) was added and the mixture was stirred for 2 hours at room temperature. The reaction mixture was diluted with water and thereafter brought to pH 1 with an aqueous solution of 1 M hydrochloric acid. The precipitating solid was recovered by filtration and thereafter washed with water to obtain the titled compound (51 mg) as a yellow solid.
MS (ESI neg.) m/z : 488 [M-H]-, RT=1.175 min (Analytical Condition 4)

### 127-(3) Ethyl 4-[[6-fluoro-4-hydroxy-8-(methylamino)-2-oxo-1,2-dihydroquinoline-3 -carbonyl] amino]benzoate

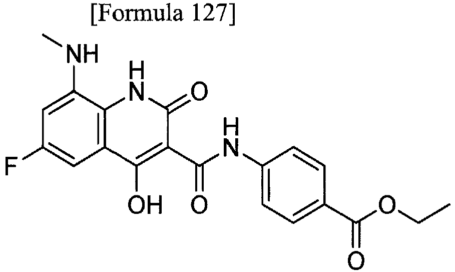

To a solution in ethanol (30 mL) and chloroform (10 mL) of the ethyl 4-([8-[benzyl(methyl)amino]-6-fluoro-4-hydroxy-2-oxo-1,2-dihydroquinoline-3-carbonyl]amino)benzoate (27 mg) obtained in Example 127-(2), 5% palladium on carbon (25 mg) was added and the mixture was stirred for 5 hours at room temperature under a hydrogen atmosphere. After removing the insoluble matter by filtration, the filtrate was concentrated under reduced pressure. The residue was purified by column chromatography (silica gel cartridge, chloroform/methanol = 97:3) to obtain the titled compound (10 mg) as a pale yellow solid.
MS (ESI neg.) m/z : 398 [M-H]-, RT=1.025 min (Analytical Condition 4)
¹H NMR (400 MHz, DMSO-d6) δ ppm 1.33 (br t, J=7.3 Hz, 3 H), 2.84 (s, 3 H), 4.32 (q, J=7.3 Hz, 2 H), 6.53 - 6.74 (m, 2 H), 6.86 - 7.00 (m, 1 H), 7.82 (d, J=8.4 Hz, 2 H), 8.00 (d, J=8.4 Hz, 2 H), 11.36 (br s, 1 H), 12.96 (br s, 1 H), 16.03 (s, 1 H)

### Example 128 3-[[Cyclohexyl(methyl)amino]methyl]-4-([[6-fluoro-8-(methylamino)-2-oxo-1,2-dihydroquinolin-3-yl]methyl]amino)benzoic acid

### 128-(1) Methyl 3-[[cyclohexyl(methyl)amino]methyl]-4-[([6-fluoro-2-[(4-methoxyphenyl)methoxy]-8-(methylamino)quinolin-3-yl]methyl)amino]benzoate

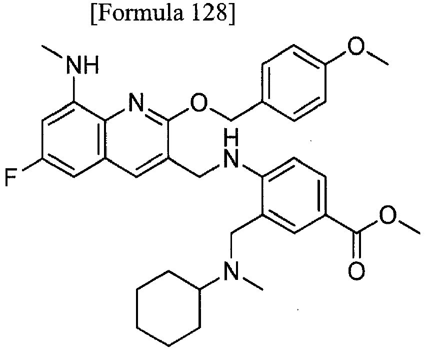

To a solution in ethanol (1 mL) of the 6-fluoro-2-[(4-methoxyphenyl)methoxy]-8-(methylamino)quinoline-3-carbaldehyde (35 mg) obtained in Production Example 9-(3), methyl 4-amino-3-[[cyclohexyl(methyl)amino]methyl]benzoate (57 mg), tetraisopropyl orthotitanate (58 mg) and triethylamine (29 µL) were added and the mixture was stirred for 5 hours at room temperature. To the stirred mixture, sodium borohydride (5.8 mg) was added, followed by 15-hr stirring at room temperature. Saturated aqueous sodium bicarbonate was added to the reaction mixture which was thereafter filtered through Celite. The aqueous layer was extracted with chloroform and separated with a phase separator. The organic layer was concentrated under reduced pressure. The residue was purified by column chromatography (silica gel cartridge, hexane/ethyl acetate = 95:5 - 30:70) to obtain the titled compound (16 mg) as a yellow oil.
MS (ESI pos.) m/z : 601[M+H]+, RT=0.960 min (Analytical Condition 3)

### 128-(2) Methyl 3-[[cyclohexyl(methyl)amino]methyl]-4-([[6-fluoro-8-(methylamino)-2-oxo-1,2-dihydroquinolin-3-yl]methyl]amino)benzoate

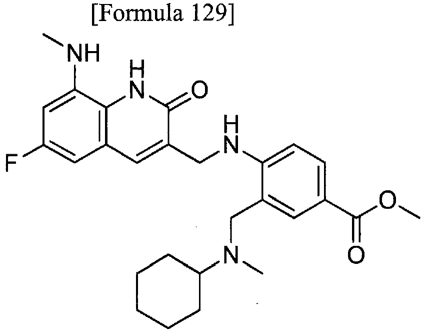

To a solution in chloroform (1 mL) of the methyl 3-[[cyclohexyl(methyl)amino]methyl]-4-[([6-fluoro-2-[(4-methoxyphenyl)methoxy]-8-(methylamino)quinolin-3-yl]methyl)amino]benzoate (19 mg) obtained in Example 128-(1), TFA (1 mL) was added and the mixture was stirred for 3 hours at room temperature. The reaction mixture was concentrated and a crude product (20 mg) containing the titled compound was obtained as a yellow solid.
MS (ESI pos.) m/z : 481[M+H]+, RT=0.693 min (Analytical Condition 3)

### 128-(3) 3-[[Cyclohexyl(methyl)amino]methyl]-4-([[6-fluoro-8-(methylamino)-2-oxo-1,2-dihydroquinolin-3-yl]methyl]amino)benzoic acid

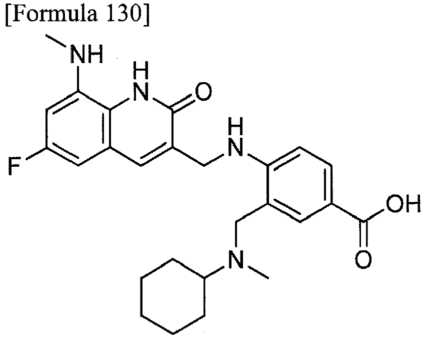

To a solution in THF (1 mL) of the methyl 3-[[cyclohexyl(methyl)amino]methyl]-4-([[6-fluoro-8-(methylamino)-2-oxo-1,2-dihydroquinolin-3-yl]methyl]amino)benzoate (20 mg) obtained in Example 128-(2), 2 M sodium hydroxide in aqueous solution (1 mL) was added and the mixture was stirred for 15 hours at room temperature, followed by 16-hr stirring at 65°C. To the reaction mixture, an aqueous solution of 1 M hydrochloric acid was added to establish pH 1. The precipitating solid was recovered by filtration. The crude product thus obtained was purified by preparative LCMS to obtain the titled compound (1.2 mg) as a light brown solid.
MS (ESI pos.) m/z : 467 [M+H]+, RT=0.608 min (Analytical Condition 3)
¹H NMR (400 MHz, METHANOL-d4) δ ppm 1.10 - 1.24 (m, 2 H), 1.24 - 1.38 (m, 2 H), 1.43 - 1.56 (m, 2 H), 1.62 - 1.71 (m, 1 H), 1.81 - 1.90 (m, 2 H), 1.97 - 2.05 (m, 2 H), 2.44 (br s, 3 H), 2.91 (s, 3 H), 4.06 (br s, 2 H), 4.40 (s, 2 H), 6.50 - 6.55 (m, 1 H), 6.60 (d, J=6.7 Hz, 1 H), 6.69 (d, J=8.4 Hz, 1 H), 7.75 - 7.87 (m, 2 H), 8.49 (s, 1 H)

### Example 130 4-([6-Fluoro-8-(methylamino)-4-[2-(methylamino)ethyl]-2-oxo-1,2-dihydroquinoline-3-carbonyl] amino)benzoic acid-trifluoroacetate

### 130-(1) tert-Butyl4-(3-[2-[benzyl(methyl)amino]-4-fluoro-6-(2,2,3,3,8,11,11-heptamethyl-9-oxo-4,10-dioxa-8-aza-3-siladodecan-5-yl)anilino]-3-oxopropanamido)benzoate

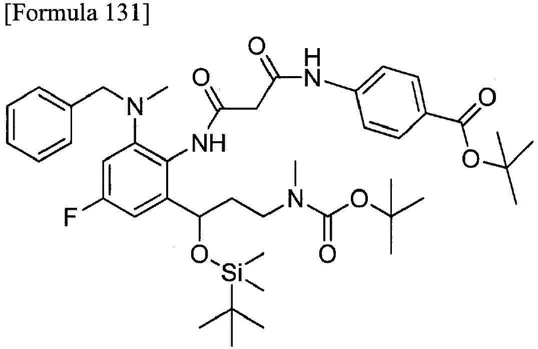

To a solution in chloroform (1 mL) of the 3-[4-(tert-butoxycarbonyl)anilino]-3-oxopropanoic acid (56 mg) obtained in Production Example 7-(2), oxalyl chloride (0.017 mL) was added at room temperature and the mixture was stirred for 2 hours at that temperature. After the end of the reaction, the solution as such was used in the next reaction. To a solution in pyridine (10 mL) of the tert-butyl (3-[2-amino-3-[benzyl(methyl)amino]-5-fluorophenyl]-3-[[tert-butyl(dimethyl)silyl]oxy]propyl)methylcarbamate (53 mg) obtained in Production Example 12-(5), the separately prepared acid chloride solution was added at room temperature and the mixture was stirred for one day and night at that temperature. After the end of the reaction, the solvent was removed by concentrating the reaction mixture under reduced pressure. The residue was dissolved in ethyl acetate and the resulting organic layer was washed with an aqueous solution of sodium hydrogencarbonate and saturated brine, dried over anhydrous magnesium sulfate and concentrated under reduced pressure to thereby obtain a crude product. The crude product thus obtained was purified by column chromatography (silica gel cartridge, n-hexane/ethyl acetate = 60:40 - 40:60), whereupon the titled compound (30 mg) was obtained as a pale yellow oil.
¹H NMR (400 MHz, CHLOROFORM-d) δ ppm -0.20 (s, 3 H), -0.12 (br s, 3 H), 0.84 (s, 9 H), 1.45 (s, 9 H), 1.60 (s, 9 H), 1.86 (br s, 1 H), 1.96 (br s, 1 H), 2.69 (s, 3 H), 2.79 (s, 3 H), 2.98 - 3.38 (m, 4 H), 3.90 - 4.26 (m, 2 H), 4.71 (br t, J=5.5 Hz, 1 H), 6.67 - 6.80 (m, 1 H), 6.81 - 6.96 (m, 1 H), 7.14 - 7.39 (m, 5 H), 7.63 (br d, J=8.6 Hz, 2 H), 7.93 (d, J=8.6 Hz, 2 H), 8.36 (br s, 1 H), 10.15 (brs, 1 H)

### 130-(2) tert-Butyl 4-[3-(2-[benzyl(methyl)amino]-6-[3-[(tert-butoxycarbonyl)(methyl)amino]-1-hydroxypropyl]-4-fluoroanilino)-3-oxopropanamido]benzoate

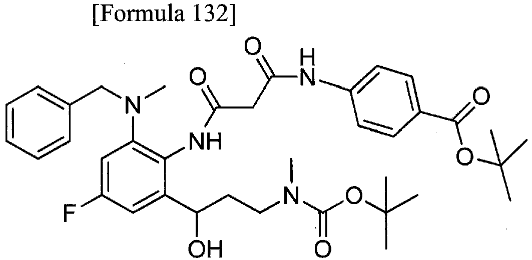

To a solution in THF (5 mL) of the tert-butyl 4-(3-[2-[benzyl(methyl)amino]-4-fluoro-6-(2,2,3,3,8,11,11-heptamethyl-9-oxo-4,10-dioxa-8-aza-3-siladodecan-5-yl)anilino]-3-oxopropanamido)benzoate (30 mg) obtained in Production Example 130-(1), 1 M TBAF in THF solution (1 mL) was added under ice cooling and the mixture was stirred for 5 hours at that temperature. After the end of the reaction, a saturated aqueous solution of sodium hydrogencarbonate was added for quenching, followed by the addition of ethyl acetate. The resulting organic layer was washed with saturated brine, dried over anhydrous magnesium sulfate and concentrated under reduced pressure to thereby obtain a crude product. The thus obtained crude product was purified by column chromatography (silica gel cartridge, n-hexane/ethyl acetate = 60:40 - 40:60) to obtain the titled compound (32 mg) as a pale yellow oil.
¹H NMR (400 MHz, CHLOROFORM-d) δ ppm 1.42 (s, 9 H), 1.59 (s, 9 H), 2.63 (s, 3 H), 2.77 (s, 3 H), 3.07 (br s, 2 H), 3.16 (br s, 2 H), 3.56 (br s, 2 H), 4.12 (s, 2 H), 4.61 (br d, J=6.2 Hz, 1 H), 6.63 - 6.81 (m, 1 H), 6.86 - 7.01 (m, 1 H), 7.16 - 7.38 (m, 5 H), 7.57 (br d, J=8.6 Hz, 2 H), 7.92 (d, J=8.6 Hz, 2 H), 8.27 (br s, 1 H), 9.79 (br s, 1 H)

### 130-(3) tert-Butyl 4-(3-[2-[benzyl(methyl)amino]-6-[N-(tert-butoxycarbonyl)-N-methyl-beta-alanyl]-4-fluoroanilino]-3-oxopropanamido)benzoate

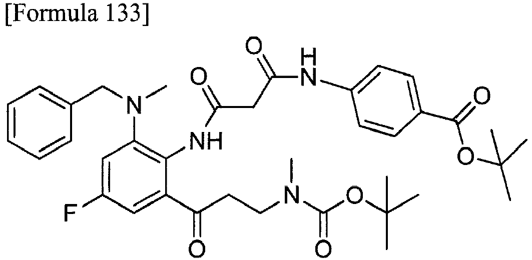

To a solution in DMSO (1 mL) of the tert-butyl 4-[3-(2-[benzyl(methyl)amino]-6-[3-[(tert-butoxycarbonyl)(methyl)amino]-1-hydroxypropyl]-4-fluoroanilino)-3-oxopropanamido]benzoate (16 mg) obtained in Production Example 130-(2), 40% IBX (52 mg) was added and the mixture was stirred for one day and night at room temperature. After the end of the reaction, a solution of sodium hydrogencarbonate was added to the resulting reaction mixture for quenching the reaction; thereafter, ethyl acetate was added. The resulting organic layer was washed with water and saturated brine, dried over anhydrous magnesium sulfate and concentrated under reduced pressure to thereby obtain a crude product. The thus obtained crude product was purified by column chromatography (silica gel cartridge, n-hexane/ethyl acetate = 60:40 - 40:60) to obtain the titled compound (5 mg) as a pale yellow oil.
¹H NMR (400 MHz, CHLOROFORM-d) δ ppm 1.36 - 1.48 (m, 9 H), 1.58 (s, 9 H), 2.66 (m, 3 H), 2.88 (s, 3 H), 3.09 (br s, 2 H), 3.26 (br s, 2 H), 3.52 - 3.61 (m, 2 H), 4.03 (s, 2 H), 6.82 - 7.03 (m, 1 H), 7.16 - 7.41 (m, 6 H), 7.64 (br d, J=8.6 Hz, 2 H), 7.93 (d, J=8.6 Hz, 2 H), 8.57 (br, 1 H), 9.42 (br, 1 H)

### 130-(4) tert-Butyl 4-[(8-[benzyl(methyl)amino]-4-[2-[(tert-butoxycarbonyl)(methyl)amino]ethyl]-6-fluoro-2-oxo-1,2-dihydroquinoline-3-carbonyl)amino]benzoate

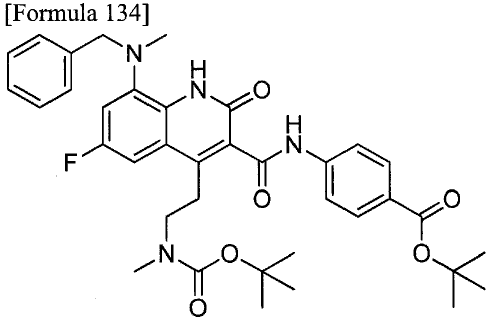

To ethanol (1 mL) of the tert-butyl 4-(3-[2-[benzyl(methyl)amino]-6-[N-(tert-butoxycarbonyl)-N-methyl-beta-alanyl]-4-fluoroanilino]-3-oxopropanamido)benzoate (30 mg) obtained in Production Example 130-(3), 20% sodium ethoxide in ethanol solution (0.043 mL) was added and the mixture was stirred for 2 hours at room temperature. After the end of the reaction, an aqueous solution of ammonium chloride was added to the reaction mixture for quenching the reaction, followed by the addition of chloroform. The resulting organic layer was washed with water and saturated brine, dried over anhydrous magnesium sulfate, and concentrated under reduced pressure to thereby obtain a crude product. The thus obtained crude product was purified by column chromatography (silica gel cartridge, chloroform/methanol = 100:0 - 95:5) to obtain the titled compound (29 mg) as a pale yellow oil.
¹H NMR (400 MHz, CHLOROFORM-d) δ ppm 1.48 (s, 9 H), 1.58 (s, 9 H), 2.69 (s, 3 H), 2.91 - 3.13 (m, 2 H), 3.41 - 3.73 (m, 5 H), 4.08 (br s, 2 H), 7.24 - 7.40 (m, 5 H), 7.57 (br d, J=8.2 Hz, 1 H), 7.76 (d, J=8.7 Hz, 2 H), 7.85 - 7.94 (m, 1 H), 7.99 (d, J=8.7 Hz, 2 H), 9.73 (br s, 1 H), 11.23 (brs, 1 H)

### 130-(5) tert-Butyl 4-[[4-[2-[(tert-butoxycarbonyl)(methyl)amino]ethyl]-6-fluoro-8-(methylamino)-2-oxo-1,2-dihydroquinoline-3-carbonyl]amino]benzoate

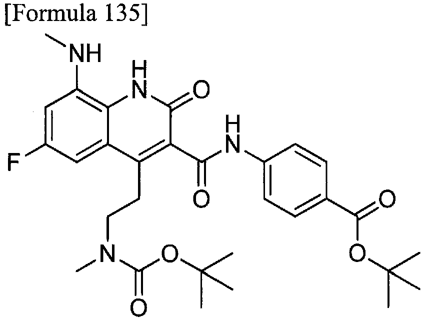

To a solution in methanol (2.5 mL) of the tert-butyl 4-[(8-[benzyl(methyl)amino]-4-[2-[(tert-butoxycarbonyl)(methyl)amino]ethyl]-6-fluoro-2-oxo-1,2-dihydroquinoline-3-carbonyl)amino]benzoate (29 mg) obtained in Production Example 130-(4), a 5% palladium on active carbon catalyst (29 mg) was added and the mixture was stirred for 2 hours at room temperature under a hydrogen atmosphere. After the end of the reaction, the reaction mixture was filtered through Celite and the filtrate was concentrated under reduced pressure, whereupon a crude product was obtained. The thus obtained crude product was purified by (silica gel cartridge, chloroform/methanol = 100:0 - 95:5) to obtain the titled compound (9 mg) as a yellow solid.
¹H NMR (400 MHz, CHLOROFORM-d) δ ppm 1.31 (br s, 9 H), 1.43 (br s, 9 H), 2.57 (br s, 3 H), 2.66 - 2.83 (m, 4 H), 2.93 (br s, 1 H), 3.23 (br s, 2 H), 6.09 - 6.28 (m, 1 H), 6.74 - 7.02 (m, 2 H), 7.85 (br s, 4 H), 9.64 (br s, 1 H), 10.30 (br s, 1 H)

### 130-(6) 4-([6-Fluoro-8-(methylamino)-4-[2-(methylamino)ethyl]-2-oxo-1,2-dihydroquinoline-3 -carbonyl] amino)benzoic acid-trifluoroacetate

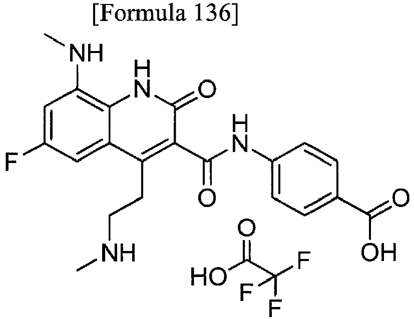

To a solution in chloroform (0.7 mL) of the tert-butyl 4-[(8-[benzyl(methyl)amino]-4-[2-[(tert-butoxycarbonyl)(methyl)amino]ethyl]-6-fluoro-2-oxo-1,2-dihydroquinoline-3-carbonyl)amino]benzoate (7.2 mg) obtained in Production Example 130-(5), TFA (0.2 mL) was added under ice cooling and the mixture was stirred for 5 hours at that temperature. After the end of the reaction, the reaction mixture was concentrated under reduced pressure to thereby obtain the titled compound (quantitatively) as a colorless solid.
MS (ESI pos.) m/z : 413 [M+H]+, RT=0.695 min (Analytical Condition 2)
¹H NMR (600 MHz, DMSO-d6) δ ppm 2.61 (t, J=5.4 Hz, 2 H), 2.84 (s, 3 H), 3.10 (br s, 5 H), 6.39 (br s, 1 H), 6.60 (br d, J=10.0 Hz, 1 H), 7.03 (dd, J=10.0, 2.3 Hz, 2 H), 7.83 (d, J=8.9 Hz, 2 H), 7.96 (d, J=8.9 Hz, 2 H), 8.47 (br s, 1 H), 10.86 (s, 1 H), 11.36 (br s, 1 H), 12.81 (br s, 1 H)

### Example 131 4-([4-[(2-Aminoethyl)amino]-6-fluoro-8-(methylamino)-2-oxo-1,2-dihydroquinoline-3-carbonyl]amino)benzoic acid-formate

### 131-(1) tert-Butyl 4-([8-[benzyl(methyl)amino]-6-fluoro-4-[(4-methylbenzene-1-sulfonyl)oxy]-2-oxo-1,2-dihydroquinoline-3-carbonyl] [(2,4-dimethoxyphenyl)methyl]amino)benzoate

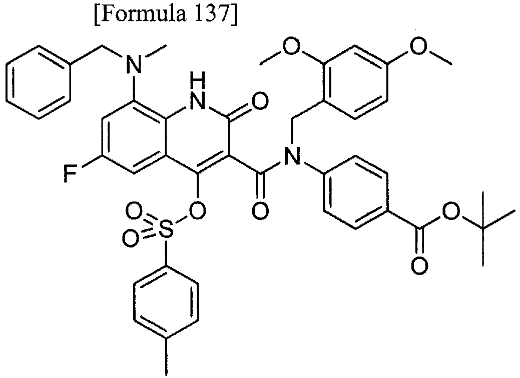

To a solution in acetonitrile (17 mL) of DIPEA (166 µL) and the tert-butyl 4-([8-[benzyl(methyl)amino]-6-fluoro-4-hydroxy-2-oxo-1,2-dihydroquinoline-3-carbonyl][(2,4-dimethoxyphenyl)methyl]amino)benzoate (580 mg) obtained in Example 118-(2), paratoluene sulfonyl chloride (174 mg) was added and the mixture was stirred for 2 hours at room temperature. The reaction mixture was diluted with a saturated aqueous solution of sodium hydrogencarbonate and extracted with ethyl acetate. The organic layer was washed with saturated brine and thereafter dried over anhydrous magnesium sulfate and concentrated under reduced pressure. The residue was purified by column chromatography (silica gel cartridge, hexane/ethyl actate = 60:40 - 50:50) to obtain the titled compound (169 mg) as a yellow amorphous.
MS (ESI pos.) m/z : 822 [M+H]+, RT=1.120 min (Analytical Condition 4)

### 131-(2) tert-Butyl 4-([8-[benzyl(methyl)amino]-4-([2-[(tert-butoxycarbonyl)amino]ethyl]amino)-6-fluoro-2-oxo-1,2-dihydroquinoline-3-carbonyl][(2,4-dimethoxyphenyl)methyl]amino)benzoate

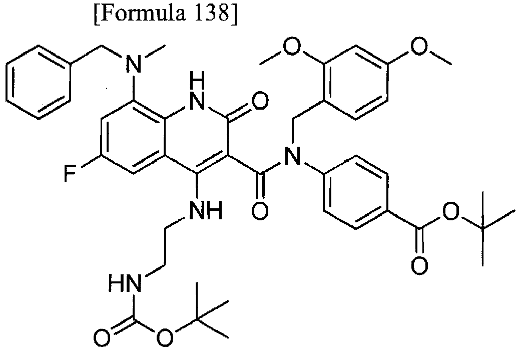

To a solution in acetonitrile (8 mL) of the tert-butyl 4-([8-[benzyl(methyl)amino]-6-fluoro-4-[(4-methylbenzene-1-sulfonyl)oxy]-2-oxo-1,2-dihydroquinoline-3-carbonyl] [(2,4-dimethoxyphenyl)methyl]amino)benzoate (157 mg) obtained in Example 131-(1), tert-butyl (2-aminoethyl)carbamate (306 mg) and DIPEA (100 µL) were added and the mixture was stirred for 2 hours at room temperature. The reaction mixture was diluted with a saturated aqueous solution of sodium hydrogencarbonate and extracted with chloroform. The organic layer was passed through a phase separator and concentrated under reduced pressure. The residue was purified by column chromatography (NH silica gel cartridge, chloroform/methanol = 100:0) to obtain the titled compound (46 mg) as a yellow oil.
MS (ESI pos.) m/z : 810 [M+H]+, RT=1.056 min (Analytical Condition 4)

### 131-(3) tert-Butyl 4-[[4-([2-[(tert-butoxycarbonyl)amino]ethyl]amino)-6-fluoro-8-(methylamino)-2-oxo-1,2-dihydroquinoline-3-carbonyl][(2,4-dimethoxyphenyl)methyl]amino]benzoate

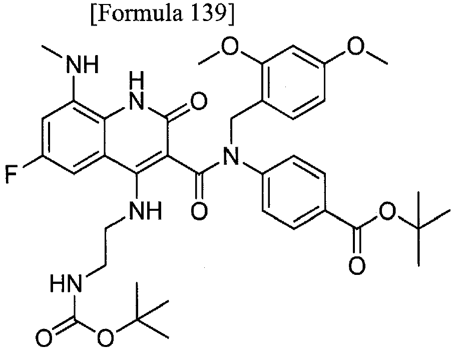

To a solution in methanol (15 mL) of the tert-butyl 4-([8-[benzyl(methyl)amino]-4-([2-[(tert-butoxycarbonyl)amino]ethyl]amino)-6-fluoro-2-oxo-1,2-dihydroquinoline-3-carbonyl][(2,4-dimethoxyphenyl)methyl]amino)benzoate (46 mg) obtained in Example 131-(2), 5% palladium on carbon (40 mg) was added and the mixture was stirred for an hour at room temperature under a hydrogen atmosphere. After removing the insoluble matter by filtration, the filtrate was concentrated under reduced pressure to obtain the titled compound (36 mg) as a colorless solid.
MS (ESI pos.) m/z : 742 [M+Na]+, RT=0.956 min (Analytical Condition 4)

### 131-(4) 4-([4-[(2-Aminoethyl)amino]-6-fluoro-8-(methylamino)-2-oxo-l,2-dihydroquinoline-3-carbonyl] amino)benzoic acid-formate

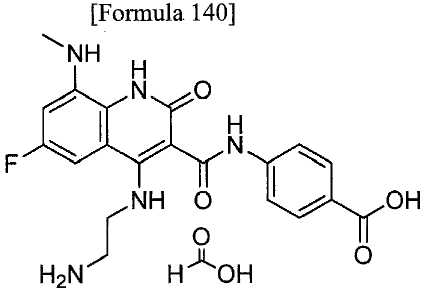

To a solution in chloroform (2.5 mL) of the tert-butyl 4-[[4-([2-[(tert-butoxycarbonyl)amino]ethyl]amino)-6-fluoro-8-(methylamino)-2-oxo-1,2-dihydroquinoline-3-carbonyl][(2,4-dimethoxyphenyl)methyl]amino]benzoate (36 mg) obtained in Example 131-(3), TFA (2.5 mL) was added and the mixture was stirred for an hour at room temperature. After concentrating the reaction mixture under reduced pressure, the residue was purified by reversed phase column chromatography to obtain the titled compound (1.9 mg) as a yellow solid.
MS (ESI pos.) m/z : 414 [M+H]+, RT=0.479 min (Analytical Condition 3)
¹H NMR (400 MHz, DMSO-d6) δ ppm 2.82 (d, J=4.3 Hz, 3 H), 2.93 (br t, J=5.9 Hz, 2 H), 3.67 (br t, J=5.9 Hz, 2 H), 6.42 (br d, J=4.3 Hz, 1 H), 6.57 (dd, J=11.4, 2.3 Hz, 1 H), 7.10 (dd, J=10.8, 2.3 Hz, 1 H), 7.74 (d, J=8.7 Hz, 2 H), 7.91 (d, J=8.7 Hz, 2 H), 12.70 (br s, 1 H)

The same procedures as in Example 131 were employed to obtain the Example compounds identified below from the corresponding raw materials.

**[Table 16-1]**

| Example | Structural Formula | Compound Name | MS | NMR |
|---|---|---|---|---|
| 131 | 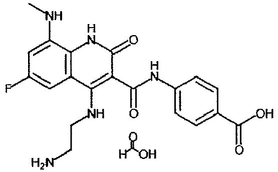 | 4-({4-[(2-aminoethyl) amino]-6-fluoro-8-(methylamino)-2-oxo-1,2-dihydroquinoline-3-carbonyl}amino)benzoic acid-formate | MS (ESI pos.) m/z: 414 [M+H]+ RT=0.479 min (Analytical Condition 3) | 1H NMR (400 MHz, DMSO-d6) δ ppm 2.82 (d, J=4.3 Hz, 3 H), 2.93 (br t, J=5.9 Hz, 2 H), 3.67 (br t, J=5.9 Hz, 2 H), 6.42 (br d, J=4.3 Hz, 1 H), 6.57 (dd, J=11.4, 2.3 Hz, 1 H), 7.10 (dd, J=10.8, 2.3 Hz, 1 H), 7.74 (d, J=8.7 Hz, 2 H), 7.91 (d, J=8.7 Hz, 2 H), 12.70 (br s, 1 H) |
| 132 | 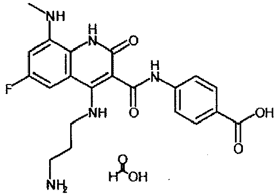 | 4-({4-[(3-aminopropyl) amino]-6-fluoro-8-(methylamino)-2-oxo-1,2-dihydroquinoline-3-carbonyl}amino)benzoic acid-formate | MS (ESI pos.) m/z: 428 [M+H]+ RT=0.534 min (Analytical Condition 3) | 1H NMR (400 MHz, DMSO-d6) δ ppm 1.83 -1.96 (m, 2 H), 2.77 - 2.88 (m, 5 H), 3.74 (br d, J=5.9 Hz, 2 H), 6.47 (brs, 1 H), 6.59 (d, J=11.4 Hz, 1 H), 7.11 (br d, J=9.3 Hz, 1 H), 7.68 (br d, J=8.6 Hz, 2 H), 7.90 (br d, J=8.6 Hz, 2 H), 12.92 (br s, 1 H) |
| 133 | 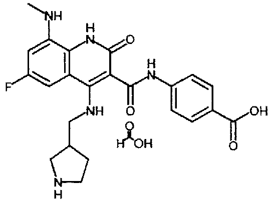 | 4-{[6-fluoro-8-(methylamino)-2-oxo-4-{[(pyrrolidine-3-yl)methyl]amino}-1,2-dihydroquinoline-3-carbonyl]amino}benzoic acid-formate | MS (ESI pos.) m/z: 454 [M+H]+ RT=0.534 min (Analytical Condition 3) | 1H NMR (400 MHz, DMSO-d6) δ ppm 1.15-2.90 (m, 12 H), 6.44 (s, 1 H), 6.53-6.67 (m, 2H), 7.07 (br d, J=9.8 Hz, 1 H), 7.66 (br d, J=8.1 Hz, 2 H), 7.88 (d, J=8.1 Hz, 2 H), 10.75 (s, 1 H), 13.06 (s, 1 H) |
| 135 | 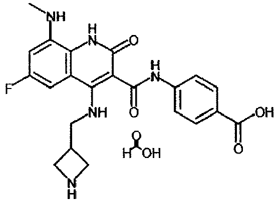 | 4-{[4-{[(azetidine-3-yl)methyl]amino}-6-fluoro-8-(methylamino)-2-oxo-1,2-dihydroquinoline-3- carbonyl]amino}benzoic acid-formate | MS (ESI pos.) m/z: 440 [M+H]+ RT=0.545 min (Analytical Condition 3) | 1H NMR (400 MHz, DMSO-d6) δ ppm 2.60-4.10 (m, 10 H), 6.46 (d, J=4.2 Hz, 1 H), 6.59 (dd, J=11.4, 1.7 Hz, 1 H), 7.08 (dd, J=10.6, 1.7 Hz, 1 H), 7.70 (d, J=8.5 Hz, 2 H), 7.91 (br d, J=8.5 Hz, 2 H), 9.95 (br s, 1 H), 12.72 (br s, 1 H) |
| 136 | 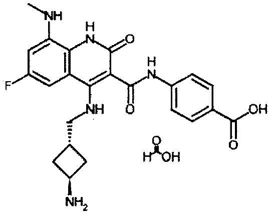 | 4-{[4-{[(trans-3-aminocyclobutyl)methyl] amino}-6-fluoro-8-(methylamino)-2-oxo-1,2-dihydroquinoline-3-carbonyl]amino}benzoic acid-formate | MS (ESI pos.) m/z: 454 [M+H]+ RT=0.592 min (Analytical Condition 3) | 1H NMR (400 MHz, DMSO-d6) δ ppm 2.06 - 2.25 (m, 4 H), 2.54 (s, 2 H), 2.67 (brs, 2 H), 2.82 (br d, J=4.2 Hz, 3 H), 6.46 (brs, 1 H), 6.60 (dd, J=11.2, 2.0 Hz, 1 H), 7.09 (dd, J=10.7, 2.0 Hz, 1 H), 7.71 (d, J=8.6 Hz, 2 H), 7.92 (d, J=8.6 Hz, 2 H), 10.68 (brs, 1 H), 13.16 (brs, 1 H) |
| 137 | 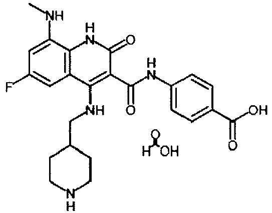 | 4-{[6-fluoro-8-(methylamino)-2-oxo-4-{[(piperidine-4-yl)methyl]amino}-1,2-dihydroquinoline-3-carbonyl]amino}benzoic acid-formate | MS (ESI pos.) m/z : 468 [M+H]+ RT=0605 min (Analytical Condition 3) | 1H NMR (400 MHz, DMSO-d6) δ ppm 1.08 - 2.04 (m, 5 H), 2.38 (s, 1 H), 2.54 (s, 1 H), 2.82 (br d, J=4.5 Hz, 3 H), 3.52 - 3.60 (m, 2 H), 3.62 - 3.86 (m, 2 H), 6.44 (d, J=4.5 Hz, 1 H), 6.58 (brdd, J=11.4,2.2 Hz, 1 H), 7.09 (dd, J=10.6, 2.2 Hz, 1 H), 7.73 (br d, J=8.6 Hz, 2 H), 7.92 (br d, J=8.6 Hz, 2 H), 10.25 (br s, 1 H), 12.82 (br s, 1 H), 13.16 (brs, 1 H) |

**[Table 16-2]**

| Example | Structural Formula | Compound Name | MS | NMR |
|---|---|---|---|---|
| 139 | 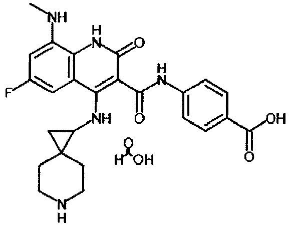 | 4-({4-[(6-azaspiro[2.5] octane-1-yl)amino]-6-fluoro-8-(methylamino)-2-oxo-1,2-dihydroquinoline-3- carbonyl}amino)benzoic acid-formate | MS (ESI pos.) m/z: 480 [M+H]+ RT=0.627 min (Analytical Condition 3) | 1 H NMR (400 MHz, DMSO-d6) δ ppm 0.59 - 0.67 (m, 1 H), 0.97 -1.03 (m, 1 H), 1.17-1.88 (m, 5 H), 2.75 - 2.86 (m, 5 H), 3.02 - 3.10 (m, 2 H), 6.46 (d, J=4.3 Hz, 1 H), 6.64 (dd, J=11.3, 2.2 Hz, 1 H), 7.57 (dd, J=11.1, 2.2 Hz, 1 H), 7.69 (d, J=8.7 Hz, 2 H), 7.91 (d, J=8.7 Hz, 2 H), 11.53 (s, 1 H), 13.37 (brs, 1 H) |
| 140 | 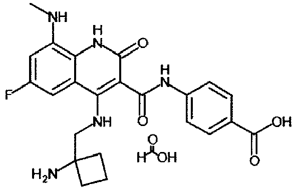 | 4-{[4-{[(1-aminocyclobutyl)methyl] amino}-6-fluoro-8-(methylamino)-2-oxo-1,2-dihydroquinoline-3-carbonyl]amino}benzoic acid-formate | MS (ESI pos.) m/z : 454 [M+H]+ RT=0.625 min (Analytical Condition 3) | 1H NMR (400 MHz, DMSO-d6) δ ppm 1.82 - 1.94 (m, 2 H), 2.04 - 2.27 (m, 4 H), 2.83 (d, J=4.3 Hz, 3 H), 3.96 (s, 2 H), 6.46 (d, J=4.3 Hz, 1 H), 6.61 (dd, J=11.5,2.1 Hz, 1 H), 7.05 (dd, J=10.5, 2.1 Hz, 1 H), 7.76 (d, J=8.6 Hz, 2 H), 7.94 (d, J=8.6 Hz, 2 H), 10.35 (br s, 1 H), 13.03 (br s, 1 H) |
| 141 | 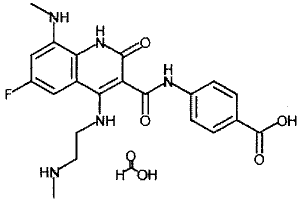 | 4-{[6-fluoro-8-(methylamino)-4-{[2-(methylamino)ethyl] amino}-2-oxo-1,2-dihydroquinoline-3-carbonyl]amino}benzoic acid-formate | MS (ESI pos.) m/z: 428 [M+H]+ RT=0.529 min (Analytical Condition 3) | 1H NMR (400 MHz, DMSO-d6) δ ppm 2.82 (d, J=4.4 Hz, 3 H), 3.01 - 3.10 (m, 2 H), 3.73 (br t, J=5.9 Hz, 2 H), 3.82 (s, 3 H), 6.40 (br d, J=4.4 Hz, 1 H), 6.57 (dd, J=11.4, 2.3 Hz, 1 H), 7.09 (dd, J=10.8, 2.3 Hz, 1 H), 7.77 (d, J=8.7 Hz, 2 H), 7.93 (d, J=8.7 Hz, 2 H), 9.34 (br s, 1 H), 12.43 (br s, 1 H) |
| 142 | 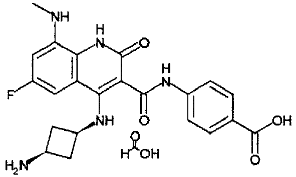 | 4-({4-[(cis-3-aminocyclobutyl)amino]-6-fluoro-8-(methylamino)-2-oxo-1,2-dihydroquinoline-3-carbonyl}amino)benzoic acid-formate | MS (ESI pos.) m/z : 440 [M+H]+ RT=0.567 min (Analytical Condition 3) | 1H NMR (400 MHz, DMSO-d6) δ ppm 2.19 (br d, J=8.9 Hz, 2 H), 2.74 - 2.88 (m, 5 H), 4.17 - 4.27 (m, 2 H), 6.47 (br d, J=4.3 Hz, 1 H), 6.61 (dd, J=11.2, 1.8 Hz, 1H), 6.80 (dd, J=10.4, 1.8 Hz, 1 H), 7.74 (d, J=8.7 Hz, 2 H), 7.94 (d, J=8.7 Hz, 2 H), 11.14 (brs, 1 H), 13.26 (brs, 1 H) |
| 143 | 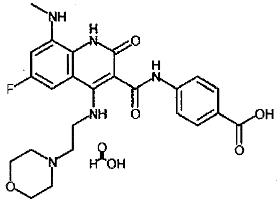 | 4-{[6-fluoro-8-(methylamino)-4-{[2-(morpholine-4-yl)ethyl]amino}-2-oxo-1,2-dihydroquinoline-3-carbonyl]amino}benzoic acid-formate | MS (ESI pos.) m/z: 484 [M+H]+ RT=0.583 min (Analytical Condition 3) | 1 H NMR (400 MHz, DMSO-d6) δ ppm 2.34 - 2.46 (m, 4 H), 2.50-2.60 (m, 2 H), 2.82 (d, J=4.4 Hz, 3 H), 3.54 - 3.64 (m, 4 H), 3.72 - 3.81 (m, 2 H), 6.42 (brs, 1 H), 6.58 (dd, J=11.4, 2.2 Hz, 1 H), 7.07 - 7.15 (m, 1 H), 7.75 (d, J=8.7 Hz, 2 H), 7.92 (d, J=8.7 Hz, 2 H), 10.48 (brs, 1 H), 10.64 (br s, 1 H), 13.05 (brs, 1 H) |

### Example 134 4-([6-Fluoro-8-(methylamino)-4-[3-(methylamino)propyl]-2-oxo-1,2-dihydroquinoline-3-carbonyl] amino)benzoic acid-trifluoroacetate

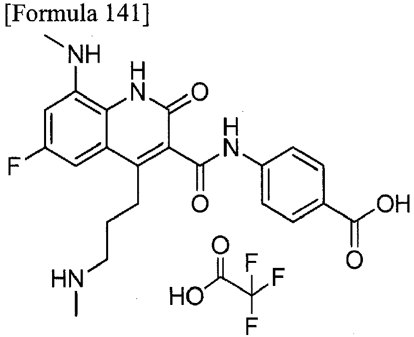

Using the tert-butyl (4-[2-amino-3-[benzyl(methyl)amino]-5-fluorophenyl]-4-[[tert-butyl(dimethyl)silyl]oxy]butyl)methylcarbamate obtained in Production Example 13-(4) as well as the 3-[4-(tert-butoxycarbonyl)anilino]-3-oxopropanoic acid obtained in Production Example 7-(2) and also applying the same procedures as in Example 130, the titled compound (24 mg) was obtained as a yellow solid.
MS (ESI pos.) m/z : 427 [M+H]+, RT=0.722 min (Analytical Condition 2)
¹H NMR (400 MHz, DMSO-d₆) δ ppm 1.78 - 2.00 (m, 2 H), 2.72 - 2.90 (m, 8 H), 3.00 (br s, 2 H), 6.36 (br s, 1 H), 6.58 (br d, J=10.3 Hz, 1 H), 6.94 (dd, J=10.3, 2.3 Hz, 1 H), 7.81 (d, J=8.8 Hz, 2 H), 7.95 (d, J=8.8 Hz, 2 H), 8.29 (br s, 1 H), 10.79 (s, 1 H), 11.27 (br s, 1 H), 12.08 (br s, 1 H)

### Example 147 6-Fluoro-4-hydroxy-N-[4-[(methanesulfonyl)carbamoyl]phenyl]-8-(methylamino)-2-oxo-1,2-dihydroquinoline-3-carboxamide

### 147-(1) 4-([8-[Benzyl(methyl)amino]-6-fluoro-4-hydroxy-2-oxo-1,2-dihydroquinoline-3-carbonyl]amino)benzoic acid

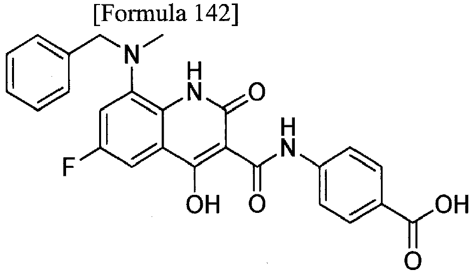

To a solution in chloroform (15 mL) of the tert-butyl 4-([8-[benzyl(methyl)amino]-6-fluoro-4-hydroxy-2-oxo-1,2-dihydroquinoline-3-carbonyl]amino)benzoate (798 mg) obtained in Example 117-(2), TFA (5 mL) was added at room temperature and the mixture was stirred for 3 hours at that temperature. After the end of the reaction, the reaction mixture was concentrated under reduced pressure to remove the solvent; to the concentrate, an aqueous solution of sodium hydrogencarbonate was added and the mixure was thoroughly stirred; thereafter, the solid was separated by filtration. The thus separated solid was thoroughly washed with water and diethyl ether, whereby the titled compound (580 mg) was obtained as a pale yellow solid.
¹H NMR (400 MHz, DMSO-d₆) δ ppm 2.68 (s, 3 H), 4.22 (br s, 2 H), 7.12 - 7.53 (m, 7 H), 7.78 (br d, J=8.4 Hz, 2 H), 7.98 (br d, J=8.4 Hz, 2 H), 10.48 - 11.49 (m, 1 H), 12.23 - 13.35 (m, 2 H)

### 147-(2) 8-[Benzyl(methyl)amino]-6-fluoro-4-hydroxy-N-[4-[(methanesulfonyl)carbamoyl]phenyl]-2-oxo-1,2-dihydroquinoline-3-carboxamide

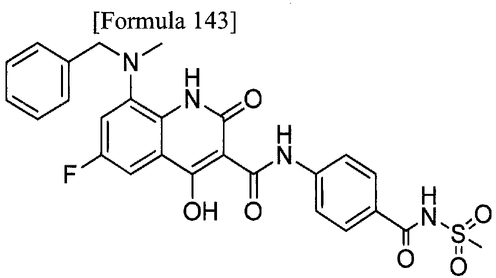

A solution in NMP (2.5 mL) and chloroform (5 mL) of 4-dimethylaminopyridine (122 mg), WSC•HCl (191 mg), methane sulfonamide (71 mg), and the 4-([8-[benzyl(methyl)amino]-6-fluoro-4-hydroxy-2-oxo-1,2-dihydroquinoline-3-carbonyl]amino)benzoic acid (230 mg) obtained in Example 147-(1) was stirred for 6 hours at 55°C, then followed by 2-day stirring at room temperature. After the end of the reaction, an aqueous solution of ammonium chloride was added to the reaction mixture and the precipitating solid was separated by filtration. The thus separated solid was thoroughly washed with diethyl ether, whereby the titled compound (100 mg) was obtained as a pale yellow solid.
¹H NMR (400 MHz, DMSO-d6) δ ppm 2.69 (s, 3 H), 3.18 (s, 3 H), 4.22 (s, 2 H), 6.85 - 7.51 (m, 7 H), 7.78 (d, J=8.8 Hz, 2 H), 8.02 (d, J=8.8 Hz, 2 H), 10.76 - 11.33 (m, 1 H), 12.70 - 13.16 (m, 1 H)

### 147-(3) 6-Fluoro-4-hydroxy-N-[4-[(methanesulfonyl)carbamoyl]phenyl]-8-(methylamino)-2-oxo-1,2-dihydroquinoline-3-carboxamide

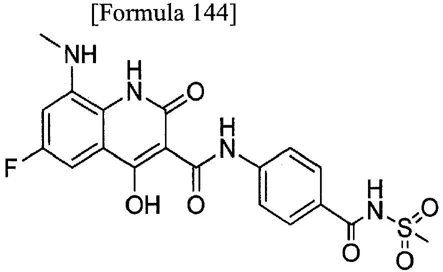

To a solution in DMF (3 mL) of the 8-[benzyl(methyl)amino]-6-fluoro-4-hydroxy-N-[4-[(methanesulfonyl)carbamoyl]phenyl]-2-oxo-1,2-dihydroquinoline-3-carboxamide (100 mg) obtained in Production Example 147-(2), a 5% palladium on active carbon catalyst (25 mg) was added and the mixture was stirred for 5 hours at 45°C under a hydrogen atmosphere. After the end of the reaction, the reaction mixture was filtered through Celite and the resulting filtrate was concentrated under reduced pressure to thereby obtain a crude product. The thus obtained crude product was thoroughly washed with chloroform, whereby the titled compound (10 mg) was obtained as a brown solid.
MS (ESI pos.) m/z : 449 [M+H]+, RT=0.797 min (Analytical Condition 4)
¹H NMR (400 MHz, DMSO-d₆) δ ppm 2.85 (br d, J=4.3 Hz, 3 H), 3.32 (s, 3 H), 6.54 (br s, 1 H), 6.61 - 6.75 (m, 1 H), 6.85 - 7.01 (m, 1 H), 7.79 (br d, J=8.7 Hz, 2 H), 8.01 (br d, J=8.7 Hz, 2 H), 11.22 - 11.41 (m, 1 H), 12.83 - 13.01 (m, 1 H)

The same procedures as in Example 147 were employed to obtain the Example compounds identified below from the corresponding raw materials.

**[Table 17]**

| Example | Structural Formula | Compound Name | MS | NMR |
|---|---|---|---|---|
| 147 | 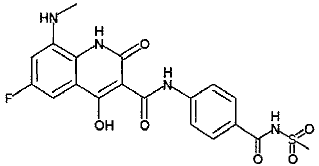 | 6-fluoro-4-hydroxy-N-{4-[(methanesulfonyl) carbamoyl]phenyl}-8-(methylamino)-2-oxo-1,2-dihydroquinoline-3-carboxamide | MS (ESI pos.) m/z : 449 [M+H]+ RT=0.797 min (Analytical Condition 4) | 1H NMR (400 MHz, DMSO-d6) δ ppm 2.85 (br d, J=4.3 Hz, 3 H), 3.32 (s, 3 H), 6.54 (br s, 1 H), 6.61 - 6.75 (m, 1 H), 6.85 - 7.01 (m, 1 H), 7.79 (br d, J=8.7 Hz, 2 H), 8.01 (br d, J=8.7 Hz, 2 H), 11.22 -11.41 (m, 1 H), 12.83 - 13.01 (m, 1 H) |
| 148 | 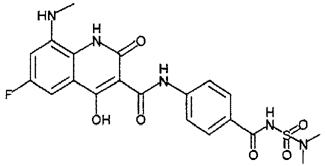 | N-{4-[(dimethylsulfamoyl) carbomoyl]phenyl}-6-fluoro-4-hydroxy-8-(methylamino)-2-oxo-1,2-dihydroquinoline-3-carboxamide | MS (ESI pos.) m/z: 478 [M+H]+ RT=0.848 min (Analytical Condition 4) | 1H NMR (400 MHz, DMSO-d6) δ ppm 2.84 (s, 3 H), 2.90 (s, 6 H), 6.57 -6.77 (m,2 H), 6.91 (dd, J=8.8, 2.4 Hz, 1 H), 7.79 (d, J=8.7 Hz, 2 H), 8.00 (d, J=8.7 Hz, 2 H), 11.38 (s, 1 H), 11.79 (s, 1 H), 12.95 (s, 1 H) |
| 164 | 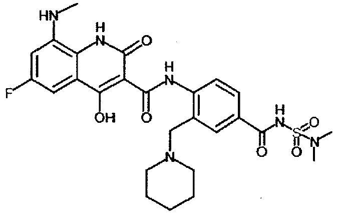 | N-{4-[(dimethylsulfamoyl) carbamoyl]-2-[(piperidine-1-yl)methyl]phenyl}-6-fluoro-4-hydroxy-8-(methylamino)-2-oxo-1,2-dihydroquinoline-3-carboxamide | MS (ESI pos.) m/z: 575 [M+H]+ RT=0.730 min (Analytical Condition 3) | 1H NMR (400 MHz, acetone-d6) δ ppm 1.49 (br s, 2 H), 1.70 (br s, 4 H), 2.81 (br s, 4 H), 2.91 -3.06 (m, 9 H), 3.87 (br s, 2 H), 5.89 (br s, 1 H), 6.70 (br dd, J=11.2, 2.3 Hz, 1 H), 7.03 (br d, J=8.0 Hz, 1 H), 8.00 (br d, J=8.4 Hz, 1 H), 8.21 (brs, 1 H), 8.37 (br d, J=8.4 Hz, 1 H), 10.10 (m, 1 H), 10.68 (brs, 1 H), 12.84 (brs, 1 H) |

### Example 150 N-(4-Carbamimidoylphenyl)-6-fluoro-4-hydroxy-8-(methylamino)-2-oxo-1,2-dihydroquinoline-3-carboxamide

### 150-(1) 8-[Benzyl(methyl)amino]-N-(4-carbamoylphenyl)-6-fluoro-4-hydroxy-2-oxo-1,2-dihydroquinoline-3-carboxamide

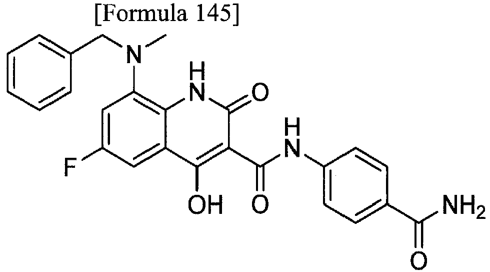

A solution in DMF (5 mL) of ammonium chloride (47 mg), WSC·HCl (166 mg), 4-dimethylaminopyridine (107 mg), and the 4-([8-[benzyl(methyl)amino]-6-fluoro-4-hydroxy-2-oxo-1,2-dihydroquinoline-3-carbonyl]amino)benzoic acid (200 mg) obtained in Example 147-1 was stirred for 2 hours at 65°C. The reaction mixture was left to cool to room temperature and to the reaction mixture, a saturated aqueous solution of ammonium chloride (40 mL) and water (10 mL) were added and the precipitating solid was recovered by filtration. The solid was washed with diethyl ether to obtain the titled compound (121 mg) as a brown solid.
MS (ESI pos.) m/z : 461 [M+H]+, RT=1.189 min (Analytical Condition 3)

### 150-(2) 8-[Benzyl(methyl)amino]-N-(4-cyanophenyl)-6-fluoro-4-hydroxy-2-oxo-1,2-dihydroquinoline-3-carboxamide

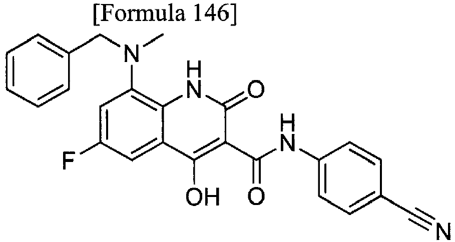

To a solution in THF (6 mL) of the 8-[benzyl(methyl)amino]-N-(4-carbamoylphenyl)-6-fluoro-4-hydroxy-2-oxo-1,2-dihydroquinoline-3-carboxamide (61 mg) obtained in Example 150-(1), pyridine (86 µL) and trifluoroacetic anhydride (112 µL) were sequentially added and the mixture was stirred for an hour at room temperature. Trifluoroacetic anhydride (56 µL) was further added and the mixture was stirred for 13.5 hours at room temperature. Yet another portion (56 µL) of trifluoroacetic anhydride was added and the mixture was stirred for 30 minutes at room temperature. To the reaction mixture, a saturated aqueous solution of ammonium chloride (20 mL) was added and extraction was conducted twice with chloroform (50 mL). The organic layers were passed through a phase separator and concentrated under reduced pressure with the aid of ISOLUTE HM-N. The residue was purified by column chromatography (silica gel cartridge, chloroform/methanol = 100:0 - 80:20) to obtain the titled compound (42 mg) as a colorless solid.
MS (ESI pos.) m/z : 443 [M+H]+, RT=1.430 min (Analytical Condition 3)

### 150-(3) 8-[Benzyl(methyl)amino]-6-fluoro-4-hydroxy-N-[4-(N'-hydroxycarbamimidoyl)phenyl]-2-oxo-1,2-dihydroquinoline-3-carboxamide

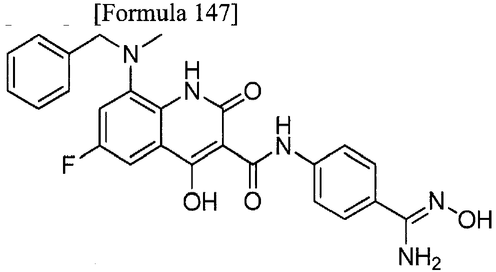

To a suspension in ethanol (2 mL) of the 8-[benzyl(methyl)amino]-N-(4-cyanophenyl)-6-fluoro-4-hydroxy-2-oxo-1,2-dihydroquinoline-3-carboxamide (40 mg) obtained in Example 150-(2), 50% hydroxylamine in aqueous solution (61 µL) was added and the mixture was stirred in a fused tube for 4 hours at 85°C. The precipitating solid was recovered by filtration and washed with ethanol (2 mL) to obtain the titled compound (28 mg) as a colorless solid.
MS (ESI pos.) m/z : 476 [M+H]+, RT=0.879 min (Analytical Condition 3)

### 150-(4) 8-[Benzyl(methyl)amino]-6-fluoro-4-hydroxy-2-oxo-N-[4-(5-oxo-4,5-dihydro-1,2,4-oxadiazol-3-yl)phenyl]-1,2-dihydroquinoline-3-carboxamide

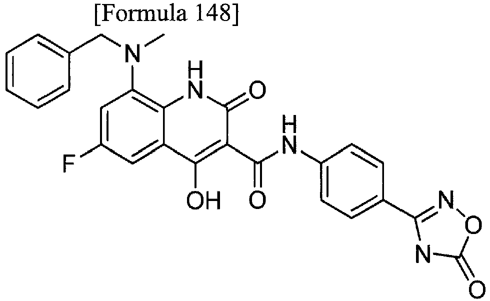

To a solution in pyridine (0.5 mL) of the 8-[benzyl(methyl)amino]-6-fluoro-4-hydroxy-N-[4-(N'-hydroxycarbamimidoyl)phenyl]-2-oxo-1,2-dihydroquinoline-3-carboxamide (26 mg) obtained in Example 150-(3), ethyl chloroformate (16 µL) was added under ice cooling and the mixture was stirred for 10 minutes at room temperature, followed by 4-hr stirring at 90°C. To the reaction mixture, a saturated aqueous solution of ammonium chloride (10 mL) was added and the precipitating solid was recovered by filtration. The solid was sequentially washed with diethyl ether, n-heptane and chloroform to obtain the titled compound (10 mg) as a light yellow solid.
MS (ESI neg.) m/z : 500 [M-H]-, RT=1.311 min (Analytical Condition 3)

### 150-(5) N-(4-Carbamimidoylphenyl)-6-fluoro-4-hydroxy-8-(methylamino)-2-oxo-1,2-dihydroquinoline-3-carboxamide

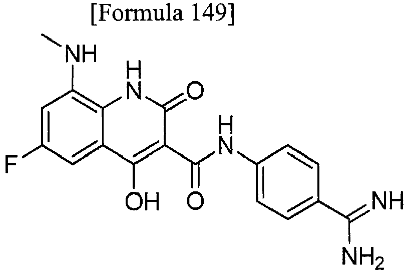

A suspension in DMF (15 mL) of 5% palladium on carbon (55 mg, containing 55% water) and the 8-[benzyl(methyl)amino]-6-fluoro-4-hydroxy-2-oxo-N-[4-(5-oxo-4,5-dihydro-1,2,4-oxadiazol-3-yl)phenyl]-1,2-dihydroquinoline-3-carboxamide (9 mg) obtained in Example 150-(4) was stirred for 3.5 hours at room temperature under a hydrogen atmosphere. The reaction mixture was filtered through Celite and the filtrate was concentrated under reduced pressure. The residue was washed with chloroform to obtain the titled compound (7 mg) as a light brown solid.
MS (ESI pos.) m/z : 370 [M+H]+, RT=0.636 min (Analytical Condition 3)
¹H NMR (600 MHz, DMSO-d6) δ ppm 2.83 (br d, J=4.1 Hz, 3 H), 6.53 - 6.70 (m, 1 H), 6.79 - 6.96 (m, 1 H), 7.84 - 7.93 (m, 4 H), 8.84 - 9.03 (m, 2 H), 9.17 - 9.35 (m, 2 H), 11.32 (br s, 1 H), 13.09 (br s, 1H)

### Example 151 6-Fluoro-N-[4-(S-methanesulfonimidoyl)phenyl]-8-(methylamino)-2-oxo-1 ,2-dihydroquinoline-3 -carboxamide

### 151-(1) 6-Fluoro-N-[4-[S-methane-N-(2,2,2-trifluoroethane)sulfonimidoyl]phenyl]-8-(methylamino)-2-oxo-1,2-dihydroquinoline-3-carboxamide

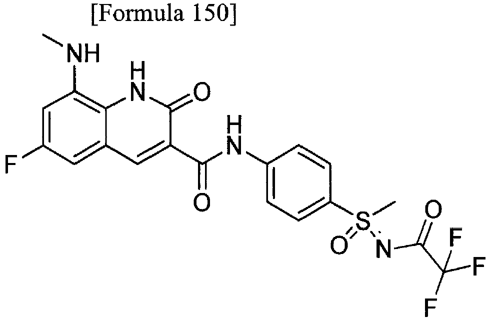

To a solution in DMF (5 mL) of N-[(4-aminophenyl)(methyl)oxo-lambda6-sulfanylidene]-2,2,2-trifluoroacetamide (110 mg), HATU (261 mg), and the 6-fluoro-8-(methylamino)-2-oxo-1,2-dihydroquinoline-3-carboxylic acid (81 mg) obtained in Production Example 2-(5), DIPEA (120 µL) was added and the mixture was stirred for 21.5 hours at room temperature, followed by 3.5-hr stirring at 70°C. To the reaction mixture, water (30 mL) was added, and the precipitating solid was recovered by filtration and washed with diethyl ether. The solid was dissolved in methanol/chloroform and concentrated under reduced pressure with the aid of ISOLUTE HM-N. The residue was purified by column chromatography (NH silica gel cartridge, chloroform/methanol = 95:5 - 90:10) to obtain the titled compound (34 mg) as a yellow solid.
MS (ESI pos.) m/z : 485 [M+H]+, RT=1.093 min (Analytical Condition 3)

### 151-(2) 6-Fluoro-N-[4-(S-methanesulfonimidoyl)phenyl]-8-(methylamino)-2-oxo-1,2-dihydroquinoline-3-carboxamide

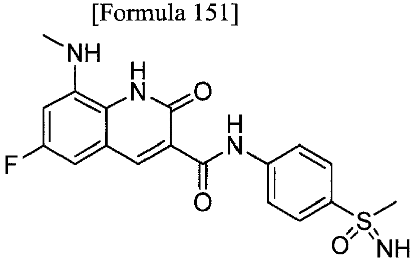

To a suspension in methanol (20 mL) of the 6-fluoro-N-[4-[S-methane-N-(2,2,2-trifluoroethane)sulfonimidoyl]phenyl]-8-(methylamino)-2-oxo-1,2-dihydroquinoline-3-carboxamide (34 mg) obtained in Example 151-(1), potassium carbonate (17 mg) was added and the mixture was stirred for 30 minutes at room temperature. After further addition of potassium carbonate (10 mg), the mixture was stirred for an additional 2 hours at room temperature. The reaction mixture was filtered and the filtrate was concentrated under reduced pressure. To the residue, water (15 mL) was added and the mixture was neutralized with a saturated aqueous solultion of ammonium chloride. Afer extraction with chloroform (200 mL) and chloroform/methanol (90/10, 200 mL), the organic layers were passed through a phase separator and concentrated under reduced pressure. The residue was stirred for 10 minutes in diethyl ether (10 mL) and, thereafter, the insoluble matter was recovered by filtration to obtain the titled compound (19 mg) as a yellow solid.
MS (ESI pos.) m/z : 389 [M+H]+, RT=0.755 min (Analytical Condition 3)
¹H NMR (600 MHz, DMSO-d6) δ ppm 2.86 (d, J=4.5 Hz, 3 H), 3.06 (s, 3 H), 4.14 (s, 1 H), 6.55 (br d, J=4.5 Hz, 1 H), 6.65 (dd, J=11.6, 2.5 Hz, 1 H), 7.07 (dd, J=8.7, 2.5 Hz, 1 H), 7.91 - 7.96 (m, 4 H), 8.89 (s, 1H), 11.87 (br s, 1H), 12.47 (br s, 1H)

### Example 152 Methyl 4-[[6-fluoro-4-hydroxy-8-(methylamino)-2-oxo-1,2-dihydroquinoline-3-carbonyl]amino]-3-[(piperidin-1-yl)methyl]benzoate

### 152-(1) Methyl 4-([8-[benzyl(methyl)amino]-6-fluoro-4-hydroxy-2-oxo-1,2-dihydroquinoline-3 -carbonyl] amino)-3 -iodobenzoate

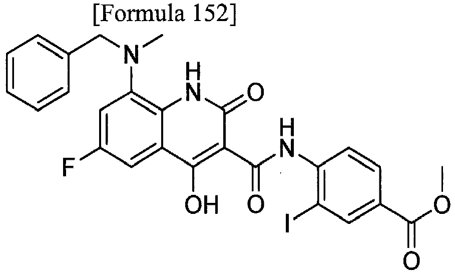

A solution in chloroform (15 mL) of 4-dimethylaminopyridine (0.81 mg), WSC· HCl (1.3 g), the methyl 2-amino-3-[benzyl(methyl)amino]-5-fluorobenzoate (1.4 g) obtained in Production Example 6-(2), and the 3-[2-iodo-4-(methoxycarbonyl)anilino]-3-oxopropanoic acid (1.2 g) obtained in Production Example 10-(2) was stirred for 17 hours at 60°C. After the end of the reaction, an aqueous solution of ammonium chloride was added to the reaction mixture for quenching the reaction. The resulting organic layer was washed with sodium hydrogencarbonate and saturated brine, dried over anhydrous magnesium sulfate, and concentrated under reduced pressure to thereby obtain a crude product. The thus obtained crude product was purified by (silica gel cartridge, chloroform/methanol = 100:0 - 95:5) to obtain the titled compound (0.718 g) as a yellow solid.
MS (ESI/APCI pos.) m/z : 602 [M+H]+, RT=3.28-3.36 min (Analytical Condition 1)

### 152-(2) Methyl 4-([8-[benzyl(methyl)amino]-6-fluoro-4-hydroxy-2-oxo-1,2-dihydroquinoline-3-carbonyl]amino)-3-ethenylbenzoate

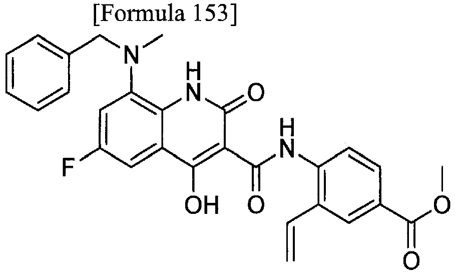

A solution in DMF (12 mL) of SUPERSTABLE Pd(0) catalyst (333 mg), tributyl(vinyl)tin (0.42 mL), and the methyl 4-([8-[benzyl(methyl)amino]-6-fluoro-4-hydroxy-2-oxo-1,2-dihydroquinoline-3-carbonyl]amino)-3-iodobenzoate (718 mg) obtained in Example 152-(1) was stirred for 2 hours at 120°C using a microwave applicator. After the end of the reaction, the reaction mixture was concentrated under reduced pressure to thereby obtain a crude product. The thus obtained crude product was purified by column chromatography (silica gel cartridge, chloroform/methanol = 100:0 - 95:5) to obtain the titled compound (115 mg).
¹H NMR (400 MHz, CHLOROFORM-d) δ ppm 2.67 (s, 3 H), 3.94 (s, 3 H), 4.09 (s, 2 H), 5.59 (d, J=11.5 Hz, 1 H), 5.86 (d, J=17.2 Hz, 1 H), 7.08 (dd, J=17.2, 11.5 Hz, 1 H), 7.21 (dd, J=8.7, 2.6 Hz, 1 H), 7.24 - 7.45 (m, 5 H), 7.59 (dd, J=8.7, 2.6 Hz, 1 H), 8.00 (dd, J=8.6, 2.0 Hz, 1 H), 8.20 (d, J=2.0 Hz, 1 H), 8.33 (d, J=8.6 Hz, 1 H), 9.20 (s, 1 H), 12.54 (s, 1H)

### 152-(3) Methyl 4-([8-[benzyl(methyl)amino]-6-fluoro-4-hydroxy-2-oxo-1,2-dihydroquinoline-3-carbonyl]amino)-3-formylbenzoate

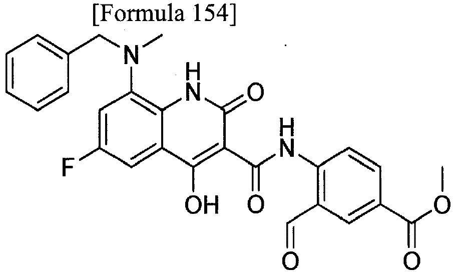

To a solution in 1,4-dioxane (5 mL) and water (1.25 mL) of 2,6-dimethylpyridine (0.05 mL), sodium periodate (207 mg), and the methyl 4-([8-[benzyl(methyl)amino]-6-fluoro-4-hydroxy-2-oxo-1,2-dihydroquinoline-3-carbonyl]amino)-3-ethenylbenzoate (115 mg) obtained in Production Example 152-(2), 4% osmium oxide in aqueous solution (0.13 mL) was added at room temperature and the mixture was stirred for 17 hours. After the end of the reaction, chloroform and water were added to the reaction mixture. The resulting organic layer was washed with water and saturated brine, dried over anhydrous magnesium sulfate and concentrated under reduced pressure to thereby obtain a crude product. The thus obtained crude product was purified by (silica gel cartridge, chloroform/methanol = 100:0 - 95:5) to obtain the titled compound (96 mg) as a colorless solid.
¹H NMR (400 MHz, CHLOROFORM-d) δ ppm 2.66 (s, 3 H), 3.97 (s, 3 H), 4.09 (s, 2 H), 7.20 (dd, J=9.0, 2.6 Hz, 1 H), 7.26 - 7.38 (m, 5 H), 7.57 (dd, J=8.0, 2.6 Hz, 1 H), 8.28 (dd, J=8.6, 2.1 Hz, 1 H), 8.46 (d, J=2.1 Hz, 1 H), 8.82 (d, J=8.6 Hz, 1 H), 9.18 (s, 1 H), 10.12 (s, 1 H)

### 152-(4) Methyl 4-([8-[benzyl(methyl)amino]-6-fluoro-4-hydroxy-2-oxo-1,2-dihydroquinoline-3-carbonyl]amino)-3-[(piperidin-1-yl)methyl]benzoate

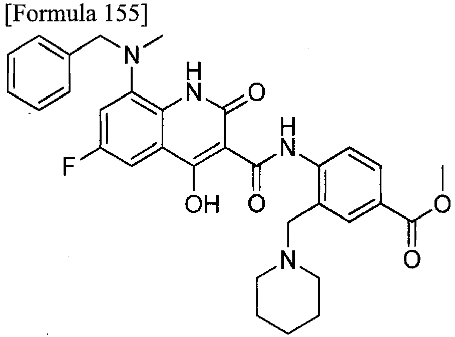

To a solution in chloroform (4 mL) of acetic acid (0.08 mL), piperidine (0.11 mL), and the methyl 4-([8-[benzyl(methyl)amino]-6-fluoro-4-hydroxy-2-oxo-1,2-dihydroquinoline-3-carbonyl]amino)-3-formylbenzoate (70 mg) obtained in Production Example 152-(3), sodium triacetoxyborohydride (147 mg) was added and the mixtrure was stirred for 6 hours at 60°C. After the end of the reaction, sodium hydrogencarbonate was added to the reaction mixture for quenching the reaction and chloroform was added thereafter. The resulting organic layer was washed with water and saturated brine and thereafer dried over anhydrous magnesium sulfate and concentrated under reduced pressure to thereby obtain a crude product. The thus obtained crude product was purified by (silica gel cartridge, chloroform/methanol = 100:0 - 95:5) to obtain the titled compound (30 mg) as a pale yellow solid.
MS (ESI pos.) m/z : 573 [M+H]+, RT=0.683 min (Analytical Condition 4)

### 152-(5) Methyl 4-[[6-fluoro-4-hydroxy-8-(methylamino)-2-oxo-1,2-dihydroquinoline-3-carbonyl]amino]-3-[(piperidin-1-yl)methyl]benzoate

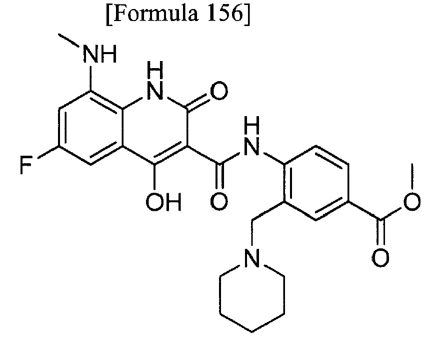

To a solution in DMF (2 mL) of the methyl 4-([8-[benzyl(methyl)amino]-6-fluoro-4-hydroxy-2-oxo-1,2-dihydroquinoline-3-carbonyl]amino)-3-[(piperidin-1-yl)methyl]benzoate (39 mg) obtained in Production Example 152-(4), a 10% palladium on active carbon catalyst (40 mg) was added and the mixtdure was stirred for 3 hours at room temperature under a hydrogen atmosphere. After the end of the reaction, the reaction mixture was passed through Celite and the resulting filtrate was concentrated under reduced pressure to obtain a crude product. The thus obtained crude product was purified by column chromatography (silica gel cartridge, chloroform/methanol = 100:0 - 95:5), whereupon the titled compound (14 mg) was obtained as a pale yellow solid.
MS (ESI pos.) m/z : 483 [M+H]+, RT=0.772 min (Analytical Condition 3)
¹H NMR (400 MHz, CHLOROFORM-d) δ ppm 1.16 - 1.36 (m, 2 H)1.40 - 1.70 (m, 4 H), 2.41 (br s, 4 H), 2.58 (br s, 3 H), 3.49 (s, 2 H), 3.94 (s, 3 H), 5.50 (br s, 1 H), 6.44 (br dd, J=9.6, 1.9 Hz, 1 H), 7.10 (br dd, J=9.6, 1.9 Hz, 1 H), 7.93 (d, J=1.3 Hz, 1 H), 8.00 - 8.11 (m, 1 H), 8.20 (d, J=8.6 Hz, 1 H), 11.94 (br s, 1H), 12.30 (br s, 1 H)

### Example 155 4-[[6-Fluoro-4-hydroxy-8-(methylamino)-2-oxo-1,2-dihydroquinoline-3 -carbonyl] amino]-3-[(morpholin-4-yl)methyl]benzoic acid

### 155-(1) Ethyl 4-([8-[benzyl(methyl)amino]-6-fluoro-4-hydroxy-2-oxo-1,2-dihydroquinoline-3-carbonyl]amino)-3-[(morpholin-4-yl)methyl]benzoate

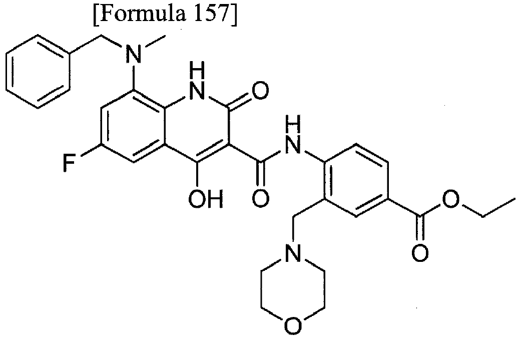

A solution in DMF (18 mL) of 4-dimethylaminopyridine (660 mg), TEA (1.0 mL), WSC·HCl (1.0 g), the methyl 2-amino-3-[benzyl(methyl)amino]-5-fluorobenzoate (520 mg) obtained in Production Example 6-(2), and the 3-[4-(methoxycarbonyl)-2-[(morpholin-4-yl)methyl]anilino]-3-oxopropanoic acid (1.2 g) obtained in Production Example 14-(6) was stirred for 46 hours at room temperature. After the end of the reaction, an aqueous solution of ammonium chloride was added to the reaction mixture for quenching the reaction. The resulting organic layer was washed with sodium hydrogencarbonate and saturated brine, dried over anhydrous magnesium sulfate and concentgrated under reduced pressure to thereby obtain a crude product. The thus obtained crude product was used in the next reaction without being further purified.

To an ethanol solution (18 mL) of the aforementioned crude product, 20% sodium ethoxide in ethanol solution (0.71 mL) was added and the mixture was stirred for 5 hours at room temperature. After the end of the reaction, the reaction mixture was concentrated under reduced pressure and, thereafter, chloroform and an aqueous solution of ammonium chloride were added. The resulting organic layer was washed with saturated brine, dried over anhydrous magnesium sulfate and concentrated under reduced pressure to obtain a crude product. The thus obtained crude product was purified by column chromatography (silica gel cartridge, chloroform/methanol = 100:0 - 95:5) to obtain the titled compound (270 mg) as a pale yellow solid.
MS (ESI pos.) m/z : 589 [M+H]+, RT=0.715 min (Analytical Condition 4)

### 155-(2) Ethyl 4-[[6-fluoro-4-hydroxy-8-(methylamino)-2-oxo-1,2-dihydroquinoline-3-carbonyl]amino]-3-[(morpholin-4-yl)methyl]benzoate

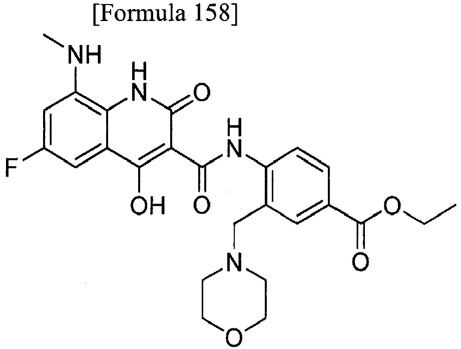

To a solution in DMF (9 mL) of the ethyl 4-([8-[benzyl(methyl)amino]-6-fluoro-4-hydroxy-2-oxo-1,2-dihydroquinoline-3-carbonyl]amino)-3-[(morpholin-4-yl)methyl]benzoate (270 mg) obtained in Production Example 155-(1), a 10% palladium on active carbon catalyst (135 mg) was added and the mixture was stirred for 3 hours at room temperature under a hydrogen atmosphere. After the end of the reaction, the reaction mixture was passed through Celite and the resulting filtrate was concentrated under reduced pressure to obtain a crude product. The thus obtained crude product was purified by column chromatography (silica gel cartridge, chloroform/methanol = 100:0 - 95:5), whereupon the titled compound (200 mg) was obtained as a brown solid.
MS (ESI pos.) m/z : 499 [M+H]+, RT=0.679 min (Analytical Condition 4)

### 155-(3) 4-[[6-Fluoro-4-hydroxy-8-(methylamino)-2-oxo-1,2-dihydroquinoline-3-carbonyl]amino]-3-[(morpholin-4-yl)methyl]benzoic acid

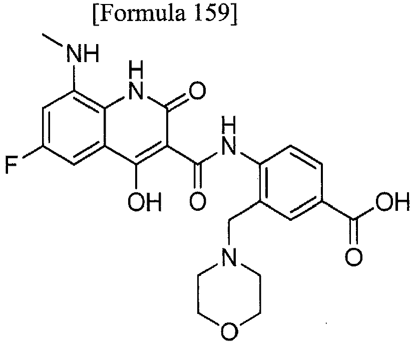

A solultion in a 1,4-dioxane (2 mL)/methanol (1 mL)/water (1 mL) mixture of lithium hydroxide monohydrate (83 mg) and the ethyl 4-[[6-fluoro-4-hydroxy-8-(methylamino)-2-oxo-1,2-dihydroquinoline-3-carbonyl]amino]-3-[(morpholin-4-yl)methyl]benzoate (197 mg) obtained in Production Example 155-(2) was stirred for 2 hours at 60°C. After the end of the reaction, the solvents were removed by concentrating the reaction mixture under reduced pressure and the resulting concentrate was dissolved in water. The resulting solution was then pH adjusted with an aqueous solution of hydrochloric acid and an aqueous solution of sodium hydrogencarbonate such that it would be at pH=5. The precipitating solid was separated by filtration and washed with a sufficient amount of water, whereby the titled compound (100 mg) was obtained as a green solid.
MS (ESI pos.) m/z : 471 [M+H]+, RT=0.715 min (Analytical Condition 3)
¹H NMR (400 MHz, DMSO-d6) δ ppm 2.43 (br s, 4 H), 2.84 (d, J=4.4 Hz, 3 H), 3.49 - 3.72 (m, 6 H), 6.56 (br d, J=4.4 Hz, 1 H), 6.61 - 6.69 (m, 1 H), 6.91 (dd, J=8.8, 2.3 Hz, 1 H), 7.88 (br s, 1 H), 7.93 (br d, J=8.6 Hz, 1 H), 8.27 (d, J=8.6 Hz, 1 H), 11.11 (br s, 1 H), 12.70 (br s, 1 H), 12.94 (br s, 1H)

The same procedures as in Example 155 were employed to obtain the Example compounds identified below from the corresponding raw materials.

**[Table 18]**

| Example | Structural Formula | Compound Name | MS | NMR |
|---|---|---|---|---|
| 155 | 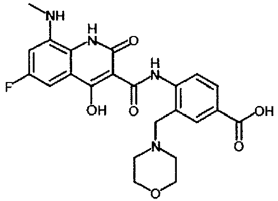 | 4-{[6-fluoro-4-hydroxy-8-(methylamino)-2-oxo-1,2-dihydroquinoline-3-carbonyl]amino)-3-[(morpholine-4-yl)methyl]benzoic acid | MS (ESI pos.) m/z: 471 [M+H]+ RT=0.715 min (Analytical Condition 3) | 1H NMR (400 MHz, DMSO-d6) δ ppm 2.43 (br s, 4 H), 2.84 (d, J=4.4 Hz, 3 H), 3.49 - 3.72 (m, 6 H), 6.56 (br d, J=4.4 Hz, 1 H), 6.61 - 6.69 (m, 1 H), 6.91 (dd, J=8.8, 2.3 Hz, 1 H), 7.88 (br s, 1 H), 7.93 (br d, J=8.6 Hz, 1 H), 8.27 (d, J=8.6 Hz, 1 H), 11.11 (br s, 1 H), 12.70 (br s, 1 H), 12.94 (br s, 1 H) |
| 156 | 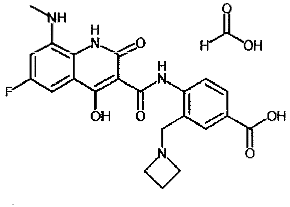 | 3-[(azetidine-1-yl)methyl]-4-{(6-fluoro-4-hydroxy-8-(methylamino)-2-oxo-1,2-dihydroquinoline-3-carbonyl]amino}benzoic acid-formate | MS (ESI pos.) m/z: 441 [M+H]+ RT=0.678 min (Analytical Condition 3) | 1H NMR (400 MHz, DMSO-d6) δ ppm 1.86 - 2.17 (m, 2 H), 2.84 (br d, J=3.5 Hz, 3 H), 3.01 - 3.84 (m, 6 H), 5.98-6.73 (m, 2 H), 6.91 (brs, 1 H), 7.84 (br d, J=7.3 Hz, 1 H), 7.91 (br s, 1 H), 8.45 (br s, 1 H) |
| 157 | 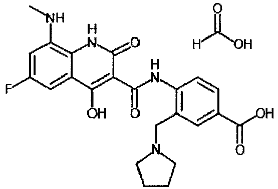 | 4-{[6-fluoro-4-hydroxy-8-(methylamino)-2-oxo-1,2-dihydroquinoline-3-carbonyl]amino}-3-[(pyrrolidine-1-yl)methyl]benzoic acid-formate | MS (ESI pos.) m/z: 455 [M+H]+ RT=0.698 min (Analytical Condition 3) | 1 H NMR (400 MHz, DMSO-d6) δ ppm 1.71 (br s, 4 H), 2.58 (br s, 4 H), 2.81 (br d, J=5.0 Hz, 3 H), 3.77 (br s, 2 H), 5.93-6.67 (m, 2 H), 6.77 - 6.93 (m, 1 H), 7.72 - 7.87 (m, 1 H), 7.91 (s, 1 H), 8.44 (br d, J=7.3 Hz, 1 H) |

### Example 159 N-[4-(2,4-Dioxo-1,3-oxazolidin-5-yl)phenyl]-6-fluoro-8-(methylamino)-2-oxo-1,2-dihydroquinoline-3-carboxamide

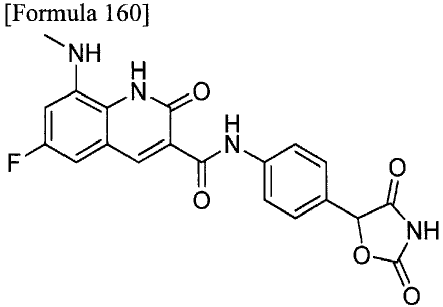

To a suspension in ethanol (10 mL) of the N-[4-(2-amino-1-hydroxy-2-oxoethyl)phenyl]-6-fluoro-8-(methylamino)-2-oxo-1,2-dihydroquinoline-3-carboxamide (18 mg) obtained in Example 158, diethyl carbonate (13 µL) and 20% sodium ethoxide in ethanol solution (55 µL) were added and the mixture was heated under reflux for 1.5 hours. Diethyl carbonate (13 µL) and 20% sodium ethoxide in ethanol solution (55 µL) were additionally supplied and the mixture was heated under reflux for an hour. Diethyl carbonate (26 µL) was additionally supplied and the mixture was heated under reflux for 19 hours. Diethyl carbonate (52 µL) and 20% sodium ethoxide in ethanol solution (110 µL) were additionally supplied and the mixture was heated under reflux for 6 hours. Further, diethyl carbonate (52 µL) and 20% sodium ethoxide in ethanol solution (110 µL) were additionally supplied and the mixture was heated under reflux for an hour. The reaction mixture was left to cool to room temperature and using an aqueous solultion of 1 M hydrochloric acid, a saturated aqueous solultion of sodium hydrogencarbonate, and a saturated aqueous solultion of ammonium chloride, pH=6 was established. Extraction was conducted with ethyl acetate (100 mL×3); the organic layers were dried over anhydrous magnesium sulfate and thereafter concentrated under reduced pressure to obtain the titled compound (5 mg) as a yellow solid. MS (ESI pos.) m/z : 411 [M+H]+, RT=0.923 min (Analytical Condition 3)
¹H NMR (600 MHz, DMSO-d6) δ ppm 2.85 (br d, J=4.5 Hz, 3 H), 6.01 (s, 1 H), 6.55 (br d, J=4.5 Hz, 1 H), 6.64 (br d, J=11.6 Hz, 1 H), 7.06 (br d, J=8.7 Hz, 1 H), 7.38 - 7.48 (m, 2 H), 7.76 - 7.86 (m, 2 H), 8.88 (s, 1 H), 11.86 (br s, 1 H), 12.24 (s, 1 H)

### Example 160 4-[(8-Hydroxy-2-oxo-1,2-dihydroquinoline-3-carbonyl)amino]benzoic acid

### 160-(1) tert-Butyl 4-[(8-methoxy-2-oxo-1,2-dihydroquinoline-3-carbonyl)amino]benzoate

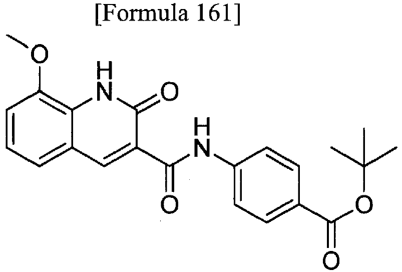

To a solution in chloroform (10 mL) of the 3-[4-(tert-butoxycarbonyl)anilino]-3-oxopropanoic acid (961 mg) obtained in Production Example 7-(2), DMF (26 µL) and oxalyl chloride (349 µL) were added and after 1-hr stirring at room temperature, the reaction mixture was concentrated under reduced pressure. To the residue, chloroform (10 mL) was added to form an acid chloride solution. To a solution of 2-amino-3-methoxybenzaldehyde (260 mg) and triethylamine (959 µL) in chloroform (10 mL), the separately prepared acid chloride solution was added dropwise under ice cooling and the mixture was stirred for 15 hours at room temperature. To the reaction mixture, chloroform was added and the precipitate was dissolved; thereafter, the solution was concentrated under reduced pressure with the aid of ISOLUTE HM-N. The residue was purified by column chromatography (NH silica gel cartridge, hexane/ethyl acetate = 80:20 - 0:100) and, thereafter, recrystallization was performed with ethyl acetate/chloroform/hexane to obtain the titled compound (209 mg) as a colorless solid.
MS (ESI pos.) m/z : 395 [M+H]+, RT=1.204 min (Analytical Condition 3)

### 160-(2) 4-[(8-Methoxy-2-oxo-1,2-dihydroquinoline-3-carbonyl)amino]benzoic acid

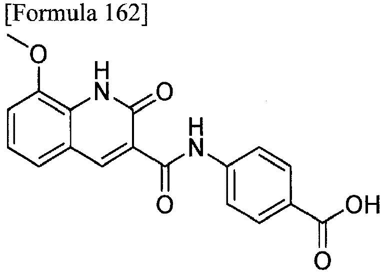

To the tert-butyl 4-[(8-methoxy-2-oxo-1,2-dihydroquinoline-3-carbonyl)amino]benzoate (100 mg) obtained in Example 160-(1), 48% hydrobromic acid (3 mL) was added and the mixtrure was stirred for 5 hours at 100°C. Following the addition of acetic acid (2 mL), the mixture was stirred for an additional 2 hours at 100°C. The precipitate was recovered by filtration and washed with water; thereafter, the titled compound (58 mg) was obtained as a colorless solid.
MS (ESI neg.) m/z : 337 [M-H]-, RT=0.859 min (Analytical Condition 3)

### 160-(3) 4-[(8-Hydroxy-2-oxo-1,2-dihydroquinoline-3-carbonyl)amino]benzoic acid

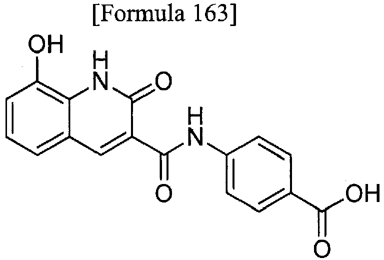

To a suspension in chloroform (6 mL) of the 4-[(8-methoxy-2-oxo-1,2-dihydroquinoline-3-carbonyl)amino]benzoic acid (56 mg) obtained in Example 160-(2), boron tribromide (1.0 mL) was added dropwise under ice cooling and the mixture was stirred for 4 hours at 60°C. The reaction mixture was ice cooled and after adding methanol (10 mL) dropwise, the mixture was concentrated under reduced pressure. To the residue, 2 M sodium hydroxide in aqueous solution (10 mL) and water (10 mL) were added and extraction was conducted with ethyl acetate (25 mL×2). To the aqueous layer, an aqueous solution of 1 M hydrochloric acid was added to establish pH=1 and, thereafter, the precipitating solid was recovered by filtration. The solid was dissolved in methanol/chloroform and filtered; thereafter, the filtrate was concentrated under reduced pressure to obtain the titled compound (12 mg) as a colorless solid.
MS (ESI neg.) m/z : 323 [M-H]-, RT=0.754 min (Analytical Condition 3)
¹H NMR (600 MHz, DMSO-d6) δ ppm 7.11 - 7.19 (m, 2 H), 7.40 - 7.51 (m, 1 H), 7.84 (d, J=8.7 Hz, 2 H), 7.97 (d, J=8.7 Hz, 2 H), 8.94 (s, 1 H), 10.77 (br s, 1 H), 11.71 (br s, 1 H), 12.53 (s, 1 H), 12.69 (br s, 1 H)

The same procedures as in Example 160 were employed to obtain the compound of Example 161 from the corresponding raw material.

**[Table 19]**

| Example | Structural Formula | Compound Name | MS | NMR |
|---|---|---|---|---|
| 160 | 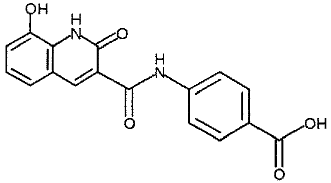 | 4-[(8-hydroxy-2-oxo-1,2-dihydroquinoline-3-carbonyl)amino]benzoic acid | MS (ESI neg.) m/z: 323 [M-H]-RT=0.754 min (Analytical Condition 3) | 1H NMR (600 MHz, DMSO-d6) δ ppm 7.11 - 7.19 (m, 2 H), 7.40 - 7.51 (m, 1 H), 7.84 (d, J=8.7 Hz, 2 H), 7.97 (d, J=8.7 Hz, 2 H), 8.94 (s, 1 H), 10.77 (br s, 1 H), 11.71 (brs, 1 H), 12.53 (s, 1 H), 12.69 (br s, 1 H) |
| 161 | 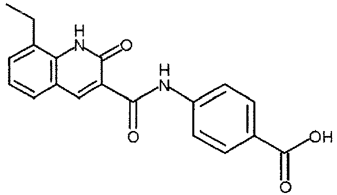 | 4-[(8-ethyl-2-oxo-1,2-dihydroquinoline-3-carbonyl)amino]benzoic acid | MS (ESI neg.) m/z: 335 [M-H]-RT=0.971 min (Analytical Condition 3) | 1H NMR (600 MHz, DMSO-d6) δ ppm 1.21 (t, J=7.4 Hz, 3 H), 2.98 (q, J=7.4 Hz, 2 H), 7.30 (t, J=7.6 Hz, 1 H), 7.59 (d, J=7.0 Hz, 1 H), 7.83 - 7.89 (m, 3 H), 7.98 (d, J=8.7 Hz, 2 H), 8.99 (s, 1 H), 11.89 (s, 1 H), 12.42 (s, 1 H), 12.78 (br s, 1 H) |

### Example163 4-([[6-Fluoro-8-(methylamino)-2-oxo-1,2-dihydroquinoline-3-yl]methyl]amino)benzoic acid

### 163-(1) Methyl 4-([[6-fluoro-8-(methylamino)-2-oxo-1,2-dihydroquinoline-3-yl]methyl] amino)benzoate

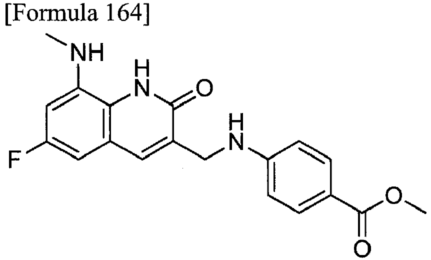

To a solution in NMP (1 mL) of the 3-(chloromethyl)-6-fluoro-8-(methylamino)quinolin-2(1H)-one (27 mg) obtained in Production Example 11-(1), methyl 4-aminobenzoate (19 mg) and potassium carbonate (62 mg) were added and the mixture was stirred for 2 hours at 80°C. After being left to cool to room temperature, the reaction mixture was diluted with chloroform and washed with water, a saturated aqueous solution of ammonium chloride, saturated aqueous sodium bicarbonate, and brine. The aqueous layer was separated by a phase separator and the organic layer was concentrated under reduced pressure. The residue was purified by column chromatography (NH silica gel cartridge, chloroform/methanol = 99:1 - 85:15) to obtain the titled compound (16 mg) as a yellow solid. MS (ESI pos.) m/z : 356 [M+H]+, RT=0.941 min (Analytical Condition 3)

### 163-(2) 4-([[6-Fluoro-8-(methylamino)-2-oxo-1,2-dihydroquinoline-3-yl]methyl]amino)benzoic acid

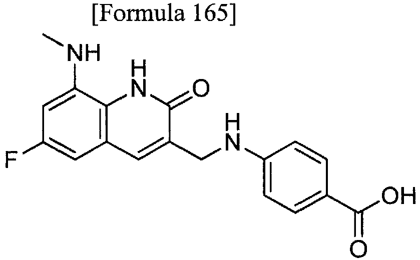

To a solution in THF (0.5 mL) of the methyl 4-([[6-fluoro-8-(methylamino)-2-oxo-1,2-dihydroquinolin-3-yl]methyl]amino)benzoate (16 mg) obtained in Example 163-(1), 2 M sodium hydroxide in aqueous solultion (0.5 mL) was added and the mixture was stirred for 19 hours at room temperature and for an additional 19 hours at 65°C. After leaving the reaction mixture to cool to room temperature, THF was concentrated under reduced pressure. To the thus obtained aqueous solution, an aqueous solution of 1 M hydrochloric acid was added to establish pH 4. The precipitating solid was recovered by filtration and washed with water. By subsequent drying, the titled compound (6.2 mg) was obtained as a colorless solid. MS (ESI pos.) m/z : 342 [M+H]+, RT=0.791 min (Analytical Condition 3)
¹H NMR (600 MHz, DMSO-d6) δ ppm 2.81 (d, J=4.5 Hz, 3 H), 4.21 (br d, J=5.4 Hz, 2 H), 6.26 (br d, J=4.5 Hz, 1 H), 6.44 (dd, J=12.0, 2.5 Hz, 1 H), 6.59 (d, J=9.1 Hz, 2 H), 6.67 (dd, J=9.1, 2.5 Hz, 1 H), 6.91 (br t, J=5.4 Hz, 1 H), 7.61 (s, 1 H), 7.66 (d, J=9.1 Hz, 2 H), 11.11 (br s, 1 H), 12.00 (br s, 1 H)

### Example 165 3-[(8-Amino-2-oxo-1,2-dihydroquinoline-3-carbonyl)amino]benzoic acid-trifluoroacetate

### 165-(1) 3-[(8-Nitro-2-oxo-1,2-dihydroquinoline-3-carbonyl)amino]benzoic acid

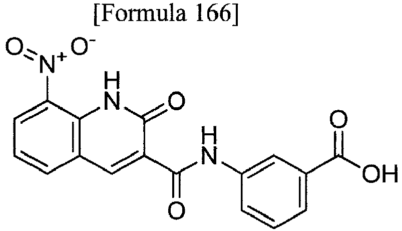

A suspension of 8-nitro-2-oxo-1,2-dihydroquinoline-3-carboxylic acid (301 mg) in thionyl chloride (10 mL) was stirred under reflux for an hour. After being left to cool to room temperature, the reaction mixture was concentrated under reduced pressure. To a solution of the residue in THF (4 mL), methyl 3-aminobenzoate (233 mg) was added under ice cooling and the mixture as such was stirred for an hour. The reaction mixture was filtered and the solid obtained was suspended in methanol, followed by 30-min stirring at room temperature. The suspended matter was recovered by filtration. To a suspension of the obtained solid in THF (3 mL), 1 M sodium hydroxide in aqueous solution (7 mL) was added amd the mixture was stirred for 3 days at room temperature. To the reaction mixture, an aqueous solution of 1 M hydrochloric acid was added and the precipitating solid was recovered by filtration. Upon washing with water, the titled compound (75 mg) was obtained as a colorless solid.
MS (ESI pos.) m/z : 354 [M+H]+, RT=0.884 min (Analytical Condition 3)

### 165-(2) 3-[(8-Amino-2-oxo-1,2-dihydroquinoline-3-carbonyl)amino]benzoic acid-trifluoroacetate

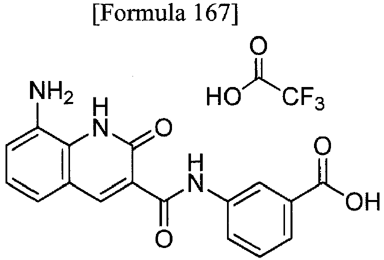

To a suspension in ethanol (5 mL) and water (1 mL) of the 3-[(8-nitro-2-oxo-1,2-dihydroquinoline-3-carbonyl)amino]benzoic acid (70 mg) obtained in Example 165-(1), an iron powder (56 mg) and ammonium chloride (32 mg) were added and the mixture was refluxed for 4 hours. To the reaction mixture, an aqueous solution of sodium hydroxide was added to render the reaction mixture basic in nature. The suspension was stirred for 30 minutes at room temperature and filtered through Celite. To the filtrate, a saturated aqueous solution of ammonium chloride was added to establish pH 4 and the precipitating solid was recovered by filtration. The thus obtained solid was purified by preparative HPLC to obtain the titled compound (1.8 mg) as a yellow solid.
MS (ESI pos.) m/z : 324 [M+H]+, RT=0.748 min (Analytical Condition 3)
¹H NMR (600 MHz, DMSO-d6) δ ppm 5.78 (br s, 2 H), 6.98 (d, J=7.6 Hz, 1 H), 7.10 (dd, J=8.3, 7.8 Hz, 1 H), 7.22 (d, J=7.0 Hz, 1 H), 7.51 (dd, J=7.6, 7.0 Hz, 1 H), 7.70 (d, J=7.8 Hz, 1 H), 7.95 (d, J=8.3 Hz, 1 H), 8.30 (s, 1 H), 8.87 (s, 1 H), 11.76 (br s, 1 H), 12.32 (s, 1 H), 13.05 (br s, 1 H)

The compounds of the present invention were checked for their actions by the following pharmacological tests.

### Test Example 1-1 Test for Evaluating the Activity of Inhibiting E. coli DNA Gyrase Enzyme (Method 1)

The enzymatic activity of E. coli DNA gyrase was measusred by a method comprising reacting DNA gyrase and its substrate, relaxed pBR322 (Inspiralis), in the presence of ATP and detecting the post-reaction supercoiled pBR322/relaxed pBR322 ratio with H19 (Profoldin).

Specifically, to 1 ng/µL of relaxed pBR322, E. coli DNA gyrase enzyme (Inspiralis) was added at a final concentration of 0.006 U/µL and the mixture was incubated for 60 minutes at room temperature. This reaction was carried out in 20 mmol/L tris-HCl buffered solution (pH 8.0) comprising 0.5 mmol/L ATP, 0.005% bridge 35, 35 mmol/L ammonium acetate, 8 mmol/L magnesium chloride, 4.6% glycerol, and 1 mmol/L dithiothreitol. To the reaction mixture, H19 was added to bring the reaction to an end; thereafter, the supercoiled pBR322/relaxed pBR322 ratio was detected at excitation wavelength/emission wavelength = 485 nm/535 nm. An inhibition curve was obtained by allowing the analyte compound to be present at varying concentrations during the reaction described above. From the inhibition curve, the concentration of the analyte compound at which it decreased the amount of the reaction product by 50% (IC₅₀) was determined and used as an index for the activity of inhibiting E. coli DNA gyrase enzyme. The test results of interest are shown in [Table 20-1] and [Table 20-2].

### Test Example 1-2 Test for Evaluating the Activity of Inhibiting E. coli DNA Gyrase Enzyme (Method 2)

The enzymatic activity of E. coli DNA gyrase was measusred by a method comprising reacting DNA gyrase and its substrate, relaxed pBR322 (Inspiralis), in the presence of ATP and detecting the post-reaction supercoiled pBR322/relaxed pBR322 ratio with H19.

Specifically, to 1 ng/µL of relaxed pBR322, E. coli DNA gyrase enzyme (the gyrA or gyrB gene was incorporated into the pET15b plasmid and after expressing them in E. coli BL21 (DE3), purification was performed using a Ni-NTA column to form a complex at room temperature) was added at a final concentration of 0.135 nmol/mL and the mixture was incubated for 60 minutes at room temperature. This reaction was carried out in 20 mmol/L tris-HCl buffered solution (pH 8.0) comprising 0.5 mmol/L ATP, 0.005% bridge 35, 35 mmol/L ammonium acetate, 8 mmol/L magnesium chloride, 4.6% glycerol, and 1 mmol/L dithiothreitol. To the reaction mixture, H19 was added to bring the reaction to an end; thereafter, the supercoiled pBR322/relaxed pBR322 ratio was detected at excitation wavelength/emission wavelength = 485 nm/535 nm. An inhibition curve was obtained by allowing the analyte compound to be present at varying concentrations during the reaction described above. From the inhibition curve, the concentration of the analyte compound at which it decreased the amount of the reaction product by 50% (IC₅₀) was determined and used as an index for the activity of inhibiting E. coli DNA gyrase enzyme. The test results of interest are shown in [Table 20-1] and [Table 20-2].

**[Table 20-1]**

| E. coli enzyme inhibition test results (E. coli gyrase) | | |
|---|---|---|
| Example No. | E.Coli Gyrase supercoiling IC50 (nM) or % inhibition | Method 1 or 2 |
| 1 | 52.30 | Method 2 |
| 2 | 1.72 | Method 2 |
| 3 | 124.40 | Method 1 |
| 4 | 164.18 | Method 2 |
| 5 | 9.75 | Method 2 |
| 6 | 33.08 | Method 2 |
| 7 | 3.35 | Method 1 |
| 8 | 30.09 | Method 1 |
| 9 | 51.67 | Method 1 |
| 10 | 459.40 | Method 1 |
| 11 | 244.99 | Method 1 |
| 12 | 21.13 | Method 1 |
| 13 | 31725.11 | Method 1 |
| 14 | 35521.65 | Method 1 |
| 15 | 35.89 | Method 1 |
| 16 | 130.81 | Method 1 |
| 17 | 293.11 | Method 1 |
| 18 | 15.65 | Method 1 |
| 19 | 426.39 | Method 1 |
| 20 | 1.19 | Method 1 |
| 21 | 44% inhibition at 3000nM | Method 1 |
| 22 | 173.84 | Method 1 |
| 23 | 696.94 | Method 1 |
| 24 | 641.24 | Method 1 |
| 25 | 65.44 | Method 1 |
| 26 | 17.83 | Method 1 |
| 27 | 257.85 | Method 1 |
| 28 | 32.04 | Method 1 |
| 29 | 28.23 | Method 1 |
| 30 | 216.27 | Method 1 |
| 31 | 321.13 | Method 1 |
| 32 | 25.34 | Method 2 |
| 33 | 0.36 | Method 2 |
| 34 | 23.20 | Method 2 |
| 35 | 1.47 | Method 2 |
| 36 | 71.90 | Method 2 |
| 37 | 9.97 | Method 2 |
| 38 | 4730.70 | Method 2 |
| 39 | 48.74 | Method 2 |
| 40 | 7.29 | Method 2 |
| 41 | 149.12 | Method 2 |
| 42 | 73.57 | Method 1 |
| 43 | 175.60 | Method 1 |
| 44 | 373.27 | Method 1 |
| 45 | 101.18 | Method 1 |
| 46 | 7.47 | Method 1 |
| 47 | 10.73 | Method 1 |
| 48 | 285.66 | Method 1 |
| 49 | 20.40 | Method 1 |
| 50 | 1.87 | Method 1 |
| 51 | 5.46 | Method 1 |
| 52 | 2.47 | Method 1 |
| 53 | 1.49 | Method 1 |
| 54 | 1.90 | Method 1 |
| 55 | 14.72 | Method 1 |
| 56 | 18.00 | Method 1 |
| 57 | 3.62 | Method 1 |
| 58 | 2.05 | Method 1 |
| 59 | 14.70 | Method 1 |
| 60 | 11.82 | Method 1 |
| 61 | 334.35 | Method 2 |
| 62 | 0.92 | Method 1 |
| 63 | 1.44 | Method 1 |
| 64 | 44.71 | Method 1 |
| 65 | 0.95 | Method 1 |
| 66 | 0.95 | Method 1 |
| 67 | 6.36 | Method 1 |
| 68 | 9.40 | Method 1 |
| 69 | 7.00 | Method 1 |
| 70 | 0.53 | Method 1 |
| 71 | 0.88 | Method 1 |
| 72 | 8.03 | Method 1 |
| 73 | 22.96 | Method 1 |
| 74 | 129.25 | Method 1 |
| 75 | 0.25 | Method 1 |
| 76 | 249.04 | Method 1 |
| 77 | 24.33 | Method 1 |
| 78 | 12% inhibition at 300nM | Method 1 |
| 79 | 174.74 | Method 1 |
| 80 | 2.50 | Method 1 |
| 81 | 110.05 | Method 2 |
| 82 | 0.47 | Method 2 |
| 83 | 1.09 | Method 2 |
| 84 | 11.41 | Method 2 |
| 85 | 0.61 | Method 2 |
| 86 | 34.43 | Method 2 |
| 87 | 1038.82 | Method 2 |
| 88 | 41% inhibition at 10000nM | Method 2 |
| 89 | 29.86 | Method 2 |
| 90 | 2.45 | Method 2 |
| 91 | 18.56 | Method 2 |
| 92 | 2.56 | Method 2 |
| 93 | 31.83 | Method 2 |
| 94 | 131.08 | Method 2 |
| 95 | 2.16 | Method 2 |
| 96 | 0.31 | Method 2 |
| 97 | 0.60 | Method 2 |
| 98 | 0.21 | Method 2 |
| 99 | 0.70 | Method 2 |
| 100 | 33.72 | Method 2 |

**[Table 20-2]**

| E. coli enzyme inhibition test results (E. coli gyrase) | | |
|---|---|---|
| Example No. | E.Coli Gyrase supercoiling IC50 (nM) or % inhibition | Method 1 or 2 |
| 101 | 0.80 | Method 2 |
| 102 | 228.35 | Method 2 |
| 103 | 12.08 | Method 2 |
| 104 | 0.29 | Method 2 |
| 105 | 29.75 | Method 2 |
| 106 | 6.98 | Method 2 |
| 107 | 0.57 | Method 2 |
| 108 | 6.05 | Method 2 |
| 109 | 15.12 | Method 2 |
| 110 | 3.68 | Method 2 |
| 111 | 2.94 | Method 2 |
| 112 | 4.13 | Method 2 |
| 113 | 0.26 | Method 2 |
| 114 | 338.93 | Method 2 |
| 115 | 4.57 | Method 2 |
| 116 | 136.80 | Method 2 |
| 117 | 1.10 | Method 2 |
| 118 | 45% inhibition at 100nM | Method 2 |
| 119 | 20.60 | Method 2 |
| 120 | 32.49 | Method 2 |
| 121 | 2.48 | Method 2 |
| 122 | 1.92 | Method 2 |
| 123 | 3.59 | Method 2 |
| 124 | 1.60 | Method 2 |
| 125 | 24.65 | Method 2 |
| 126 | 22.04 | Method 2 |
| 127 | 214.34 | Method 2 |
| 128 | 339.72 | Method 2 |
| 129 | 386.51 | Method 2 |
| 130 | 109.23 | Method 2 |
| 131 | 0.94 | Method 2 |
| 132 | 0.52 | Method 2 |
| 133 | 0.82 | Method 2 |
| 134 | 131.11 | Method 2 |
| 135 | 0.66 | Method 2 |
| 136 | 1.28 | Method 2 |
| 137 | 1.31 | Method 2 |
| 138 | 128.53 | Method 2 |
| 139 | 6.94 | Method 2 |
| 140 | 2.15 | Method 2 |
| 141 | 2.09 | Method 2 |
| 142 | 2.66 | Method 2 |
| 143 | 14.56 | Method 2 |
| 144 | 0.48 | Method 2 |
| 145 | 0.19 | Method 2 |
| 146 | 0.43 | Method 2 |
| 147 | 1.75 | Method 2 |
| 148 | 2.66 | Method 2 |
| 149 | 0.55 | Method 2 |
| 150 | 89.28 | Method 2 |
| 151 | 21.45 | Method 2 |
| 152 | 1229.66 | Method 2 |
| 153 | 7.53 | Method 2 |
| 154 | 9.78 | Method 2 |
| 155 | 109.28 | Method 2 |
| 156 | 13.39 | Method 2 |
| 157 | 5.61 | Method 2 |
| 158 | 7.78 | Method 2 |
| 159 | 1.08 | Method 2 |
| 160 | 11667.60 | Method 2 |
| 161 | 2228.03 | Method 2 |
| 162 | 3452.66 | Method 2 |
| 163 | 2097.54 | Method 2 |
| 164 | 4.44 | Method 2 |
| 165 | 27% inhibition at 50000nM | Method 1 |
| 166 | 18.07 | Method 2 |
| 167 | 134.21 | Method 2 |
| 168 | 1671.94 | Method 2 |
| Reference Example 1 | 7010.13 | Method 2 |

### Test Example 2 Test for Evaluating the Activity of Inhibiting E. coli Topoisomerase IV Enzyme

The enzymatic activity of E. coli topoisomerase IV was measusred by a method comprising reacting topoisomerase IV and its substrate, supercoiled pBR322 (Inspiralis), in the presence of ATP and detecting the post-reaction supercoiled pBR322/relaxed pBR322 ratio with H19.

Specifically, to 2 ng/µL of supercoiled pBR322, E. coli topoisomerase IV enzyme (Inspiralis) was added at a final concentration of 0.0075 U/µL and the mixture was incubated for 30 minutes at room temperature. This reaction was carried out in 20 mmol/L tris-HCl buffered solution (pH 8.0) comprising 0.5 mmol/L ATP, 0.005% bridge 35, 35 mmol/L ammonium acetate, 8 mmol/L magnesium chloride, 4.6% glycerol, and 1 mmol/L dithiothreitol. To the reaction mixture, H19 was added to bring the reaction to an end; thereafter, the supercoiled pBR322/relaxed pBR322 ratio was detected at excitation wavelength/emission wavelength = 485 nm/535 nm. An inhibition curve was obtained by allowing the analyte compound to be present at varying concentrations during the reaction described above. From the inhibition curve, the concentration of the analyte compound at which it decreased the amount of the reaction product by 50% (IC₅₀) was determined and used as an index for the activity of inhibiting E. coli topoisomerase IV enzyme. The test results for the compounds under measurement are shown in [Table 21].

**[Table 21]**

| E. coli Enzyme Inhibition Test Results (E. coli TopoIV) | |
|---|---|
| Example No. | E.Coli TopoIV relaxation IC50 (nM) |
| 2 | 979.97 |
| 5 | 16973.99 |
| 33 | 218.58 |
| 40 | 1304.04 |
| 52 | 518.66 |
| 65 | 32.45 |
| 70 | 47.39 |
| 85 | 176.36 |
| 96 | 15.26 |
| 98 | 41.38 |
| 101 | 51.52 |
| 104 | 23.40 |
| 107 | 76.29 |
| 117 | 62.89 |
| 123 | 614.67 |
| 124 | 95.88 |
| 133 | 159.92 |
| 146 | 51.92 |
| 148 | 126.65 |
| 153 | 4927.80 |

### Test Example 3 Test for Evaluating Antibacterial Activity (E. coli)

Minimum inhibitory concentration (MIC) was measured using the broth microdilution method described below in accordance with a modification of the standard method specified by CLSI (Clinical & Laboratory Standards Institute).

Bacterial cells under test were cultured overnight on a heart infusion agar medium, scraped off and suspended at a concentration equivalent to McFarland 0.5 turbidity; the suspension thus obtained was diluted 10 folds to give a liquid inoculum. The inoculum (0.005 mL) was inoculated on a cation-adjusted Mueller-Hinton medium containing the analyte compound and cultured at 35°C for 18 hours. A minimum drug concentration at which no bacterial growth was macroscopically visible was designated MIC. Test results for typical compounds are shown in [Table 22-1] and [Table 22-2].

### Test Example 4 Test for Evaluating Antibacterial Activity (N. gonorrhoeae)

Minimum inhibitory concentration (MIC) was measured using the broth microdilution method described below in accordance with a modification of the method of Geers et al. (Antimicrob. Agents. Chemother. (1989), 33, 233-234).

Specifically, proteose peptone No. 3 (15 g), sodium chloride (5 g), dipotassium hydrogenphosphate (4 g), potassium dihydrogenphosphate (1 g), starch, soluble, Bacto (1 g), sodium hydrogencarbonate (0.15 g) and glucose (5 g) were dissolved in 1 L of water and thereafter adjusted to pH 7.4 using sodium hydroxide. After sterilization, BD. Difco supplement C was added to give a final concentration of 1%, whereby a gonococcal broth was prepared.

Bacterial cells under test were cultured overnight on a chocolate II agar medium, scraped off and suspended at a concentration equivalent to McFarland 0.5 turbidity; the suspension thus obtained was diluted 5 folds to give a liquid inoculum. The inoculum (0.005 mL) was inoculated on a gonococcal broth containing the analyte compound and cultured at 35°C for 24 hours. A minimum drug concentration at which no bacterial growth was macroscopically visible was designated MIC. Test results for typical compounds are shown in [Table 22-1] and [Table 22-2].

**[Table 22-1]**

| E. coli/N. gonorrhoeae MIC Test Resuts | | | | |
|---|---|---|---|---|
| Example No. | MIC (µg/mL) E.Coli ATCC 25922 | | MIC (µg/mL) N. gonorrhoeae ATCC 49226 | |
| 1 | > | 64 | > | 4 |
| 2 | | 64 | > | 1 |
| 3 | > | 64 | > | 4 |
| 4 | > | 64 | > | 4 |
| 5 | > | 64 | | NT |
| 6 | > | 64 | | 4 |
| 7 | > | 32 | > | 4 |
| 8 | > | 64 | | 4 |
| 9 | > | 64 | | NT |
| 10 | > | 64 | | NT |
| 11 | > | 64 | | NT |
| 12 | > | 64 | | NT |
| 13 | | NT | | NT |
| 14 | | NT | > | 4 |
| 15 | > | 64 | | NT |
| 16 | > | 64 | > | 4 |
| 17 | > | 64 | > | 1 |
| 18 | > | 0.5 | | 0.25 |
| 19 | > | 16 | > | 4 |
| 20 | > | 16 | | NT |
| 21 | | NT | | NT |
| 22 | > | 8 | > | 4 |
| 23 | > | 4 | | NT |
| 24 | > | 32 | | NT |
| 25 | > | 16 | | NT |
| 26 | > | 32 | | NT |
| 27 | > | 8 | | NT |
| 28 | > | 16 | > | 4 |
| 29 | > | 64 | > | 4 |
| 30 | > | 16 | | NT |
| 31 | > | 16 | | NT |
| 32 | > | 64 | > | 4 |
| 33 | | 16 | | 0.004 |
| 34 | > | 64 | | NT |
| 35 | > | 4 | | NT |
| 36 | > | 32 | | NT |
| 37 | > | 4 | | 1 |
| 38 | | NT | | NT |
| 39 | > | 8 | | NT |
| 40 | | 4 | | NT |
| 41 | > | 64 | | NT |
| 42 | > | 64 | | NT |
| 43 | > | 32 | | NT |
| 44 | > | 16 | | NT |
| 45 | > | 16 | | NT |
| 46 | > | 1 | | NT |
| 47 | > | 32 | | NT |
| 48 | > | 16 | | NT |
| 49 | > | 8 | | NT |
| 50 | > | 8 | | 0.06 |

| E. coli/N. gonorrhoeae MIC Test Results | | | | |
|---|---|---|---|---|
| Example No. | MIC (µg/mL) E.Coli ATCC 25922 | | MIC (µg/mL) N. gonorrhoeae ATCC 49226 | |
| 51 | | 16 | | NT |
| 52 | | 16 | | 0.5 |
| 53 | | 0.25 | | NT |
| 54 | > | 16 | | 1 |
| 55 | > | 16 | | NT |
| 56 | > | 64 | | 0.12 |
| 57 | > | 16 | | NT |
| 58 | > | 32 | | 0.12 |
| 59 | > | 2 | | NT |
| 60 | > | 32 | | NT |
| 61 | | NT | | NT |
| 62 | > | 16 | | 2 |
| 63 | > | 16 | | NT |
| 64 | > | 8 | | NT |
| 65 | > | 16 | | 0.5 |
| 66 | > | 32 | | 0.25 |
| 67 | > | 16 | | NT |
| 68 | > | 8 | | NT |
| 69 | | 8 | | NT |
| 70 | | 8 | | 0.03 |
| 71 | > | 8 | | NT |
| 72 | > | 4 | | 0.12 |
| 73 | > | 8 | | NT |
| 74 | > | 64 | | NT |
| 75 | | 8 | | NT |
| 76 | > | 32 | | 0.25 |
| 77 | > | 16 | | 1 |
| 78 | | NT | | NT |
| 79 | | NT | | NT |
| 80 | > | 16 | | NT |
| 81 | | NT | > | 1 |
| 82 | | 0.25 | | NT |
| 83 | > | 16 | > | 4 |
| 84 | > | 32 | | NT |
| 85 | > | 4 | | 2 |
| 86 | > | 4 | | NT |
| 87 | | NT | | NT |
| 88 | | NT | | NT |
| 89 | > | 8 | | NT |
| 90 | > | 16 | | NT |
| 91 | > | 16 | | 1 |
| 92 | > | 4 | | NT |
| 93 | > | 2 | | 0.25 |
| 94 | > | 2 | | NT |
| 95 | > | 16 | | 1 |
| 96 | | 4 | | 0.015 |
| 97 | > | 8 | | NT |
| 98 | | 4 | | 0.06 |
| 99 | | 64 | | 0.25 |
| 100 | > | 64 | > | 1 |

**[Table 22-2]**

| E. coli/N. gonorrhoeae MIC Test Results | | | | |
|---|---|---|---|---|
| Example No. | MIC (µg/mL) E.Coli ATCC 25922 | | MIC (µg/mL) N. gonorrhoeae ATCC 49226 | |
| 101 | | 16 | | 0.03 |
| 102 | | NT | | NT |
| 103 | > | 64 | | 4 |
| 104 | | 8 | | 0.015 |
| 105 | > | 32 | | NT |
| 106 | > | 8 | | 0.5 |
| 107 | > | 64 | | 0.5 |
| 108 | > | 32 | > | 1 |
| 109 | > | 32 | | NT |
| 110 | > | 64 | > | 1 |
| 111 | > | 32 | | NT |
| 112 | > | 64 | | NT |
| 113 | | 32 | | NT |
| 114 | > | 32 | | NT |
| 115 | > | 4 | | NT |
| 116 | > | 64 | > | 1 |
| 117 | | 1 | | 0.0005 |
| 118 | > | 64 | | 1 |
| 119 | > | 64 | | NT |
| 120 | > | 32 | | NT |
| 121 | > | 32 | | 0.25 |
| 122 | | 16 | | 0.5 |
| 123 | | 8 | | NT |
| 124 | | 8 | | 0.12 |
| 125 | > | 32 | | NT |
| 126 | > | 8 | | NT |
| 127 | > | 16 | | NT |
| 128 | > | 16 | | NT |
| 129 | > | 16 | | 1 |
| 130 | > | 8 | | NT |
| 131 | > | 16 | | NT |
| 132 | > | 8 | | 0.25 |
| 133 | > | 16 | | NT |
| 134 | > | 32 | | NT |
| 135 | > | 16 | | 0.12 |
| 136 | > | 32 | | NT |
| 137 | > | 32 | | NT |
| 138 | | 32 | | 0.06 |
| 139 | > | 64 | | NT |
| 140 | > | 32 | | NT |
| 141 | > | 16 | | NT |
| 142 | > | 32 | | NT |
| 143 | > | 64 | | NT |
| 144 | > | 8 | | 0.004 |
| 145 | > | 32 | | 0.06 |
| 146 | | 16 | | 0.008 |
| 147 | > | 32 | | 0.008 |
| 148 | | 1 | ≦ | 0.0005 |
| 149 | > | 8 | | 0.03 |
| 150 | > | 16 | | 0.03 |
| 151 | > | 16 | | 0.5 |
| 152 | > | 16 | | 4 |
| 153 | | 8 | | 0.06 |
| 154 | > | 16 | | 0.06 |
| 155 | > | 32 | | 0.015 |
| 156 | | 32 | | 0.5 |
| 157 | | 16 | | 0.12 |
| 158 | > | 16 | | 0.03 |
| 159 | > | 16 | | 0.002 |
| 160 | > | 32 | > | 4 |
| 161 | > | 32 | > | 4 |
| 162 | > | 32 | > | 1 |
| 163 | > | 32 | > | 1 |
| 164 | | 64 | | 0.12 |
| 165 | | NT | | NT |
| 166 | > | 16 | | NT |
| 167 | > | 32 | | NT |
| 168 | > | 32 | | NT |
| Reference Examlpe 1 | > | 64 | > | 1 |

### INDUSTRIAL APPLICABILITY

The present invention provides novel compounds useful as antibacterial agents that inhibit GyrB of DNA gyrase and ParE of topoisomerase IV to thereby exhibit strong antibacterial activity against Gram-positive and Gram-negative bacteria as well as drug-resistant variants thereof. These compounds are applicable as pharmaceuticals and the like.

## Claims

1. A compound represented by formula [1] : 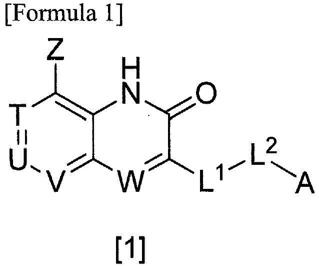 {wherein
Z represents NH-R¹, a C₁₋₄ alkyl group (said C₁₋₄ alkyl group may be substituted with an amino group or a hydroxy group) or a hydroxy group;
R¹ represents a hydrogen atom or a C₁₋₄ alkyl group;
T, U, V and W all represent C-R² or either one of them represents N while the other represent C-R²;
each R² independently represents a hydrogen atom, a halogen atom, a hydroxy group, a C₁₋₆alkyl group, a C₁₋₆ alkoxy group (said C₁₋₆ alkyl group and said C₁₋₆alkoxy group may be substituted with -N(R²¹)(R²²)) or an amino group [said amino group may be substituted with a C₁₋₆ alkyl group (said C₁₋₆ alkyl group may be substituted with a piperidin-4-yl group, a pyrrolidin-3-yl group, an azetidin-3-yl group, a 1-amino-cyclobutan-3-yl group, a morpholinyl group or -N(R²³)(R²⁴)), a 1-amino-cyclobutan-3-yl group or any of the substituents described by formula [2]]: 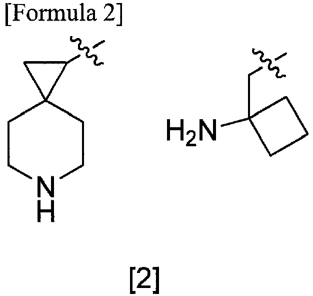
R²¹, R²², R²³ and R²⁴ are the same or different and each represents a hydrogen atom or a C₁₋₆alkyl group;
L¹ represents -CONR³-, -COO-, -(CH₂)ₙNR³- or -NR³CO-;
R³ represents a hydrogen atom or a C₁₋₆ alkyl group;
n represents an integer of 1 to 4;
L² represents a group selected from the group consisting of a bond, a C₁₋₆ alkylene group, a piperidinediyl group, a pyrrolidinediyl group and an azetidinediyl group (said C₁₋₆ alkylene group, said piperidinediyl group, said pyrrolidinediyl group and said azetidinediyl group may be substituted with a carboxy group or an oxo group);
A represents an aryl group, a heterocyclic group or a C₃₋₈ cycloalkyl group (said aryl group, said heterocyclic group and said C₃₋₈cycloalkyl group may be substituted with one to four substituents which are the same or different and are each selected from the following substituent group R^{a});
the substituent group R^{a} represents a C₁₋₆alkyl group (said C₁₋₆ alkyl group may be substituted with one or two substituents selected from the group consisting of a carboxy group, a hydroxy group, a C₃₋₈cycloalkyl group, a carbamoyl group and -N(R¹¹)(R¹²)), a carboxy group, a hydroxy group, a heterocyclic group, an amidino
group, -N(R¹³)(R¹⁴), -CON(R¹⁵)(R¹⁶), a C₁₋₆alkoxy group (said C₁₋₆alkoxy group may be substituted with one or two substituents selected from the group consisting of an amino group, an N-methylpiperazinyl group and a morpholinyl group), a C₂₋₆alkenyl group (said C₂₋₆alkenyl group may be substituted with a hydroxy group or -N(R¹⁷)(R¹⁸)), -COOR¹⁹, a 3-aminoazetidinyl group, a piperazinyl group (said piperazinyl group may be substituted with one methyl group), a 4-aminopiperidinyl group or any of the substitutents described by formula [3]: 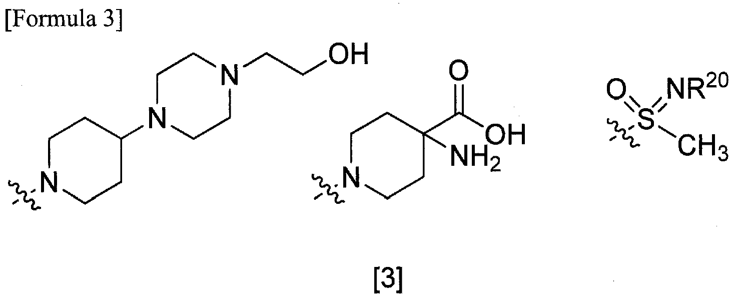
R¹¹ and R¹² are the same or different and each represents a hydrogen atom, a C₁₋₁₂alkyl group, a C₃₋₈cycloalkyl group, a hydroxyethyl group, an N-methylpiperidin-4-yl group, a carboxymethyl group, an N,N-dimethylaminopropyl group or an aminoethyl group; or alternatively
R¹¹ and R¹², when taken together with the nitrogen atom to which they bind, may form a saturated 3- to 7-membered ring, where said saturated 3- to 7-membered ring may further contain one or more nitrogen atoms, oxygen atoms or sulfur atoms in the ring, and said saturated 3- to 7-membered ring may be substituted with an amino group;
R¹³ and R¹⁴ are the same or different and each represents a hydrogen atom, a C₁₋₆alkoxy carbonyl group, a C₃₋₈cycloalkyl group, -CONHSO₂Me, a C₁₋₆ alkyl group [said C₁₋₆ alkyl group may be substituted with one or two substituents selected from among an amino group, an N-methylpiperazinyl group, a morpholinyl group, -N(CH₂CH₂OH)₂ and a heterocyclic group (said heterocyclic group may be substituted with an amino group)];
R¹⁵ and R¹⁶ are the same or different and each represents a hydrogen atom, a hydroxy group, a 1,3-dihydroxypropan-2-yl group, a methanesulfonyl group, an N,N-dimethylsulfamoyl group and a C₁₋₆ alkyl group (said C₁₋₆ alkyl group may be substituted with one or two substituents which are the same or different and are each selected from the group consisting of an amino group, a morpholinyl group, a piperidinyl group, a carboxy group, a hydroxy group and the substituent described by formula [4]): 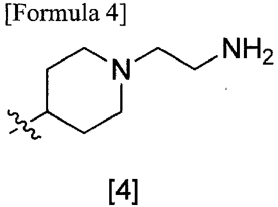
R¹⁵ and R¹⁶, when taken together with the nitrogen atom to which they bind, may form a saturated 3- to 7-membered ring, where said saturated 3- to 7-membered ring may further contain one or more nitrogen atoms, oxygen atoms or sulfur atoms in the ring;
R¹⁷ and R¹⁸ are the same or different and each represents a hydrogen atom or a C₁₋₆ alkyl group;
R¹⁹ represents a C₁₋₆ alkyl group; and
R²⁰ represents a hydrogen atom or a C₁₋₆ alkyl group}
or a pharmaceutically acceptable salt thereof.

2. The compound according to claim 1 wherein Z is NH-R¹, a C₁₋₄ alkyl group or a hydroxy group and R¹ is a C₁₋₄ alkyl group, or a pharmaceutically acceptable salt thereof.

3. The compound according to claim 2 wherein Z is NH-R¹, an ethyl group or a hydroxy group and R¹ is a methyl group, or a pharmaceutically acceptable salt thereof.

4. The compound according to claim 3 wherein Z is NH-R¹ and R¹ is a methyl group, or a pharmaceutically acceptable salt thereof.

5. The compound according to claim 4 wherein T, U, V and W are each C-R², or a pharmaceutically acceptable salt thereof.

6. The compound according to claim 5 wherein L¹ is -CONR³-, -COO- or -(CH₂)ₙNR³-, or a pharmaceutically acceptable salt thereof.

7. The compound according to claim 6 wherein L¹ is -CONR³-, or a pharmaceutically acceptable salt thereof.

8. The compound according to claim 7 wherein R³ is a hydrogen atom, or a pharmaceutically acceptable salt thereof.

9. The compound according to claim 8 wherein L² is a bond or a group selected from the group consisting of a bond, a methylene group or an ethylene group, a piperidinediyl group, a pyrrolidinediyl group, and an azetidinediyl group, or a pharmaceutically acceptable salt thereof.

10. The compound according to claim 9 wherein L² is a bond or an ethylene group, or a pharmaceutically acceptable salt thereof.

11. The compound according to claim 10 wherein L² is a bond, or a pharmaceutically acceptable salt thereof.

12. The compound according to any one of claims 1 to 11 wherein T, U, V and W are each C-R² and each R² is independently a hydrogen atom, a halogen atom, a hydroxy group, a C₁₋₆ alkyl group (said C₁₋₆ alkyl group may be substituted with -N(R²¹)(R²²)), a C₁₋₆alkoxy group or an amino group [said amino group may be substituted with a C₁₋₆ alkyl group (said C₁₋₆alkyl group may be substituted with a piperidin-4-yl group, a pyrrolidin-3-yl group, an azetidin-3-yl group, a 1-amino-cyclobutan-3-yl group, a morpholino group or -N(R²³)(R²⁴)), a 1-amino-cyclobutan-3-yl group or any of the substituents described by formula [2]]: 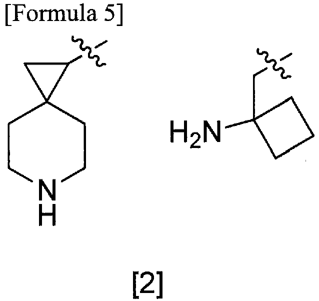 R²¹, R²², R²³ and R²⁴ are the same or different and each is a hydrogen atom or a C₁₋₆ alkyl group, or a pharmaceutically acceptable salt thereof.

13. The compound according to claim 12 wherein each R² is independently a hydrogen atom, a fluorine atom, a hydroxy group, an ethyl group, a n-propyl group (said ethyl group and said n-propyl group may be substituted with -N(R²¹)(R²²)), a C₁₋₆alkoxy group, an amino group [said amino group may be substituted with a methyl group, an ethyl group, a n-propyl group (said methyl group, said ethyl group and said n-propyl group may be substituted with one or two substituents selected from the group consisting of a piperidin-4-yl group, a pyrrolidin-3-yl group, an azetidin-3-yl group, a 1-amino-cyclobutan-3-yl group, a morpholino group and -N(R²³)(R²⁴)), a 1-amino-cyclobutan-3-yl group or any of the substituents described by formula [2]]: 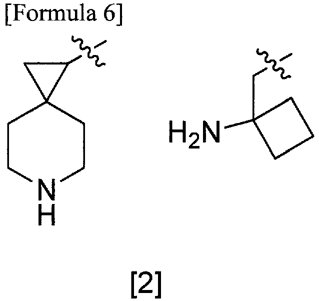 R²¹, R²², R²³ and R²⁴ are the same or different and each is a hydrogen atom or a methyl group, or a pharmaceutically acceptable salt thereof.

14. The compound according to claim 13 wherein each R² is independentlyl a hydrogen atom, a fluorine atom or a hydroxy group, or a pharmaceutically acceptable salt thereof.

15. The compound according to claim 13 wherein U is C-F or C-H, T and V are each C-H, and W is C-R², or a pharmaceutically acceptable salt thereof.

16. The compound according to any one of claims 1 to 15 wherein A is an aryl group or a heterocyclic group (said aryl group and said heterocyclic group may be substituted with one to four substituents which are the same or different and are each selected from the substituent group R^{a}), or a pharmaceutically acceptable salt thereof.

17. The compound according to claim 16 wherein A is an aryl group or a heterocyclic group (said aryl group and said heterocyclic group may be substituted with one or two substituents which are the same or different and are each selected from the substituent group R^{b});
R^{b} is a C₁₋₆ alkyl group (said C₁₋₆ alkyl group may be substituted with one or two substituents which are the same or different and are each selected from the group consisting of a carboxy group, a hydroxy group, a carbamoyl group and -N(R¹¹)(R¹²)), a carboxy group, a hydroxy group, a heterocyclic group, an amidino group, -N(R¹³)(R¹⁴), -CON(R¹⁵)(R¹⁶), a C₁₋₆alkoxy group (said C₁₋₆alkoxy group may be substituted with an amino group, an N-methylpiperazinyl group or a morpholino group), a C₂₋₆alkenyl group (said C₂₋₆alkenyl group may be substituted with a hydroxy group or -N(R¹⁷)(R¹⁸)), a 3-aminoazetidino group, piperazinyl group (said piperazinyl group may be substituted with one methyl group), a 4-aminopiperidino group or any of the substituents described by formula [3]: 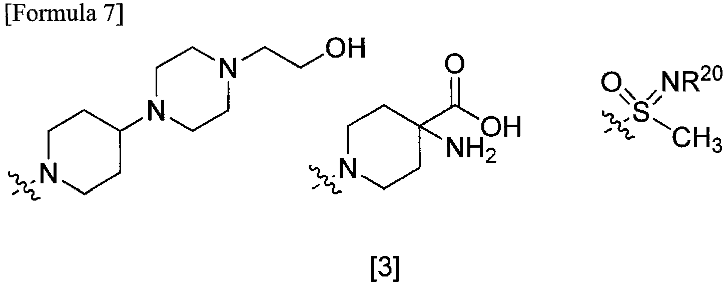 R¹¹ and R¹² are the same or different and each is an atom or a group selected from the group consisting of a hydrogen atom, a methyl group, a n-pentyl group, a n-octyl group, a cyclopropyl group, a cyclohexyl group, a hydroxyethyl group, an N-methylpiperidin-4-yl group, a carboxymethyl group, an N,N-dimethylaminopropyl group and an aminoethyl group, or alternatively
R¹¹ and R¹², when taken together with the nitrogen atom to which they bind, form a saturated 3- to 7-membered ring which is an azetidinyl group, a pyrrolidinyl group, a piperidinyl group or a morpholino group;
R¹³ and R¹⁴ are the same or different and each is a hydrogen atom, a t-butoxycarbonyl group, a cyclohexyl group, -CONHSO₂Me, a methyl group, an ethyl group or a n-propyl group (said methyl group, said ethyl group and said n-propyl group may be substituted with one or two substituents selected from the group consisting of an amino group, an N-methylpiperazino group, a morpholinyl group, -N(CH₂CH₂OH)₂ and a heterocyclic group);
R¹⁵ and R¹⁶ are the same or different and each is a hydrogen atom, a hydroxy group, a 1,3-dihydroxypropan-2-yl group, a methanesulfonyl group, an N,N-dimethylsulfamoyl group and a C₁₋₆ alkyl group (said C₁₋₆ alkyl group may be substituted with one or two substituents which are the same or different and are each selected from the group consisting of an amino group, a morpholinyl group, a piperidinyl group, a carboxy group, a hydroxy group and the substituent described by formula [4]): 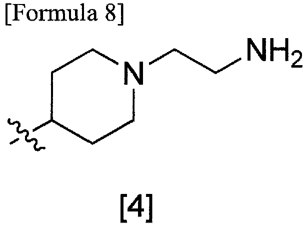 or alternatively, R¹⁵ and R¹⁶, when taken together with the nitrogen atom to which they bind, form a saturated 3- to 7-membered ring which is a morpholinyl group;
R¹⁷ and R¹⁸ are the same or different and each is a hydrogen atom or a methyl group;
R²⁰ is a hydrogen atom or a methyl group, or a pharmaceutically acceptable salt thereof.

18. The compound according to claim 17 wherein A is a phenyl group or a heterocyclic group (said phenyl group and said heterocyclic group may be substituted with one or two substituents which are the same or different and are each selected from the following substituent group R^{c});
R^{c} is a methyl group (said methyl group may be substituted with one or two substituents which are the same or different and are each selected from the group consisting of a carboxy group, a hydroxy group, a carbamoyl group and -N(R¹¹)(R¹²)), a carboxy group, a heterocyclic group and -CON(R¹⁵)(R¹⁶);
R¹¹ and R¹² are the same or different and each is an atom or a group selected from the group consisting of a hydrogen atom, a methyl group, a n-pentyl group, a n-octyl group, a cyclopropyl group, a cyclohexyl group, a hydroxyethyl group, an N-methylpiperidin-4-yl group, a carboxymethyl group, an N,N-dimethylaminopropyl group and an aminoethyl group, or alternatively
R¹¹ and R¹², when taken together with the nitrogen atom to which bind, form a saturated 3- to 7-membered ring which is an azetidinyl group, a pyrrolidinyl group, a piperidinyl group or a morpholino group;
R¹⁵ and R¹⁶ are the same or different and each is a hydrogen atom, a methanesulfonyl group, an N,N-dimethylsulfamoyl group, a methyl group, an ethyl group or a n-propyl group (said methyl group, ethyl group or n-propyl group may be substituted with one or two substituents selected from the group consisting of an amino group, a morpholinyl group, a piperidinyl group and a hydroxy group), or a pharmaceutically acceptable salt thereof.

19. A pharmaceutical composition comprising the compound of claims 1 to 18 or a pharmaceutically acceptable salt thereof.

20. A GyrB/ParE inhibitor comprising the compound of claims 1 to 18 or a pharmaceutically acceptable salt thereof.

21. An antibacterial agent comprising the compound of claims 1 to 18 or a pharmaceutically acceptable salt thereof.
